(19) Europäisches Patentamt
European Patent Office
Office européen des brevets

(11) EP 1 542 942 B1

(12) **FASCICULE DE BREVET EUROPEEN**

(45) Date de publication et mention de la délivrance du brevet:
**27.12.2006 Bulletin 2006/52**

(21) Numéro de dépôt: **03798215.4**

(22) Date de dépôt: **23.09.2003**

(51) Int Cl.:
*C07B 37/04* (2006.01) *C07B 37/12* (2006.01)
*C07B 37/02* (2006.01) *C07C 213/08* (2006.01)
*C07C 219/10* (2006.01) *C07C 219/12* (2006.01)
*C07C 209/08* (2006.01) *C07C 211/63* (2006.01)
*C07C 213/02* (2006.01) *C07C 215/44* (2006.01)
*C07C 67/03* (2006.01)

(86) Numéro de dépôt international:
**PCT/FR2003/002795**

(87) Numéro de publication internationale:
**WO 2004/029004 (08.04.2004 Gazette 2004/15)**

(54) **COMPOSITIONS CONTENANT DES LIQUIDES IONIQUES ET LEURS UTILISATIONS, NOTAMMENT EN SYNTHESE ORGANIQUE**

ZUSAMMENSETZUNGEN, DIE IONISCHE FLÜSSIGKEITEN ENTHALTEN, UND DEREN VEWENDUNGEN, INSBESONDERE IN DER ORGANISCHEN SYNTHESE

COMPOSITIONS CONTAINING IONIC LIQUIDS AND USES THEREOF, ESPECIALLY IN ORGANIC SYNTHESIS

(84) Etats contractants désignés:
**AT BE BG CH CY CZ DE DK EE ES FI FR GB GR HU IE IT LI LU MC NL PT RO SE SI SK TR**

(30) Priorité: **26.09.2002 FR 0211910**

(43) Date de publication de la demande:
**22.06.2005 Bulletin 2005/25**

(73) Titulaires:
- **CENTRE NATIONAL DE LA RECHERCHE SCIENTIFIQUE (CNRS)**
  **75794 Paris Cedex 16 (FR)**
- **UNIVERSITE DE RENNES I**
  **F-35065 Rennes (FR)**

(72) Inventeurs:
- **VAULTIER, Michel**
  **F-35410 Châteaugiron (FR)**
- **GMOUH, Said**
  **20250 Casablanca (MA)**

(74) Mandataire: **Grosset-Fournier, Chantal Catherine et al**
**Grosset-Fournier & Demachy,**
**54, rue Saint-Lazare**
**75009 Paris (FR)**

(56) Documents cités:
- **FRAGA-DUBREUIL J ET AL: "Grafted ionic liquid-phase-supported synthesis of small organic molecules" TETRAHEDRON LETTERS, ELSEVIER SCIENCE PUBLISHERS, AMSTERDAM, NL, vol. 42, no. 35, 27 août 2001 (2001-08-27), pages 6097-6100, XP004298190 ISSN: 0040-4039**
- **DUBREUIL J F ET AL: "Rate accelerations of 1,3-dipolar cycloaddition reactions in ionic liquids" TETRAHEDRON LETTERS, ELSEVIER SCIENCE PUBLISHERS, AMSTERDAM, NL, vol. 41, no. 38, 16 septembre 2000 (2000-09-16), pages 7351-7355, XP004211923 ISSN: 0040-4039**
- **WASSERSCHEID P ET AL: "Ionic Liquids - New Solutions for Transition Metal Catalysis" ANGEWANDTE CHEMIE. INTERNATIONAL EDITION, VERLAG CHEMIE. WEINHEIM, DE, vol. 39, no. 21, 27 octobre 2000 (2000-10-27), pages 3772,3778,3780-3787, XP002218385 ISSN: 0570-0833 cité dans la demande**
- **WELTON T: "Room-temperature ionic liquids. Solvents for synthesis and catalysis" CHEMICAL REVIEWS, AMERICAN CHEMICAL SOCIETY. EASTON, US, vol. 99, 1999, pages 2071-2083, XP002162959 ISSN: 0009-2665 cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 542 942 B1

Printed by Jouve, 75001 PARIS (FR)

## Description

La présente invention concerne des compositions contenant des liquides ioniques, ainsi que leurs utilisations, notamment en synthèse organique.

La synthèse sur support solide est devenue une méthode très efficace et très utilisée pour la fabrication de banques combinatoires de produits (Wilson et al., 1997 ; Charken et al., 1996 ; Sammelson et al., 2001 ; Gravet et al., 1997 ; Went Worth et al., 1999). Grâce au développement des tests à haut débit pour l'évaluation biologique et la découverte de nouveaux produits biologiquement actifs, les banques combinatoires sont devenues très importantes en chimie pharmaceutique et en agrochimie. L'utilisation de résines de Merrifield convenablement fonctionnalisées a permis la mise au point d'une multitude de méthodologies de synthèse sur support solide (Thompson et al., 1996 ; Dôrwald, 2000). Ces méthodologies sur support solide ont de nombreux avantages comme la purification aisée par simple lavage, la possibilité d'utiliser diverses techniques comme la synthèse parallèle ou celle de séparation-mélange ("split and mix") permettant de produire une grande quantité de produits en même temps. Cependant, de nombreux problèmes sont associés à ces méthodes, tels que le prix des résines fonctionnalisées et leur faible charge spécifique très souvent inférieure à 1 mmole/gr de résine et n'atteignant que très rarement 2 mmoles/gr de résine. Une autre difficulté est due au fait que les réactions se font en phase hétérogène, ce qui demande une mise au point particulière dans chaque cas, les conditions de réactions étant généralement différentes de celles utilisées en solution. De plus, les moyens d'analyse performants pour suivre les réactions sont peu nombreux. D'autres difficultés peuvent surgir en particulier lors du décrochage des produits recherchés et il est difficile d'utiliser des réactions à haute température, conditions qui détruisent les supports solides de même d'ailleurs qu'une agitation magnétique ou mécanique mal adaptée.

L'utilisation de polymères solubles (Sammelson et al., 2001 ; Gravet et al., 1997) s'avère une alternative intéressante. En effet, la substitution des résines insolubles par un polymère soluble tel que le poly(éthylène glycol) ou PEG, permet de retrouver les procédures expérimentales familières à la chimie en solution, tout en conservant une purification simple. En outre, il est possible d'utiliser les différentes méthodes d'analyse pour la caractérisation du produit obtenu, sans décrochage préalable du support. masse des polymères comprise entre 2000 et 20 000 daltons. La conséquence directe est une faible charge spécifique puisqu'une solution décimolaire contient déjà 500 grammes de polymère par litre pour un PEG de masse 5000 qui conduirait au plus à une décimole de produit attendu pour un litre de solution. De telles concentrations ne sont que rarement utilisées car elles entraînent des problèmes de viscosité du milieu. Un autre problème est la purification des produits et le recyclage des PEG.

Ainsi, il existe un réel besoin de nouveaux supports pour la synthèse organique supportée.

Depuis quelques années, les liquides ioniques (Welton et al., 1999; Wasserscheid et al., 2000) sont de plus en plus utilisés en synthèse organique et en catalyse car ils présentent un certain nombre de propriétés physico-chimiques intéressantes et importantes telles que leur grande stabilité thermique, leurs faibles volatilités et leurs tensions de vapeur très faibles, leur faible inflammabilité, leur fort pouvoir de solubilisation aussi bien des sels que des molécules organiques neutres et des polymères et enfin la possibilité d'un recyclage aisé.

L'article de Fraga-Dubreuil et al. (Tetrahedron Letters, vol. 42, no. 35, pages 6097-6100, 2001) décrit l'utilisation de liquide ionique comme support de réaction. Plus particulièrement, il décrit l'utilisation de sels d'imidazolium fonctionnalisés dans le cadre de réactions de cycloadditions dipolaires et de la réaction de Knoevenagel, en l'absence de solvant et en utilisant des irradiations micro-onde.

L'article de Fraga-Dubreuil et al. (Tetrahedron Letters, vol. 41, no. 38, pages 7351-7355, 2000) décrit les réactions de cycloaddition 1,3-dipolaire entre un imidate dérivé de diéthyl aminomalonate et de 2-éthoxybenzaldéhyde à 70°C, sans solvant traditionnel et en utilisant des liquides ioniques, stables à l'air, comme solvants. Les réactions décrites sont effectuées dans un bain d'huile ou en présence d'irradiations par micro-onde.

La présente invention a pour but de fournir une nouvelle utilisation des liquides ioniques en tant que nouvelles matrices pour la synthèse organique en phase homogène sur support(s) soluble(s).

La présente invention a pour but de fournir de nouvelles matrices pour la synthèse organique sur support(s) soluble(s), facilement recyclables, liquides dans une vaste gamme de températures, présentant une tension de vapeur très faible et possédant un très fort pouvoir de solubilisation.

La présente invention a pour but de fournir de nouvelles matrices pour la synthèse organique sur support(s) soluble(s), le(s)dit(s) support(s) soluble(s) étant dissous dans lesdites matrices.

La présente invention a pour but de fournir de nouvelles matrices pour la synthèse organique sur support(s) soluble(s) en remplacement des résines mais sans avoir les inconvénients liés aux réactions en phase hétérogène sur support solide.

La présente invention a pour but de fournir une nouvelle utilisation des liquides ioniques, en conférant à ces liquides ioniques des propriétés de type résine.

La présente invention concerne l'utilisation d'un liquide ionique, comme matrice liquide pour la synthèse organique en phase homogène sur support soluble, sans solvant organique volatil, ledit liquide ionique se présentant sous forme liquide ou solide à température ambiante, de formule $A_1^+X_1^-$, $A_1^+$ représentant un cation, fonctionnel ou non, ou

un mélange de cations dans lequel soit aucun des cations n'est fonctionnel soit l'un au moins des cations est fonctionnel, et $X_1^-$ un anion, fonctionnel ou non, ou un mélange d'anions dans lequel soit aucun des anions n'est fonctionnel soit l'un au moins des anions est fonctionnel.

L'expression "liquide ionique" désigne un sel ou un mélange de sels dont le point de fusion est compris entre -100°C et 250°C.

L'expression "matrice liquide" désigne un liquide ionique capable de solubiliser une ou plusieurs espèces chimiques telles que les sels minéraux ou organiques, les molécules organiques, les polymères d'origine naturelle ou synthétique. L'expression "matrice liquide" désigne donc un solvant constitué d'un liquide ionique. Ces nouveaux solvants sont non volatils et ont une très faible tension de vapeur. Ils sont également polaires et ont la capacité de dissoudre des sels d'onium fonctionnels pouvant alors servir de supports solubles.

L'expression "support soluble" désigne un sel dissous dans la matrice liquide ionique portant une ou plusieurs fonctions permettant l'accrochage de molécules et leur fonctionnalisation ultérieure ainsi que le décrochage en fin de séquence réactionnelle.

L'expression "synthèse organique en phase homogène sur support soluble" désigne la ou les transformation (s) de la ou des fonctions chimiques portée(s) par le support soluble sans modifier la matrice liquide, suivie d'une réaction de clivage libérant la ou les molécules recherchées.

L'expression "cation fonctionnel" désigne un groupe moléculaire qui possède au moins une fonction chimique, une partie de ce groupe portant une charge positive.

L'expression "anion fonctionnel" désigne un groupe moléculaire qui possède au moins une fonction chimique, une partie de ce groupe portant une charge négative.

L'expression "cation non fonctionnel" désigne un groupe moléculaire qui ne possède pas de fonction chimique, une partie de ce groupe portant une charge positive.

L'expression "anion non fonctionnel" désigne un groupe moléculaire qui ne possède pas de fonction chimique, une partie de ce groupe portant une charge négative.

Lorsque la matrice $A_1^+X_1^-$ ne comporte aucun ion fonctionnel, elle sert de milieu réactionnel inerte vis-à-vis des réactifs mais est capable de les dissoudre.

Lorsque la matrice $A_1^+X_1^-$comporte au moins un ion fonctionnel, elle peut servir pour une part de milieu réactionnel et pour une autre part de support soluble.

La matrice $A_1^+X_1^-$ peut contenir plusieurs cations et/ou anions non fonctionnels pour les raisons qui suivent :

D'une part, le mélange de cations peut provenir de l'industrie. En effet, de nombreux détergents à base de cations ammonium ou phosphonium sont des mélanges de sels produits en tant que tels par synthèse. Ils correspondent à des coupes. Des milliers de tonnes sont produits ainsi à bas prix. L'intérêt d'utiliser de tels mélanges dans le cadre de la présente invention est donc économique.

Le fait d'avoir des mélanges n'est pas gênant, si tous les constituants du mélange sont inertes chimiquement dans les conditions d'utilisation. Par exemple, un mélange de sels de tétralkyl ammonium ou de phosphonium non fonctionnels peut être utilisé.

D'autre part, le point de fusion d'un mélange est inférieur au point de fusion du constituant du mélange qui fond à la température la plus basse. Il peut donc être très important d'avoir recours à un mélange pour disposer d'un liquide ionique à température de fusion raisonnable.

Certains sels fonctionnalisés, en particulier ceux avec de gros anions tels que $NTf_2^-$, $PF_6^-$, $BF_4^-$ ou $CF_3SO_3^-$, peuvent être liquides à température ambiante ou fondre à basse température, par exemple

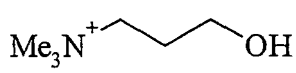 ,

$NTf_2^-$ est liquide à température ambiante. Ce liquide ionique est préparé par alkylation de $Me_3N$ selon la réaction suivante :

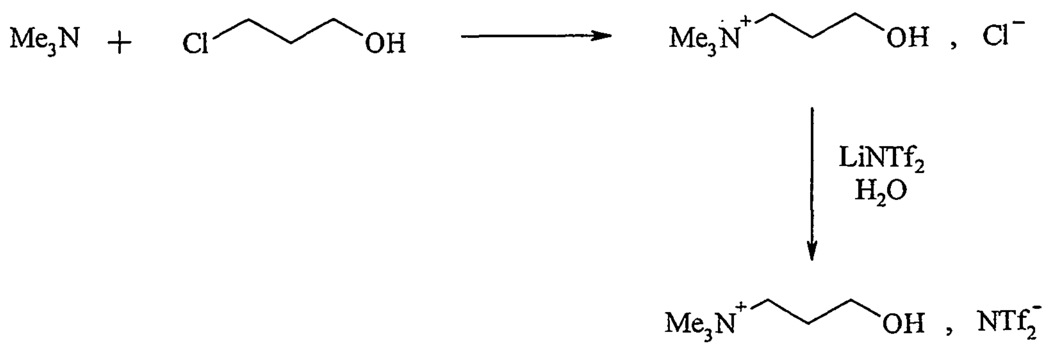


Tf représentant $CF_3SO_2$.

La présente invention concerne l'utilisation telle que définie ci-dessus, caractérisée en ce que $A_1^+$ représente un cation non fonctionnel ou un mélange de cations non fonctionnels et $X_1^-$ un anion non fonctionnel ou un mélange d'anions non fonctionnels.

La présente invention concerne également l'utilisation telle que définie ci-dessus, caractérisée en ce que :

$A_1^+$ représente un cation fonctionnel ou un mélange de cations dont l'un au moins est fonctionnel,
et/ou $X_1^-$ représente un anion fonctionnel ou un mélange d'anions dont l'un au moins est fonctionnel,
lesdits cations fonctionnels et anions fonctionnels correspondant à une entité ionique, à savoir respectivement cationique ou anionique, liée à au moins une fonction $F_i$, $F_i$ variant de $F_0$ à $F_n$, n étant un nombre entier variant de 1 à 10.

L'expression "entité ionique" désigne la partie du cation ou de l'anion, qui porte la charge, respectivement positive ou négative.

La fonction $F_i$ est notamment choisie parmi les fonctions suivantes :

hydroxyle, carboxylique, amide, sulfone, amine primaire, amine secondaire, aldéhyde, cétone, éthényle, éthynyle, diényle, éther, époxyde, phosphine (primaire, secondaire ou tertiaire), azoture, imine, cétène, cumulène, hétérocumulène, thiol, thioéther, sulfoxyde, groupements phosphorés, hétérocycles, acide sulfonique, silane, stannane ou aryle fonctionnel, et toute fonction résultant d'une transformation chimique, thermique, photochimique ou par irradiation micro-onde des fonctions précédentes.

La présente invention concerne l'utilisation d'un liquide ionique tel que défini ci-dessus, pour la préparation d'une composition stable contenant en solution :

- au moins ledit liquide ionique de formule $A_1^+X_1^-$, jouant le rôle de matrice liquide et,
- au moins un sel fonctionnalisé (sel à tâche dédiée), notamment sel d'onium fonctionnalisé, de formule $A_2^+X_2^-$, comme support de réaction,

le sel fonctionnalisé, notamment le sel d'onium fonctionnalisé, étant dissous dans la matrice liquide, pour former une phase homogène,
$A_1^+$ représentant un cation non fonctionnel ou un mélange de cations dans lequel aucun des cations n'est fonctionnel, et $X_1^-$ représentant un anion non fonctionnel ou un mélange d'anions dans lequel aucun des anions n'est fonctionnel, $A_2^+$ représentant un cation, fonctionnel ou non, ou un mélange de cations dans lequel aucun des cations n'est fonctionnel ou dans lequel l'un au moins des cations est fonctionnel, et $X_2^-$ représentant un anion, fonctionnel ou non, ou un mélange d'anions dans lequel aucun des anions n'est fonctionnel ou dans lequel l'un au moins des anions est fonctionnel,
sous réserve que $A_2^+$ et/ou $X_2^-$ représente(nt) ou comporte(nt) respectivement un cation fonctionnel et un anion fonctionnel,
lesdits cations fonctionnels et anions fonctionnels correspondant à une entité ionique Y-, à savoir respectivement cationique $Y^+$- ou anionique $Y^-$-, liée éventuellement par l'intermédiaire d'un bras L, notamment un groupe alkyle comprenant de 1 à 20 atomes de carbone, à au moins une fonction $F_i$, $F_i$ variant de $F_0$ à $F_n$, n étant un nombre entier variant de 1 à 10, le cation fonctionnel pouvant être représenté sous la forme $Y^+$-L-$F_i$, et l'anion fonctionnel sous la forme $Y^-$-$(L)_k$-$F_i$, k étant égal à 0 ou 1, et l'anion fonctionnel pouvant représenter, lorsque k est égal à 0, un anion simple, correspondant à $Y^-$-$F_i$, notamment choisi parmi : $OH^-$, $F^-$, $CN^-$, RO- ou $RS^-$, R représentant un groupement alkyle comprenant de 1 à

20 atomes de carbone ou un groupement aryle comprenant de 6 à 30 atomes de carbone.

L'expression "composition stable" désigne le mélange homogène composé de la matrice liquide $A_1^+X_1^-$ et du ou des sel(s) fonctionnalisé(s) $A_2^+X_2^-$. Cette composition est dite stable dans la mesure où elle ne subit pas de transformations spontanées au cours du temps.

On peut vérifier que cette composition est stable par analyse spectroscopique à l'aide de la RMN, de l'IR, de l'UV visible, de la spectrométrie de masse ou de méthodes de chromatographie.

L'expression "sel fonctionnalisé (sel à tâche dédiée)" désigne une entité de type $A_2^+X_2^-$ dans laquelle le cation et/ou l'anion porte une fonction $F_i$ telle que définie précédemment. Cette fonction confère audit sel fonctionnalisé et à la composition stable, dont il fait partie, des propriétés chimiques et/ou physico-chimiques.

L'expression "sel d'onium fonctionnalisé" désigne les sels d'ammonium, de phosphonium, de sulfonium, ainsi que tous les sels résultant de la quaternarisation d'une amine, d'une phosphine, d'un thioéther ou d'un hétérocycle contenant l'un ou plusieurs de ces hétéroatomes, et portant au moins une fonction $F_i$. Cette expression désigne aussi un sel d'onium dont le cation tel que défini ci-dessus n'est pas fonctionnalisé mais dont l'anion porte une fonction $F_i$. Cette expression peut également désigner un sel dont l'anion et le cation portent une fonction $F_i$.

Un sel d'onium fonctionnalisé préféré est notamment choisi parmi les suivants :

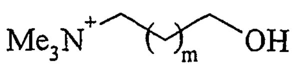 , $Cl^-$ ou $NTf_2^-$ ou $PF_6^-$ ou $BF_4^-$

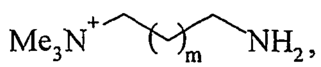 , $Cl^-$ ou $NTf_2^-$ ou $PF_6^-$ ou $BF_4^-$

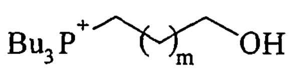 , $Cl^-$ ou $NTf_2^-$ ou $PF_6^-$ ou $BF_4^-$

m étant un nombre entier compris de 0 à 20.

Un sel d'onium non fonctionnalisé préféré est notamment choisi parmi les suivants : cations imidazolium, pyridinium $Me_3N^+$- Bu ou $Bu_3P^+$- Me, anions $NTf_2^-$, $PF_6^-$ ou $BF_4^-$.

L'expression "support de réaction" désigne tout sel de type $A_2^+X_2^-$ fonctionnalisé par une fonction $F_i$ transformable et clivable de façon à libérer la molécule recherchée en fin de séquence réactionnelle. Cette expression désigne notamment tout sel de type $A_2^+X_2^-$ fonctionnalisé par une fonction $F_n$ (dernière fonction dans l'enchaînement réactionnel) clivable de façon à libérer la molécule recherchée en fin de séquence réactionnelle

Dans le terme "Y-", le tiret "-" représente la liaison éventuelle entre l'entité ionique et le bras L.

Dans le terme "$Y^+$-", le tiret "-" représente la liaison éventuelle entre l'entité cationique et le bras L.

Dans le terme "$Y^-$-", le tiret "-" représente la liaison éventuelle entre l'entité anionique et le bras L.

Le terme "bras L" désigne une chaîne alkyle ou aralkyle pouvant contenir un ou plusieurs hétéroatomes tels que l'azote, le phosphore, le soufre, l'oxygène, le silicium, l'étain, contenant entre 1 et 30 atomes de carbone, et ledit bras est notamment choisi parmi une chaîne alkyle contenant de 2 à 10 atomes de carbone et de 1 à 6 atomes d'oxygène ou d'azote.

Dans ce mode de réalisation de l'invention, la matrice ionique est non fonctionnelle et il est nécessaire que le sel d'onium $A_2^+X_2^-$ soit fonctionnalisé, soit par l'intermédiaire du cation $A_2^+$, soit par l'intermédiaire de l'anion $X_2^-$, soit par l'intermédiaire du cation $A_2^+$ et de l'anion $X_2$-.

La fonctionnalisation du cation seul implique que l'anion est simplement "spectateur" lors des modifications fonctionnelles et n'est là que pour assurer la neutralité électrique. Le fait de n'avoir une fonction que sur le cation avec un anion inerte entraîne une simplification de la maîtrise des réactions.

De même, si le cation est inerte chimiquement, il est possible de modifier l'anion sans se soucier du cation. L'utilisation d'un anion fonctionnel présente des intérêts supplémentaires :

- une simplicité de synthèse du sel fonctionnalisé par simple neutralisation ou métathèse d'anion, par exemple :

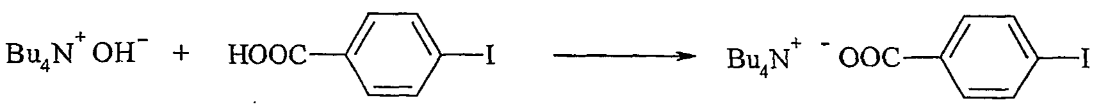

on peut utiliser la solution de carboxylate d'ammonium ainsi obtenue directement dans un couplage de Heck ou de Suzuki ;

- en terme de clivage, il suffit de laver par une solution de HCl, notamment pour libérer, dans le cas de l'exemple susmentionné, un acide carboxylique :

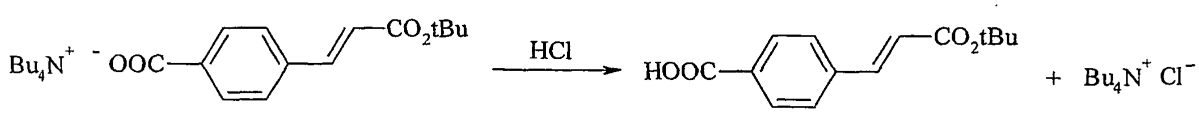


composé obtenu après un
couplage de Heck
cet exemple concerne un acide de Bronsted ;

- l'anion peut également réagir par neutralisation d'un acide de Lewis par exemple :

$$Bu_4N^+OH^- + Ar_1B(OH)_2 \rightarrow Bu_4N^+\ Ar_1\overline{B}(OH)_3$$

l'anion ainsi obtenu peut alors servir d'intermédiaire dans une réaction, notamment le couplage de Suzuki, selon la réaction suivante :

$$Bu_4N^+OH^- + Ar_1B(OH)_2 \rightarrow Bu_4N^+Ar_1\overline{B}^-(OH)_3$$

on peut noter que ce couplage est "décrochant" dans la mesure où il n'y a pas besoin d'une réaction de clivage pour récupérer le produit final.

L'utilisation d'un sel fonctionnalisé mettant en jeu à la fois l'anion et le cation est intéressante dans des séquences plus compliquées. Il est possible de modifier le cation ou l'anion sélectivement et de provoquer la réaction de l'anion avec le cation dans une transformation ultime via les fonctions que portent le cation et l'anion. Il est également possible de partir d'un sel fonctionnalisé dont seul le cation est fonctionnalisé. On modifie la fonction $F_0$ pour obtenir la fonction $F_i$ et, par métathèse, on introduit un anion fonctionnel dont la fonction pourra réagir avec la fonction $F_i$ portée par le cation fonctionnalisé.

La présente invention concerne l'utilisation telle que définie ci-dessus, pour la préparation d'une composition stable contenant en solution :

- au moins une première partie dudit liquide ionique de formule $A_1{}^+X_1{}^-$, dont le cation et/ou l'anion correspond(ent) à une entité ionique liée à une ou des fonctions initiales $F_0$, jouant le rôle de matrice liquide, et
- au moins une deuxième partie dudit liquide ionique de formule $A_1{}^+X_1$-, dans laquelle ladite ou lesdites fonctions initiales $F_0$ sont transformées en de premières nouvelles fonctions, conférant à ladite deuxième partie dudit liquide ionique le rôle de sel fonctionnalisé et de support de réaction,

le sel fonctionnalisé et la matrice liquide formant une phase homogène,
les susdites premières nouvelles fonctions de la deuxième partie dudit liquide ionique étant susceptibles d'être transformées ultérieurement en d'autres fonctions, sans qu'il y ait affectation de la ou des fonctions initiales $F_0$ de la première partie dudit liquide ionique.

L'expression "sans qu'il y ait affectation de la ou des fonctions initiales $F_0$ de la première partie dudit liquide ionique" signifie que les premières nouvelles fonctions de la deuxième partie dudit liquide ionique sont transformées ultérieurement en d'autres fonctions, par des transformations chimiosélectives.

Ce mode de réalisation particulier de l'invention correspond au cas où le liquide ionique $A_1{}^+X_1{}^-$ joue à la fois le rôle de matrice liquide et de sel fonctionnalisé.

Le cas où le liquide ionique $A_1{}^+X_1{}^-$ joue à la fois le rôle de matrice liquide et de sel fonctionnalisé est intéressant

dans la mesuré où on part d'un seul produit initial. De plus, après réaction, lors du clivage par transestérification ou transamidation, par exemple, le sel de départ est régénéré et donc recyclé. Par ailleurs, la fonction $F_0$ du sel de départ peut conférer au milieu des propriétés particulières d'activation de réaction, par exemple par liaison hydrogène ou par toute autre activation dépendant de la fonction $F_0$.

La présente invention concerne l'utilisation d'un liquide ionique tel que défini ci-dessus, caractérisé en ce que le cation $A_2^+$ et/ou l'anion $X_2^-$ du ou des sels fonctionnalisés, correspondant à une entité ionique Y- liée à au moins une fonction $F_i$, sont immobilisés dans la matrice liquide et ne peuvent pas être extraits de la matrice liquide par des moyens conventionnels d'extraction, notamment par solvant, et dans laquelle la(les) fonction(s) $F_i$ du ou des sels fonctionnalisés peuvent être transformée(s) à l'issue d'au moins une réaction résultant de l'addition d'au moins un réactif dans la susdite composition.

Le terme "immobilisés" signifie que le sel fonctionnalisé ne peut être extrait de la matrice par des moyens conventionnels tels que l'extraction ou la distillation.

L'expression "ne peuvent pas être extraits de la matrice liquide par des moyens conventionnels d'extraction" désigne le fait que le mélange formé par la matrice liquide et le sel fonctionnalisé peut être lavé à l'aide de solvants classiques ou chauffé sous vide sans perte dudit sel fonctionnalisé. Cela permet notamment l'utilisation d'excès de réactifs qui peuvent être éliminés lorsque la réaction est terminée comme dans le cas des résines.

La présente invention repose sur l'aspect inattendu selon lequel le mélange d'un sel fonctionnalisé dans une matrice liquide $A_1^+X_1^-$ aboutit à l'immobilisation dudit sel fonctionnalisé dans ladite matrice liquide.

La présente invention concerne l'utilisation d'un liquide ionique tel que défini ci-dessus, caractérisé en ce que plusieurs sels fonctionnalisés sont immobilisés.

Il est possible de conférer à une solution de plusieurs sels dans une matrice liquide ionique plusieurs propriétés que l'on pourra utiliser en cascade ou en multicomposant.

La présente invention concerne l'utilisation telle que définie ci-dessus, caractérisée en ce que le cation $A_2^+$ est fonctionnel.

Selon un mode de réalisation avantageux, la présente invention concerne l'utilisation telle que définie ci-dessus, caractérisée en ce que le cation $A_2^+$ est fonctionnel et l'anion $X_2^-$ est non fonctionnel.

La présente invention concerne l'utilisation telle que définie ci-dessus, caractérisée en ce que l'anion $X_2^-$ est fonctionnel.

Selon un mode de réalisation avantageux, la présente invention concerne l'utilisation telle que définie ci-dessus, caractérisée en ce que le cation $X_2^-$ est fonctionnel et l'anion $A_2^+$ est non fonctionnel.

La présente invention concerne l'utilisation telle que définie ci-dessus, caractérisée en ce que $A_2^+$ et $X_2^-$ sont fonctionnels.

La présente invention concerne l'utilisation telle que définie ci-dessus, caractérisée en ce que :

- soit le liquide ionique de formule $A_1^+X_1$- est solide à température ambiante et est liquéfiable dans une gamme de températures allant d'environ 25°C à environ 250°C, notamment d'environ 30°C à environ 150°C, et le sel fonctionnalisé $A_2^+x_2^-$ est solide à température ambiante et est soluble dans le liquide ionique $A_1^+X_1^-$ liquéfié, pour former une phase homogène,
- soit le liquide ionique de formule $A_1^+X_1^-$ est solide à température ambiante et est liquéfiable dans une gamme de températures allant d'environ 25°C à environ 250°C, notamment d'environ 30°C à environ 150°C, et le sel fonctionnalisé $A_2^+X2^-$ est liquide à température ambiante, et est miscible avec le liquide ionique $A_1^+X_1^-$ liquéfié pour former une phase homogène,
- soit le liquide ionique $A_1^+X_1^-$ est liquide à température ambiante et le sel fonctionnalisé $A_2^+X_2^-$ est liquide à température ambiante et miscible avec le liquide ionique $A_1^+X_1^-$, pour former une phase homogène,
- soit le liquide ionique $A_1^+X_1^-$ est liquide à température ambiante et le sel fonctionnalisé $A_2^+X_2^-$ est solide à température ambiante et est soluble ou partiellement soluble dans le liquide ionique $A_1^+X_1^-$ dans une gamme de températures allant d'environ 25°C à environ 250°C, notamment d'environ 30°C à environ 150°C, pour former une phase homogène.

Lorsque le liquide ionique de formule $A_1^+X_1^-$ et le sel fonctionnalisé $A_2^+X_2^-$ sont solides à température ambiante, on obtient, après les transformations de la fonction initiale $F_0$ en des fonctions $F_i$ et le clivage, un mélange formé d'un solide et de la molécule recherchée d'où il est possible d'extraire cette molécule, par simple ajout d'un solvant solubilisant la molécule et non les sels fonctionnalisés, et par simple filtration. Ce mode de réalisation permet donc d'ajouter quelques avantages propres à la technique sur supports solides, tout en conservant les avantages des réactions sur supports solubles.

Lorsque le liquide ionique de formule $A_1^+X_1^-$ est solide à température ambiante et le sel fonctionnalisé $A_2^+X_2^-$ est liquide à température ambiante, on se trouve dans le cas où on ajoute un sel fonctionnalisé liquide à une matrice solide à température ambiante. Une phase homogène est obtenue à la fusion du mélange qui peut soit rester liquide, soit donner un solide, soit donner un mélange hétérogène solide/liquide.

Le cas où le liquide ionique $A_1^+X_1$- et le sel fonctionnalisé $A_2^+X_2^-$ sont liquides à température ambiante correspond au cas standard permettant de faire des réactions à température ambiante ou sub-ambiante.

Lorsque le liquide ionique $A_1^+X_1$- est liquide à température ambiante et le sel fonctionnalisé $A_2^+X_2$- est solide à température ambiante, on peut rencontrer plusieurs cas distincts, et notamment :

- soit le solide $A_2^+X_2^-$ est soluble dans $A_1^+X_1^-$ et on travaille en solution comme dans le cas précédent ; ce cas est très fréquent en raison par exemple de l'utilisation de chlorures, de bromures ou d'iodures solides à température ambiante, peu coûteux et faciles à purifier par recristallisation ;
- soit le solide $A_2^+X_2^-$ est soluble dans $A_1^+X_1^-$ à une température supérieure à la température ambiante, ce qui oblige à travailler à cette température afin d'obtenir une phase homogène et d'éviter une reprécipitation ; sinon, une fois que le sel fonctionnalisé est dissous, on récupère une solution à température ambiante sans reprécipitation ;
- soit il est nécessaire de dissoudre le sel fonctionnalisé $A_2^+X_2^-$ dans un solvant traditionnel, de mélanger la solution à $A_1^+X_1^-$ liquide, puis d'éliminer le solvant sous vide pour obtenir une solution de $A_2^+X_2^-$.

La présente invention concerne l'utilisation telle que définie ci-dessus, caractérisée en ce que :

- soit le liquide ionique de formule $A_1^+X_1$- est liquide à température ambiante,
- soit le liquide ionique de formule $A_1^+X_1^-$ est solide à température ambiante et est liquéfiable dans une gamme de températures allant d'environ 25°C à environ 250°C, notamment d'environ 30°C à environ 150°C.

La présente invention concerne. également l'utilisation telle que définie ci-dessus, caractérisée en ce que le liquide ionique de formule $A_1^+X_1^-$, jouant le rôle de matrice liquide, présente une viscosité inférieure ou égale à environ 1500 cp (15 N.s/m$^2$), notamment inférieure à environ 500 cp (5 N.s/m$^2$) et de préférence inférieure à environ 200 cp (2 N.s/m$^2$).

Ainsi, le tableau ci-dessous correspond aux viscosités à 23°C des liquides ioniques préférés de l'invention.

| Liquide Ionique | Viscosité en (cP) à 23 °C |
|---|---|
| [btma][$NTf_2$] | 58 |
| [htma][$NTf_2$] | 64 |
| [$C_3$OHtma] [$NTf_2$] | 94,3 |
| [$C_3$CNtma][$NTf_2$] | 62 |
| [$C_5$CNtma] [$NTf_2$] | 69 |

[btma][$NTf_2$] = [$C_4$tma][$NTf_2$] = [$Me_3N^+$-$C_4H_9$] [$N(SO_2CF_3)_2$]

[htma][$NTf_2$] = [$C_6$tma][$NTf_2$] = [$Me_3N^+$-$C_6H_{11}$] [$N^-(SO_2CF_3)_2$]

[$C_3$OHtma][$NTf_2$] = [$Me_3N^+$-$(CH_2)_2CH_2OH$] [$N(SO_2CF_3)_2$]

[$C_3$CNtma][$NTf_2$] = [$Me_3N^+$-$(CH_2)_2CH_2CN$] [N ($SO_aCF_3)_2$]

[$C_5$CNtma][$NTf_2$] = [$Me_3N^+$-$(CH_2)_4CH_2CN$] [$M(So_2CF_3)_2$]

Méthode et appareil de mesures de viscosité dynamique :

Les mesures de viscosité dynamique ont été réalisées à 23°C sur un microviscosimètre de type Rheolyst AR 1000 (TA instruments) muni d'un cône plat en acier de diamètre P = 40 mm et d'angle t = 1 degré 1 minute. La procédure est appliquée de 0,06 à 200$s^{-1}$ pendant 3 minutes avec 20 points par décade. L'appareil est piloté par ordinateur avec le logiciel Rheology Advantage Instrument Control AR.

La présente invention concerne une composition stable contenant en solution :

- au moins ledit liquide ionique de formule $A_1^+X_1^-$, jouant le rôle de matrice liquide et,
- au moins un sel fonctionnalisé (sel à tâche dédiée), notamment sel d'onium fonctionnalisé, de formule $A_2^+X_2^-$, comme support de réaction,

le sel fonctionnalisé, notamment le sel d'onium fonctionnalisé, étant dissous dans la matrice liquide, pour former une phase homogène,

$A_1^+$ représentant un cation non fonctionnel ou un mélange de cations dans lequel aucun des cations n'est fonctionnel, et $X_1^-$ représentant un anion non fonctionnel ou un mélange d'anions dans lequel aucun des anions n'est fonctionnel, $A_2^+$ représentant un cation, fonctionnel ou non, ou un mélange de cations dans lequel aucun des cations n'est fonctionnel ou dans lequel l'un au moins des cations est fonctionnel, et $X_2^-$ représentant un anion, fonctionnel ou non, ou un mélange d'anions dans lequel aucun des anions n'est fonctionnel ou dans lequel l'un au moins des anions est fonctionnel, sous réserve que $A_2^+$ et/ou $X_2$- représente(nt) où comporte(nt) respectivement un cation fonctionnel et un anion fonctionnel,

lesdits cations fonctionnels et anions fonctionnels correspondant à une entité ionique Y-, à savoir respectivement cationique $Y^+$- ou anionique $Y^-$-, liée éventuellement par l'intermédiaire d'un bras L, notamment un groupe alkyle comprenant de 1 à 20 atomes de carbone, à au moins une fonction $F_i$, $F_i$ variant de $F_0$ à $F_n$, n étant un nombre entier variant de 1 à 10, le cation fonctionnel pouvant être représenté sous la forme $Y^+$-L-$F_i$, et l'anion fonctionnel sous la forme $Y^-$-$(L)_k$-$F_i$, k étant égal à 0 ou 1, et l'anion fonctionnel pouvant représenter, lorsque k est égal à 0, un anion simple, correspondant à $Y^-$-$F_i$, notamment choisi parmi : $OH^-$, $F^-$, $CN^-$, RO- ou $RS^-$, R représentant un groupement alkyle comprenant de 1 à 20 atomes de carbone ou un groupement aryle comprenant de 6 à 30 atomes de carbone.

La présente invention concerne une composition stable contenant en solution :

- au moins une première partie dudit liquide ionique de formule $A_1^+X_1^-$, dont le cation et/ou l'anion correspond(ent) à une entité ionique liée à une ou des fonctions initiales $F_0$, jouant le rôle de matrice liquide, et
- au moins une deuxième partie dudit liquide ionique de formule $A_1^+X_1^-$, dans laquelle ladite ou lesdites fonctions initiales $F_0$ sont transformées en de premières nouvelles fonctions, conférant à ladite deuxième partie dudit liquide ionique le rôle de sel fonctionnalisé et de support de réaction,

le sel fonctionnalisé et la matrice liquide formant une phase homogène,

les susdites premières nouvelles fonctions de la deuxième partie dudit liquide ionique étant susceptibles d'être transformées ultérieurement en d'autres fonctions, sans qu'il y ait affectation de la ou des fonctions initiales $F_0$ de la première partie dudit liquide ionique.

Selon un mode de réalisation avantageux de la présente invention, une composition de l'invention est caractérisée en ce que le cation $A_2^+$ et/ou l'anion $X_2^-$ du

ou des sels fonctionnalisés, correspondant à une entité ionique Y-liée à au moins une fonction $F_i$, sont immobilisés dans la matrice liquide et ne peuvent pas être extraits de la matrice liquide par des moyens conventionnels d'extraction, notamment par solvant.

Une composition avantageuse de la présente invention est caractérisée en ce que la matrice liquide est non réactive vis-à-vis du sel fonctionnalisé.

La propriété de non-réactivité de la matrice vis-à-vis du sel est vérifiée par exemple à l'aide des techniques spectroscopiques habituelles telles que la RMN $^1H$, $^{13}C$, la spectrométrie de masse, ou par analyse HPLC.

Une autre composition avantageuse de la présente invention est caractérisée en ce que $A_2^+$ est un cation fonctionnel.

Une autre composition avantageuse de la présente invention est caractérisée en ce que les anions $X_1^-$ et $X_2^-$ sont identiques.

Ce cas particulier concerne notamment le cas des gros anions tels que $NTf_2^-$, $BF_4^-$, $PF_6^-$, $CF_3SO_3^-$ couramment utilisés pour la préparation de liquides ioniques. Ce mode de réalisation particulier favorise également la solubilité d'un sel dans l'autre.

Une autre composition avantageuse de la présente invention est caractérisée en ce que les anions $X_1^-$ et $X_2^-$ sont différents. Ce cas particulier concerne le cas général où un sel fonctionnalisé, par exemple un halogénure ($Cl^-$, $Br^-$, $I^-$, $F^-$) est dissous dans une matrice liquide ionique. Ce mode de réalisation particulier présente l'avantage particulier de permettre la dissolution de sels peu coûteux.

Selon un mode de réalisation avantageux, une composition selon la présente invention est caractérisée en ce que :

- les anions $X_1$- et $X_2^-$ sont choisis parmi les deux familles suivantes :

  * les anions non complexes, choisis notamment parmi les anions $BF_4^-$, $PF_6^-$, $CF_3SO_3^-$, $CH_3COO^-$, $CF_3CO_2^-$, $^-N(SO_2CF_3)_2$ (ou $NTf_2^-$), les halogénures, les anions $BR_4^-$, $RCO_2^-$ ou $RSO_3^-$, R étant un groupe alkyle comprenant de 1 à 20 atomes de carbone ou un groupe aryle comprenant de 6 à 30 atomes de carbone, ledit groupe R pouvant également représenter un groupe perfluoré ou partiellement fluoré, ou les anions $R'SO_4^-$, R' étant un atome d'hydrogène, un groupe méthyle ou un groupe éthyle ;

* les anions complexes, résultant de la combinaison d'un acide de Lewis et d'un halogénure, de préférence $Cl^-$ ou $F^-$, de formule générale $MX_j$, j étant un nombre entier compris entre 1 et 7, et M représentant un métal, notamment choisi parmi l'aluminium, l'étain, le zinc, le bismuth, le manganèse, le fer, le cuivre, le molybdène, l'antimoine, le gallium ou l'indium ;

- les cations $A_1^+$ et $A_2^+$ sont choisis parmi les cations onium, tels que les cations pyridinium, imidazolium, ammonium, phosphonium ou sulfonium, substitués ou non, et de préférence ammonium ou phosphonium.

Une composition avantageuse de la présente invention est caractérisée en ce que le cation fonctionnel $A_2^+$ correspond à une entité cationique $Y^+$-, liée, éventuellement par l'intermédiaire d'un bras L, notamment un groupe alkyle comprenant de 1 à 20 atomes de carbone, à une fonction $F_0$, ladite fonction $F_0$ étant choisie parmi les fonctions classiques de la chimie organique, telles que les fonctions hydroxyle, carboxylique, amide, sulfone, amine primaire, amine secondaire, aldéhyde, cétone, éthényle, éthynyle, diényle, éther, époxyde, phosphine (primaire, secondaire ou tertiaire), azoture, imine, cétène, cumulène, hétérocumulène, thiol, thioéther, sulfoxyde, groupements phosphorés, hétérocycles, acide sulfonique, silane, stannane ou aryle fonctionnel.

Selon un mode de réalisation avantageux, une composition de l'invention est caractérisée en ce que le liquide ionique est choisi parmi les suivants :

$(Ra)_3N^+\text{-}R_b, X_1^-$

$(Ra)_3P^+\text{-}R_b, X_1^-$

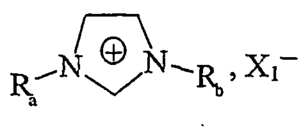

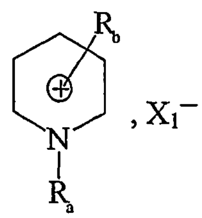

$R_a$ et $R_b$ représentant des groupes alkyle linéaires ou ramifiés, comprenant de 1 à 20 atomes de carbone, notamment un groupe éthyle, propyle, butyle, pentyle, hexyle, heptyle ou octyle, ou des groupes alkyle fonctionnels comprenant de 1 à 20 atomes de carbone, ou des groupes aryle fonctionnels ou non comprenant de 6 à 30 atomes de carbone,

$Bu_3P^+\text{-}Me , X_1^-$

$^{\oplus}P(C_6H_{13})_3C_{14}H_{29}, X_1\text{-}$

$(C_8H_{17})_3N^+Me , X_1^-$

$X_1^-$ étant notamment choisi parmi : $NTf_2^-$, $PF_6^-$, $BF_4^-$ ou $CF_3SO_3^-$,

Une composition avantageuse de l'invention est caractérisée en ce que le sel fonctionnalisé est choisi parmi les suivants :

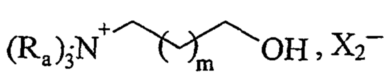

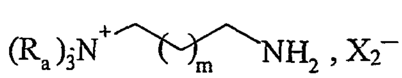

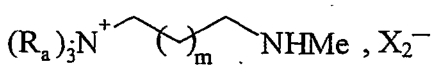
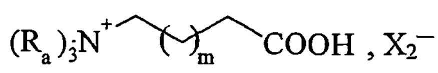

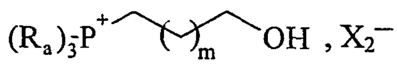
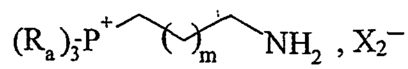

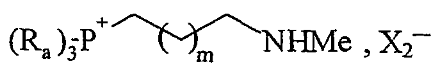
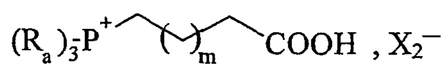

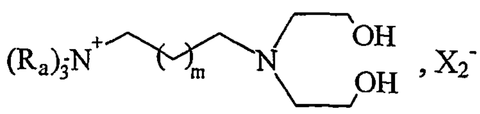
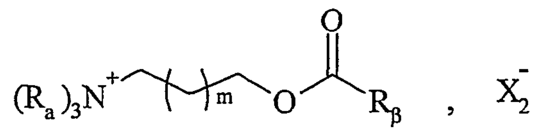

$X_2^-$ étant choisi parmi : $NTf_2^-$, $PF_6^-$, $BF_4^-$, $Cl^-$, $Br^-$, $I^-$, $CF_3SO_3^-$, $MeSO_4^-$, $EtSO_4^-$, $MeSO_3^-$, $C_6H_5SO_3^-$, $pMeC_6H_4SO_3^-$ ,
m étant un nombre entier compris de 0 à 20,
$R_\beta$ représentant un groupe vinyle, substitué ou non, aryle fonctionnel comprenant de 1 à 20 atomes de carbone, ou alkyle fonctionnel comprenant de 6 à 30 atomes de carbone,
et $R_a$ représentant un groupe alkyle ramifié ou non comprenant de 1 à 20 atomes de carbone, notamment un groupe éthyle, propyle, butyle, pentyle, hexyle, heptyle ou octyle.

Selon un mode de réalisation avantageux, une composition de l'invention est caractérisée en ce que $X_2^-$ est un anion fonctionnel, correspondant en particulier à un anion dont le $pK_A$ de l'acide conjugué est inférieur à 30, et est notamment choisi parmi les anions suivants :

$OH^-$, $F^-$, $R_cBZ_3^-$, $N_3^-$, $CN^-$, ou

Z représentant un groupement -F, -OH, -OR, R représentant un groupe alkyle comprenant de 1 à 20 atomes de carbone,
V et W représentant, indépendamment l'un de l'autre, un groupement électroattracteur, notamment un groupement cyano, alcoxycarbonyle comprenant de 2 à 20 atomes de carbone, acyle comprenant de 2 à 20 atomes de carbone, benzoyle, alkyle sulfonyle comprenant de 1 à 20 atomes de carbone, aryle sulfonyle comprenant de 6 à 30 atomes de carbone, dialcoxyphosphonyle comprenant de 2 à 20 atomes de carbone,
$R_c$ représentant un groupement alkyle, ramifié ou non, cyclique ou non, comprenant de 1 à 20 atomes de carbone, ou un groupement aryle comprenant de 6 à 30 atomes de carbone,
et en ce que le cation $A_2^+$ est choisi parmi les cations ammonium et phosphonium, notamment parmi les cations suivants :

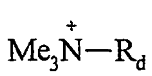
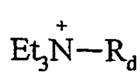
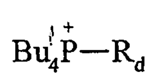

$R_d$ étant un groupe alkyle comprenant de 1 à 20 atomes de carbone.

La présente invention concerne également l'utilisation d'une composition telle que définie ci-dessus, pour la synthèse organique, en continu, en discontinu, combinatoire, ou parallèle, et/ou pour la préparation de banques de produits.

La présente invention concerne également l'utilisation d'une composition telle que définie ci-dessus, pour la mise en oeuvre d'un procédé de préparation d'une molécule G d'une fonction initiale $F_0$, liée, éventuellement par l'intermédiaire d'un bras L, notamment un groupe alkyle comprenant de 1 à 20 atomes de carbone, à une entité ionique $Y^+$-, faisant partie du cation $A_2^+$ du sel fonctionnalisé $A_2^+X_2^-$, et/ou $Y^-$-, faisant partie de l'anion $X_2^-$ du sel fonctionnalisé $A_2^+X_2^-$, le cation étant sous la forme $Y^+$-L-$F_0$ et/ou l'anion étant sous la forme $Y^-$ -$(L)_k$-$F_0$, k étant égal à 0 ou 1, lequel procédé comprend les étapes :

- d'une première addition d'un réactif $B_1$ dans la composition susmentionnée et la réaction entre ladite fonction $F_0$, et le réactif $B_1$, conduisant à une fonction $F_1$, liée à l'entité ionique $Y^+$-, faisant partie du cation $A_2^+$ du sel fonctionnalisé $A_2^+X_2^-$, et/ou à l'entité ionique $Y^-$-, faisant partie de l'anion $X_2^-$ du sel fonctionnalisé $A_2^+X_2^-$, selon l'un des schémas réactionnels suivants :

$$Y^+\!-L-F_0\,,\,X_2^- \xrightarrow{B_1} Y^+\!-L-F_1\,,\,X_2^-$$

ou

$$A_2^+\,,\,Y^-\!-(L)_k-F_0 \xrightarrow{B_1} A_2^+\,,\,Y^-\!-(L)_k-F_1$$

- de n-1 additions successives de réactifs $B_i$, $1 < i \leq n$, n variant de 2 à 10, à la composition susmentionnée, permettant, à chaque addition, la réaction entre le réactif $B_i$ et une fonction $F_{i-1}$, conduisant à l'obtention d'une fonction $F_i$, la (n-1ème) addition du réactif $B_n$ sur la fonction $F_{n-1}$ conduisant à l'obtention de la fonction $F_n$, les n-1 additions pouvant être représentées selon l'un des schémas réactionnels suivants :

$$Y^+\!-L-F_1\,,\,X_2^- \xrightarrow{B_2} Y^+\!-L-F_2\,,\,X_2^- \cdots\cdots Y^+\!-L-F_{i-1}\,,\,X_2^- \xrightarrow{Bi} Y^+\!-L-F_i\,,\,X_2^- \cdots\cdots \xrightarrow{B_n} Y^+\!-L-F_n\,,\,X_2^-$$

ou

$$A_2^+\,,Y^-\!-(L)_k-F_1 \xrightarrow{B_2} A_2^+\,,\,Y^-\!-(L)_k-F_2 \cdots\cdots A_2^+\,,Y^-\!-(L)_k-F_{i-1} \xrightarrow{Bi} A_2^+\,,Y^-\!-(L)_k-F_i \cdots\cdots \xrightarrow{B_n} A_2^+\,,Y^-\!-(L)_k-$$

- de clivage de la fonction $F_n$, liée à l'entité ionique $Y^+$- ou $Y^-$- respectivement du cation $A_2^+$ et/ou de l'anion $X_2^-$, permettant de récupérer d'une part le sel fonctionnalisé $A_2^+X_2^-$ sous la forme $Y^+$-L-$F_0$, $X_2^-$ ou

$A_2^+$, $Y^-$-$(L)_k$-$F_0$, en solution dans la matrice liquide ionique $A_1^+X_1^-$, ou sous la forme $Y^+$-L-$F'_0$, $X_2^-$ ou $A_2^+$, $Y^-$-$(L)_k$-$F'_0$, dans lesquelles $F'_0$ représente une fonction différente de $F_0$,
et d'autre part la molécule G,
selon l'un des schémas réactionnels suivants :

$$Y^+\!-L-F_n\,,\,X_2^- \xrightarrow{\text{clivage}} G + Y^+\!-L-F_0\,,\,X_2^-$$

ou

$$Y^+\!-L-F'_n\,,\,X_2^- \xrightarrow{\text{clivage}} G + Y^+\!-L-F'_0\,,\,X_2^-$$

ou

$$A_2^+,\ Y^-\!-\!(L)_k\!-\!F_n \xrightarrow{\text{clivage}} G + A_2^+,\ Y^-\!-\!(L)_k\!-\!F_0$$

ou

$$A_2^+,\ Y^-\!-\!(L)_k\!-\!F'_n \xrightarrow{\text{clivage}} G + A_2^+,\ Y^-\!-\!(L)_k\!-\!F'_0$$

L'utilisation des liquides ioniques dans un tel enchaînement de réactions présente les avantages suivants :

- les réactions sont effectuées en solution, et donc toutes les techniques d'analyse, incluant la RMN $^{1}H$, $^{13}C$, $^{19}F$, $^{31}P$, $^{11}B$, $^{15}N$ etc..., la HPLC, l'IRTF, l'UV, visible, fluorescence, les techniques électrochimiques, l'électrophorèse, la spectrométrie de masse etc..., peuvent être utilisées dans les conditions normales sans complications particulières ;
- les réactions sont effectuées aux concentrations habituelles de 0,5 à 1 mole par litre (voir plus), ce qui représente un énorme avantage en terme de charge spécifique ;
- le recyclage de la solution de sel fonctionnalisé est facile ;
- les solutions sont facilement transférables à l'aide des techniques de seringues et (ou) de pompage ;
- la synthèse des liquides ioniques et des sels fonctionnalisés est très simple et certains sont disponibles dans le commerce ;
- une immense variété de liquides ioniques et de sels fonctionnalisés est facilement accessible ;
- les solutions se prêtent aisément aux techniques de partition et donc aux techniques de synthèse parallèle ou combinatoire, ce qui permet d'accéder à des bibliothèques de produits ;
- des réactivités et des sélectivités nouvelles ont été observées dans ces milieux ;
- la montée en échelle (scale up) ne pose pas de problèmes différents de ceux observés lors du travaux dans les solvants usuels ;
- un parallèle est facilement établi entre cette nouvelle technologie et les techniques de synthèse sur support solide et synthèse sur polymères solubles. On en déduit immédiatement et évidemment que les sels fonctionnalisés à l'identique des résines de Wang, Rink, silylalkyle, carbonate, carboxyliques, formyle, hydroxy, amino, oxime etc... peuvent être utilisés de façon beaucoup plus aisée et avantageuse.

La présente invention concerne l'utilisation telle que définie ci-dessus, pour la mise en oeuvre de la réaction de Diels-Alder, selon l'un des schémas réactionnels suivants :

a) 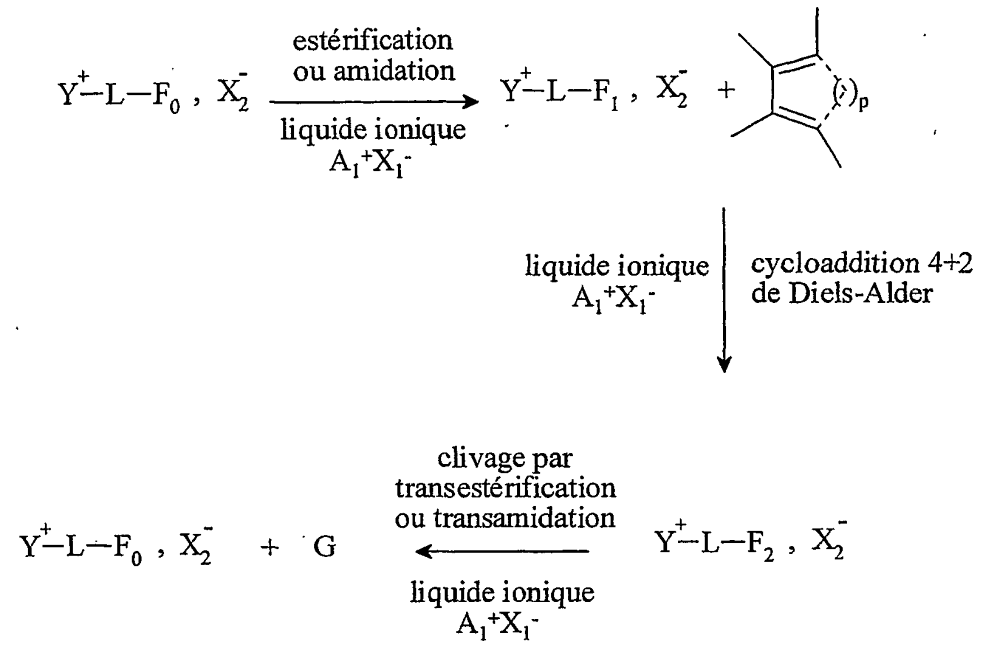


p étant un nombre entier variant de 0 à 2,
$Y^+$- représentant un cation onium tel que défini ci-dessus, et étant de préférence un cation triméthylalkylammonium, triéthylalkylammonium ou tributylalkylphosphonium,
L représentant un bras, notamment un groupe alkyle linéaire ou ramifié comprenant de 1 à 20 atomes de carbone, ou un groupe aralkyle éventuellement fonctionnel, comprenant de 6 à 30 atomes de carbone, et étant de préférence un groupe alkyle linéaire de préférence un groupe alkyle linéaire de type $(CH_2)_r$, r variant de 1 à 20, et de préférence de 3 à 6,
$X_2^-$ étant tel que défini ci-dessus, et étant notamment $NTf_2^-$, $BF_4^-$, $PF_6^-$, $Cl^-$, $Br^-$, $CH_3COO^-$, $CF_3CO_2^-$, $CF_3SO_3^-$, $BR_4^-$, R étant tel que défini ci-dessus,
le liquide ionique $A_1^+X_1^-$ étant notamment de la forme :

$(Ra)_3N^+-R_b, X_1^-$

$(Ra)_3P^+-R_b, X_1^-$

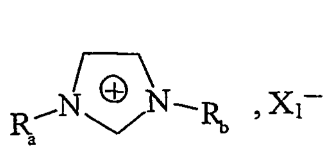

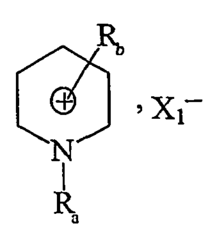

$R_a$ et $R_b$ étant tels que définis ci-dessus, et représentant de préférence des groupes alkyle comprenant de 1 à 20 atomes de carbone,
$X_1$- étant choisi parmi : $BF_4^-$, $PF_6^-$, $NTf_2^-$, $Cl^-$, $Br^-$, $CH_3COO^-$, $CF_3CO_2^-$, $CF_3SO_3^-$, $BR_4^-$, R étant tel que défini ci-dessus,
les fonctions $F_0$, $F_1$ et $F_2$ étant telles que définies ci-dessous :

- $F_0$ correspond à un groupe $-\chi_1H$, dans lequel $\chi_1$ représente un atome d'oxygène ou un groupe $-NR_f$, $R_f$ correspondant à un groupe alkyle, linéaire ou ramifié, comprenant de 1 à 20 atomes de carbone, ou un groupe aryle comprenant de 6 à 30 atomes de carbone,
- $F_1$ répond à la formule suivante :

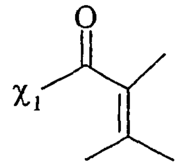

$\chi_1$ étant tel que défini ci-dessus,

- $F_2$ répond à la formule suivante :

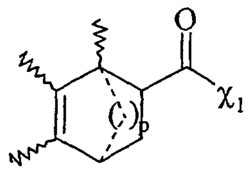

$\chi_1$ étant tel que défini ci-dessus, G répondant à la formule suivante :

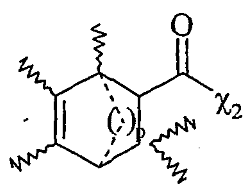

dans laquelle $\chi_2$ représente soit un groupe $OR_g$, $R_g$ représentant un atome d'hydrogène ou un groupe alkyle comprenant de 1 à 20 atomes de carbone, soit un groupe $-NR_hR_u$, $R_h$ et $R_u$ représentant indépendamment l'un de l'autre un atome d'hydrogène, un groupe alkyle comprenant de 1 à 20 atomes de carbone ou un groupe aryle comprenant de 6 à 30 atomes de carbone,

b) 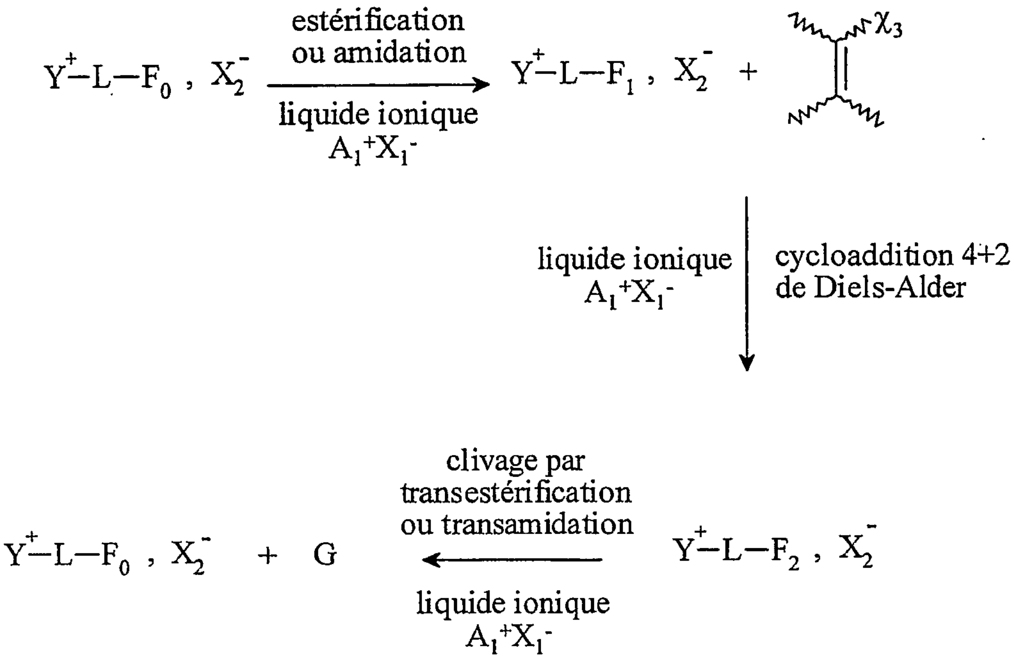


$Y^+$-, L, $X_2$- et le liquide ionique $A_1{}^+X_1$- étant tels que définis précédemment,
les fonctions $F_0$, $F_1$ et $F_2$ étant telles que définies ci-dessous :

- $F_0$ représente toute fonction permettant d'agrafer un diène-1,3, et est notamment choisi parmi les fonctions carbonyles, amines, alcoxy, silanes, stannanes et boranes, comprenant de 1 à 20 atomes de carbone,

- $F_1$ répond à la formule suivante :

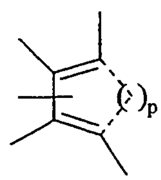

p étant un nombre entier variant de 0 à 2,

- $F_2$ répond à la formule suivante :

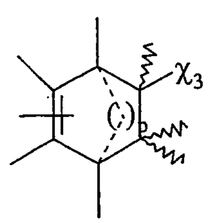

$\chi_3$ représentant un groupement électroattracteur, notamment choisi parmi les groupes cyano, alkoxycarbonyle, comprenant de 1 à 20 atomes de carbone, acyle comprenant de 2 à 20 atomes de carbone, benzoyle, sulfonyle, dialkoxyphosphonyle comprenant de 1 à 10 atomes de carbone,
G répondant à la formule suivante :

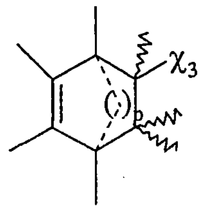

$\chi_3$ étant tel que défini ci-dessus.

La présente invention concerne également l'utilisation telle que définie ci-dessus, pour la mise en oeuvre de la réaction de Heck, selon le schéma réactionnel suivant :

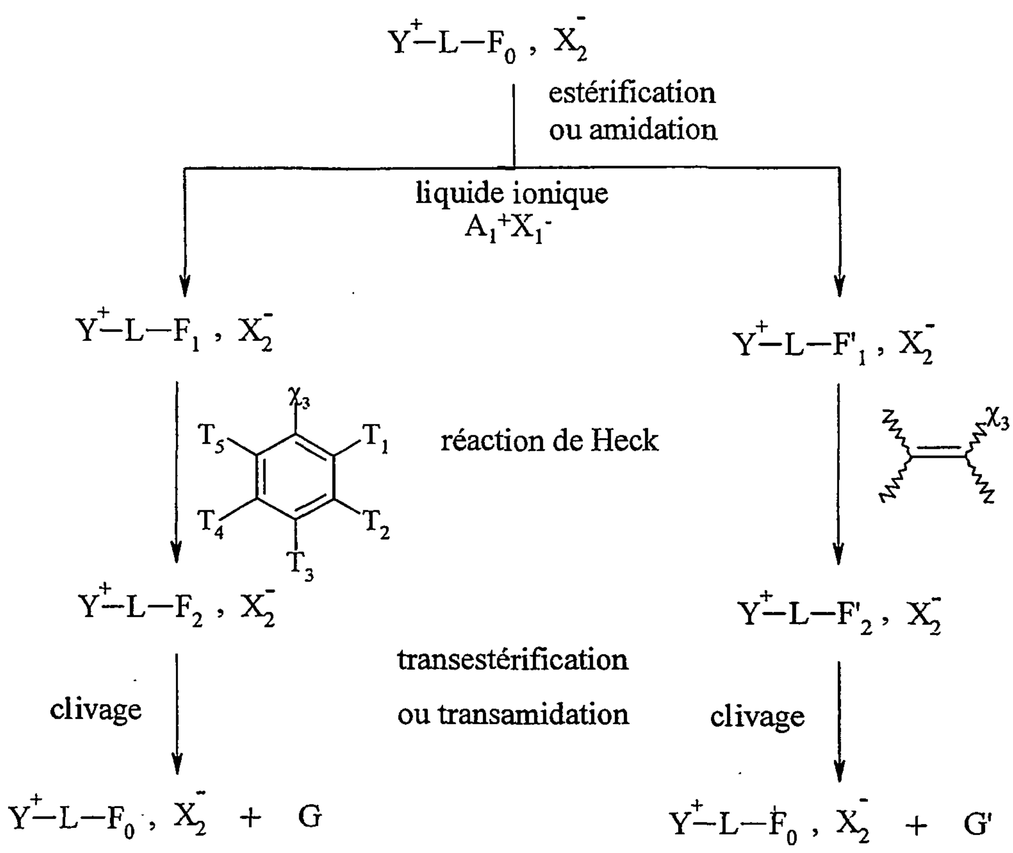


$Y^+$- représentant un cation onium tel que défini ci-dessus, et étant de préférence un cation triméthylalkylammonium, triéthylalkylammonium ou tributylalkylphosphonium,

L représentant un bras, notamment un groupe alkyle linéaire ou ramifié comprenant de 1 à 20 atomes de carbone, ou un groupe aralkyle éventuellement fonctionnel comprenant de 1 à 20 atomes de carbone, et étant de préférence un groupe alkyle linéaire de préférence un groupe alkyle linéaire de type $(CH_2)_r$, r variant de 1 à 20, et de préférence de 3 à 6,

$X_2^-$ étant tel que défini ci-dessus, et étant notamment $BF_4^-$, $PF_6^-$, $NTf_2^-$, $CF_3SO_3^-$, $Cl^-$, $Br^-$, ou $I^-$,

le liquide ionique $A_1^+X_1^-$ étant notamment de la forme :

$(Ra)_3N^+\text{-}R_b, X_1^-$

$(Ra)_3P^+\text{-}R_b,X_1^-$

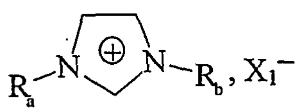 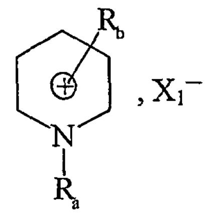

$R_a$ et $R_b$ étant tels que définis ci-dessus, et représentant de préférence des groupes alkyle comprenant de 1 à 20 atomes de carbone,
$X_1^-$ étant choisi parmi : $BF_4^-$, $PF_6^-$, $NTf_2^-$, $Cl^-$, $Br^-$, $CH_3COO^-$, $CF_3CO_2^-$, $CF_3SO_3^-$, $BR_4^-$, R étant tel que défini ci-dessus, les fonctions $F_0$, $F_1$, $F'_1$, $F_2$ et $F'_2$ étant telles que définies ci-dessous :

- $F_0$ correspond à un groupe $-\chi_1H$, dans lequel $\chi_1$ représente un atome d'oxygène ou un groupe $-NR_f$, $R_f$ correspondant à un groupe alkyle, linéaire ou ramifié, comprenant de 1 à 20 atomes de carbone, ou un groupe aryle comprenant de 6 à 30 atomes de carbone,

- $F_1$ répond à la formule suivante :

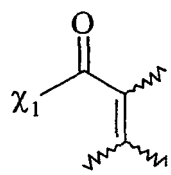

  $\chi_1$ étant tel que défini ci-dessus,

- $F_2$ répond à la formule suivante :

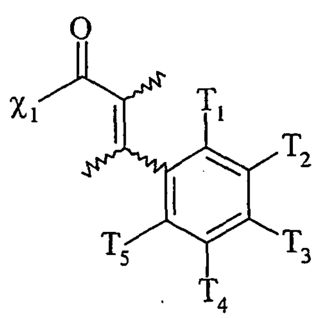

  $\chi_1$ étant tel que défini ci-dessus,
  G répondant à la formule suivante :

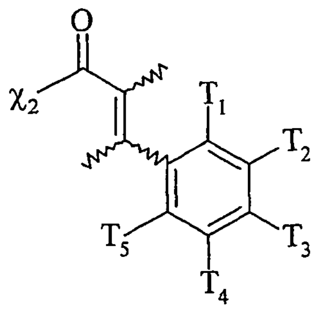

dans laquelle $\chi_2$ représente soit un groupe $-OR_g$, $R_g$ représentant un atome d'hydrogène ou un groupe alkyle comprenant de 1 à 20 atomes de carbone, soit un groupe $-NR_hR_u$, $R_h$ et $R_u$ représentant indépendamment l'un de l'autre un atome d'hydrogène, un groupe alkyle comprenant de 1 à 20 atomes de carbone ou un groupe aryle comprenant de 6 à 30 atomes de carbone,

$\chi_3$ représentant un groupement partant, notamment choisi parmi les halogénures I, Cl et Br, les groupes mésylate, tosylate, triflate, sulfonate, sulfate ou phosphate,

$T_1$, $T_2$, $T_3$, $T_4$ et $T_5$ représentant indépendamment les uns des autres un atome d'hydrogène, un groupe alkyle, linéaire ou ramifié, comprenant de 1 à 20 atomes de carbone ou un groupe aryle comprenant de 6 à 30 atomes de carbone, ou un groupe fonctionnel notamment choisi parmi $NO_2$, CN, COOR, OR, COR, NHCOR, NRR", $SO_2R$, I, Br, R et R" représentant indépendamment l'un de l'autre un groupe alkyle comprenant de 1 à 20 atomes de carbone ou un groupe aryle comprenant de 6 à 30 atomes de carbone,

l'entité

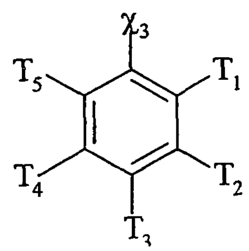

représentant notamment les groupes suivants:

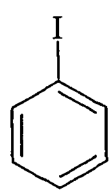
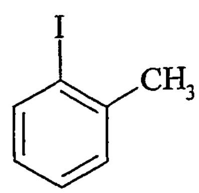
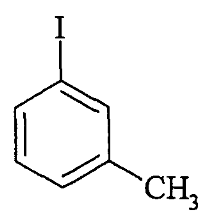
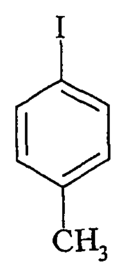
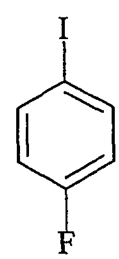

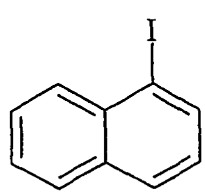
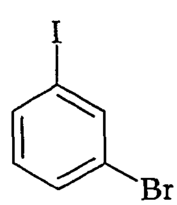
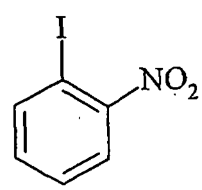
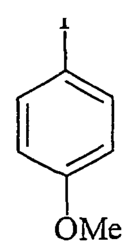

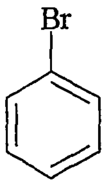 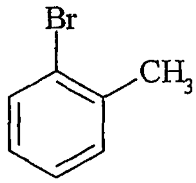 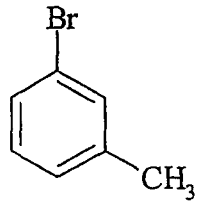 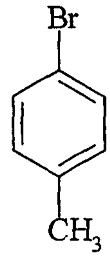 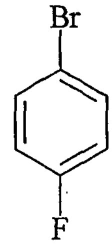

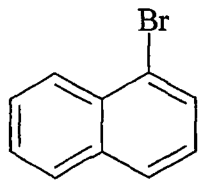 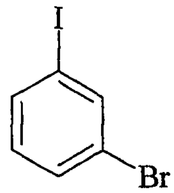 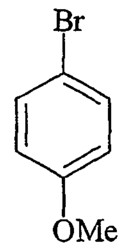

- $F'_1$ répond à la formule suivante :

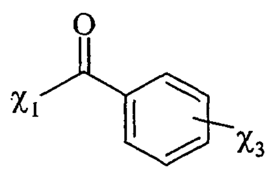

$\chi_1$ et $\chi_3$ étant tels que définis ci-dessus,

- $F'_2$ répond à la formule suivante :

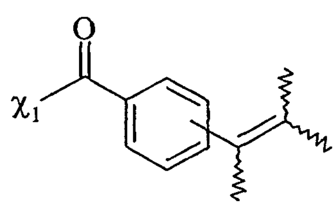

$\chi_1$ étant tel que défini ci-dessus,
G' répondant à la formule suivante :

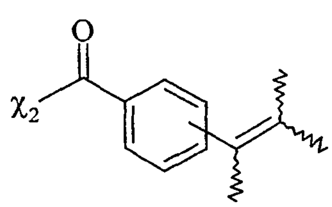

$\chi_2$ étant tel que défini ci-dessus.

La présente invention concerne l'utilisation telle que définie ci-dessus, pour la mise en oeuvre de la réaction

de Baylis-Hilman, selon le schéma réactionnel suivant :

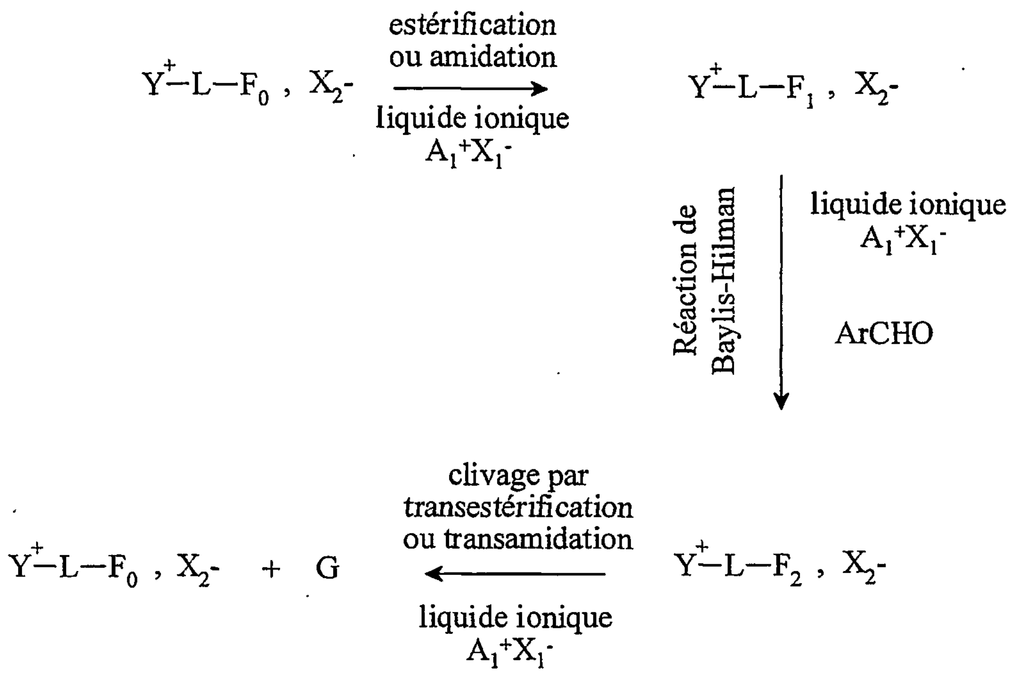


$Y^+$- représentant un cation onium tel que défini ci- dessus, et étant de préférence un cation triméthylalkylammonium, triéthylalkylammonium ou tributylalkylphosphonium,

L représentant un bras, notamment un groupe alkyle linéaire ou ramifié comprenant de 1 à 20 atomes de carbone, ou un groupe aralkyle éventuellement fonctionnel, comprenant de 6 à 30 atomes de carbone, et étant de préférence un groupe alkyle linéaire de préférence un groupe alkyle linéaire de type $(CH_2)_r$, r variant de 1 à 20, et de préférence de 3 à 6,

$X_2$- étant tel que défini ci-dessus, et étant notamment $BF_4^-$ , $PF_6^-$, $NTf_2^-$ , $CF_3SO_3^-$, $Cl^-$, $Br^-$, $I^-$, $CH_3CO_2^-$ ou $CF_3CO_2^-$, le liquide ionique $A_1^+X_1^-$ étant notamment de la forme :

$$(Ra)_3\overset{+}{N}-R_b\,,\, X_1^- \qquad (Ra)_3\overset{+}{P}-R_b\,,\, X_1^-$$

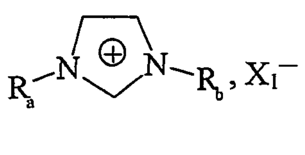

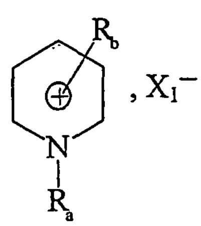

$R_a$ et $R_b$ étant tels que définis ci-dessus, et représentant de préférence des groupes alkyle comprenant de 1 à 20 atomes de carbone,

$X_1^-$ étant choisi parmi : $BF_4^-$; $PF_6^-$, $NTf_2^-$, $Cl^-$, $Br^-$, $CH_3COO^-$, $CF_3CO_2^-$, $CF_3SO_3^-$, $BR_4^-$, R étant tel que défini ci-dessus,

les fonctions $F_0$, $F_1$ et $F_2$ étant telles que définies ci-dessous :

- $F_0$ représente un groupe -OH,
- $F_1$ répond à la formule suivante :

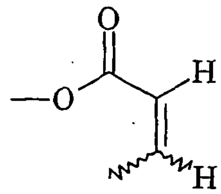

- $F_2$ répond à la formule suivante :

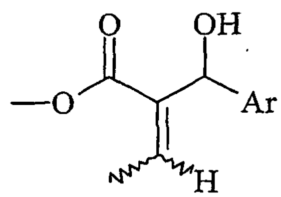

G répondant à la formule suivante :

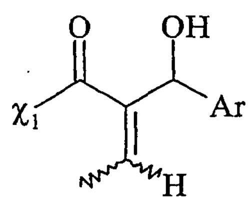

$\chi_1$ représentant un groupe -OH, ou un groupe -$OR_g$, $R_g$ représentant un groupe alkyle, linéaire ou ramifié, comprenant de 1 à 20 atomes de carbone,
Ar représentant un groupe aromatique ou hétéroaromatique, substitué ou non, ArCHO étant notamment choisi parmi :

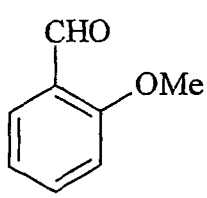 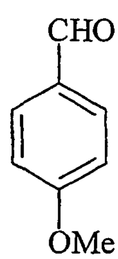 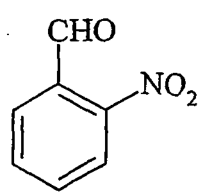 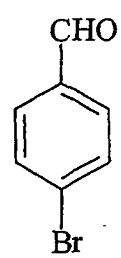

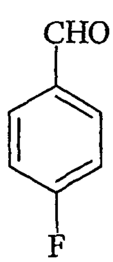 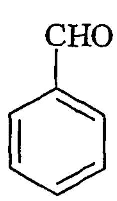 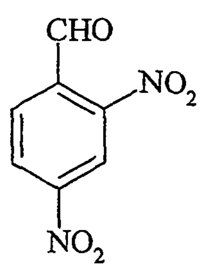 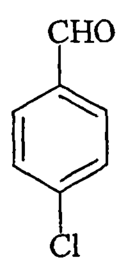

La présente invention concerne l'utilisation telle que définie ci-dessus, pour la misé en oeuvre du couplage de Suzuki, selon l'un des schémas réactionnels suivants :

a)

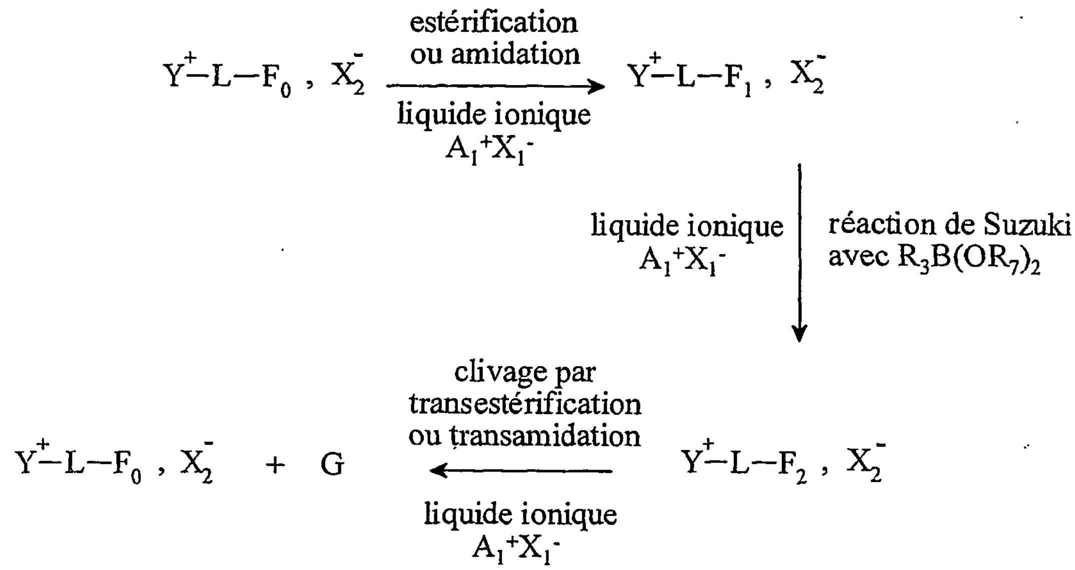


$R_3$ étant choisi parmi les groupes aryle, hétéroaryle, éthényle, diényle, allyle, éthynyle, substitués ou non, comprenant de 2 à 30 atomes de carbone,

$R_7$ représentent un groupe alkyle, ramifié ou linéaire ou un groupe cycloalkyle comprenant de 1 à 12 atomes de carbone,

$Y^+$- représentant un cation onium tel que défini ci-dessus, et étant de préférence un cation triméthylalkylammonium, triéthylalkylammonium ou tributylalkylphosphonium,

L représentant un bras, notamment un groupe alkyle linéaire ou ramifié comprenant de 1 à 20 atomes de carbone; ou un groupe aralkyle éventuellement fonctionnel comprenant de 6 à 30 atomes de carbone, et étant de préférence un groupe alkyle linéaire de préférence un groupe alkyle linéaire de type $(CH_2)_r$, r variant de 2 à 20, et de préférence de 3 à 6,

$X_2^-$ étant tel que défini ci-dessus, et étant notamment $NTf_2^-$, $BF_4^-$, $PF_6^-$, $Cl^-$, $Br^-$, $CH_3COO^-$, $CF_3CO_2^-$, $CF_3SO_3^-$, $BR_4^-$, R étant tel que défini ci-dessus,

le liquide ionique $A_1^+X_1^-$ étant notamment de la forme :

$(Ra)_3\overset{+}{N}-R_b , X_1^-$ $(Ra)_3\overset{+}{P}-R_b , X_1^-$

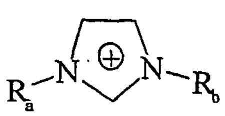 , $X_1^-$

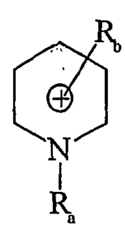 , $X_1^-$

$R_a$ et $R_b$ étant tels que définis ci-dessus, et représentant de préférence des groupes alkyle comprenant de 1 à 20 atomes de carbone,

$X_1^-$ étant choisi parmi: $BF_4^-$, $PF_6^-$ , $NTf_2^-$ , $Cl^-$ , $Br^-$, $CH_3COO^-$, $CF_3CO_2^-$, $CF_3SO3^-$, $BR_4^-$, R étant tel que défini ci-dessus,

les fonctions $F_0$, $F_1$ et $F_2$ étant telles que définies ci-dessous :

- $F_0$ est de la forme $-\chi_1H$, $\chi_1$ représentant un atome d'oxygène ou un groupe $-NR_f$, $R_f$ correspondant à un groupe alkyle, linéaire ou ramifié, comprenant de 1 à 20 atomes de carbone, ou un groupe aryle comprenant de 6 à 30 atomes de carbone,
- $F_1$ est de la forme $-R_e-\chi$, $R_e$ représentant un groupe aromatique ou hétéroaromatique comprenant de 6 à 30 atomes de carbone, $\chi$ représentant un groupe partant choisi de préférence parmi Cl, Br, I, OTf, $O-CO_2R^5$ ou $OSO_3-R^5$, $R^5$ représentant un groupe alkyle comprenant de 1 à 10 atomes de carbone ou un groupe aralkyle comprenant de 6 à 30 atomes de carbone, $F_1$ répondant de préférence à la formule suivante :

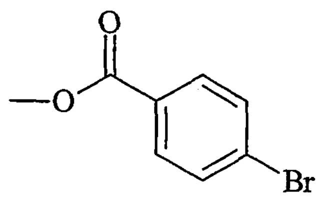

- $F_2$ est de la forme $-R_e-R_2$, $R_e$ étant tel que défini ci-dessus et $R_2$ étant choisi parmi les groupes aryle, hétéroaryle, éthényle, diényle, allyle, éthynyle, substitués ou non, comprenant de 2 à 30 atomes de carbone, $F_2$ répondant de préférence à la formule suivante :

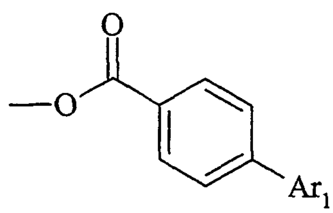

$Ar_1$ représentant un groupe aromatique choisi de préférence parmi :

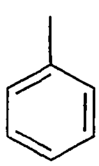 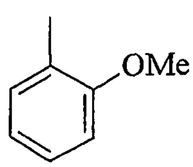 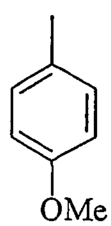 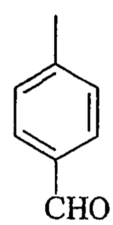 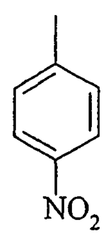

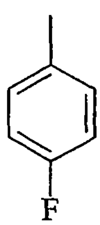 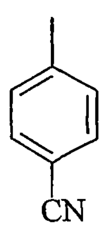 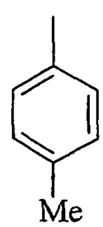 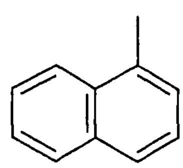

la molécule G étant de la forme $R_2$ $R_3$, $R_2$ et $R_3$ étant tels que définis ci-dessus, et répond notamment à la formule suivante :

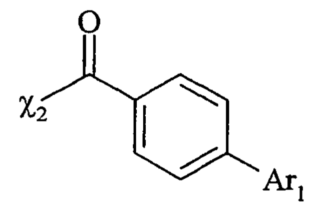

dans laquelle $\chi_2$ représente soit un groupe -ORg, Rg représentant un atome d'hydrogène ou un groupe alkyle comprenant de 1 à 20 atomes de carbone, soit un groupe $-NR_hR_u$, $R_h$ et $R_u$ représentant indépendamment l'un de l'autre un atome d'hydrogène, un groupe alkyle comprenant de 1 à 20 atomes de carbone ou un groupe aryle comprenant de 6 à 30 atomes de carbone,
$Ar_1$ est tel que défini ci-dessus,

b)

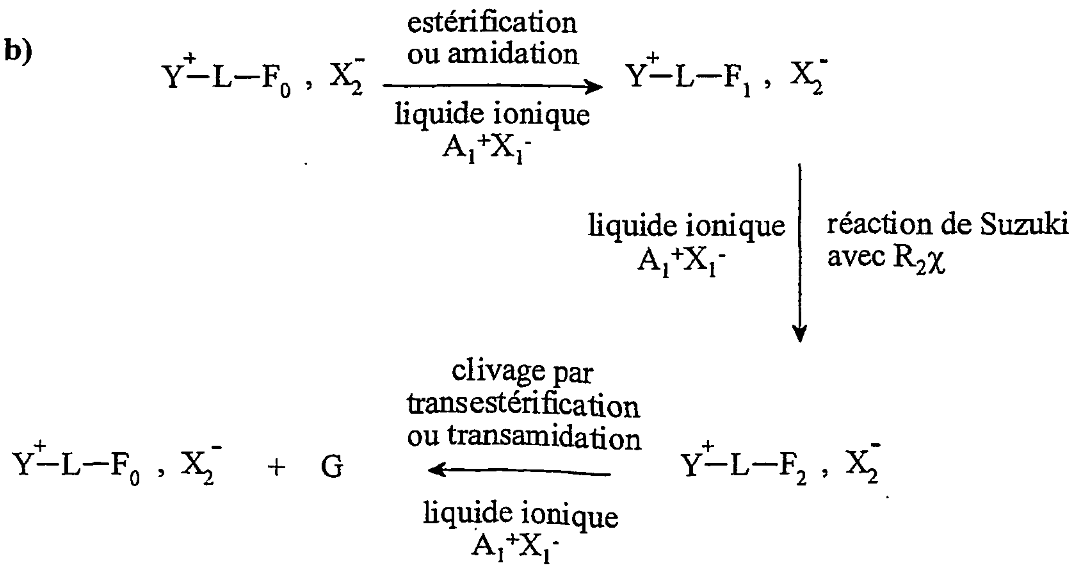


$Y^+$-, L, $X_2^-$, $A_1^+X_1^-$ et $R_2$ étant tels que définis ci-dessus,
les fonctions $F_0$, $F_1$ et $F_2$ étant telles que définies ci-dessous :

- $F_0$ est de la forme $-\chi_1H$, $\chi_1$ étant tel que défini ci-dessus,
- $F_1$ est de la forme $-R_q-B(OR_7)_2$, $R_7$ étant tel que défini ci-dessus, et $R_q$ correspondant à un groupe aryle comprenant de 6 à 30 atomes de carbone, hétéroaryle comprenant de 4 à 20 atomes de carbone, éthényle comprenant de 2 à 20 atomes de carbone, diényle comprenant de 3 à 20 atomes de carbone, allyle comprenant de 3 à 20 atomes de carbone, éthynyle comprenant de 2 à 20 atomes de carbone, substitués ou non, $F_1$ répondant de préférence à la formule suivante :

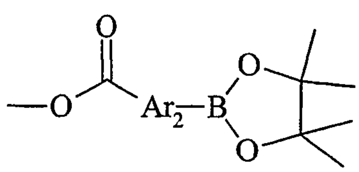

$Ar_2$ correspondant à un groupe aryle substitué ou non comprenant de 6 à 30 atomes de carbone,

- $F_2$ est de la forme $-R_q-R_e$, $R_q$ et $R_e$ étant tels que définis ci-dessus, $F_2$ répondant de préférence à la formule suivante :

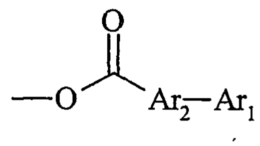

$Ar_1$ représentant un groupe aromatique choisi de préférence parmi :

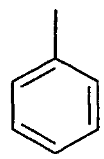 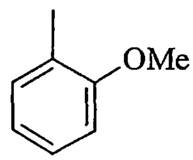 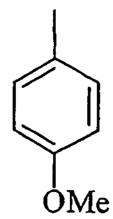 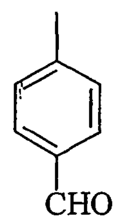 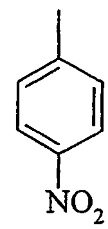

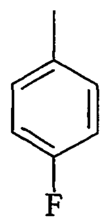 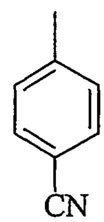 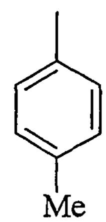 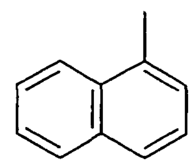

la molécule G étant de la forme $R_2-R_3$, $R_2$ et $R_3$ étant tels que définis ci-dessus, et répondant notamment à la formule suivante :

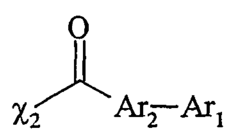

dans laquelle $\chi_2$, $Ar_1$ et $Ar_2$ sont tels que définis ci-dessus,

c)

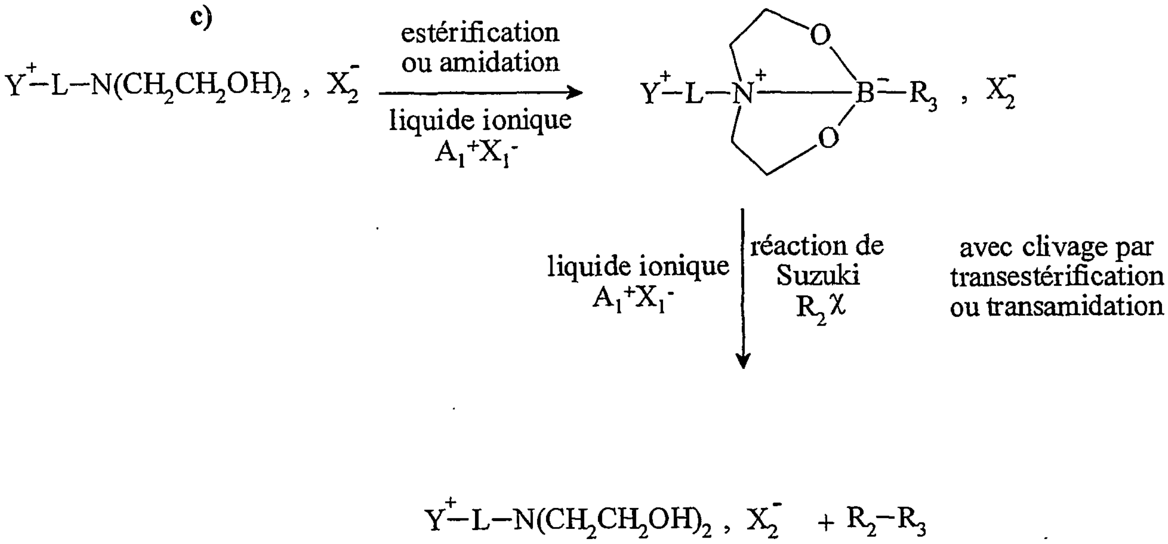


$Y^{+}$-, L, $X_2^{-}$, $A_1^{+}X_1^{-}$, $R_2$ et $R_3$ étant tels que définis ci-dessus,
$R_3$ étant de préférence un groupe phényle,

d)

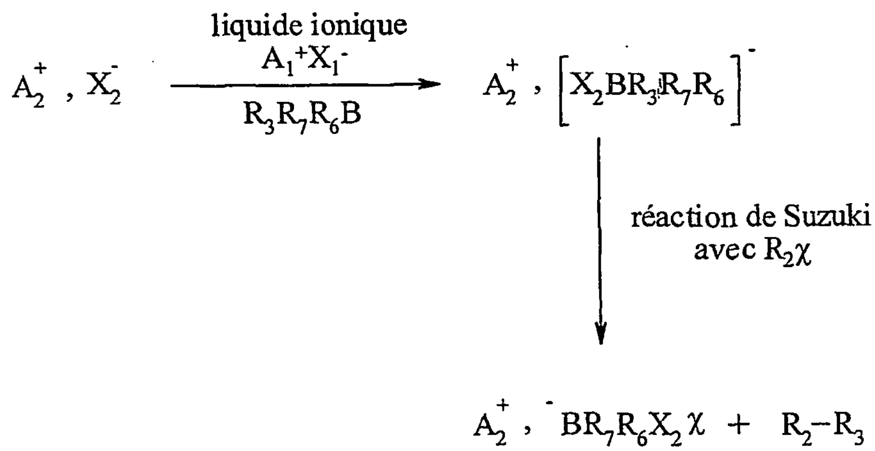


$A_2^{+}$ étant un cation ammonium $(R_a)_3N^{+}R_b$ ou phosphonium $(R_a)_3P^{+}R_b$, de préférence tétrabutylammonium et tétraméthylammonium, $R_a$ et $R_b$ étant tels que définis ci-dessus,
$X_2^{-}$ étant notamment choisi parmi $OH^{-}$, $F^{-}$, $CN^{-}$, $R_sO^{-}$, $R_sS^{-}$, de préférence $OH^{-}$ ou $F^{-}$, $R_s$ représentant un groupe alkyle comprenant de 1 à 20 atomes de carbone ou un groupe aryle comprenant de 6 à 30 atomes de carbone,
$R_3$ et $R_4$ étant tels que définis ci-dessus,
$R_6$ et $R_7$ représentant indépendamment l'un de l'autre un groupe alkyle comprenant de 1 à 20 atomes de carbone ou un groupe aryle comprenant de 6 à 30 atomes de carbone,
la molécule borée de formule $R_3R_7R_6B$ étant un trialkyle ou aryle borane, le groupe alkyle comprenant de 1 à 20 atomes de carbone et le groupe aryle comprenant de 6 à 30 atomes de carbone, un acide ou ester borinique, de préférence un acide ou ester boronique choisi comme étant l'acide phényl boronique,
$R_2$ et $\chi$ sont tels que définis précédemment, $R_2\chi$ correspondant de préférence à un halogénure d'aryle choisi parmi :

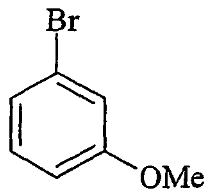

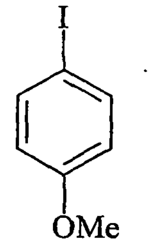

La présente invention concerne également l'utilisation telle que définie ci-dessus, pour la synthèse de banques de molécules selon la technique de synthèse parallèle, selon le schéma suivant :

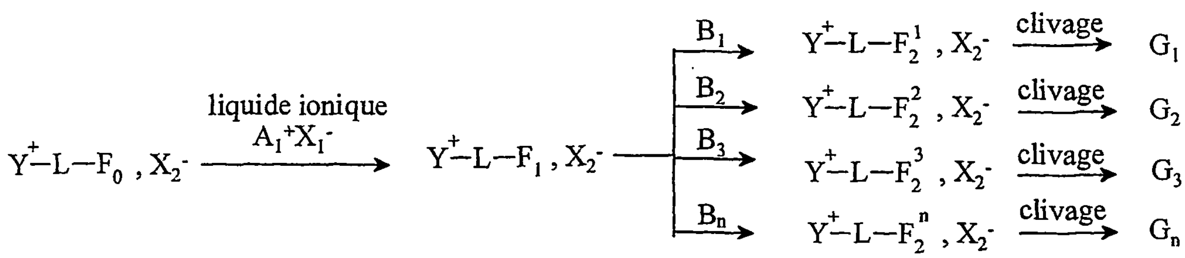


caractérisée en ce que le sel fonctionnalisé $Y^+$-L-$F_1$, $X_2$- dans le liquide ionique $A_1^+$, $X_1^-$ est séparé en n parties sensiblement égales, n variant de 2 à 1024, et en ce que chacune de ces parties est ensuite transformée selon une réaction de synthèse organique, de préférence une réaction de couplage de Heck ou de Suzuki, à l'aide chacune d'un réactif différent $B_i$ pour donner n solutions contenant chacune un composé défini $Y^+$-L-$F_2^i$, $X_2^-$, $F_2^i$ représentant une fonction choisie parmi les fonctions telles que définies ci-dessus, i variant de 1 à n, chaque solution étant traitée pour libérer les molécules $G_i$, i variant de 1 à n, qui sont chacune isolées et purifiées, constituant une banque de molécules.

La technique de synthèse parallèle consiste à préparer en parallèle et simultanément des banques de produits uniques parfaitement identifiés à raison d'un produit par réacteur ou par puits, après une séquence de réactions effectuées avec des réactifs propres à chaque produit préparé.

L'expression "sensiblement égales" désigne une partition en volumes égaux, aux erreurs d'expérience près.

L'expression "banque de molécules" désigne un ensemble de produits tous identifiés, non mélangés, chacun d'entre eux étant disposé dans un contenant qui lui est propre. Ce type de banque de molécules résulte de la synthèse parallèle. Cette expression peut également désigner un mélange de produits identifiés par les techniques d'analyse à la disposition des chimistes et résultant de la réaction d'un mélange de réactifs avec un seul produit ou d'un mélange de produits avec un seul réactif selon la technique séparation/mélange.

La présente invention concerne également l'utilisation telle que définie ci-dessus, pour la mise en oeuvre de la synthèse de banques de molécules par la technique de séparation-mélange selon le schéma suivant :

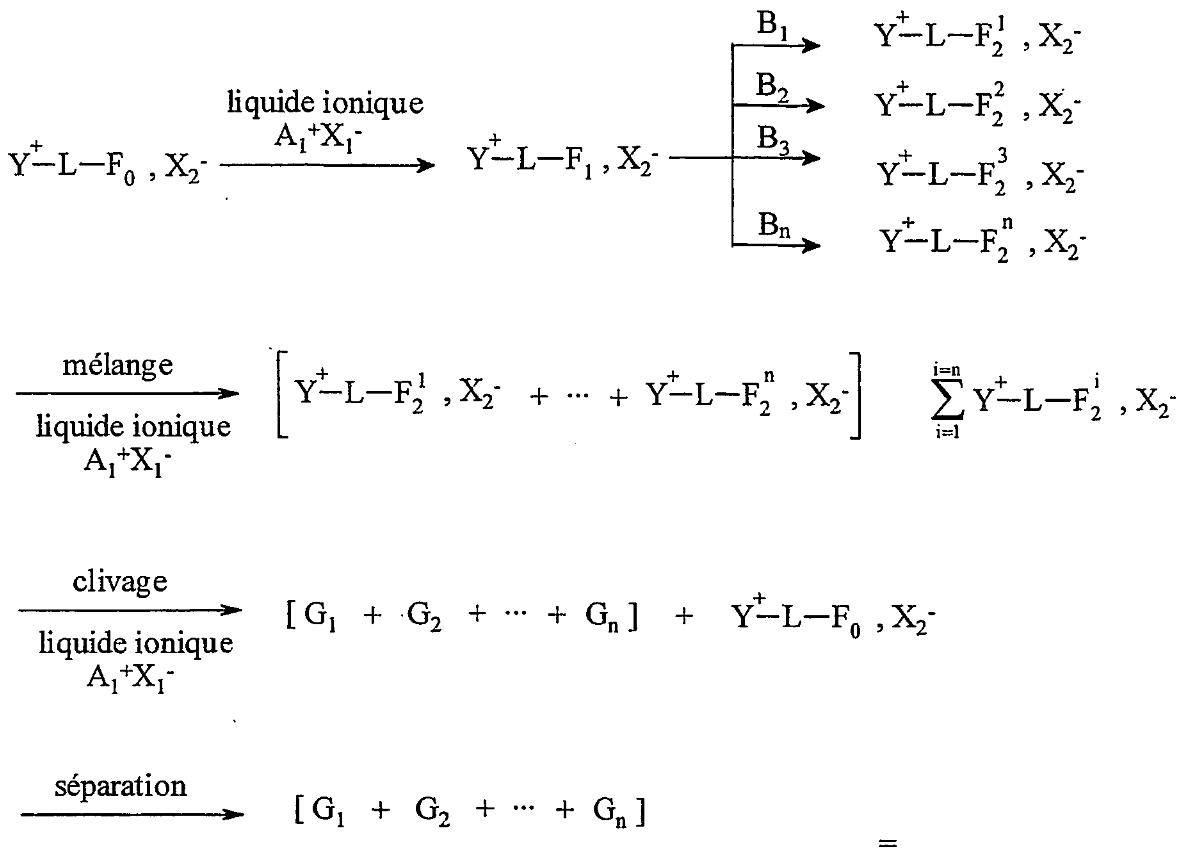


caractérisée en ce que :

- n fractions de la solution $Y^+$-L-$F_1$, $X_2^-$, obtenue à partir du sel fonctionnalisé de départ $Y^+$-L-$F_0$, $X_2^-$, dans le liquide ionique $A_1^+X_1^-$ sont transformées en parallèle selon une réaction de la chimie organique, de préférence une réaction de couplage de Heck ou de Suzuki, chacune à l'aide d'un réactif différent $B_i$ pour donner n solutions contenant chacune un composé défini $Y^+$-L-$F_2^i$, $X_2^-$, i variant de 1 à n, n variant de 2 à 1024, de préférence de 2 à 96, $F_2^i$ représentant une fonction choisie parmi les fonctions telles que définies ci-dessus,
- les n solutions obtenues à l'étape précédente sont mélangées pour donner une solution dans le liquide ionique $A_1^+X_1^-$ contenant les n produits $Y^+$-L-$F_2^i$, $X_2^-$, i variant de 1 à n, notée $\sum_{i=1}^{i=n} Y^+\text{—L—}F_2^i\ ,X_2^-$ , et cette solution est soumise à une étape de clivage, de préférence une transestérification ou une transamidation, afin d'obtenir en solution dans le liquide ionique $A_1^+X_1^-$, un mélange des n molécules $G_i$, i variant de 1 à n, et du sel fonctionnalisé de départ $Y^+$-L-$F_0$, $X_2^-$,
- le mélange tel qu'obtenu à l'étape précédente est séparé du liquide ionique $A_1^+X_1^-$ et du sel fonctionnalisé de départ $Y^+$-L-$F_0$, $X_2^-$, par les méthodes usuelles de séparation, de préférence par distillation sous vide, par extraction par un solvant classique tel que l'heptane ou le toluène suivie d'une évaporation de solvant, par chromatographie sur colonne, plaques ou sous pression, afin d'obtenir une banque contenant n molécules $G_i$,

cet enchaînement d'étapes mentionné ci-dessus pouvant être répété j fois, j étant compris de 2 à 10, afin d'obtenir j banques différentes de n produits.

La technique de séparation-mélange (ou "split and mix")(O'Brecht et al., 1998) consiste à faire réagir n fractions d'une solution d'un produit, chacune avec un réactif différent conduisant à n nouveaux produits qui sont mélangés après identification. Ce nouveau mélange est séparé en m fractions qui sont alors mises à réagir en parallèle chacune avec un réactif différent conduisant à m mélanges de n nouveaux produits, soit m x n produits. Ces opérations sont répétées autant de fois que nécessaire.

Selon un mode de réalisation avantageux, la solution dans le liquide ionique $A_1^+X_1^-$ contenant les n produits

$Y^+\text{-}L\text{-}F_2^i$, $X_2^-$, i variant de 1 à n, telle qu'obtenue après l'étape de mélange, et notée $\sum_{i=1}^{i=n} Y^+\!-\!L\!-\!F_2^i\ ,\ X_2^-$ est séparée en m parties, m varient de 2 à 1024. Chacune desdites parties est ensuite traitée respectivement par un réactif $B'_j$, j variant de 2 à m, selon le schéma suivant :

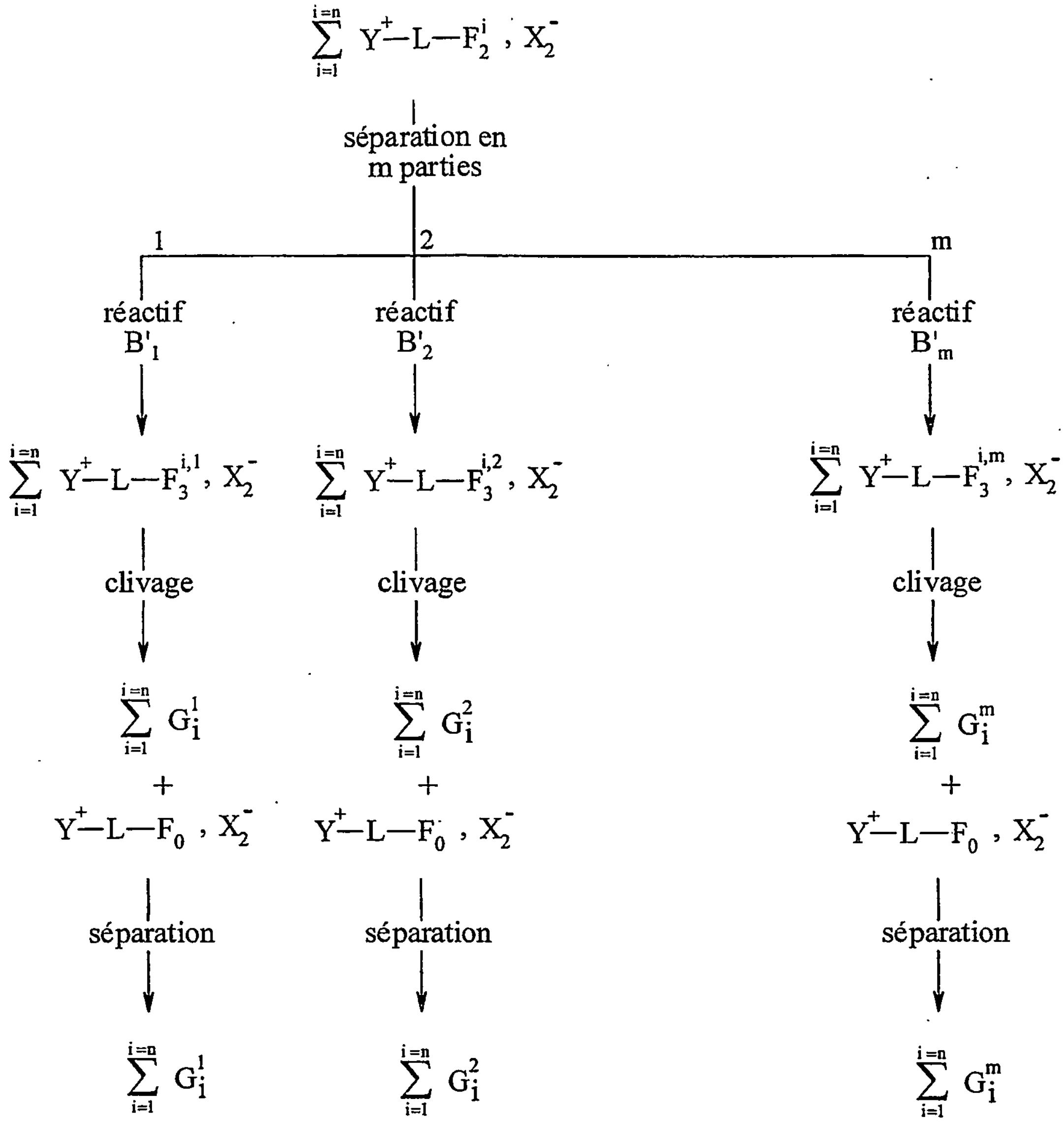


Ainsi, on obtient m nouvelles banques $G^j_i$ de n nouveaux produits.

**DESCRIPTION DES FIGURES**

La Figure 1 représente des spectres de RMN du proton enregistrés à 200 MHz dans l'acétone D6, correspondant au suivi de la réaction de couplage de Heck entre l'acrylate supporté <u>**6**</u> et le 1-iodonaphtalène.

La Figure 2 représente un chromatogramme correspondant au mélange des neuf esters méthyliques <u>**13a**</u> à <u>**13i**</u> dont les spectres de masse sont décrits dans le tableau II.

La Figure 3 représente un chromatogramme correspondant au mélange des neuf esters éthyliques **14a** à **14i** dont les spectres de masse sont décrits dans le tableau III.

La Figure 4 représente un chromatogramme correspondant au mélange des neuf esters propyliques **15a** à **15i** dont les spectres de masse sont décrits dans le tableau IV.

La Figure 5 représente un chromatogramme correspondant au mélange des neuf esters butyliques **16a** à **16i** dont les spectres de masse sont décrits dans le tableau V.

La Figure 6 représente des spectres de RMN du proton enregistrés à 200 MHz dans l'acétone D6, correspondant au suivi de la réaction de couplage de Heck entre l'iodure d'aryle supporté **7** et l'acrylate de tertiobutyle.
Le spectre supérieur correspond au spectre de 7b en solution 0,85 M dans $Me_3N$-$(CH_2)_2Me$,$NTf_2^-$. Le spectre inférieur est celui du mélange réactionnel une fois le couplage de Heck terminé.

La Figure 7 représente un chromatogramme correspondant au mélange des esters propyliques biaryliques **23a** à **23i** dont les spectres de masse sont décrits dans le tableau XI.

La figure 8 représente un chromatogramme correspondant au mélange des esters méthyliques biaryliques du tableau XII.

La figure 9 représente un chromatogramme correspondant au mélange des esters éthyliques biaryliques du tableau XIII.

La figure 10 représente des spectres de RMN du proton enregistrés à 200 MHz dans l'acétone D6, correspondant au suivi de la réaction de Grieco avec l'aniline supportée **1** et de 4-nitrobenzaldéhyde.

La figure 11 représente des spectres de RMN du proton enregistrés à 200 MHz dans l'acétone D6, correspondant au suivi du couplage de Sonogashira avec R = phényle.

La figure 12 représente un chromatogramme correspondant au mélange des esters méthyliques dont les spectres de masse sont décrits dans le tableau XX.

La figure 13 représente un chromatogramme correspondant au mélange des esters éthyliques dont les spectres de masse sont décrits dans le tableau XXI.

## PARTIE EXPÉRIMENTALE - PRÉPARATION DES COMPOSÉS

### I) SYNTHÈSE DES SELS FONCTIONNALISÉS :

#### 1/ Alcools 1 :

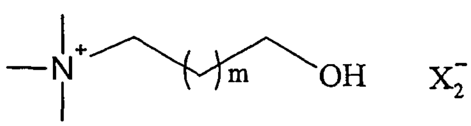

(1)

**1a** m = 1 ; $X_2$ = Cl
**1b** m = 3 ; $X_2$ = Cl
**1c** m = 1 ; $X_2$ = $PF_6$
**1d** m = 1 ; $X_2$ = $NTf_2$

(suite)

| | |
|---|---|
| 1e | m = 3 ; $X_2$ = $NTf_2$ |
| 1f | m = 1 ; $X_2$ = $BF_4$ |

* **1a:**

Dans un ballon de 250 ml on met 25 g (0,1 mol) de 3-chloropropanol, 30 ml d'une solution de triméthyle amine à 45% dans l'eau (0,2 mol) et 100 ml d'acétonitrile pour homogénéiser le milieu. Le mélange est ensuite porté à reflux pendant 36 heures. Le mélange eau/acétonitrile est évaporé sous vide et le solide blanc obtenu est lavé par 2 fois 30 ml d'éther.

Solide blanc **Rdt** = 82% **Pf =** 158-160°C

***RMN $^1$H (200 MHz, $D_2O$) :*** 1,80 - 2,05 (m, 2H) ; 3,00 (s, 9H) ; 3,20 - 3,41 (m, 2H) ; 3,60 (t, 2H, J = 7,1 Hz)
***RMN $^{13}$C (50 MHz, $D_2O$)*** *:* 25,68 ; 53,31 (t, $J_{C-N}$ = 4,1 Hz) ; 58,52 ; 64,52.

* **1b :**

Dans un ballon de 250 ml on met 5g (36 mmol) de 6-chlorohexanol, 10 ml d'une solution de triméthyle amine à 45% dans l'eau (0,1 mol) et 100 ml d'acétonitrile pour homogénéiser le milieu. Le mélange est ensuite porté à reflux pendant 36 heures. Le mélange eau/acétonitrile est évaporé sous vide et le solide blanc obtenu est lavé par 2 fois 30 ml d'éther.

Solide blanc **Rdt** = 62% **Pf** =178-180°C

***RMN$^1$H (200 MHz, MeOH) :*** 1,30 - 1,65 (m, 6H) ; 1,80 - 1,95 (m, 2H) ; 3,18 (s, 9H) ; 3,4 - 3,6 (m, 2H) ; 3,55 (t, 2H, J = 6,1 Hz).
***RMN $^{13}$C (50 MHz, MeOH) :*** 22,93 ; 25,48 ; 26,15 ; 32,35 ; 52,60 (t ; J=4,1 Hz) ; 61,67; 66,76.

* **1c:**

Un mélange d'une solution de 10 g (65,3 mol) de chlorure de N,N',N"-triméthyle-3-hydroxypropylammonium dans 15ml d'eau et 13,23 ml (0,15 mol) de l'acide hexafluorophosphorique en solution 60% dans l'eau est agité à température ambiante pendant 2 heures. Le milieu devient hétérogène immédiatement et le précipité formé est filtré et lavé à l'éther. Le solide blanc obtenu est séché sous vide.

Solide blanc **Rdt** =67% **Pf** =124-126°C

***RMN $^1$H (200 MHz, $CD_3CN$) :*** 1,70 (m, 2H) ; 2,82 (s, 9H) ; 3,15(m, 2H) ; 3,40 (t, 2H, J = 6,1 Hz).
***RMN $^{13}$C (50 MHz, $CD_3CN$)*** *:* 25,44, ; 52,59 (t, J = 4,2 Hz) ; 57,67 ; 64,26 (t, J = 3,8 Hz).

* **1d:**

Dans un bécher, on prépare une solution de 10g du sel d'ammonium **(1a)**(65,3 mmol) dans 10 mL d'eau. Dans un autre bécher, on dissout de la même manière 20 g de bis-trifluorométhanesulfonamidure de lithium (71,9 mmol). Les deux solutions sont mélangées et agitées pendant 2 heures à température ambiante pour que l'échange soit total. Les deux phases obtenues sont séparées dans une ampoule à décanter, et la phase aqueuse est extraite 2 fois par 15 ml de chlorure de méthylène. Enfin le solvant est évaporé et le produit est séché sous vide.

Huile visqueuse incolore **Rdt** = 86%

***RMN$^1$H (200 MHz, Acétone $D_6$)*** : 2,00 - 2,21 (m, 2H) ; 3,25 (s, 9H) ; 3,50 - 3,80 (m, 4H).
***RMN$^{13}$C (50 MHz, Acétone $D_6$)*** : 29,14 ; 54,27(t ; $J_{C-N}$ = 4,1 Hz) ; 60.05 ; 66.09 ; 121,05 (q, J = 321,2 Hz).

*** 1e:**

Un mélange d'une solution de 10 g (51,2 mmol) de chlorure de N,N',N''-triméthyle-4-hydroxybutylammonium dans 15 ml d'eau et 18,7 g (6,66 mmol) de lithium de trifluorométhane sulfonamidure est agité à température ambiante. Le milieu devient hétérogène immédiatement, et les deux phases sont séparées dans une ampoule à décanter. L'huile incolore obtenue est ensuite lavée deux fois par 3 ml d'eau et séchée à 50°C sous vide poussé.

Huile incolore **Rdt** = 93%.

***RMN $^1$H (200 MHz, Acétone, D6) :*** 1,41 - 1,60 (m, 6H); 1,88 - 2,01 (m, 2H) ; 3,30 (s, 9H) ; 3,50 - 3,65 (m, 4H) ; 3,55 (t, 2H, J = 6,1 Hz).
***RMN $^{13}$C (50 MHz, Acétone, D6) :*** 23,02 ; 25,60 ; 26,22 ; 53,01 (t, J = 4,1 Hz) ; 61,73 ; 66,99 ; 121,05 (q, J = 324,2 Hz).

*** 1f :**

Un mélange d'une solution de 10 g (65 mmol) de chlorure de N,N',N"-triméthyle-3-hydroxypropylammoinum dans 15 ml d'eau et 9,1 ml (0,15 mol) de l'acide tétrafluoroborique à 50% dans l'eau est agité à température ambiante. Le milieu reste homogène. Après 12 heures, l'eau est évaporée à sec, le solide blanc obtenu est lavé par 2 fois 15 ml d'éther anhydre.

Solide blanc **Rdt** = 92% **Pf =** 110-112°C

***RMN $^1$H (200 MHz, Acétone $D_6$):*** 2,10 - 2,241 (m, 2H); 3,05 (s, 9H) ; 3,24 - 3,45 (m, 4H) ; 3,61 (t, J=7,1 Hz, 2H).
***RMN $^{13}$C(50MHz, Acétone $D_6$):*** 27,52 ; 53,35 (t, $J_{C-N}$=4,1 Hz) ; 58,25,05 ??; 64,58.

**2/ Acide carboxylique 2 :**

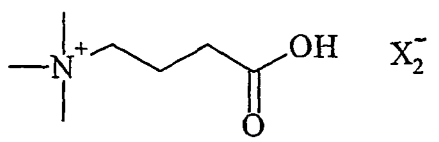

**(2a)**

**2a** $X_2$=Br
**2b** $X_2$=$NTf_2$

*** 2a:**

Un mélange de 30 ml d'une solution aqueuse 6,65 molaire (0,2 mol) et de 14,3 ml (0,1 mol) de bromobutyrate d'éthyle est porté à reflux pendant 24 heures. On évapore ensuite le solvant à sec et on lave le solide blanc obtenu par 3 fois 25 ml d'éther.
Le solide ainsi obtenu est dissous dans 15 ml d'une solution d'acide bromhydrique (6 N), puis porté à reflux pendant 12 heures. On évapore ensuite à sec et le solide obtenu est lavé à l'éther et séché ensuite sous vide.

Solide blanc **Rdt** = 75% **Pf** = 188-190°C

***RMN $^1$H (200 MHz, $CD_3OD$) :*** 1,95 - 2,21 (m, 2H) ; 2,49 (t, 2H, J = 7,0 Hz) ; 3,25 (s, 9H) ; 3,45 - 3,55 (m, 2H).
***RMN $^{13}$C (50 MHz, $CD_3OD$) :*** 18,58 ; 30,04 ; 52,86 (t, J = 4,1 Hz) ; 65,84 (t, J = 4,1 Hz) ; 174,40.

*** 2b:**

On dissout, dans un bécher, 4 g de l'acide de N,N',N''-triméthyl-3-butanoïque (**2a**) (17,7 mmol) dans 10 mL d'eau. Dans un autre bécher, on dissout de la même manière 5,6 g de $LiNTf_2$ (19,5 mmoles) et on mélange les 2 solutions. Le milieu devient trouble et on observe l'apparition de 2 phases. On laisse agiter pendant 2 heures à température ambiante pour que l'échange soit total. Le contenu du bécher est transvasé dans une ampoule à décanter et laissé décanter jusqu'à avoir 2 phases bien séparées. Le liquide ionique (phase inférieure) est extrait et la phase aqueuse est lavée deux fois par 20 ml de chlorure de méthylène. Enfin, le ballon est placée sous vide pour sécher le produit après évaporation du solvant.

Huile visqueuse incolore **Rdt** = 88%

***RMN $^1H$ (200 MHz, Acétone $D_6$) :*** 2,15 - 2,30 (m, 2H) ; 2,5 (t, J = 6,7 Hz, 2H); 3,40 (s, 9H) ; 3,55 - 3,70 (m, 2H)
***RMN $^{13}C$ (50 MHz, Acétone $D_6$) :*** 19,52 ; 23,73 ; 54,09 (t, $J_{C-N}$ = 4,0 Hz) ; 67,02 (t, $J_{C-N}$ = 3,0 Hz) ; 118,4 ($q_{C-F}$, J = 324,0 Hz) ; 172,89.

**3/ Halogénure 3 :**

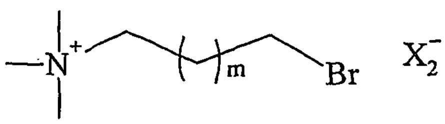

(3)

**3a** m=1;$X_2$=Br
**3b** m=3;$X_2$=Br
**3c** m = 1 ; $X_2$ = $NTf_2$
**3d** m=1;$X_2$=$PF_6$
**3e** m=3;$X_2$=$PF_6$
**3f** m=3;$X_2$=$BF_4$

**1) $X_2$=Br:**

Dans un monocol de 100 mL, surmonté d'un réfrigérant, on introduit 41 ml (0,6 mol) d'une solution aqueuse de triméthylamine à 45%. On agite et le bain d'huile est chauffé à une température de 40°C. A la sortie du réfrigérant, les vapeurs de triméthylamine passent par un piège à pastilles de KOH pour retenir les traces d'eau présentes dans les vapeurs et vont barboter dans un bicol contenant 0,2 mole du dibromoalcane dissous dans 100 mL de THF anhydre sous agitation.
Filtrer le contenu du ballon et laver le solide avec de l'éther. Le solide blanc ainsi obtenu est séché sous vide.

*** 3a:**

Solide blanc **Rdt = 99%** **Pf =** 212-215°C.

***RMN $^1H$ (20 0MHz, $CD_3OD$) :*** 2,31 - 2,59 (m, 2H) ; 3,32 (s, 9H) ; 3,52 - 3,69 (m, 4H)
***RMN $^{13}C$ (50 MHz, $CD_3OD$) :*** 26,42 ; 28,96 ; 53,11 (t, $J_{C-N}$ = 4,0 Hz) ; 65,50 (t, J = 3,6 Hz).

*** 3b:**

Solide blanc **Rdt =** 96% **Pf =** 139-140°C.

***RMN $^1H$ (200 MHz, $D_2O$) :*** 1,50 - 1,75 (m, 2H) ; 1,85 - 2,15 (m, 4H) ; 3,25 (s, 9H) ; 3,4 - 3,55 (m, 2H) ; 3,65 (t, J = 6,6Hz, 2H)
***RMN $^{13}C$(50MHz,$D_2O$) :*** 21,91 ; 24,51 ; 31,72 ; 34,61 ; 53,22 (t, $J_{C-N}$=4,0 Hz) ; 66,80

**2) $X_2$=$NTf_2$:**

On dissout dans un bécher 5,5 g de $LiNTf_2$ (19 mmoles) dans 10 mL d'eau. De la même manière, on dissout dans un autre bécher environ 17,3 mmoles de bromure correspondant dans l'eau. Les deux solutions sont mélangées et laissées agiter durant deux heures.

Le contenu du bécher est transvasé dans une ampoule à décanter. La phase aqueuse est extraite avec 2 fois 15 mL de chlorure de méthylène. Les phases organiques sont rassemblées et séchées sur $MgSO_4$. Le solvant est ensuite évaporé à sec et le produit est séché sous vide.

* **<u>3c</u>**:

Huile visqueuse incolore **Rdt** = 84%.

***RMN $^1H$ (200 MHz, Acétone $D_6$):*** 1,40 - 1,70 (m, 2H) ; 1,90 - 2,20 (m, 4H) ; 3,35 (s, 9H) ; 3,50 - 3,70 (m, 4H)
***RMN $^{13}C$ (50 MHz, Acétone $D_6$):*** 23,13 ; 25,86 ; 33,24 ; 34,58 ; 54,06 (t, $J_{C-N}$ = 4,0 Hz) ; 67,65 ; 121,37 (q, $J_{C-F}$ = 320,9 Hz)

**3) $X_2$ = $PF_6$ :**

On dissout dans un bécher 23 mmoles du bromure dans 10 mL d'eau, et on ajoute 6 mL (68 mmol) d'$HPF_6$ à 60% dans l'eau. On laisse agiter pendant 2 heures à température ambiante pour que l'échange soit total puis le précipité est filtré. Le filtrat est lavé ensuite par 2 fois 15 ml de chlorure de méthylène. Le solvant est évaporé à sec et on ajoute le solide obtenu au précipité de départ. Enfin on lave le solide blanc obtenu par l'éther et on le sèche sous vide.

*** <u>3d</u> :**

Poudre blanche **Rdt** = 88% **Pf =** 144 - 146°C

***RMN $^1H$ (200 MHz, Acétone $D_6$) :*** 2,1 - 2,5 (m, 2H) ; 3,05 (s, 9H) ; 3,4 - 3,6 (m, 4H)
***RMN $^{13}C$ (50 MHz, Acétone $D_6$) :*** 27,29 ; 30,27 ; 54,27 (t, $J_{C-N}$ = 4,1 Hz) ; 66,54 (t, $J_{C-N}$ = 3,5 Hz)
***RMN$^{19}F$ (282 MHz, Acétone $D_6$):*** -71,65 (d, J= 7,07 Hz)
***Spectre RMN $^{31}P$ (300 MHz, Acétone $D_6$) :*** -142,69 (septuplet, J= 708,2 Hz)

*** <u>3e:</u>**

Solide blanc **Rdt** = 97% **Pf** = 139 - 140°C.

***RMN $^1H$ (200 MHz, $D_2O$) :*** 1,30 - 1,55 (m, 2H) ; 1,61-1,93 (m, 4H) ; 3,05 (s, 9H) ; 3,10 - 3,30 (m, 2H) ; 3,45 (t, J = 6,7Hz, 2H)
***RMN $^{13}C$ (50 MHz, $D_2O$) :*** 21,89 ; 24,49 ; 31,69 ; 34,54 ; 53,18 (t, $J_{C-N}$ = 4,0 Hz) ; 66,78
***RMN$^{19}F$ (282 MHz, $D_2O$) :*** -71,78 (d, J = 703 Hz)
***Spectre RMN $^{31}P$ (300 MHz, $D_2O$) :*** -144,38 (septuplet, J = 703 Hz)

**4) $X_2$ = $BF_4$**

On dissout dans un bécher 5 g du bromure (**3b**)(17,3 mmol) dans 10 mL d'eau. On ajoute 2,1 mL (34,6 mmol) d'une solution d'$HBF_4$ à 50% et on laisse agiter à température ambiante durant environ 2 heures pour que la réaction soit totale. Le contenu du bécher est transvasé dans un ballon et l'eau est évaporée à sec au rotavapor et on finit de sécher sous vide poussé.

*** 3f:**

Huile visqueuse de couleur orangée **Rdt** = 78%.

***RMN $^1H$ (200 MHz, $D_2O$) :*** 1,31-1,53 (m, 2H) ; 1,60-1,94 (m, 4H) ; 3 (s, 9H) ; 3,15-3,32 (m, 2H) ; 3,45 (t, J = 6,7 Hz, 2H)
***RMN$^{13}C$ (50 MHz, $D_2O$)*** : 21,92 ; 24,55 ; 31,79 ; 34,85; 53,23 (t, $J_{C-N}$ = 3,5 Hz) ; 66,72 (t, $J_{C-N}$ = 3,02 Hz)

**4/ Amine 4 :**

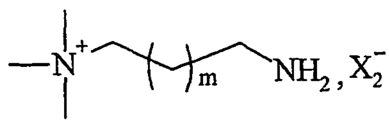

(4)

**1) $X_2$ = Br**

Dans un monocol de 250 mL, on introduit 10 mmol de bromure (**3a** ou **3b**) et 30 ml d'une solution aqueuse d'ammoniaque à 25%. On adapte un réfrigérant et le mélange réactionnel est porté à reflux durant une nuit (14 heures). On laisse refroidir la solution à température ambiante, et on ajoute 5 ml d'une solution de NaOH (1 N) puis on évapore à sec l'eau et l'excès d'ammoniaque.
Le solide obtenu est dissous dans l'acétone, filtré sur $MgSO_4$, et évaporé à sec. On obtient un solide blanc qui est séché dans un dessiccateur sous vide en présence de $P_2O_5$.

*** 4a :**

Solide blanc **Rdt** = 82% **Pf** =172-174°C

***RMN$^1H$ (200 MHz, $D_2O$) :*** 1,55 - 1,63 (m, 2H) ; 2,85 (t, J = 6,5 Hz, 2H) ; 3,21 (s, 9H) ; 3,35 - 3,60 (m, 2H).
***RMN $^{13}C$ (50 MHz, $D_2O$):*** 27,95 ; 34,85 ; 52,65 (t, $J_{C-N}$ = 3,4 Hz) ; 63,58 (t, $J_{C-N}$ = 3 Hz).

*** 4b:**

Solide blanc **Rdt** = 95% **Pf** =132-134°C

***RMN $^1H$ (200 MHz, $D_2O$) :*** 1,30-1,52 (m, 2H); 1,64-1,90 (m, 4H); 3,07 (s, 9H) ; 3,15-3,33 (m, 2H) ; 3,45 (t, J = 6,7 Hz, 2H)
***RMN$^{13}C$ (50 MHz, $D_2O$) :*** 21,92 ; 24,55 ; 31,79 ; 34,85 ; 53,23 (t, $J_{C-N}$ = 3,5 Hz) ; 66,72 (t, $J_{C-N}$ = 3,0 Hz).

**2) $X_2$ = $NTf_2$ :**

On dissout dans un bécher 5,5 g de $LiNTf_2$ (19 mmoles) dans 10 mL d'eau. De la même manière, on dissout dans un autre bécher environ 17,3 mmoles du bromure d'ammonium correspondant dans l'eau. Les deux solutions sont mélangées et laissées agiter durant deux heures.

Le contenu du bécher est transvasé dans une ampoule à décanter et la phase aqueuse est extraite avec 2 fois 15 mL de chlorure de méthylène. Les 2 phases organiques sont rassemblées et séchées sur $MgSO_4$. Le solvant est ensuite évaporé à sec et le produit est séché sous vide.

* **4c**:

Huile visqueuse orangée **Rdt** = 87%

***RMN $^1H$ (200 MHz, Acétone)*** : 1,41-1,70 (m, 2H) ; 1,95-2,15 (m, 4H) ; 2,82 (s élargi, 2H) ; 3,35 (s, 9H) ; 3,25-3,40 (m, 2H); 3,50-3,70 (m, 2H).
***RMN $^{13}C$ (50 MHz, Acétone) :*** 23,02 ; 26,45 ; 30,79 ; 35,64 ; 53,56 (t, $J_{C-N}$ = 3,5 Hz) ; 67,82 (t, $J_{C-N}$ = 3,0 Hz) ; 121,65 (q, J = 321 Hz)

**5/ Diéthanolamine 5 :**

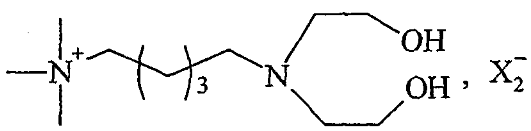

(5)

| | |
|---|---|
| **5a** | $X_2$ = $NTf_2$ |
| **5b** | $X_2$ = $BF_4$ |
| **5c** | $X_2$ = $PF_6$ |

Dans un ballon monocol de 50mL, on introduit 10g du sel ((III) avec $X_2$ = Br et m = 3), 40 mL d'éthanol et 1 équivalent de diéthanolamine. On adapte un réfrigérant et le mélange réactionnel est porté à 90°C pendant une nuit (14 heures). Le montage est ensuite refroidi à température ambiante, et on additionne une solution de NaOH (1N) jusqu'à atteindre un pH basique. Le mélange (eau / éthanol) est ensuite évaporé à sec et le ballon est placé sous vide poussé jusqu'à élimination totale de l'eau. La pâte blanche obtenue est ensuite lavée par 3 fois 30 ml d'acétone anhydre pour extraire le maximum de produit. Enfin, le solvant est évaporé à sec.

* **5a**:

Huile visqueuse orangée **Rdt** = 91%.

***RMN$^1H$ (200 MHz, $D_2O$)***: 1,25 - 1,5 (m, 2H) ; 1,55 - 1,95 (m, 4H) ; 2,8 - 2,95 (m, 4H); 3,0 (t, J = 5,8 Hz, 4H) ; 3,05 (s, 9H) ; 3,2 - 3,35 (m, 2H) ; 3,75 (t, J = 5,7 Hz, 4H).
***RMN$^{13}C$ (50 MHz, $D_2O$)*** : 21,26 ; 22,31 ; 23,17 ; 48,31 ; 51,86 (t, $J_{C-N}$ = 4,0 Hz) ; 52,83 ; 54,05 ; 56,60 ; 56,80 ; 65,53.

* **5b** :

Pâte blanche **Rdt** = 91%.

***RMN $^{1}H$ (200 MHz, $D_2O$) :*** 1,25 - 1,45 (m, 2H) ; 1,55 - 1,90(m, 4H) ; 2,8 - 2,95 (m, 4H) ; 3,05 (s, 9H) ; 3,25 (t, J = 8,7 Hz, 2H) ; 3,80 (t, J = 5,8 Hz, 4H) ; 2,95 - 3,15 (m, 4H)
***RMN 13C (50 MHz, $D_2O$) :*** 22,41 ; 23,37 ; 23,93 ; 49,42 ; 53,15 ; 53,95 ; 55,22 ; 57,19 ; 57,53 ; 66,68.
***RMN $^{19}F$ (282MHz, $D_2O$) :*** -71,64 (d, $J_{P-F}$ = 707,6 Hz).
***Spectre RMN $^{31}P$ : (300MHz, $D_2O$) :*** -144,35.

* **5c :**

Huile visqueuse incolore **Rdt=** 94%.

***RMN $^{1}H$ (200 MHz, $D_2O$):*** 1,25-1,65 (m, 4H) ; 1,67-1,95 (m, 4H) ; 3,05 (s, 9H) ; 3,15 (t, J = 5,6 Hz, 2H) ; 3,2-3,3 (m, 4H) ; 3,4-3,5 (m, 2H) ; 3,8 (t, J = 5,6 Hz, 4H)
***RMN $^{13}C$ (50 MHz, $D_2O$) :*** 21,90 ; 22,32 ; 28,47 ; 31,75 ; 34,63 ; 49,29 ; 53,17 (t, $J_{C-N}$ = 4,0 Hz) ; 56,87 ; 66,58 ; 70,30.
***RMN$^{19}F$(282 MHz, $D_2O$) :*** -149,98 (t, $J_{B-F}$ = 1,13 Hz).

**II) FONCTIONNALISATION DES SELS PRECEDENTS :**

**A) Ester acrylique 6 :**

***Procédure générale de l'estérification par l'acide acrylique :***

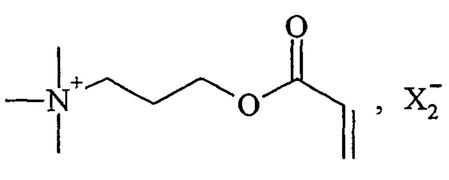

**6**

**6a** $X_2$ = $NTf_2$
**6b** $X_2$ = Cl
**6c** $X_2$ = $BF_4$

Une solution du sel N,N',N"-triméthyl-3-hydroxypropylammonium et de 3 équivalents de chlorure d'acryloyle dans l'acétonitrile est agitée en présence de 5 équivalents de $K_2CO_3$ solide pendant 2 heures à une température comprise entre 18 et 22°C. Le mélange est ensuite filtré et placé sous vide pour éliminer le solvant et l'excès du réactif. L'acrylate d'ammonium ainsi obtenu est stable à 4°C et peut être stocké pendant plusieurs mois.

* **6a** :

Huile incolore **Rdt** =100%

***RMN $^{1}H$ (200 MHz, Acétone $D_6$) :*** 2,22 - 2,25 (m, 2H) ; 3,25 (s, 9H) ; 3,60 - 3,75 (m, 2H) ; 4,15 (t, 2H, J = 6,0 Hz) ; 5,80

(dd, 1H, $J_1$ = 1,92, $J_2$ = 10,68) ; 6,05 (dd, 1H, $J_1$ = 17,2, $J_2$ = 10,7) ; 6,15 (dd, 1H, $J_1$ = 1,9, $J_2$ =17,2).
***RMN $^{13}$C (50 MHz, Acétone $D_6$) :*** 29,17 ; 54,16 (t, J = 4,0) ; 65,16 ; 65,23 ; 121,05 (q, $J_{CF}$ = 374,2 Hz) ; 129,40 ; 132,15 ; 165,61
***RMN$^{19}$F (282 MHz, Acétone $D_6$) :*** -79,8
**Spectrométrie de masse (FAB) pour $C_9H_{18}NO_2$ ($C^+$)**

| | |
|---|---|
| **Masse Théorique calculée :** | 172,13375 |
| **Masse Trouvée :** | 172,1346 |

* **6b:**

Solide blanc **Rdt** = 100% **Pf** =175-177°C

***RMN$^1$H (200 MHz, Acétone $D_6$) :*** 2,15 - 2,20 (m, 2H) ; 3,15 (s, 9H) ; 3,48 - 3,52 (m, 2H) ; 4,18 (t, 2H, J = 6,0 Hz) ; 5,75 (dd, 1H, $J_1$ = 1,92 Hz, $J_2$ = 10,5 Hz) ; 6,15 (dd, 1H, $J_1$ = 10,5 Hz, $J_2$ = 17,3 Hz) ; 6,15 (dd, 1H, $J_1$ =1,9 Hz, $J_2$ = 17,3 Hz)
***RMN $^{13}$C(50 MHz, Acétone $D_6$):*** 21,74 ; 52,23 (t, J = 4,2 Hz) ; 60,44 (t, J = 3,02) ; 62,6 ; 127,41 ; 130,65 ; 165,04

* **6c :**

Huile incolore **Rdt** = 93 %

***RMN$^1$H (200 MHz, Acétone $D_6$)*** : 2,28 - 3,31 (m, 2H) ; 3,32 (s, 9H); 3,06 - 3,15 (m, 2H) ; 4,52 (t, 2H, J=6,6 Hz) ; 5,80 (dd, 1H, $J_1$ = 1,9 Hz, $J_2$ = 10,0 Hz) ; 6,05 (dd, 1H, $J_1$ = 18,3 Hz, $J_2$ =10,0 Hz) ; 6,15 (dd, 1H, $J_1$ = 1,9 Hz, $J_2$ = 18,3 Hz)
***RMN $^{13}$C (50 MHz, Acétone $D_6$):*** 22,81, ; 53,28 ; 61,46 ; 63,83 ; 128,51 ; 131,72 ; 167,31.

**B/ Esters 3-iodobenzoïques 7 :**

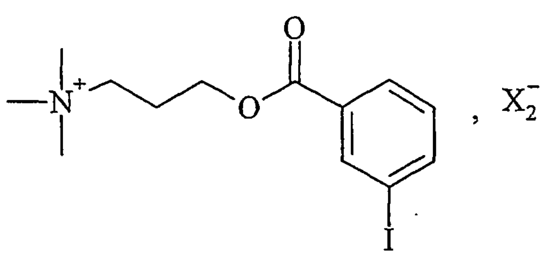

(7)

| | |
|---|---|
| **7a** | $X_2$ = Cl |
| **7b** | $X_2$ = $NTf_2$ |

* **7a:**

Dans un ballon de 100 ml on introduit 1,3 g (8,4 mmol) de chlorure N,N',N"-triméthyl-3-hydroxypropylammonium, 10 ml d'acétonitrile, 6,5 g de $K_2CO_3$ et 3 g du chlorure d'acide 3-iodobenzoïque. Après une nuit d'agitation à température ambiante, on filtre et on lave $K_2CO_3$ par 3 fois 15 ml de chlorure de méthylène. Après évaporation de ce dernier, on isole le produit par filtration après cristallisation dans l'acétone. Le solide blanc ainsi obtenu est lavé par 3 x 10 ml d'éther afin d'éliminer les traces d'acide restant.

Solide blanc **Rdt**= 85% **Pf** = 180-182°C

***RMN$^1$H (200 MHz, $D_2O$)**:* 2,15 (m, 2H); 3,01 (s, 9H) ; 3,25 - 3,45 (m, 2H); 4,10 - 4,20 (t, 2H, J = 6,6 Hz) ; 7,05 (t, 1H, J = 7,1 Hz); 7,75 (dd, 2H, $J_1$ = 7,1 Hz, $J_2$ = 2 Hz) ; 8,0 (d, 1H, J = 2,0 Hz).
***RMN$^{13}$C (50 MHz, $D_2O$)*** : 22,56 ; 53,35 (t, $J_{C-N}$ = 4,1 Hz) ; 62,83 ; 64,15 (t, $J_{C-N}$ = 3,3 Hz) ; 93,87 ; 129,07 ; 130,72 ; 131,08 ; 138,31 ; 142,83 ; 166,91.

*** 7b:**

Dans un ballon de 100 ml on solubilise 0,7g (1,8 mmol) du chlorure ((**VII**) avec $X_2$ = Cl) dans 5 ml d'eau. A cette solution on ajoute 0,8g (2,8 mmol) de $LiNTf_2$ dans 3 ml d'eau. Le mélange réactionnel est agité 2 heures à température ambiante avant d'extraire le produit de l'eau par le chlorure de méthylène. Après évaporation de ce dernier, on obtient un solide blanc.

Solide blanc **Rdt** = 88% **Tf=** 78-80°C

***RMN$^1$H (200 MHz, Acétone $D_6$)**:* 2,20 - 2,30 (m, 2H); 3,31 (s, 9H) ; 3,65 - 3,77 (m, 2H) ; 4,39 (t, 2H, J = 6,7 Hz) ; 7,21 (t, 1H, J = 7,1 Hz) ; 7,90 (dd, 2H, $J_1$ = 7,1 Hz, $J_2$ = 1,9 Hz) ; 8,22 (d, 1H, J = 1,9 Hz)
*RMN $^{13}$C (50MHz, Acétone $D_6$)* : 23,97 ; 54,23 (t, $J_{C-N}$ = 4,1 Hz) ; 55,40 ; 63,27 ; 65,31 (t, $J_{C-N}$ = 3,3 Hz) ; 94,70 ; 121,17 (q, $J_{C-F}$ = 320,9 Hz) ; 130,11 ; 131,94 ; 133,29 ; 139,32 ; 143,37 ; 165,65
***RMN$^{19}$F (282 MHz, Acétone $D_6$)*:** -79,23
Spectrométrie de masse (FAB) pour $C_{15}H_{19}F_6N_2O_6S_2$

| | |
|---|---|
| Masse Théorique calculée pour ($2C^+$,A) : | 976,0094 |
| Masse Trouvée : | 976,0094 |

**C/ Esters 4- bromobenzoïques 8 :**

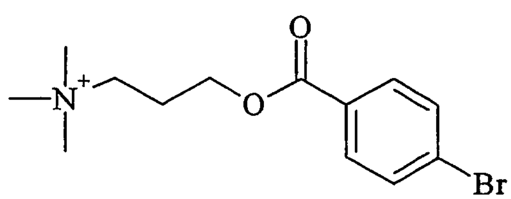

$X_2^-$ (**8**)

**8a** $X_2$ = Cl **8b** $X_2$ = $NTf_2$ **8c** $X_2$ = $PF_6$ 6 **8d** $X_2$ = $BF_4$ 4

*** 8a:**

Dans un ballon de 250 ml, on introduit 2 g (13,1 mmol) de chlorure de N,N',N"-triméthyl-3-hydroxypropylammonium, 25 ml d'acétonitrile, 20 g de $K_2CO_3$ en poudre et 4 g (17,5 mmol) du chlorure d'acide du 4-bromobenzoïque. Après une nuit d'agitation à température ambiante, on filtre et $K_2CO_3$ est lavé par 3 fois 15 ml de chlorure de méthylène et enfin on évapore à sec. On reprend à l'eau et l'excès de l'acide 4-bromobenzoique qui cristallise par filtration est éliminé. Le produit est ensuite cristallisé dans l'acétone après évaporation d'eau.

Solide blanc **Rdt** = 60% **Pf** =164-166°C

***RMN $^1$H (200 MHz, $D_2O$) :*** 2,21 - 2,34 (m, 2H) ; 3,12 (s, 9H) ; 3,30 - 3,58 (m, 2H) ; 4,35 (t, 2H, J=6,8 Hz) ; 7,57 (d, 2H, J = 7,4 Hz) ; 7,80 (d, 2H, $J_1$ = 7,4 Hz)
***RMN $^{13}$C (50 MHz, $D_2O$) :*** 22,55 ; 30,61 ; 53,34 (t, $J_{C-N}$ = 4,2 Hz) ; 62,69; 64,41 (t, $J_{C-N}$ = 4,09 Hz); 128,34; 128,66;

131,37; 132,20; 167,84
Spectrométrie de masse (FAB) pour $C_{11}$ $H_{19}$ $NO_2Cl$

| | |
|---|---|
| Masse Théorique pour ($C^+$): | 302,0580 |
| Masse Trouvée : | 302,0585 |

*** 8b:**

Dans ce cas la synthèse de substrat a été envisagée selon deux approches : par estérification directe du bis-trifluorométhanesulfonamidure de N,N,N-triméthyl-3-hydroxypropylammonium ou par métathèse à partir du chlorure correspondant.

**Estérification** :

Dans un ballon de 250 ml, on introduit 4 g (10,5 mmol) de l'alcool, 20 ml d'acétonitrile, 2 ml d'une solution saturée de $NaCO_3$ dans l'eau et 4 g (17,5 mmol) de chlorure de l'acide 4-bromobenzoïque. Le mélange réactionnel est chauffé à 60°C durant une nuit. On évapore ensuite à sec, et le résidu obtenu est solubilisé dans le chlorure de méthylène. Cette solution est lavée successivement par 2 × 20ml d'eau, 2 × 20ml d'une solution de soude (1N) et enfin par 2 × 20ml d'eau. La solution est séchée sur le sulfate de magnésium et le solvant est évaporé à sec. On reprend dans l'acétone et les traces d'acides qui restent sont éliminées par précipitation à 4°C. Après évaporation de ce dernier, on obtient un solide blanc pur.
Rdt = 90 %

**Métathèse**

Dans un ballon de 100 ml on solubilise 1 g (2,98 mmol) de **(8a)** dans 5 ml d'eau. A cette solution on ajoute 1,1 g (3,19 mmol) de bis-trifluorométhanesulfonamidure de lithium ($LiNTf_2$) en solution dans 3 ml d'eau. Le mélange réactionnel est agité 2 heures à température ambiante avant d'extraire notre produit par 20 ml de chlorure de méthylène. Après évaporation de ce dernier, on obtient un solide blanc qui est séché sous vide.

Solide blanc **Rdt** = 90% **Tf** = 86-88°C

***RMN$^1$H (200 MHz, Acétone $D_6$)*** : 2,64 - 2,83 (m, 2H) ; 3,59 (s, 9H) ; 3,96 - 4,06 (m, 2H) ; 4,71 (t, 2H, J = 6,76 Hz) ; 7,90 (d, 2H, J = 8,9 Hz) ; 8,19 (dd, 2H, J = 8,9Hz).
***RMN $^{13}$C (50 MHz, Acétone $D_6$)* :** 23,96 ; 54,24 (t, $J_{C-N}$ = 4,2 Hz) ; 63,09; 65,35 (t, $J_{C-N}$ = 4,0 Hz) ; 121,46 (q, $J_{C-F}$ = 322,0 Hz) ; 128,95 ; 130,42 ; 132,58 ; 133,12 ; 166,34.
Spectrométrie de masse (FAB) pour $C_{15}$ $H_{19}$ $F_6N_2O_6$ $S_2$

| | |
|---|---|
| Masse Théorique calculée pour ($2C^+$, $A^-$) : | 880,03713 |
| Masse Trouvée : | 880,0375 |

*** 8c :**

A une solution de 1 g (2,98 mmol) de (**8a**) dans 3 ml d'eau on ajoute 0,5 ml (5,7 mmol) de $HPF_6$ à 60% dans l'eau. Le mélange réactionnel est laissé agiter deux heures à température ambiante pour que l'échange soit total. Le solide blanc obtenu après filtration est lavé à l'eau puis deux fois par 30 ml d'éther et enfin séché sous vide.

Solide blanc **Rdt** = 96% **Pf** =154-156°C

***RMN$^1$H (200 MHz, Acétone $D_6$):*** 2,45 - 2,59 (m, 2H) ; 3,40 (s, 9H) ; 3,79 - 3,85 (m, 2H) ; 4,50 (t, 2H, J = 5,96 Hz), 7,55 (dd, 2H, $J_1$= *1,91* Hz, $J_2$ = 7,73 Hz) ; 8,00 (dd, 2H, $J_1$ = *1,91* Hz, $J_2$ =7 ,74 Hz)
***RMN$^{13}$C (50 MHz, Acétone $D_6$)*:** 22,96 ; 53,23 (t, J = 4,01 Hz) ; 62,31 ; 64,34 ; 128,06 ; 129,48 ; 131,79 ; 132,25 ; 165,63
***RMN$^{19}$F (282MHz, Acétone $D_6$):*** -71,6 (d, J = 707,3 Hz ; P-F)
***RMN31P(Acétone,121,SMlzz)*** $\delta$*:* -142 (m, J = 0,7 Hz, $P-F_6$)
Spectrométrie de masse (FAB) pour $C_{15}$ $H_{19}$ $F_6NO_2P$

Masse Théorique calculée pour ($2C^+$, $A^-$): 747,0822
Masse Trouvée : 747,0824

* **<u>8d:</u>**

A une solution de 1 g (2,98 mmol) de (**<u>8a</u>**) dans 3 ml d'eau on ajoute 1 ml de $HBF_4$ en solution dans l'eau à 40%. Après l'ajout de ce dernier, on observe la formation d'un solide blanc. Le mélange réactionnel est laissé agiter deux heures à température ambiante. Le solide blanc obtenu après filtration est lavé par l'eau (pour éliminer l'excès de $HBF_4$) puis deux fois par 30 ml d'éther et enfin séché sous vide.

Solide blanc **Rdt** = 98% **Tf** = 154-156°C

***RMN$^1$H (200 MHz, Acétone $D_6$)*** : 2,39 - 2,57 (m, 2H); 3,35 (s, 9H) ; 3,70 - 3,87 (m, 2H) ; 4,50 (t, 2H, J = 5,91 Hz) ; 7,73 (dd, 2H, $J_1$ = 1,97 Hz, $J_2$ = 6,77 Hz) ; 8,02 (dd, 2H, $J_1$ = 1,77 Hz, $J_2$ = 6,47 Hz)
***RMN $^{13}$C (50 MHz, Acétone $D_6$)* :** 22,96 ; 53,14 (4,1 Hz) ; 62,35 ; 64,29 ; 128,01 ; 129,52 ; 131,85 ; 132,24 ; 165,61.
***RMN$^{19}$F (282 MHz, Acétone $D_6$)* :** -150,16 (s, B-F)
Spectrométrie de masse (FAB) pour $C_{15}H_{19}F_4NO_2B$

Masse Théorique calculée pour ($2C^+$, $A^-$): 689,1214
Masse Trouvée : 689,1211

**D/ Boratrane <u>9</u> :**

1/ Greffage sur le cation :

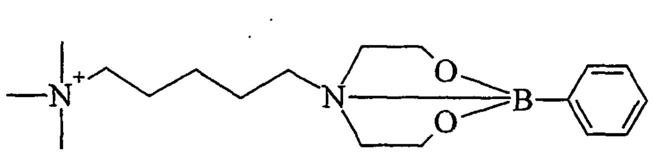

$X_2^-$ **(9)**

**<u>9a</u>** $X_2 = NTf_2$
**<u>9b</u>** $X_2 = BF_4$
**<u>9c</u>** $X_2=PF_6$

Dans un ballon monocol de 150mL, on introduit 2 g de (**<u>5a</u>** à **<u>5c</u>**) et un barreau aimanté. Le ballon est placé sous vide poussé durant environ 3 heures à une température de 50°C. Le ballon est ensuite placé sous Argon, et on ajoute environ 2,8 ml d'isopropanol pour solubiliser le sel. Dans un autre ballon et sous argon, dissoudre 1 g de l'acide phénylboronique dans 60 mL de chloroforme. On ajoute ensuite cette solution sur la première. Et enfin, on additionne 43 mL d'éther anhydre et on laisse agiter 18 heures à température ambiante.
Le milieu devient biphasique, on élimine la phase supérieure et on lave 3 fois par 20 ml d'éther anhydre la pâte blanche.

* **<u>9a:</u>**

Huile visqueuse jaune claire.

***RMN $^1$H (200 MHz, Acétone $D_6$)* :** 1,40-2,20 (m, 6H) ; 3,30 (s, 9H); 3,35-3,70 (m, 6H) ; 3,95-4,10 (m, 6H) ; 7,25-7,5 (m, 3H) ; 7,70-7,90 (m, 2H)

***RMN $^{13}C$ (50 MHz, Acétone $D_6$) :*** 24,14; 26,58 ; 30,24 ; 56,7 ; 57,05 ; 60,7 ; 64,04 ; 66,8 ; 67,3 ; 67,8 ; 128,97 ; 135,45
***Spectre RMN$^{11}$ B (96,25 MHz, Acétone):*** 13,06

* **9b**:

Pâte blanche

***RMN$^1$H (200 MHz, Acétone. $D_6$):*** 1,42 - 2,25 (m, 6H) ; 3,01 (s, 9H) ; 3,29 - 3,62 (m, 6H; 3,95 - 4,1 (m, 6H) ; 7,23 - 7,58 (m, 3H) ; 7,70 - 7,91 (m, 2H)
***RMN$^{11}$B (96,25 MHz, Acétone)***: 29,96 (60%) ; 4,40 (40%)

* **9c**:

Pâte blanche

***RMN $^1$H (200 MHz, Acétone) :*** 1,40 - 2,21 (m, 6H) ; 3,16 (s, 9H); 3,34-3,73 (m, 6H) ; 3,91-4,12 (m, 6H) ; 7,25-7,51 (m, 3H) ; 7,72-7,89 (m, 2H)

2/ Greffage sur l'anion :

***a- Quaternarisation par un hydroxyle***

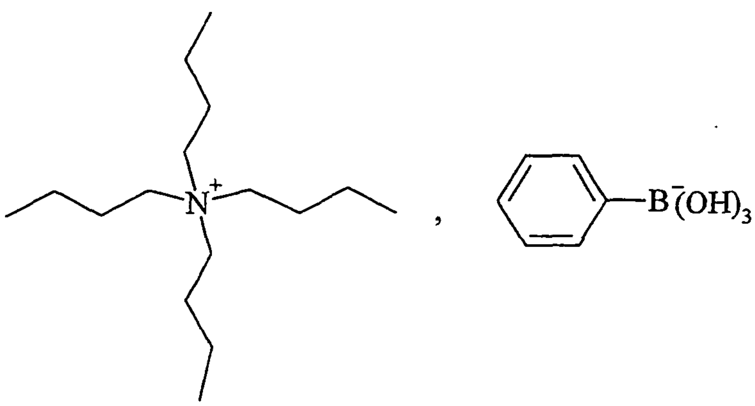

**(10)**

0,1 g (0,38 mmol) de l'hydroxyde de tétrabutylammonium est dissous dans 0,6 g du bis-trifluorométhanesulfonamidure de N,N',N''-triméthylbutylammonium comme matrice dans un ballon de 5 ml. A cette solution on ajoute 47 mg (0,38 mmol) d'acide phénylboronique et enfin 0,5 mL de THF (anhydre). On laisse agiter la solution durant 2 heures à température ambiante. On évapore ensuite THF à sec et on sèche la solution sous vide.

Huile visqueuse teintée.

***RMN $^1$H (200 MHz, Acétone $D_6$) :*** 0,9-1,05 (m, 12H); 1,21-1,52 (m, 8H) ; 1,5-1,8 (m, 8H); 3,0 (singulet élargi, 8H); 6,90-7,30 (m, 3H); 7,60-7,70 (m, 2H).
***RMN $^{13}C$ (50 MHz, Acétone $D_6$)***: 14,21 ; 20,50 ; 24,78 ; 25,75 ; 26,56 ; 53,78 ; 59,67 ; 67,45 ; 68,56 ; 127,94 ; 134,60
***Spectre RMN$^{11}$B (96,25MHz, Acétone)*** : 3,97

***b- Quaternarisation par le fluorure*** :

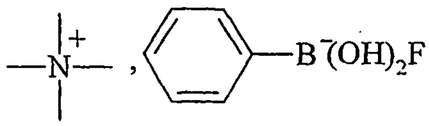

**(11)**

Dans un ballon monocol de 10 mL, on introduit 0,1 g (2,1 mmol) de fluorure de tétraméthylammonium anhydre, puis on ajoute 1 mL de THF (anhydre) et on homogénéise la solution en chauffant si nécessaire. Enfin on introduit 0,13 g d'acide phénylboronique (2,1 mmol). On laisse agiter durant environ 2 heures à température ambiante.

Après 2 heures d'agitation, on ajoute de l'éther anhydre pour une meilleure cristallisation et on filtre le solide sur verre fritté. Le solide est lavé 2 à 3 fois par 20 ml d'éther. Et pour finir, le solide est placé sous vide pour le sécher.

Solide blanc **Rdt** = 82% **Pf =** 162-164°C

***RMN $^1H$ (200 MHz, Acétone $D_6$) :*** 3,15 (s, 12H) ; 6,8 - 7,4 (m, 3H) ; 7,50 - 7,70 (m,2H)
***RMN $^{13}C$ (50 MHz, Acétone $D_6$):*** 56,19 (t, $J_{C-N}$ = 3,97 Hz) ; 127,47 ; 128,36 ; 130,91 ; 132,98; 135,96.
***Spectre RMN $^{19}F$ (282 MHz, Acétone $D_6$)*** : - 136,40 (multiplet).
***Spectre RMN $^{11}B$ (96,25 MHz, Acétone $D_6$) :*** 4,66 (D, $J_{B-F}$ = ***27,2Hz****)(56%) ;* 28,5 (44%).

**E/ Synthèse de la triéthylamine supportée (TEAS)(12) :**

Dans un ballon monocol, on introduit 3 g (6,1 mmol) de **(3b)**, 12 mL d'éthanol et 13 g (18 mmol) de diéthylamine. On adapte un réfrigérant et on porte ensuite le mélange réactionnel à reflux durant environ 14 heures. Après quoi, l'éthanol et l'excès de diéthylamine sont évaporés. L'huile obtenue est dissoute dans le dichlorométhane et la solution lavée avec 2 fois 5 mL de $K_2CO_3$ dilué. On sépare la phase organique, on sèche sur $MgSO_4$ et on évapore le solvant à sec.

Huile visqueuse orangée **Rdt** = 83%

***Spectre RMN$^1H$ (200 MHz, Acétone D6) :*** 1,05 (t, J = 7,1 Hz, 2H); 1,40 - 1,70 (m, 2H); 1,90-2,10 (m, 4H); 2,50 - 2,70 (m, 6H); 3,35 (s, 9H); 3,50 - 3,70 (m, 2H).
***Spectre RMN$^{13}C$ (50MHz, Acétone) :*** 12,34 ; 23,813 ; 25,08 ; 27,48 ; 48,140 ; 53,543 ; 54,04 (t, $J_{C-N}$ = 4,02 Hz) ; 67,93 ; 124,594 (q, J = 319,9 Hz)

**EXEMPLES**

Les différents sels fonctionnalisés utilisés ont été préparés selon les procédures décrites dans la littérature.

Pour illustrer ce principe, nous avons choisi de supporter :

- un ester acrylique par l'intermédiaire d'un sel d'ammonium dont la préparation est décrite dans le schéma ci-dessous ; ce sel a été ensuite engagé dans trois exemples de réactions de grand intérêt en chimie organique ;

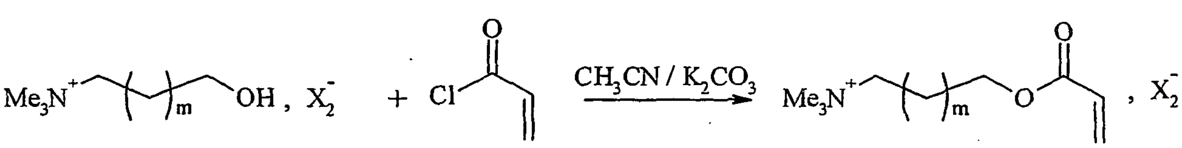


**1** **6**

- un ester arylique substitué par un halogène préparé selon le schéma réactionnel ci-dessous, qui a été testé dans deux exemples de réactions de couplages ;

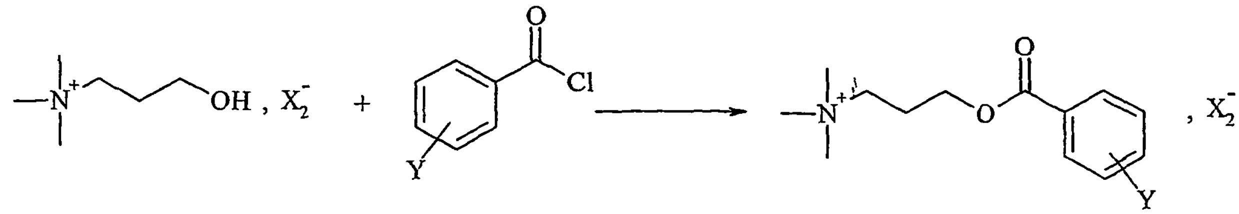

1

7 (avec Y = Br)

8 (avec Y = I)

- un ester d'acide arylboronique sous forme de boratrane (fonctionnalisation de la partie cationique) ou sous forme de borate (fonctionnalisation de la partie anionique du sel fonctionnalisé).

La synthèse de sels fonctionnalisés et de substrats ioniques est décrite en détail dans la partie expérimentale qui suit.

**EXEMPLE 1 : RÉACTION DE HECK**

La formation de liaisons carbone-carbone est une opération fondamentale en chimie organique. Parmi le grand nombre de réactions possibles, les méthodes utilisant des catalyseurs organométalliques sont extrêmement importantes. En particulier, nous avons appliqué le principe de l'invention de synthèse organique supportée sur liquide ionique (SOSLI) dans le couplage d'alcènes catalysé par le palladium avec les iodures d'aryle connu sous le nom de couplage de Heck.

Cette réaction a fait l'objet de plusieurs travaux utilisant les liquides ioniques comme solvants (Abbott et al., 2002 ; Murphy et al., 2000 ; Fraga-Dubreuil et al., 2001 ; Visser et al., 2002 ; Visser et al., 2001). En 1999, Xiao J. et al, ont montré la possibilité du recyclage du système catalytique et l'effet de l' anion sur la cinétique et la sélectivité de la réaction (Jeffery et al., 1996 ; Howarth et al., 2000 ; Bayer et al., 1991).

Dans cet exemple on utilise l'ester acrylique, et un ester arylique substitué par un atome d'iode comme substrats séparément.

**1- Ester acrylique:**

L'acrylate (6) a été engagé dans Heck en présence d'acétate de palladium comme catalyseur, de bicarbonate de potassium comme base et d'un iodure d'aryle en large excès comme réactif (voir schéma ci-après).

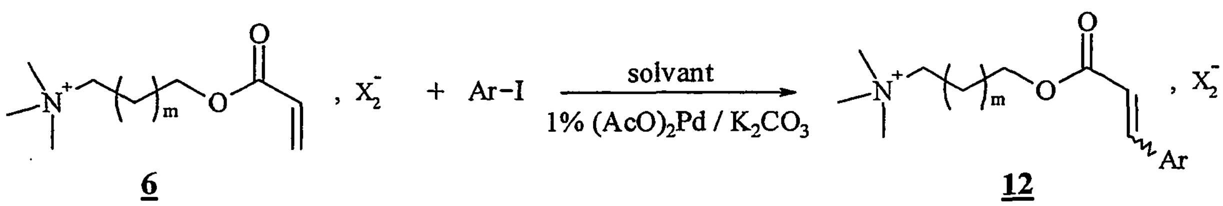


6

12

Toutes les réactions de couplage ont été effectuées à 80°C avec comme matrice liquide $A_1^+X_1^-$ = $Me_3N^+$ Bu, $^-NTf_2$. Le suivi des réactions a été effectué par RMN du proton à 200MHz et la figure 1 montre l'exemple de la réaction du sel 6 ($X_2^-$ = $NTf_2^-$) en solution dans le triflimidure de triméthylbutylammonium avec le 1-iodonaphtalène (essai 10 dans le tableau I ci-dessous).

D'après la Figure 1, on constate qu'il est possible et simple de suivre la réaction par RMN $^1H$. En effet, on constate la disparition totale des signaux entre 5,9 et 6,5 ppm correspondant aux trois protons de la double liaison du substrat 6, et l'apparition des signaux de la double liaison du produit résultant du couplage de Heck 12.

Ce type de suivi est impossible dans le cas de l'utilisation d'un support solide insoluble et moins évident dans le cas du support soluble (PEG) décrit dans la littérature en raison de signaux de RMN élargis.

Les résultats obtenus pour un temps de réaction de 2 heures à 80°C sont regroupés dans le tableau I ci-dessous :

**TABLEAU I**

| essai | Ar | X[i] | Solvant ou matrice ionique[i] | Conversion (%)[ii] | Rapport E/Z[iii] |
|---|---|---|---|---|---|
| 1 | 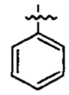 | | $CH_3CN$ | 26 | > 98/2 |
| 2 | 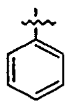 | $NTf_2$ | $CH_3CN$ | 70 | 88/12 |
| 3 | 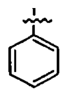 | $BF_4$ | 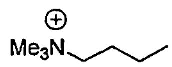 , $NTf_2^{-}$ | 75 | 84/16 |
| 4 | 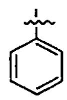 | $NTf_2$ | 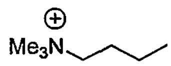 , $NTf_2^{-}$ | 100 | > 99/1 |
| 5 | 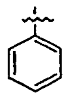 | $NTf_2$ | 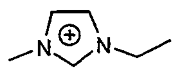 , $NT_2^{-}$ | 100 | > 99/1 |
| 6 | 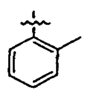 | $NTf_2$ | 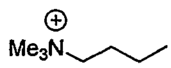 , $NTf_2^{-}$ | 100 | > 99/1 |
| 7 | 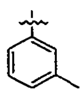 | $NTf_2$ | 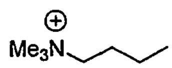 , $NTf_2^{-}$ | 100 | > 99/1 |
| 8 | 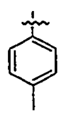 | $NTf_2$ | 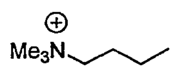 , $NTf_2^{-}$ | 100 | > 99/1 |
| 9 | 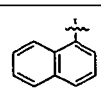 | $NTf_2$ | 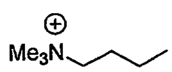 , $NTf_2^{-}$ | 100 | > 99/1 |
| 10 | 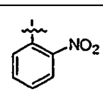 | $NTf_2$ | 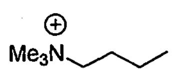 , $NTf_2^{-}$ | 100 | > 99/1 |
| 11 | 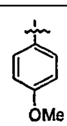 | $NTf_2$ | 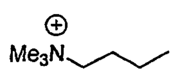 , $NTf_2^{-}$ | 100 | > 99/1 |
| 12 | 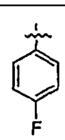 | $NTf_2$ | 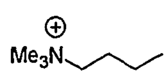 , $NTf_2^{-}$ | 100 | > 99/1 |

(suite)

| essai | Ar | $X^i$ | Solvant ou matrice ionique$^i$ | Conversion (%)$^{ii}$ | Rapport E/Z$^{iii}$ |
|---|---|---|---|---|---|
| 13 | 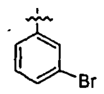 | $NTf_2$ | 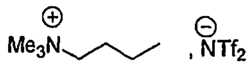 | 100 | > 99/1 |
| i : $NTf_2 = N(SO_2CF_3)_2$<br>ii : déterminé par RMN<br>iii : déterminé par RMN et confirmé par GC. | | | | | |

Les résultats du tableau ci-dessus montrent que l'emploi du substrat supporté permet d'avoir une meilleure réactivité par rapport à l'utilisation d'un substrat classique (comparer les essais 1 et 2).

L'utilisation du liquide ionique comme solvant a nettement augmenté la vitesse de réaction et permis d'avoir une meilleure stéréosélectivité comparée à l'acétonitrile souvent utilisé comme solvant pour cette réaction (essais 2 et 4).

On remarque une relation directe entre la réactivité / sélectivité et la nature de l'anion du substrat supporté (essais 4 et 5).

La nature du réactif semble n'avoir aucune influence sur la réaction.

En dernière étape pour cet exemple, le produit est libéré du support par transestérification avec un alcool (Schéma ci-dessous), et on a testé la possibilité d'adapter cette méthodologie à la technique combinatoire.

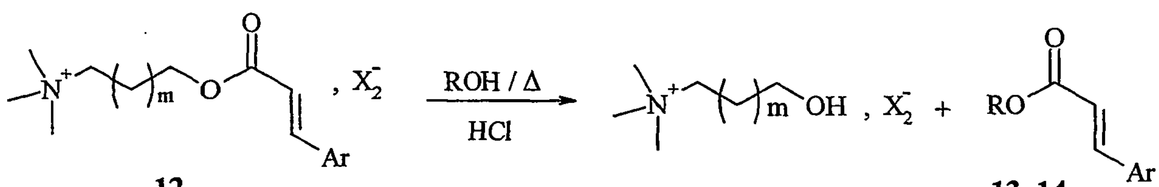


12 13, 14, 15 ou 16

Ainsi, une transestérification a été effectuée avec différents alcools sur un mélange des produits 12a à 12i isolés lors de la dernière étape, selon la procédure ci-dessous. Les alcools utilisés sont le méthanol, l'éthanol, le propanol et le butanol.

***Procédure:***

Quatre mélanges synthétiques constitués de 100mg de chacun des essais : 5, 6, 7, 8, 9, 10, 11, 12, 13 (tableau I) sont dissous respectivement dans 5 ml de méthanol, d'éthanol, de propanol et de butanol. On ajoute ensuite 5 gouttes d'acide chlorhydrique concentré (12N) dans chaque solution et on porte à reflux pendant 12 heures. Le suivi de la réaction est effectué par RMN $^1H$ où on observe une différence des déplacements chimiques entre les protons des sels à tache dédiée 12 et 1, tel que représenté ci-après :

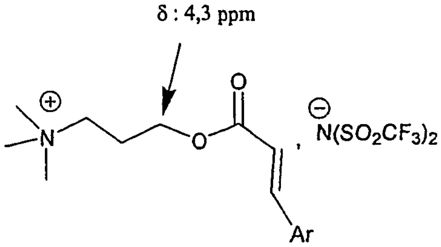


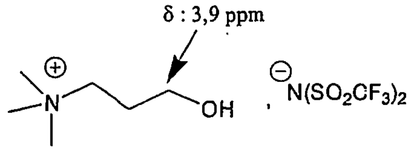


Après 12 heures, on évapore à sec chaque solution et on extrait les différents mélanges par 3 fois 15 ml d'éther diéthylique séparément. Les produits extraits sont ensuite analysés en CPG-SM, et les chromatogrammes correspondants à chaque mélange sont présentés ci-dessous.

Sur les chromatogrammes représentés dans les Figures 2 à 5, on retrouve les 36 produits attendus (13a à

**13i ; 14a** à **14i ; 15a** à **15i; 16a** à **16i).** Ceci valide le principe SOSLI et montre bien son applicabilité à la synthèse combinatoire.

Les composés **13a** à **13i** correspondent au produit de formule

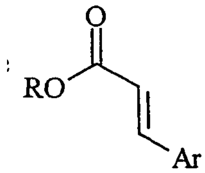

dans lequel R est un groupe méthyle.

Le tableau II ci-dessous regroupe ces 9 esters méthyliques, en indiquant pour chacun la signification de Ar. Ce tableau correspond au chromatogramme de la Figure 2.

**TABLEAU II**

| Temps de rétention (min) | Masse trouvée | 13 | Ar |
|---|---|---|---|
| 12,50 | 180 | **13a** | 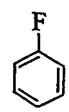 |
| 12,55 | 162 | **13b** | 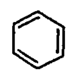 |
| 13,22 | | **13c** | |
| 13,38 | 176 | **13d** | 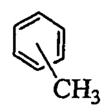 |
| 13,47 | | **13e** | |
| 14,69 | 240 | **13f** | 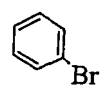 |
| 14,80 | 192 | **13g** | 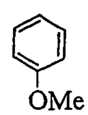 |
| 15,47 | 207 | **13h** | 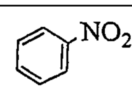 |
| 17,91 | 212 | **13i** | 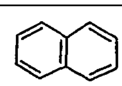 |

Les composés **14a** à **14i** correspondent au produit de formule

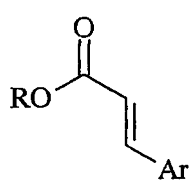

dans lequel R est un groupe éthyle.

Le tableau III ci-dessous regroupe ces 9 esters éthyliques, en indiquant pour chacun la signification de Ar. Ce tableau correspond au chromatogramme de la Figure 3.

**TABLEAU III**

| Temps de rétention (min) | Masse trouvée | 14 | Ar |
|---|---|---|---|
| 13,136 | 194 | **14a** | 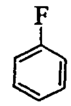 |
| 13,196 | 176 | **14b** | 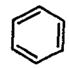 |
| 13,800 | | **14c** | 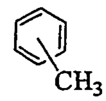 |
| 13,980 | 190 | **14d** | |
| 14,087 | | **14e** | |
| 15,475 | 254 | **14f** | 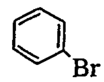 |
| 15,577 | 206 | **14g** | 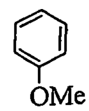 |
| 16,318 | 221 | **14h** | 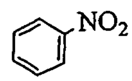 |
| 19,434 | 226 | **14i** | 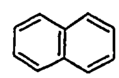 |

Les composés **15a** à **15i** correspondent au produit de formule

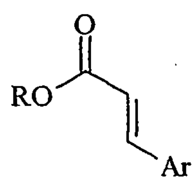

dans lequel R est un groupe propyle.

Le tableau IV ci-dessous regroupe ces 9 esters propyliques, en indiquant pour chacun la signification de Ar. Ce tableau correspond au chromatogramme de la Figure 4.

**TABLEAU IV**

| Temps de rétention (min) | Masse trouvée | 15 | Ar |
|---|---|---|---|
| 13,902 | 208 | **15a** | 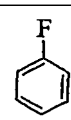 |
| 13,992 | 1190 | **15b** | 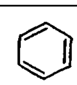 |

(suite)

| Temps de rétention (min) | Masse trouvée | 15 | Ar |
|---|---|---|---|
| 14,608 | 204 | **15c** | 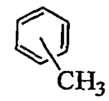 |
| 14,859 | | **15d** | |
| 14,973 | | **15e** | |
| 16,731 | 270 | **15f** | 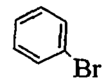 |
| 16,905 | 120 | **15g** | 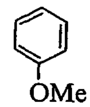 |
| 17,814 | 234 | **15h** | 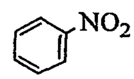 |
| 22,264 | 240 | **15i** | 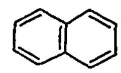 |

Les composés **16a** à **16i** correspondent au produit de formule

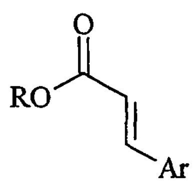

dans lequel R est un groupe butyle.

Le tableau V ci-dessous regroupe ces 9 esters butyliques, en indiquant pour chacun la signification de Ar. Ce tableau correspond au chromatogramme de la Figure 5.

**TABLEAU V**

| Temps de rétention (min) | Masse trouvée | 16 | Ar |
|---|---|---|---|
| 14,752 | 222 | **16a** | 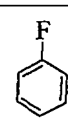 |
| 14,853 | 204 | **16b** | 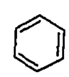 |
| 15,625 | 218 | **16c** | 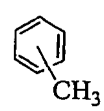 |
| 15,930 | | **16d** | |
| 116,109 | | **16e** | |
| 18,544 | 284 | **16f** | 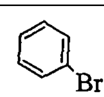 |

(suite)

| Temps de rétention (min) | Masse trouvée | <u>16</u> | Ar |
|---|---|---|---|
| 18,783 | 234 | <u>16g</u> | 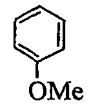 |
| 19,931 | 249 | <u>16h</u> | 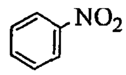 |
| 26,390 | 254 | <u>16i</u> | 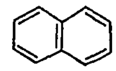 |

**2 - Ester iodo-arylique :**

Le deuxième exemple de **sel fonctionnalisé** qui a été testé dans la réaction de Heck est un iodure d'aryle supporté sur le (TMHPA, $NTf_2$) (<u>**1**</u>) selon le schéma suivant :

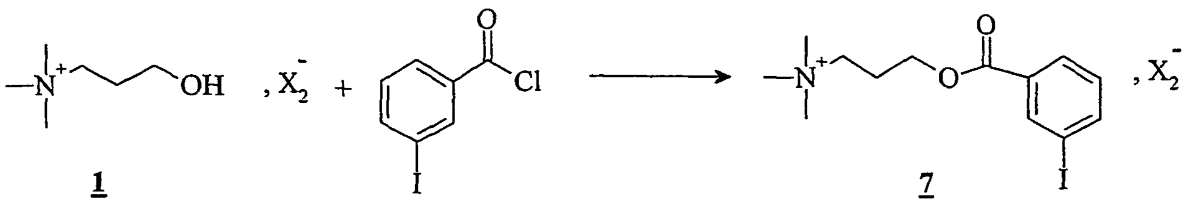

Au cours de cet exemple, nous avons porté à 80˚C et pendant 3 heures un mélange constitué :

- d'une solution de 0,85% molaire du sel <u>7</u> dissous dans le trifluorométhanesulfonamidure de triméthylbutylammonium (TMBA, $NTf_2$) comme matrice,
- d'acrylate de tertiobutyle comme alcène,
- d'acétate de palladium comme catalyseur
- de $K_2CO_3$ solide comme base

La réaction correspond au schéma suivant :

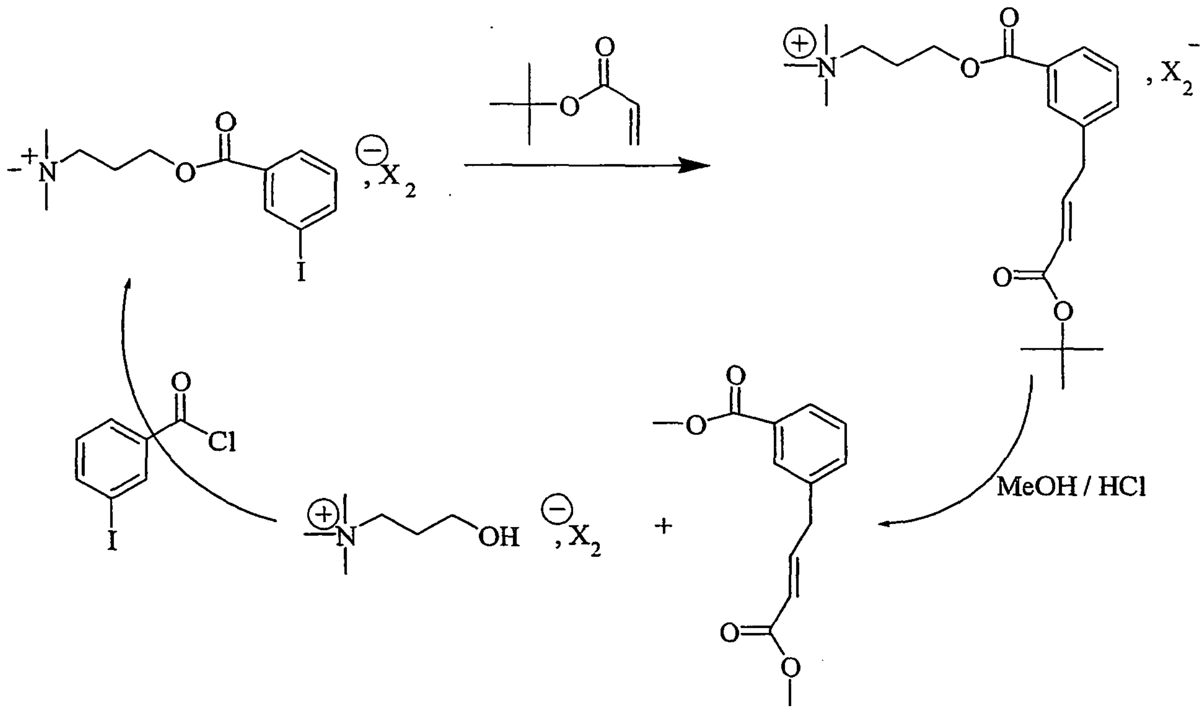


Le suivi de la réaction est effectué par RMN $^1H$ et $^{13}C$ et la Figure 6 illustre et confirme la simplicité du suivi par cette technique d'analyse, chose plus délicate dans le cas des supports solides ou solubles décrits dans la littérature.

***3*** **- Essai sur 6 grammes (15 mmol) de sel fonctionnalisé 6a.**

Tous les essais de couplage ont été effectués sur des quantités très faibles. Dans le but de montrer que le principe SOSLI peut être extrapolé à l'échelle du gramme ou même à des grandes quantités ("large scale"), nous avons testé la réaction de couplage de Heck sur 6 g (15 mmol) de l'ester acrylique (**6a**), dans les mêmes conditions opératoires que celles décrites dans la première partie de cet exemple, utilisant l'iodobenzène comme substrat (5 éq), l'hexafluorophosphate de butylméthyl imidazolium [BMIM][$PF_6$] comme matrice (11 g), et l'acétate de palladium comme catalyseur (25 mg). Après 3 heures, le spectre RMN du proton montre que la réaction est totale.

Après un lavage à l'éther pour éliminer l'excès d'iodobenzène, on effectue une transestérification par le méthanol.

Le rendement en produit isolé pur est de 86%.

Le premier recyclage du sel fonctionnalisé conduit à un rendement de 88%.

**EXEMPLE 2 : RÉACTION DE DIELS-ALDER :**

La richesse et le potentiel de la réaction de Diels-Alder ont incité les chimistes à rechercher des méthodes permettant d'augmenter d'une part la vitesse et le rendement, d'autre part la régio et la stéréosélectivité. Récemment, des travaux utilisant les liquides, ioniques comme solvant ont montré l'influence de leur polarité sur le rapport endo/exo (Xiao et al., 2000). Cette réaction est le deuxième exemple choisi pour montrer l'intérêt de la stratégie de synthèse organique supportée sur liquide ionique SOSLI.

La réaction de Diels-Alder entre un diénophile **6** et le cyclopentadiène a donc été étudiée. Le Schéma ci-après représente les différentes étapes.

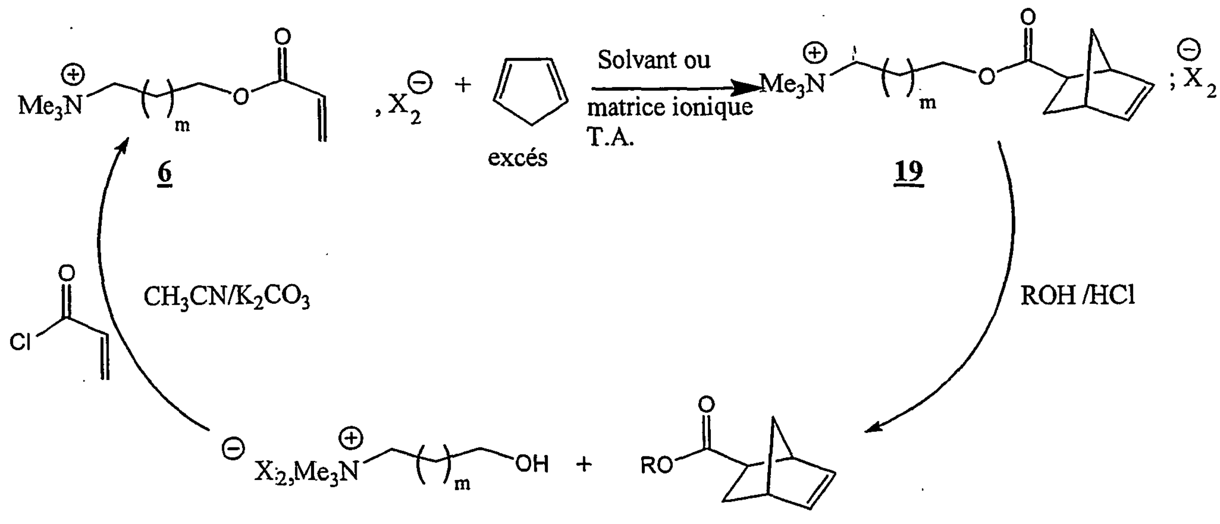


**6** **19** **1** **20**

***Procédure** :*

Une solution de (6) et de 10 équivalents du cyclopentadiène, dans un solvant ou dans une matrice ionique, est agitée pendant deux heures à température ambiante. L'excès du réactif est ensuite éliminé sous vide et le produit de réaction ainsi obtenu est mis en solution dans du méthanol, éthanol ou butanol en présence de cinq .gouttes d'acide chlorhydrique 12 N.

Après douze heures à reflux, la transestérification est totale et le produit est alors extrait avec du pentane. Les résultats obtenus sont regroupés dans le tableau VI ci-dessous :

**TABLEAU VI**

| essai | n | $X_2$ | Solvant ou Matrice liquide | R | Taux de conv (%) | Endo/exo % |
|---|---|---|---|---|---|---|
| 1 | 4 | $NTf_2$ | 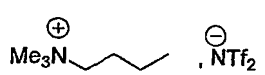 | Me | 85 | 80/20 |
| 2 | 1 | $NTf_2$ | 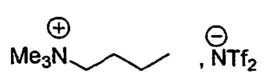 | Me | 94 | 81/19 |
| 3 | 1 | $NTf_2$ | Sans solvant[i] | Me | 96 | 83/17 |
| 4 | 1 | $NTf_2$ | $CH_2Cl_2$ | Me | 86 | 80/20 |
| 5 | 1 | $NTf_2$ | 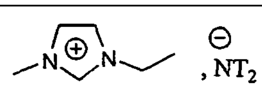 | Me | 93 | 80/20 |
| 6 | 1 | $NTf_2$ | 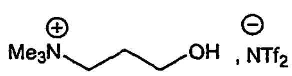 | Me | 95 | 82/18 |
| 7 | 1 | $NTf_2$ | " | Et | 89 | 82/18 |
| 8 | 1 | $NTf_2$ | " | Pr | 97 | 81/19 |
| 9 | 1 | Cl | " | Me | 95 | 78/22 |
| 10 | 1 | $BF_4$ | " | Me | 92 | 80/20 |
| **i** : **2**, $^{-}$**N(SO$_2$CF$_3$)$_2$** est liquide à température ambiante. | | | | | | |

Les résultats de ce tableau montrent que la longueur de la chaîne alkyle du bras L influence la vitesse de la réaction. En effet en augmentant la chaîne de 3 carbones, on réduit la vitesse de réaction sans l'inhiber (comparer les essais 1 et 2). En revanche, aucune influence sur la réactivité et sur la sélectivité n'est observée pour les différentes matrices ioniques et anions du support (essais 5 à 10).

La régiospécificité de cette réaction est comparable à celle observée dans le cas du substrat non supporté, c'est à dire l'acrylate de méthyle.

Enfin, nous avons testé la possibilité de recyclage de la solution du support ionique, dans deux cas différents. Les résultats que nous avons obtenus sont regroupés dans le tableau VII qui suit :

**TABLEAU VII**

| essai | $X_2$ | Matrice liquide | Rendement de 2 étapes |
|---|---|---|---|
| 1ère réaction | $NTf_2$ | 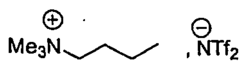 | 78 |
| 1er recyclage | $NTf_2$ | 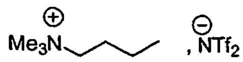 | 75 |
| 2éme | $NTf_2$ | 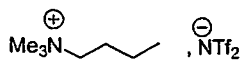 | 77 |
| 3éme | $NTf_2$ | 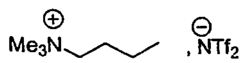 | 81 |
| 1er réaction | Cl | 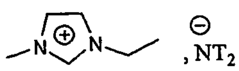 | 83 |
| 1er recyclage | Cl | 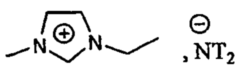 | 80 |
| 2éme | Cl | 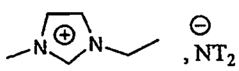 | 85 |

Quelle que soit la nature de l'anion les rendements en produit **18** isolé sont identiques ainsi que le pourcentage des deux isomères. Il faut aussi noter que dans le deuxième cas **(6b),** après trois recyclages, plus de 85% du sel fonctionnalisé **1a** est isolé de la matrice par simple filtration après précipitation dans l'acétone. Cette opération de récupération du sel fonctionnalisé peut s'avérer intéressante pour le recyclage de la matrice et du sel fonctionnalisé.

**EXEMPLE 3 : REACTION DE BAYLIS-HILMAN**

Le troisième exemple utilisé pour valider le principe SOSLI est la réaction de Baylis-Hilman, qui consiste en la condensation d'un aldéhyde sur la double liaison du substrat acrylique **6** en présence de 3-hydroxyquinuclidine (voir schéma ci-après).

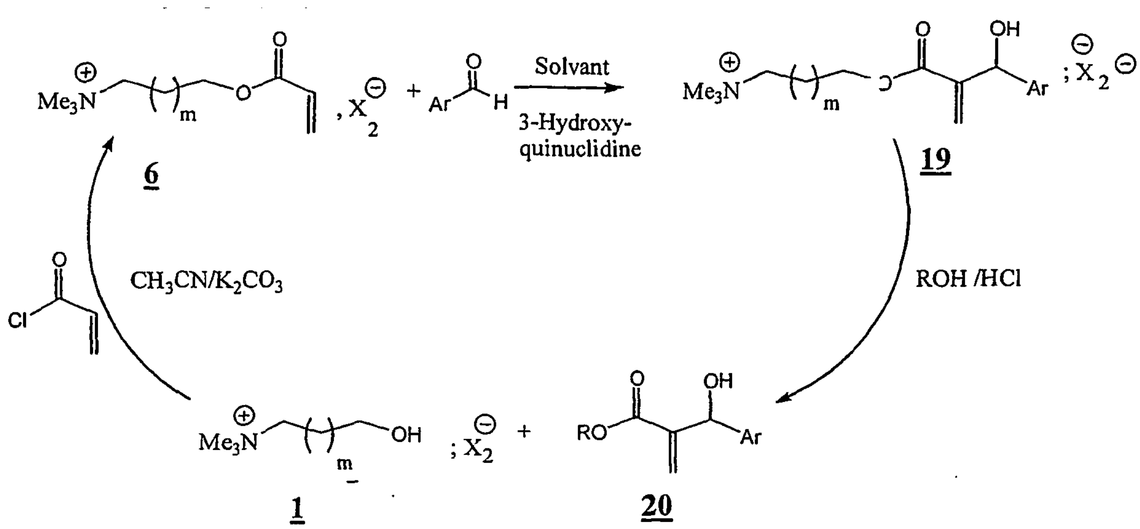


***Procédure générale :***

Un mélange de 2 mmol de 6 et de 10 mmoles d'aldéhyde dans un solvant ou dans une matrice ionique est agité à température ambiante en présence de 2 mmoles de 3-hydroxyquinuclidine comme base.

Après 24 heures, on lave à l'éther pour éliminer l'excès de réactif et le taux de conversion est déterminé par RMN. Les résultats sont regroupés dans le tableau VIII qui suit.

**TABLEAU VIII**

| essai | Ar | Solvant ou matrice liquide | Rdt % en 19[i] |
|---|---|---|---|
| 9 | 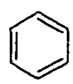 | $C_2H_5OH$ | 40 |
| 1 | 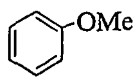 | 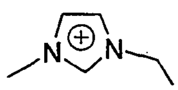 , $NTf_2^-$ | 45 |
| 2 | 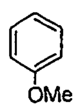 | 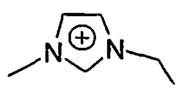 , $NTf_2^-$ | 42 |
| 3 | 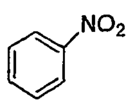 | 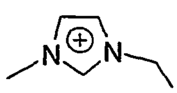 , $NTf_2^-$ | 65 |
| 4 | 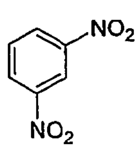 | 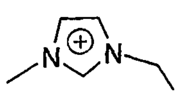 , $NTf_2^-$ | 75 |
| 5 | 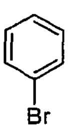 | 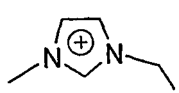 , $NTf_2^-$ | 64 |

(suite)

| essai | Ar | Solvant ou matrice liquide | Rdt % en 19[i] |
|---|---|---|---|
| 6 | 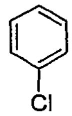 | " | 60 |
| 7 | 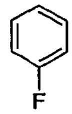 | " | 75 |
| 8[ii] | 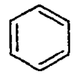 | 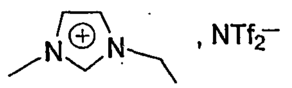 | 39 |
| 10 | 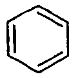 | 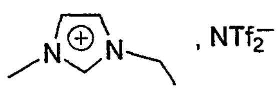 | 50 |
| 11 | 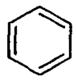 | 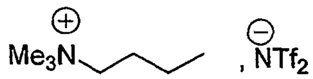 | 46 |
| 12 | 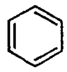 | 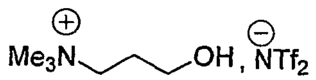 | 60 |
| 13 | 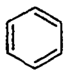 | 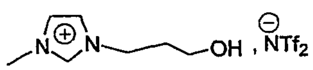 | 71 |
| 14 | 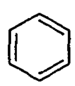 | 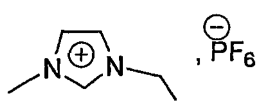 | 55 |
| 15 | 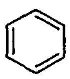 | $^{\oplus}P(C_6H_{13})_3C_{14}H_{29}$ ; $^{\ominus}NTf_2$ | 25 |

i : rendement en produit isolé après transestérification.
ii : essai avec l'acrylate de méthyle comme substrat.

On constate une meilleure réactivité du substrat ionique 6 comparé aux résultats réalisés avec l'acrylate de méthyle comme substrat dans un liquide ionique comme solvant (comparer essais 8 et 10). La nature de ce dernier influence nettement la vitesse de réaction. En effet, l'utilisation d'une matrice ionique portant une fonction alcool augmente la réactivité (comparer essais 10, 11 et 12, 13).

L'influence de la nature du réactif sur les rendements est très importante, elle est comparable à celle décrite dans des travaux concernant des substrats non supportés ou lors de l'emploi des supports solides. Il faut toutefois noter que les rendements que nous avons obtenus sont nettement meilleurs comparés à ces derniers.

**EXEMPLE 4 : COUPLAGE DE SUZUKI**

Un autre exemple où nous avons appliqué le principe SOSLI est la réaction de couplage de Suzuki qui consiste au couplage d'un halogénure d'aryle et d'un acide aryle boronique.

Cette étude a été effectuée selon deux approches distinctes :

1- en utilisant un halogénure d'aryle supporté ;
2- en utilisant un acide boronique supporté.

**A- Halogénure d'aryle supporté ;**

Dans cette étude on a utilisé comme 'halogénure d'aryle supporté, l'acide 3-iodobenzoïque et l'acide 4-bromobenzoïque. Dans un premier temps et afin de mettre au point les conditions optimales, on a étudié l'influence des différents paramètres sur la réaction de couplage. L'effet de la matrice ionique, de la température et du contre ion du support ionique a donc été étudié.

Ces différentes études ont été effectuées en utilisant l'acide phénylboronique et l'acétate de palladium comme catalyseur, selon le schéma réactionnel suivant :

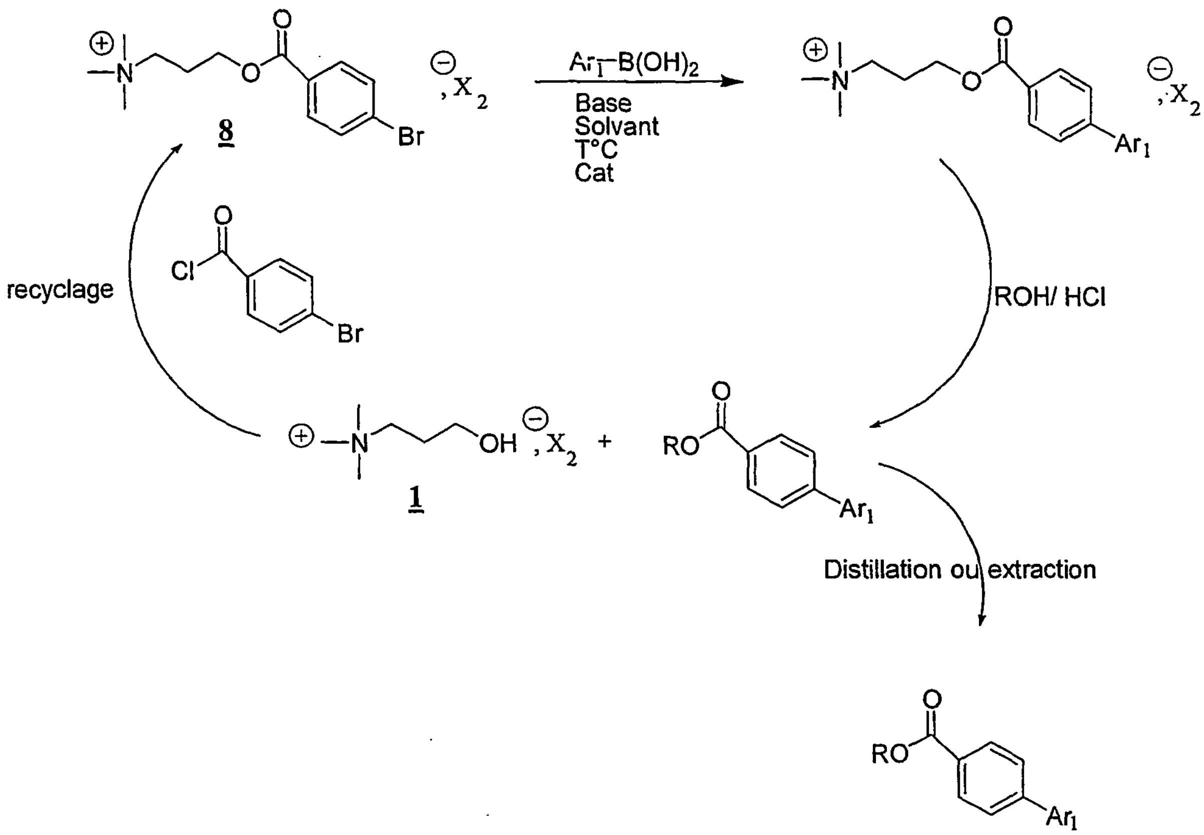

8
1

21 (R = méthyle)
22 (R = éthyle)
23 (R = propyle)

***1- Effet de la matrice :***

La réaction de couplage a été effectué avec le bis-trifluorométhane sulfonamidure du 4-bromobenzoate de N, N,N-triméthylpropylammonium et de $K_2CO_3$ solide ou en solution aqueuse comme base. Les résultats obtenus après 18 heures d'agitation à température ambiante sont regroupés dans le tableau IX suivant :

**TABLEAU IX**

| essai | Matrice | Taux de conversion (%) | Ar-Ar' (%) | Ar-Ar (homocouplage) (%) |
|---|---|---|---|---|
| 1 | 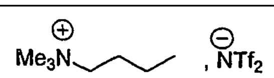 | 45,5 | 34,9 | 10,6 |
| 2 | 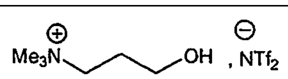 | 76,5 | 64,9 | 10,6 |

(suite)

| essai | Matrice | Taux de conversion (%) | Ar-Ar' (%) | Ar-Ar (homocouplage) (%) |
|---|---|---|---|---|
| 3 | 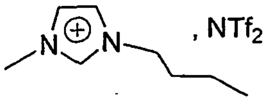 , $NTf_2$ | 62,3 | 51,6 | 10,7 |
| 4 | 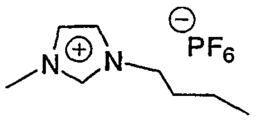 $PF_6$ | 20 | < à 2% | 2,2 |
| 5 | 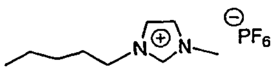 $PF_6$ | 20,7 | < à 2% | 4,73 |
| 6 | 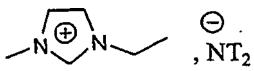 , $NT_2$ | 30,2 | 19,7 | 10,56 |
| 7 | $^{\oplus}P(C_6H_{13})_3C_{14}H_{29}$ ; $\overset{\ominus}{NTf_2}$ | 50,7 | 47,9 | 3,3 |
| 8 | $(Bu)_3\overset{\oplus}{P}-Me\ \overset{\ominus}{NTf_2}$ | 73 | 62,4 | 8,45 |

L'examen du tableau précédent montre que l'utilisation des matrices avec l'anion bis-trifluorométhanesulfonamidure ($NTf_2$) permet d'observer une meilleure réactivité comparée à l'emploi de l'hexafluorophosphate ($PF_6$) comme anion (comparer les essais 3 et 4). De même, dans le cas des matrices composées de cations ammonium et phosphonium, la réactivité est nettement meilleure que celle observée dans le cas des cations imidazoliums (comparer les essais 2, 8 et 3, 4, 5).

Il faut toutefois noter que la présence d'une fonction alcool sur la matrice ionique permet aussi bien d'améliorer le taux de conversion de 45 à 76% que de réduire le taux du produit d'homocouplage de 10 à 1% (comparer les essais 1 et 2).

Enfin, cette étude préliminaire montre que la nature de la matrice ionique influence la réactivité. Une étude poussée permet le choix d'une matrice adéquate.

***2- Effet de l'anion du support, de la température et de la base :***

Dans cette partie, on s'est intéressé à étudier l'influence de quelques paramètres sur la réactivité des substrats supportés sur un support ionique. Toutes choses étant égales par ailleurs, on a fait varier la température, la base et/ou le milieu :

Les différents essais effectués sont regroupés dans le tableau X suivant :

**TABLEAU X**

| essai | $X_2$ | T (°C) | base | Solvant ou matrice liquide[ii] | Taux Conv. (%) | Ar-Ar' (%) | Ar-Ar (%)[i] |
|---|---|---|---|---|---|---|---|
| 1 | $NTf_2$ | 20 | $K_2CO_3$(s) | 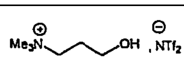 , $NTf_2$ | 28,0 | 20,6 | 7,4 |
| 2 | $PF_6$ | 20 | $K_2CO_3$(s) | 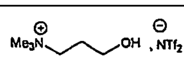 , $NTf_2$ | 100 | 91,6 | 8,4 |
| 3 | $BF_4$ | 20 | $K_2CO_3$(s) | 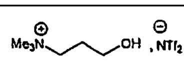 , $NTf_2$ | 100 | 99,0 | 0,1 |
| 4 | Cl | 20 | $K_2CO_3$(s) | 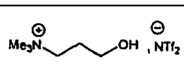 , $NTf_2$ | 23,4 | 16,2 | 7,1 |

(suite)

| essai | $X_2$ | T (°C) | base | Solvant ou matrice liquide[ii] | Taux Conv. (%) | Ar-Ar' (%) | Ar-Ar (%)[i] |
|---|---|---|---|---|---|---|---|
| 5 | $NTf_2$ | 20 | $K_2CO_3$(1) | 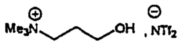 | 65,0 | 55,1 | 9,8 |
| 6 | $NTf_2$ | 20 | NaOAc (s) | 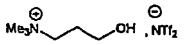 | 30,1 | 25,4 | 4,6 |
| 7 | $NTf_2$ | 20 | TEA[III] | 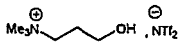 | 63,0 | 56,1 | 6,9 |
| 8 | $NTf_2$ | 20 | TEAS[IV] | 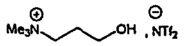 | 59,3 | 50,5 | 8,9 |
| 11 | $NTf_2$ | 80 | $K_2CO_3$ (s) | 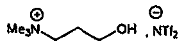 | 40,6 | 26,5 | 14,1 |

i : Produit d'homocouplage.
ii : le sel fonctionnalisé est utilisé pur et sert de matrice ionique.
iii : TEA : triéthylamine.
iv : TEAS : amine tertiaire de formule : $Me_3N^+(CH_2)_2CH_2NEt_2$ , $NTf_2^-$

Au cours de cette étude on a utilisé le bis-trifluorométhanesulfonamidure de N,N',N''-triméthyl-3-hydroxypropylammonium comme matrice. Par contre, dans le cas de l'utilisation du DMF comme solvant, le support ionique est utilisé pur, c'est à dire que le sel fonctionnalisé par le groupement 4-bromobenzoate est dissous dans le DMF pur. Au cours de cette étude on a aussi utilisé en plus des bases classiques la triéthylamine supportée sur un sel (TEAS) qui a été synthétisé par simple condensation de la diéthylamine sur le sel **(3c)** selon le schéma réactionnel suivant :

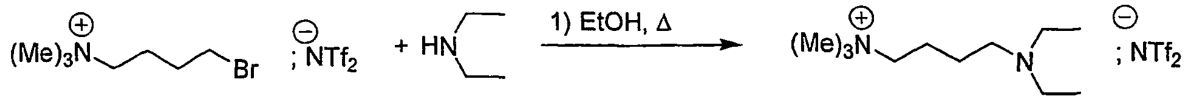


**3c** **24**

Afin d'avoir une étude comparative concluante réduit le temps des réactions a été réduit à 10 heures dans les essais effectués à température ambiante (essais 1 à 8), et à 5 heures pour ceux portés à 80°C (essai 11).

Les essais 1 à 4 montrent que la réactivité des sels fonctionnalisés (supports ioniques) dont l'anion est le tétrafluoroborate ou l'hexafluorophosphate est beaucoup plus importante que celle observée avec un anion chlorure ou bis-trifluorométhanesulfonamidure. Le fait que tous les supports ioniques soient dissous dans la même matrice prouve que, du point de vue du mécanisme, c'est la partite ionique du support qui intervient probablement au niveau du palladium. Cette réactivité observée est en plus accompagnée d'une très grande sélectivité dans le cas de l'anion du tétrafluoroborate.

En plus des résultats déjà observés lors de l'étude de l'effet de la matrice, les essais 1 à 4 montrent que le mélange de sels portant des cations ou anions différents ne réduit en rien ni la réactivité ni la sélectivité, ce qui offre un grand choix et permet de réduire le coût des cations et d'anions parfois onéreux : un chlorure coûte peut-être 50 fois moins cher, par exemple, qu'un triflimide.

On constate aussi que la présence d'eau dans le milieu augmente nettement la vitesse de la réaction. En effet, on passe de 28 à 64 % de taux de conversion en présence respectivement de $K_2CO_3$ solide et d'une solution de $K_2CO_3$ dans l'eau (essais 1 et 5), ce qui peut être expliqué par une meilleure homogénéité du milieu en présence d'eau. De même, l'emploi de la triéthylamine comme base permet d'observer une réactivité comparable à celle en présence d'eau (comparer essais 5 et 7). Le greffage de cette dernière sur un support ionique n'influence pas trop sa réactivité. En effet, le résultat obtenu lors de l'essai 8 est comparable à celui de l'essai 7, que ce soit en terme de réactivité ou en terme de sélectivité. Ceci représente un résultat majeur, sachant que le recyclage de cette forme de base est facile. En effet, un simple lavage par une solution basique permet de régénérer la TEAS. Ce qui réduira donc le coup de revient et en même temps les rejets nuisibles.

***3- Application en chimie combinatoire:***

Pour la préparation de cette banque d'esters biaryliques on a opéré comme dans le cas de l'exemple 1.

Nous avons dans un premier temps effectué une série de réactions de couplage en parallèle avec 9 acides arylboroniques et l'acide 4-bromobenzoique supporté. Ensuite, on a mélangé les 9 réactions pour former une solution homogène, qui est alors divisée en trois portions égales, après quoi chacune des solutions est solubilisée dans un alcool. On ajoute ensuite quelques gouttes d'acide chlorhydrique concentré (12 N) et on porte au reflux de l'alcool pendant 18 heures. Après évaporation de l'alcool, on extrait le mélange des biarylesters par l'éther. On obtient donc 3 séries de 9 esters qui sont alors analysées en GC/MS. Les différents biarylesters attendus sont tous identifiés sans ambiguïté.

Ci-dessous sont représentés tous les résultats sous forme de tableaux. Un chromatogramme correspondant au mélange des esters propyliques biaryliques est représenté ci-dessous.

***1/ Esters propyliques biaryliques:***

Le tableau XI ci-après correspond au chromatogramme de la Figure 7.

**TABLEAU XI**

| Bibliothèque des esters propyliques 23 | | |
|---|---|---|
| **R** | **Temps de rétention en minutes et secondes** | **Masse moléculaire** |
| 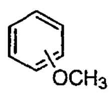 o et p | 26,42 ; 27,36 | 270 |
| 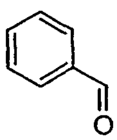 | 28,13 | 268 |
| 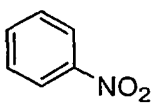 | 32,29 | 285 |
| 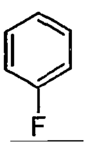 | 19,28 | 258 |
| 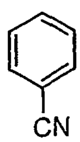 | 28,96 | 265 |
| 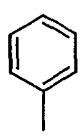 | 22,82 | 254 |
| 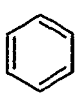 | 19,53 | 240 |
| 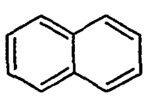 | 34,52 | 290 |

***2/ Esters méthyliques biaryliques :***

**TABLEAU XII**

| Bibliothèque des esters méthyliques | | |
|---|---|---|
| **R** | **Temps de rétention** | **Masse moléculaire** |
| 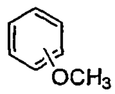 o et p | 21,31 ; 22,35 | 242 |
| 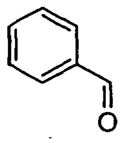 | 23,24 | 240 |
| 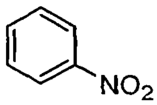 | 28,58 | 257 |
| 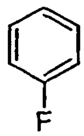 | 16,28 | 230 |
| 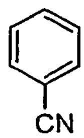 | 24,23 | 237 |
| 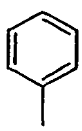 | 18,25 | 226 |
| 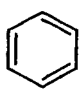 | 16,41 | 212 |
| 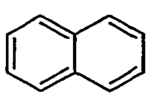 | 27,74 | 262 |

***3/ Esters éthyliques biaryliques :***

**TABLEAU XIII**

| Bibliothèque des esters éthyliques | | |
|---|---|---|
| **Ar** | **Temps de rétention** | **Masse moléculaire** |
| 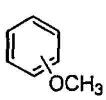 o et p | 23,76 ; 24,73 | 256 |

(suite)

| Bibliothèque des esters éthyliques | | |
|---|---|---|
| **Ar** | **Temps de rétention** | **Masse moléculaire** |
| 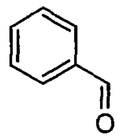 | 25,19 | 254 |
| 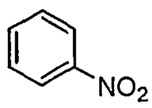 | 28,72 | 271 |
| 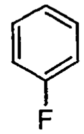 | 17,33 | 244 |
| 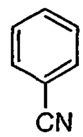 | 25,95 | 251 |
| 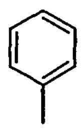 | 19,82 | 240 |
| 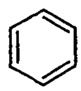 | 17,50 | 226 |
| 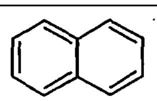 | 30,12 | 276 1 |

**B- Halogénure d'aryle supporté :**

Dans un deuxième temps, on a essayé de greffer un acide arylboronique sur un sel afin de l'engager ensuite dans une réaction de couplage de Suzuki. Cette étude peut être effectuée selon deux approches différentes :

- la première, est de supporter un acide arylboronique sur le cation du sel fonctionnalisé par la diéthanolamine pour former des boratranes (voir ci- dessous) :

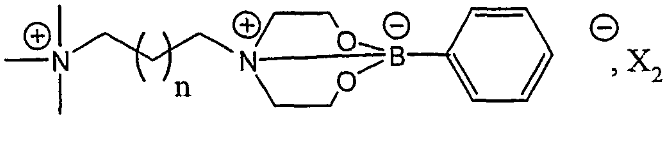

- la seconde est de le supporter via l'anion du sel fonctionnalisé. En effet, si l'anion $X_2^-$ du sel fonctionnalisé servant de support est assez nucléophile, il va réagir avec l'acide phénylboronique en quatemarisant l'atome de bore pour donner un borate :

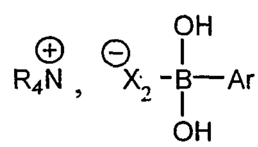

**I - Sel à cation fonctionnalisé :**

**1) Synthèse des sels fonctionnalisés :**

Les sels fonctionnalisés ont été synthétisés selon le schéma réactionnel suivant :

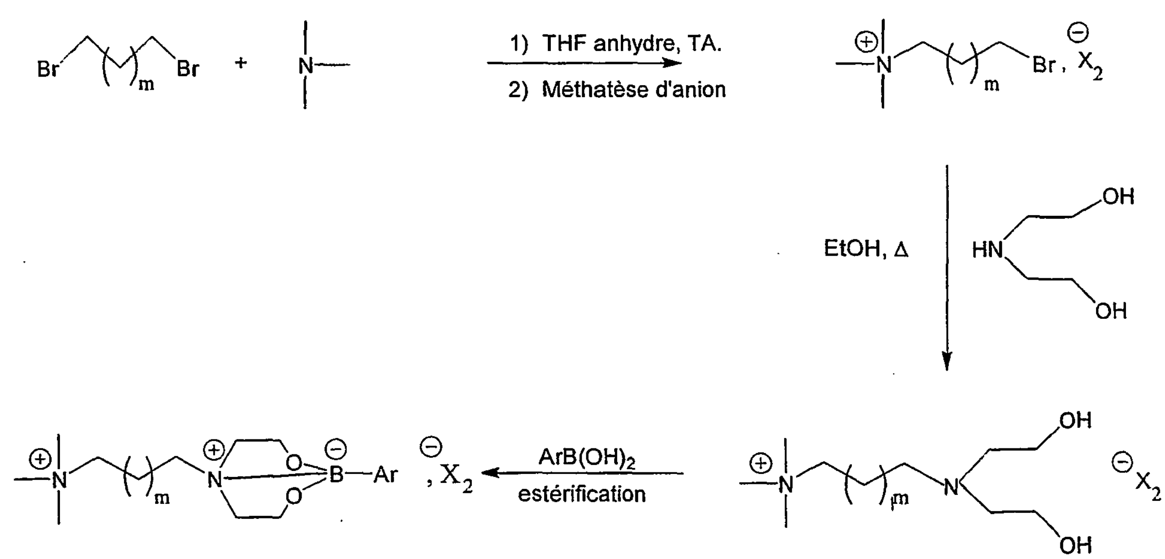


La réaction de condensation de la triméthylamine sur un dibromure d'alkyle est effectuée dans des conditions anhydres avec de très bons rendements (> 95%). Le bromure d'ammonium ainsi obtenu subit une réaction d'échange d'anions (métathèse) dans les conditions classiques. La deuxième étape a été effectuée en présence d'un équivalent de diéthanolamine avec un rendement quantitatif. De même, l'étape de greffage de l'acide arylboronique a été effectuée avec des bons rendements et permet d'obtenir un produit sous deux formes différentes. En effet, en fonction du solvant et de l'anion du support on obtient ou non un équilibre entre les bores tri et tétravalent. Ainsi, si on opère dans le THF et en présence d'un anion bis-trifluorométhanesulfonamidure l'équilibre obtenu est de 80/20 en faveur du bore tétravalent. En revanche, l'utilisation d'un mélange éther, chloroforme et isopropanol conduit exclusivement au bore tétravalent (boratrane). Dans les mêmes conditions et en présence de l'anion tétrafluoroborate, on obtient un équilibre 60/40 en faveur du bore trivalent, selon le schéma suivant :

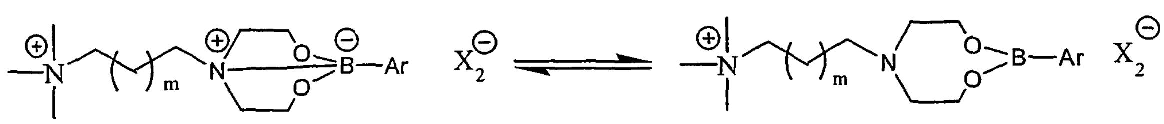

Bore tétravalent

Bore trivalent

Il faut noter que les déplacements chimiques du bore obtenus dépendent de la nature de l'anion. En effet, dans le cas du bis-trifluorométhanesulfonamidure le signal du bore tétravalent apparaît à 13,06 ppm. En revanche, avec l'anion tétrafluoroborate il apparaît à 4 ppm. Ceci montre que la nature du contre ion influe aussi sur la nature de la liaison entre les atomes d'azote et de bore, et donc sur le transfert d'électrons depuis l'azote vers le bore.

Ensuite, les boratranes synthétisés ont été engagés dans la réaction de couplage de Suzuki selon le schéma

réactionnel suivant :

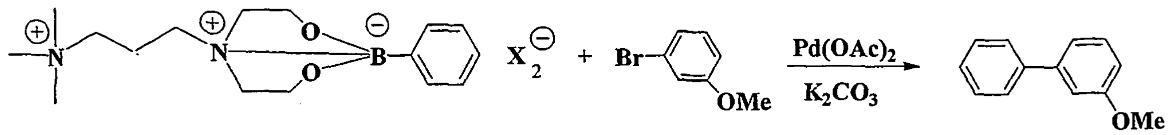


Solution 0.85 M dans $(Me)_3\overset{+}{N}Bu$, $\overset{-}{NTf_2}$

Une solution de boratrane (0,85 mol/l) dans le bis-trifluorométhanesulfonamidure de N,N',N"-triméthylbutylammonium servant de matrice est mise en contact avec le 1-bromo-3-méthoxybenzéne, en présence de $K_2CO_3$ solide comme base et d'acétate de palladium comme catalyseur. Le mélange est chauffé à 80°C pendant 18 heures. Les résultats obtenus sont regroupés dans le tableau XIV suivant :

**TABLEAU XIV**

| essai | **boratrane** | Taux de conversion | 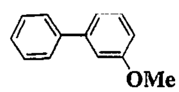 Rdt | 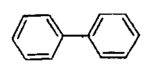 Homo-Couplage |
|---|---|---|---|---|
| 1 | 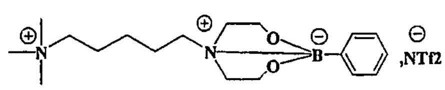 | 72% | 66,8% | 5,1% |
| 2 | 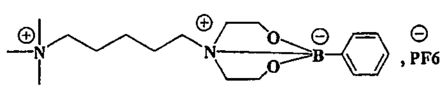 | 82% | 64,4% | 18,0% |
| 3 | 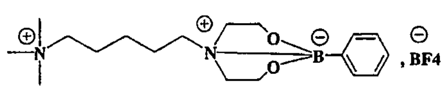 | 54% | 32,3% | 22,1% |

Les essais préliminaires qui ont été réalisés montrent que les acides boroniques supportés peuvent subir le couplage de Suzuki. Par contre, cette réaction nécessite un apport énergétique, car à température ambiante aucune réaction n'a été observée pour les différents substrats.

En revanche, à 80°C comme indiqué dans le tableau précédent la réaction a lieu et dépend de la nature du contre ion du support. On remarque aussi l'influence de ce dernier sur la sélectivité (essai 1 et 2). Il faut toutefois signaler que cette réactivité peut être influencée par la nature de l'équilibre existant entre les bores tri et tétravalent, ce qui peut expliquer la faible réactivité du boratrane possédant le tétrafluoroborate comme contre ion comparé à celle observée dans le cas où on est en présence d'un équilibre déplacé vers le bore tétravalent (cas du bis-trifluorométhane sulfonamidure).

**II - Sel à anion fonctionnalisé :**

Dans le cas précédent, on a fonctionnalisé la partie cationique du sel à tâche dédiée pour supporter un acide arylboronique sous forme d'un boratrane à atome de bore tétravalent. Ce dernier représente l'espèce intermédiaire dans la réaction de couplage de Suzuki. On peut aussi envisager l'utilisation de sels à anions nucléophiles ($OH^-$ et $F^-$) susceptibles de quaternariser l'atome de bore de l'acide arylboronique pour donner des borates, dérivés de bore tétravalent, intermédiaire dans la réaction de couplage de Suzuki. On a donc utilisé les sels d'ammonium commerciaux suivants :

- l'hydroxyde de tétrabutylammonium :

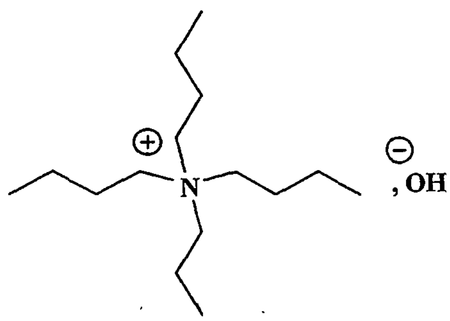

Ou $Bu_4N^+OH^-$
(TEBAOH)

- le fluorure de tétraméthylammonium :

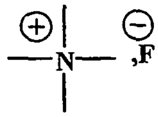

Ou $Me_4N^+F^-$
(TEMAF)

**1) Cas de $Bu_4N^+OH^-$ :**

La synthèse du substrat supporté est réalisée selon le schéma réactionnel suivant :

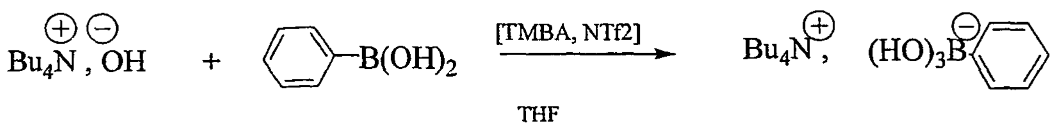


Le $Bu_4N^+OH^-$, séché sous vide après évaporation de l'eau, est solubilisé dans le bis-trifluorométhanesulfonamidure de TMBA servant de matrice pour donner une solution à 0,85 mol/l. A cette solution, on ajoute une quantité stoechiométrique d'acide phénylboronique en solution dans le THF anhydre à la température ambiante. Le suivi de la réaction par la RMN $^{11}B$ après évaporation du THF montre qu'elle est totale après deux heures. On observe alors un seul signal à 3,97 ppm correspondant à un borate.

***2) Cas de $Me_4N^+F$:***

De la même manière que dans le cas de l'hydroxyde, l'acide phénylboronique est quatemarisé par $Me4N^+F^-$ selon le schéma suivant :

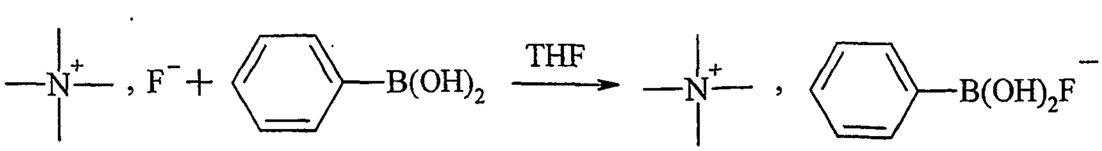


Pour cette réaction, on a solubilisé, à température ambiante, le sel dans le THF (anhydre) puis on a additionné l'acide phénylboronique. Après 18 heures d'agitation du mélange à température ambiante, le précipité qui se forme est filtré puis lavé à l'éther. Le rendement en produit isolé dépend de la quantité d'acide phénylboronique utilisé. En effet, en présence d'un excès de ce dernier on obtient 82% de rendement. En revanche un défaut le fait chuter à moins de 50%. Le suivi de cette réaction est effectué à l'aide de la RMN du Bore et du fluore. Le tableau XV ci-dessous regroupe les résultats obtenus :

**TABLEAU XV**

| essai | nb d'équivalent en acide boronique | Rdt(%) | **Analyses RMN** |
|---|---|---|---|
| **1** | 1 éq | 65% | ***Spectre RMN $^{11}$B :*** (Acétone), δ ppm : $B_4$ =4,66 ppm (60%) $B_3$ =28,5 ppm (40%) |
| | | | ***Spectre RMN$^{19}$F :*** (Acétone), δ ppm : -136,4 ppm |
| **2** | 0,75 éq | 48% | ***Spectre RMN $^{11}$B:*** (Acétone), δ ppm : $B_4$ =4,76 ppm (60%) $B_3$ =28,5 ppm (40%) |
| | | | ***Spectre RMN$^{19}$F:*** (Acétone), δ ppm :-136,05 ppm |
| **3** | 2 éq | 82% | ***Spectre RMN $^{11}$B :*** (Acétone), δ ppm : $B_4$ =4,4 ppm (60%) $B_3$ =28,5 ppm (40%) |

Dans les différents cas, la RMN du bore montre qu'on est en présence d'un équilibre entre les formes tri et tétravalente et en faveur de cette dernière (Schéma)

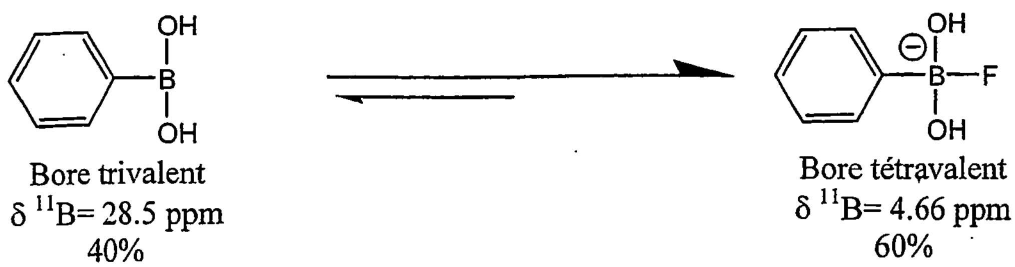


***a/- Réactions de couplage de Suzuki avec [TEMA][PhB(OH)$_2$F]***:

Le protocole est le même que celui utilisé dans la partie précédente et se fait selon le schéma réactionnel suivant :

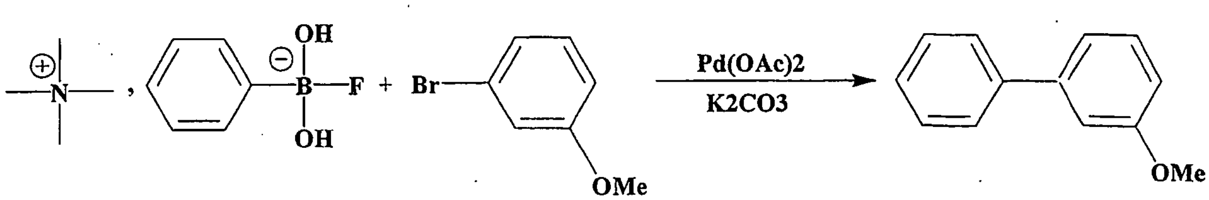


La réaction a été effectuée à deux températures distinctes pour étudier l'effet de la température aussi bien sur la réactivité, que sur la sélectivité. Voir tableau XVI ci-après :

**TABLEAU XVI**

| essai | T˚C | Taux de conversion en (%) | 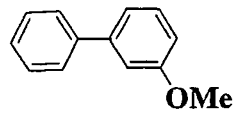 Rdt | 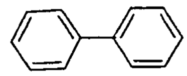 Rdt |
|---|---|---|---|---|
| 1 | TA | 92,4% | 86,3% | 5,7% |
| 2 | 80˚C | 96% | 77,7% | 18,3% |

On remarque que le rendement obtenu à 80˚C est voisin de celui obtenu à la température ambiante. Par contre, le pourcentage en produit d'homocouplage est moins important à température ambiante qu'à 80˚C (essais 1 et 2).

***b-/ Réaction de couplage avec [TEBA][PhB((OH)$_3$]:***

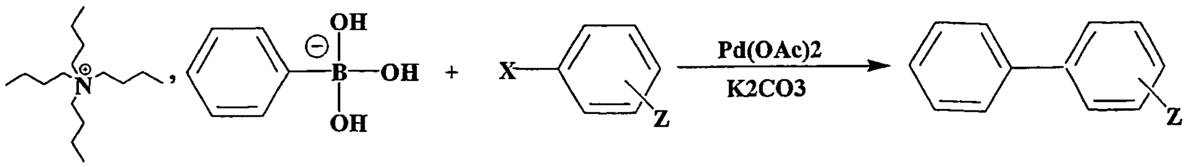


Comme dans les cas précédents, on a utilisé le même protocole et on a mis au point les meilleures conditions opératoires pour pouvoir lancer un test en chimie combinatoire.

Pour cela, on a testé l'influence de plusieurs paramètres qui sont la température et l'ajout d'un solvant tel que le DMF.

Les résultats sont regroupés dans le tableau XVII suivant :

TABLEAU XVII

| essai | Aryle | Taux de conversion | **T (˚C)** | **solvant** | 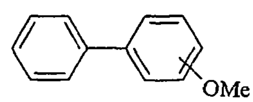 Rdt | 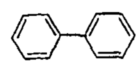 Rdt |
|---|---|---|---|---|---|---|
| 1 | 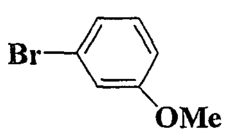 | 51 % | 40˚C | [TMBA][X$^-$] | 44,2% | 6,4% |
| 5 | 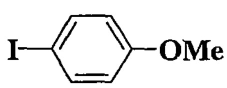 | 66% | TA | [TMBA] [X$^-$] | 55,4% | 10,2% |

**EXEMPLE 5 : UTILISATION DE LA COMBINAISON [MATRICE + SEL FONCTIONNALISE] SOLIDE A LA TEMPERATURE AMBIANTE**

Dans les différents exemples que nous avons étudiés jusqu'à présent, seules les matrices ioniques liquides à température ambiante ont été utilisées. Cependant, on a testé et montré la possibilité d'employer une matrice solide à température ambiante.

En effet, l'utilisation d'un liquide ionique dont la température de fusion est supérieure à l'ambiante, diversifie

les choix de matrice et élargit le champ d'application du principe SOSLI. Les matrices solides à température ambiante peuvent avoir un intérêt très important supplémentaire : la cristallisation du milieu rend plus simple la récupération aussi bien de l'excès des réactifs que des produits de réaction. Le choix de la matrice solide peut être effectué selon les critères suivants :

- en l'absence de solvant organique classique, le point de fusion du mélange sel ionique fonctionnalisé / matrice ionique doit être inférieur à la température de la réaction,
- en présence d'un solvant organique classique, une matrice à tache dédiée (matrice ionique + sel fonctionnalisé) doit être de préférence soluble dans le solvant.

Pour illustrer cet exemple on a réalisé l'essai de couplage de Heck en utilisant une matrice solide à température ambiante, selon le schéma suivant :

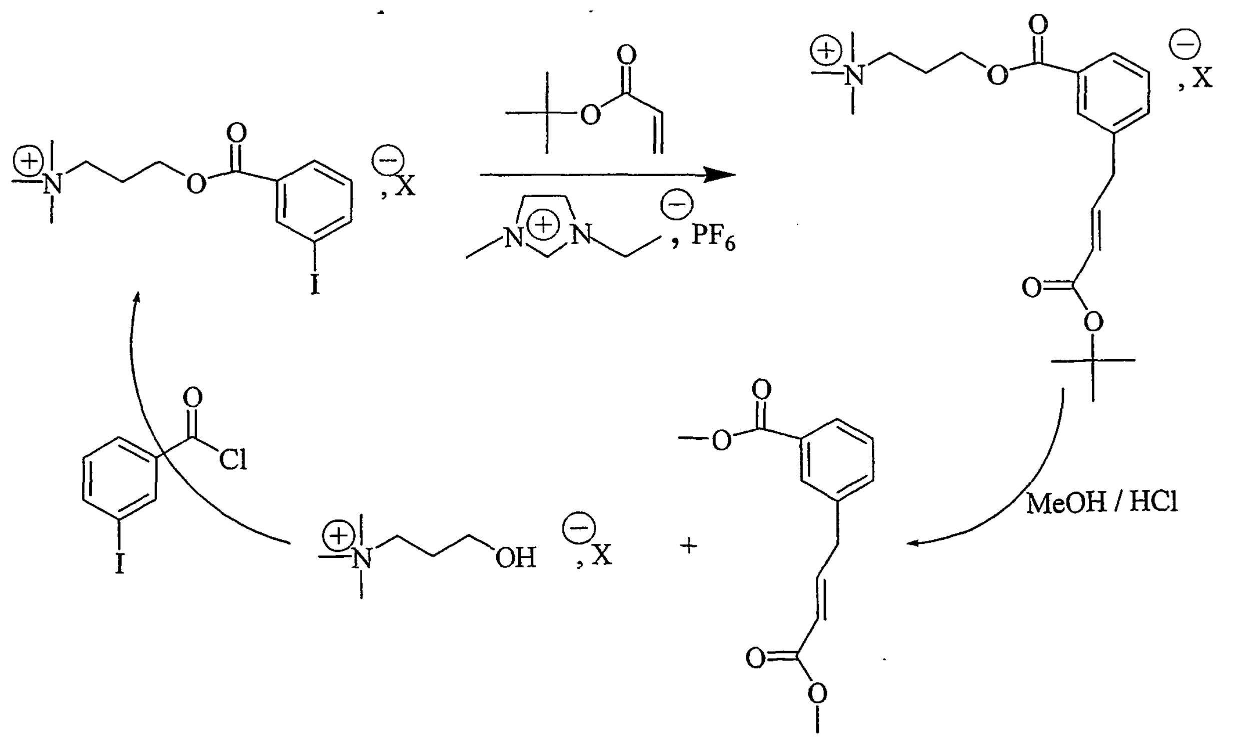


Dans cet essai on a effectué le couplage de Heck en utilisant l'iodure d'aryle supporté comme substrat et l'acrylate de tertiobutyle comme alcène dans les mêmes conditions que celles décrites lors des exemples où on a utilisé des matrices ioniques liquides à température ambiante.

Dans ce cas on a utilisé l'hexafluorophosphate de N-méthyl, N'-éthylimidazolium (EMIM, $PF_6$) comme matrice ionique, dont le point de fusion est de l'ordre de 56°C.

**Procédure:**

1 mmol de sel ionique fonctionnalisé (**7a**) est mélangé à 1g de la matrice ionique et chauffé à 70°C pour obtenir une solution. Au refroidissement, ce mélange homogène est solide à température ambiante. En chauffant de nouveau et à partir de 65°C, le milieu redevient liquide et parfaitement homogène. A cette solution et comme pour les différents testes cités auparavant, on ajoute la base et le catalyseur, puis on chauffe à 80°C. Après 5 heures la RMN $^1H$ du mélange montre une disparition totale de l'iodure de départ.

Le mélange réactionnel est refroidi à température ambiante et on obtient un mélange hétérogène (solide/liquide). On ajoute ensuite de l'éther et on filtre le solide, qui est relavé ensuite pour extraire la totalité d'acrylate. Le produit est ensuite libéré du sel fonctionnalisé par transestérification selon la procédure décrite lors des différents exemples cités auparavant. Après élimination sous vide du méthanol en excès, l'ester cinnamique est isolé par ajout d'éther et filtration du mélange solide constitué du sel fonctionnalisé et de la matrice solide de départ qui peut être réutilisé.

En conclusion cet essai montre que l'application du principe SOSLI à des matrices ioniques à tâche dédiée

solide à température ambiante est parfaitement possible, et permet par conséquent d'élargir le choix de la nature de l'anion. En outre, ce système de mélange solide à température ambiante ouvre de nouveaux horizons et permettra en plus d'adapter facilement le principe SOSLI à toutes les technologies déjà mises au point dans le cas des supports solides.

On notera la très grande diversité de sels d'onium facilement accessibles et plus particulièrement des sels de phosphonium, d'ammonium, de pyridinium et d'imidazolium.

**EXEMPLE 6 : COUPLAGE DE SONOGASHIRA.**

Un autre exemple de couplage où le principe SOSLI a été testé est celui de Sonogashira qui consiste en un couplage d'un halogénure d'aryle et d'un alcyne vrai.

Cette étude a été réalisée en supportant l'acide 4-iodobenzoïque sur un sel à tâche dédiée. Plusieurs tests sont effectués dans le but de déterminer l'influence de la nature de l'anion du support et celle de la matrice sur cette réaction de couplage (voir schéma ci-dessous).

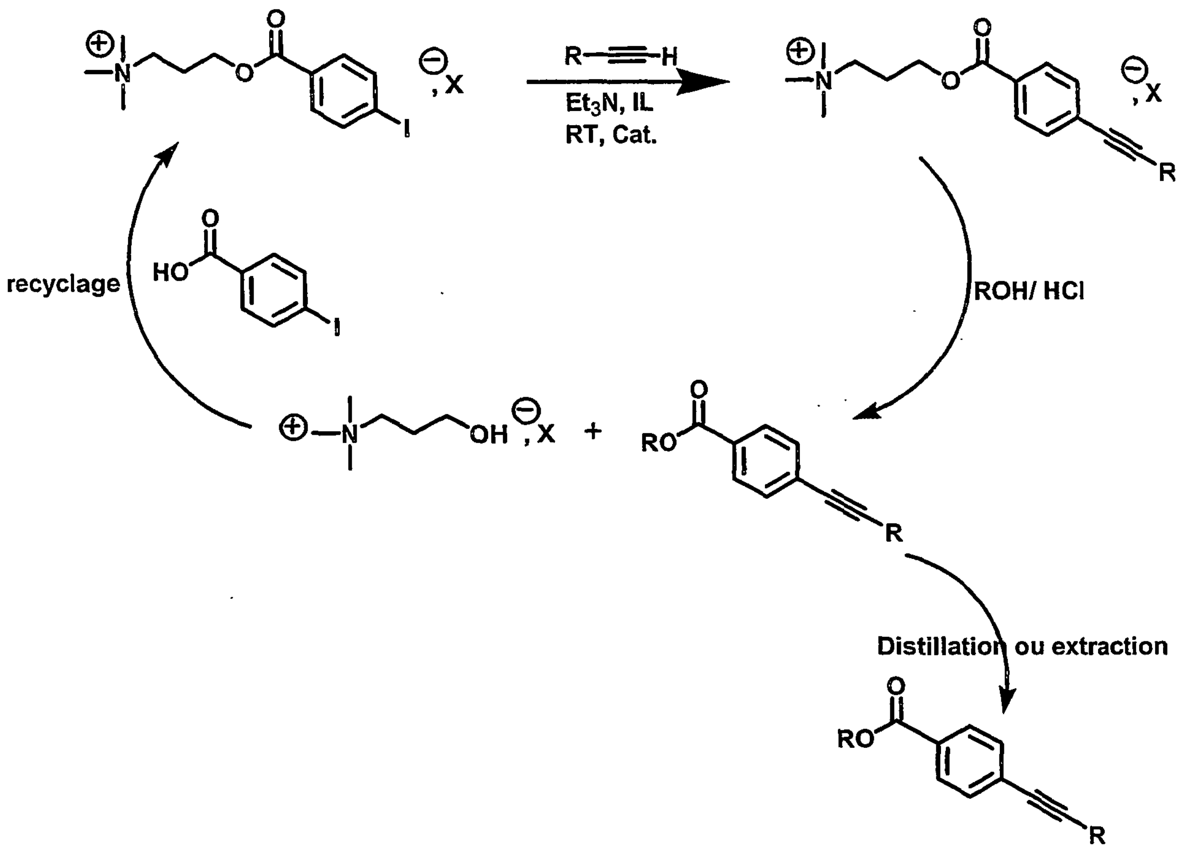


Les résultats regroupés dans le tableau XIX ci-dessous ont été déterminés grâce au suivi des réactions par RMN$^1$H.

**Tableau XIX**

| Entrée | X | R | matrice | Temps (h) | Conv.(%) | Rdt (%) |
|---|---|---|---|---|---|---|
| 1 | OTf | Ph | [tmba][$NTf_2$] | 1 | 75 | 72 |
| 2 | $NTf_2$ | Ph | [tmba][$NTf_2$] | 1 | 78 | 77 |
| 3 | $NTf_2$ | Ph | [bmim][$BF_4$] | 1 | 70 | 66 |
| 4 | $NTf_2$ | Ph | [tmba][$NTf_2$] | 2 | 100 | 95 |
| 5 | $NTf_2$ | $CH_3OCH_2$ | [tmba][$NTf_2$] | 2 | 92 | 92 |
| 6 | $NTf_2$ | $CH_3(CH_2)_3$ | [tmba][$NTf_2$] | 2 | 100 | 98 |
| 7 | $NTf_2$ | $CH_3(CH_2)_4$ | [tmba][$NTf_2$] | 2 | 94 | 90 |

(suite)

| Entrée | X | R | matrice | Temps (h) | Conv.(%) | Rdt (%) |
|---|---|---|---|---|---|---|
| 8 | $NTf_2$ | $CH_3(CH_2)_6$ | [tmba][$NTf_2$] | 2 | 93 | 88 |

[tmba] = [$Me_3N^+$-Bu]

[bmim] = butylméthylimidazolium 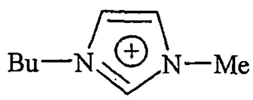

Les résultats qui figurent sur le tableau montrent que la nature de l'anion du support et de la matrice n'influence pas trop la vitesse de réaction et les rendements en produit isolé. La figure 11 illustre la simplicité du suivi de réaction par RMN du proton (Cas de R = Ph).

Le clivage des produits après couplage a été réalisé par deux transestérifications, une par le méthanol, l'autre par l'éthanol. Pour cela les produits des essais 4, 5, 6, 7 et 8 sont mélangés et mis en réaction selon le principe « Split and mix » en présence de l'alcool et d'une quantité catalytique de l'acide chlorhydrique. Cette étape permet également le recyclage du sel à tache dédiée ainsi que celui de la matrice utilisée pour la réaction.

Après 12 heures les deux réactions sont totales, les produits sont isolés par extraction à l'éther puis injectés en GC/MS. Dans les deux cas les quatre produits ont été détectés.

***1/ Esters méthyliques:***

Le tableau XX ci-après correspond au chromatogramme de la Figure 12.

**Tableau XX**

| Bibliothèque des esters méthyliques | | |
|---|---|---|
| **R** | **Temps de rétention en minutes** | **Masse moléculaire** |
| 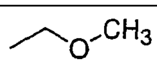 | 14,57 | 204 |
| 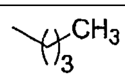 | 15,35 | 216 |
| 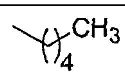 | 16,56 | 230 |
| 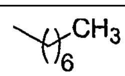 | 20,53 | 236 |
| 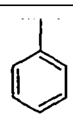 | 21,16 | 258 |

***2/ Esters éthyliques :***

Le tableau XXI ci-après correspond au chromatogramme de la Figure 13.

**Tableau XXI**

| Bibliothèque des esters éthyliques | | |
|---|---|---|
| **R** | **Temps de rétention en minutes** | **Masse moléculaire** |
| 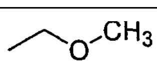 | 15,24 | 218 |
| 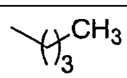 | 16,18 | 230 |

(suite)

| Bibliothèque des esters éthyliques | | |
|---|---|---|
| **R** | **Temps de rétention en minutes** | **Masse moléculaire** |
| 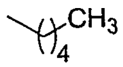 | 17,68 | 244 |
| 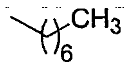 | 22,77 | 250 |
| 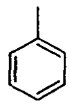 | 23,57 | 272 |

**Mode opératoire :**

A un mélange constitué d'une solution 0,85 M de [3-(4-iodobenzoyloxy)-propyl]-triméthylammonium (100 mg ; 0,156 mmol) dans un liquide ionique (matrice) et de la triéthylamine (0,92 ml), on ajoute l'alcyne (0,64 mmol) et l'iodure de cuivre (0,8 mg ; 0,04 mmol), on agite à température ambiante pendant 5 minutes puis on ajoute le complexe de palladium $PdCl_2(Ph_3P)_2$ (1,4 mg ; 0,02 mmol). On laisse sous agitation à température ambiante pendant 2 heures. Le milieu est ensuite lavé plusieurs fois à l'éther et séché sous vide.

**EXEMPLE 7 : RÉACTIONS MULTI-COMPOSANTS (RMC)**

Les réactions multi-composants mettent en présence simultanément au moins trois partenaires de réaction dans des conditions expérimentales qui ne varient pas au cours du temps et permettent la création de plusieurs liaisons covalentes en une seul étape, à la différence des réactions classiques ou deux réactifs conduisent à un produit par création de nouvelles liaisons. Ainsi il est possible d'accéder en une seule étape à une molécule hautement fonctionnalisée à partir d'entités relativement simples. De plus les RMC allient convergence et économie d'atomes, deux principes fondamentaux en synthèse organique importants pour la chimie combinatoire. Signalons enfin que ces réactions ont généralement lieu avec un rendement élevé, puisqu'elles évitent la succession d'étapes qui, font, à chaque pas, chuter le rendement.

Les RMC les plus connues et les plus développées sont celles de Passérini et de Ugi (Ugi, 1976). Un des éléments clefs de ces réactions est un isonitrile, dont la structure électronique, comptant un doublet et une lacune électronique, permet le passage d'un atome de carbone formellement divalent à un atome de carbone tétravalent par addition d'un électrophile et d'un nucléophile. Le schéma ci-dessous présente un exemple de réaction de Passérini.

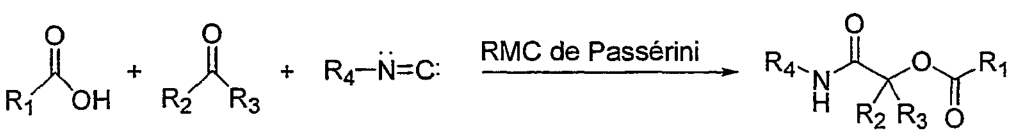


α-acyloxyamide

Bien sûr les RMC ont été transposées sur support solide, par exemple une résine à terminaison amine a été engagée dans une réaction de type Ugi pour conduire après clivage à une série d'adduits d'une grande pureté avec des rendements s'échelonnant de moyens à excellents (voir schéma ci-dessous)(Lhoel et Nielsen, 1999).

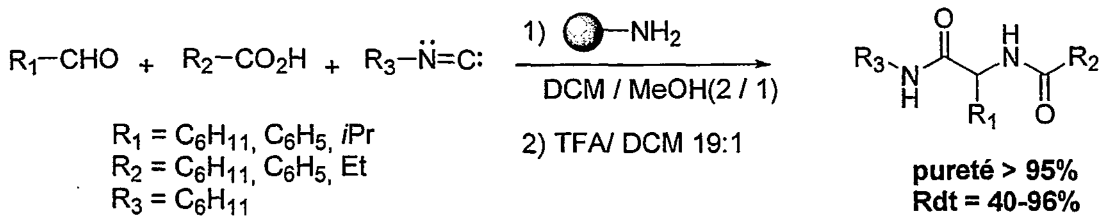


Bien que les réactions de Ugi et Passérini soient les plus connues et les plus développées, il existe d'autres RMC, qui répondent au critère essentiel à savoir : tous les réactifs sont présents dès le début de la réaction et les conditions ne varient pas au cours de celles-ci. A la différence des réactions de Ugi et Passérini, ces autres réactions ne reposent pas sur l'utilisation d'un isonitrile comme une des acteurs centraux de la création de nouvelles liaisons covalentes. Ces différents types de réactions permettent d'accéder à des structures hautement fonctionnalisées variées en une seule étape.

Les quinoléines substituées sont des pharmacophores intéressants. Leur synthèse sur support solide a été réalisée par une RMC dite de Doebner, mettant en jeu une aniline, un aldéhyde et un composé α-dicarbonylé (voir schéma ci-après)(Gopalsamy et Pallai, 1997). Les quinoléines sont obtenues avec une grande pureté et de très bons rendements.

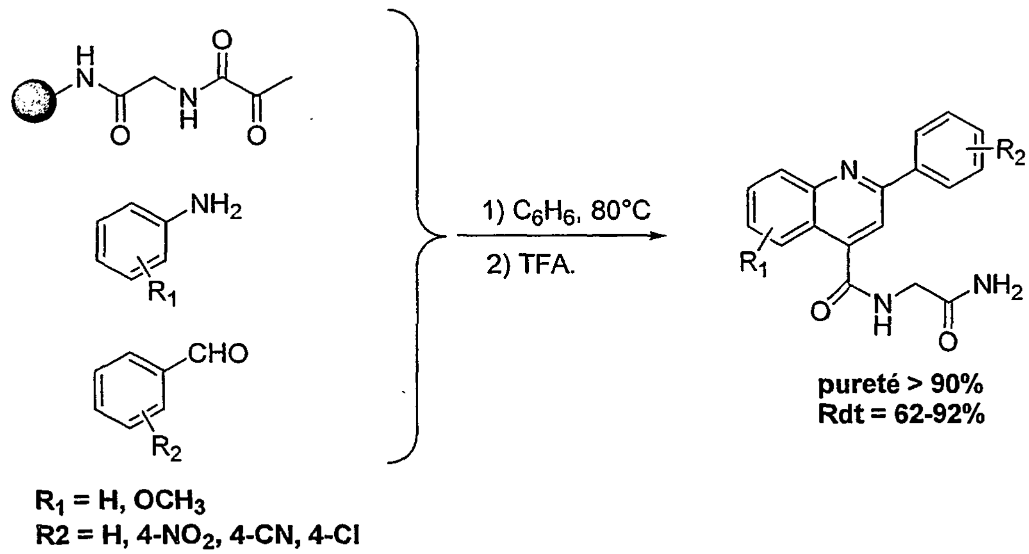


Le principe de la présente invention a été appliqué dans le cadre des RMC de type Grieco (Grieco et Bahas, 1988). Cet exemple a fait l'objet de plusieurs travaux décrits par W. Armstrong *et al.* (Kiselyov et al., 1998) sur support solide et il a permis la préparation d'une librairie de 80 produits avec des rendements allant de 50 à 93%.

Pour ce faire, l'aniline 1 a été supportée et mise en jeu avec un aldéhyde et le cyclopentadiène en présence de butyltriméthylammoniumtriflimide comme matrice pour conduire à des tétrahydroquinoléines (voir schéma ci-après). Cet exemple à trois composants, consiste en une première condensation de l'aldéhyde et de l'aniline pour conduire à l'imine. Celle-ci réagit ensuite dans ce qui est formellement une réaction de Diels-Alder avec le cyclopentadiène en présence d'une quantité catalytique d'acide trifluoroacétique.

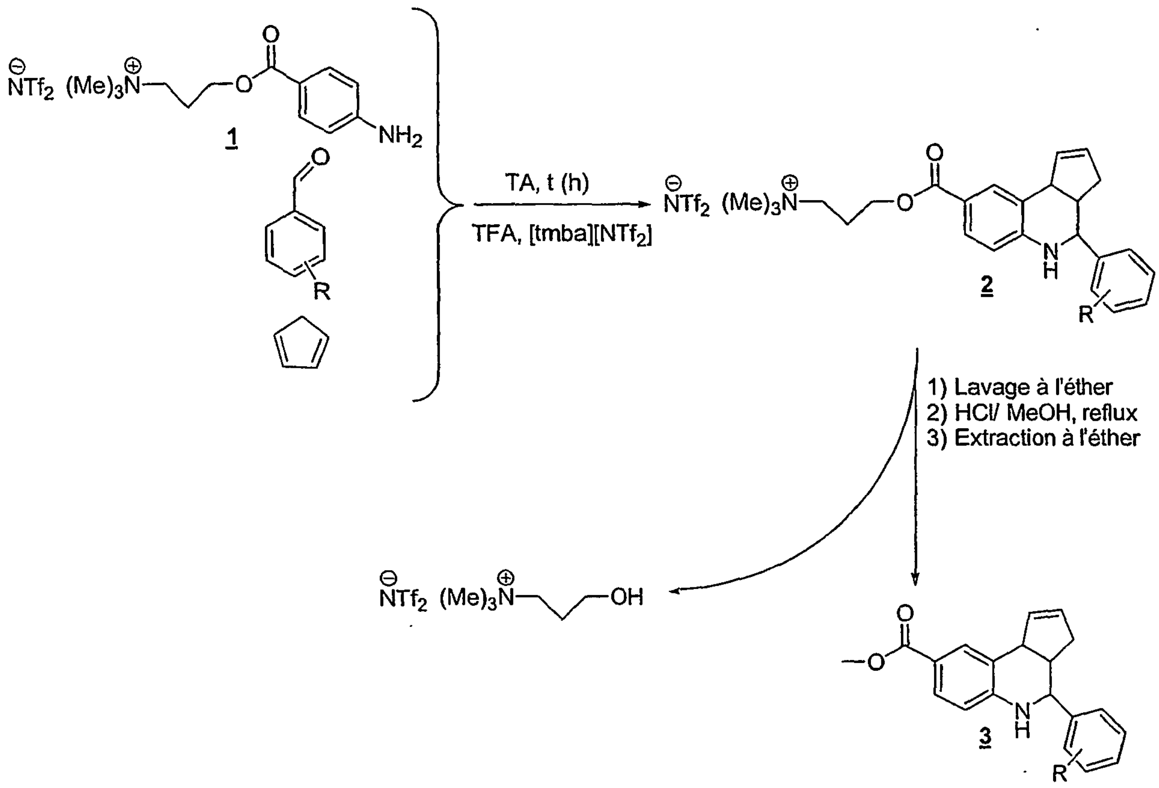


**Avec R =4-$NO_2$, H, 4-Cl, 2-MeO**

Le suivi des différentes réactions a été effectué par RMN $^1$H et nous avons observé une conversion allant de 80% à 100% selon la nature de l'aldéhyde. Ainsi, en présence du 4-nitrobenzaldéhyde, la réaction est totale au bout de 30 minutes alors qu'elle ne l'est qu'à 75% dans le cas du 4-méthoxy-benzaldéhyde (aldéhyde riche en électrons). La figure 10 illustre le cas du 4-nitrobenzaldéhyde, après lavage à l'éther pour éliminer l'excès des deux réactifs et de l'acide trifluoroacétique. Cette figure montre aussi que le suivi d'une réaction qui conduit à des composés complexes est possible et d'une clarté remarquable. Il faut aussi noter que la transestérification par le méthanol conduit à des produits très propres qui sont extraits à l'éther et purifiés par filtration sur silice.

Les différents exemples réalisés et les résultats obtenus sont regroupés dans le tableau XVIII ci-dessous.

**Tableau XVIII**

| R | matrice | temps de réaction (min) | taux de conversion (%) |
|---|---|---|---|
| H | [tmba] [$NTf_2$] | 30 | 100 |
| H | [$C_3$OHtma][$NTf_2$] | 30 | 100 |
| $NO_2$ | [tmba][$NTf_2$] | 30 | 100 |
| Cl | [tmba] [$NTf_2$] | 30 | 100 |
| Br | [tmba] [$NTf_2$] | 30 | 100 |
| OMe | [tmba] [$NTf_2$] | 75 | 82 |

[tmba] = [$Me_3N^+$-Bu]
[$C_3$OHtma] = [$Me_3N^+$-$(CH_2)_2CH_2OH$]

Au cours de ce travail aucun effet de la nature de matrice n'a été observé. En revanche, la présence de celle-ci influence nettement la vitesse de la réaction et permet une conversion totale en moins d'une heure, alors que, dans le cas des travaux décrits dans la littérature sur support solide, ce taux de conversion n'est obtenu qu'après 12 heures.

Mode opératoire de la réaction de Grieco :

Une solution 0,85 M de [3-(4-ammobenzoyloxy)-propyl]-triméthylammonium (100 mg ; 0,2 mmol) dans un

liquide ionique (matrice) est placée sous vide puis sous argon. L'aldéhyde (0,5 mmol), le cyclopentadiène (132 mg, 2 mmol) et l'acide trifluoroacétique TFA (20 µl; 0,27 mmol) sont ajoutés. Le mélange est agité à température ambiante. A la fin de la réaction, le milieu est lavé plusieurs fois à l'éther et séché sous vide.

L'huile visqueuse obtenue est ensuite dissoute dans le méthanol et portée à reflux en présence de 3 gouttes d'acide chlorhydrique concentré. Après 12 heures le produit est extrait par l'éther (2 × 30 ml) après évaporation du méthanol.

**RÉFÉRENCES BIBLIOGRAPHIQUES**

- Abbott P.; Capper, G.; Davies, L.; Rasheed, R. K.; Tambyrajah, V. *W00226701,2002,*
- Bayer, E. (1991) *Angew. Chem., Int. Ed. Engl.,* **30**, 113-129,
- Charken I. M. and Janda K. D. (1996) "Molecular Diversity and Combinatorial Chemistry" American Chemical Society, Washington, DC.,
- Dôrwald (2000) Organic synthesis on solid phase, Wiley-VCH, Weinheim,
- Fraga-Dubreuil, J.; Bazureau, J. P. (2001) *Tetrahedron Letters,* **42**(35), 6097-6100,
- Gopalsamy, A.; Pallai, P. V. (1997) *Tetrahedron Lett.,* **38**, 907,
- Gravet, D. J.; Janda, K. D. (1997) *Chem. Rev.,* **97**, 489-5 10,
- Grieco, A.; Bahas (1988) *Tetrahedron Lett.,* **29,** 5855,
- Howarth, J.; Dallas, A. (2000) *molecules,* **5**,851-855,
- Jeffery, Tuyet. (1996) *Tetrahedron,* 52*(30),*
- Kiselyov, A. S.; Smith II, L.; Virgilio, A.; Amstrong, R. W. (1998) *Tetrahedron,* **54,** 7987,
- Kiselyov, A. S.; Smith II, L.; Amstrong, R. W. (1998) *Tetrahedron,* **54,** 5089,
- Lhoel, A. M.; Nielsen, J. (1999) *Tetrahedron Lett.,* **40, 3941,**
- Murphy, Vince; Hagemeyer, Alfred; Poojary, Damodara M. *WO0032572,* 2000,
- O'Brecht, D.; Villalgordo, J.M. (1998) "Supported combinatorial and parallel synthesis of small-molecular weight compound libraries", *Tetrahedron Organic Chemistry Pergamon,* volume **17,**
- P. Wentworth, Jr. K. D. Janda, (1999) *Chem Comm.,* 1917-24,
- Sammelson, R. E.;. Kurth, M. (2001) J. *Chem. Rev.,* **101**, 137-202,
- Thompson, L. A.; Elhman, J. A. (1996) *Chem. Rev.,* **96,** 555-600,
- Ugi, I. (1976) "Isonitrile", *Academic Press, Inc.,* pp 133-199,
- Visser, A. E.; Swatloski, R. P.; Reichert, W. M. ; Davis, James H., Jr.; Rogers, R. D.; Mayton, R.; Sheff, S.; Wierzbicki, A. (2001) *Chemical Communications* (2001), (1), 135-136.
- Visser, A.E.; Swatloski, R. P.; Reichert, W. M.; Mayton, Rebecca; Sheff, Sean; Wierzbicki, Andrzej; Davis, James H. Jr.; Rogers, Robin D. (2002) *Environmental Science and Technology,* **36**(11), 2523-2529,
- Wasserscheid, P.; Keim, W. (2000) *Ang. Chem. Int. Ed.,* **39**, 3772-3789,
- Welton, T. (1999) *Chem. Rev.,* **99,** 2071-2083,
- Wilson, S. R. ; Czarnik, A. W. (1997) "Combinatorial Chemistry : Synthesis and Application" ; John Wiley & Sons New York,
- Xiao. J. ; Chen W. ; Xu L. (2000) *Organornetallics,* **19**, 1123-1127.


**Revendications**

1. Utilisation d'un liquide ionique, comme matrice liquide pour la synthèse organique en phase homogène sur support soluble, sans solvant organique volatil, ledit liquide ionique se présentant sous forme liquide ou solide à température ambiante, de formule $A_1^+X_1^-$, $A_1^+$ représentant un cation, fonctionnel ou non, ou un mélange de cations dans lequel soit aucun des cations n'est fonctionnel soit l'un au moins des cations est fonctionnel, et $X_1^-$ un anion, fonctionnel ou non, ou un mélange d'anions dans lequel soit aucun des anions n'est fonctionnel soit l'un au moins des anions est fonctionnel.

2. Utilisation selon la revendication 1, **caractérisée en ce que** $A_1^+$ représente un cation non fonctionnel ou un mélange de cations non fonctionnels et $X_1^-$ un anion non fonctionnel ou un mélange d'anions non fonctionnels.

3. Utilisation selon la revendication 1, **caractérisée en ce que**
$A_1^+$ représente un cation fonctionnel ou un mélange de cations dont l'un au moins est fonctionnel,
et/ou $X_1^-$ représente un anion fonctionnel ou un mélange d'anions dont l'un au moins est fonctionnel,

lesdits cations fonctionnels et anions fonctionnels correspondant à une entité ionique, à savoir respectivement cationique ou anionique, liée à au moins une fonction $F_i$, $F_i$ variant de $F_0$ à $F_n$, n étant un nombre entier variant de 1 à 10.

**4.** Utilisation d'un liquide ionique selon l'une des revendications 1 ou 2, pour la préparation d'une composition stable contenant en solution :

- au moins ledit liquide ionique de formule $A_1^+X_1^-$, jouant le rôle de matrice liquide et,
- au moins un sel fonctionnalisé (sel à tâche dédiée), notamment sel d'onium fonctionnalisé, de formule $A_2^+X_2^-$, comme support de réaction,

le sel fonctionnalisé, notamment le sel d'onium fonctionnalisé, étant dissous dans la matrice liquide, pour former une phase homogène,
$A_1^+$ représentant un cation non fonctionnel ou un mélange de cations dans lequel aucun des cations n'est fonctionnel, et $X_1^-$ représentant un anion non fonctionnel ou un mélange d'anions dans lequel aucun des anions n'est fonctionnel, $A_2^+$ représentant un cation, fonctionnel ou non, ou un mélange de cations dans lequel aucun des cations n'est fonctionnel ou dans lequel l'un au moins des cations est fonctionnel, et $X_2^-$ représentant un anion, fonctionnel ou non, ou un mélange d'anions dans lequel aucun des anions n'est fonctionnel ou dans lequel l'un au moins des anions est fonctionnel,
sous réserve que $A_2^+$ et/ou $X_2^-$ représente(nt) ou comporte(nt) respectivement un cation fonctionnel et un anion fonctionnel,
lesdits cations fonctionnels et anions fonctionnels correspondant à une entité ionique $Y^-$, à savoir respectivement cationique $Y^+$- ou anionique $Y^-$-, liée, éventuellement par l'intermédiaire d'un bras L, notamment un groupe alkyle comprenant de 1 à 20 atomes de carbone, à au moins une fonction $F_i$, $F_i$ variant de $F_0$ à $F_n$, n étant un nombre entier variant de 1 à 10, le cation fonctionnel pouvant être représenté sous la forme $Y^+$-L-$F_i$, et l'anion fonctionnel sous la forme $Y^-$-$(L)_k$-$F_i$, k étant égal à 0 ou 1, et l'anion fonctionnel pouvant représenter, lorsque k est égal à 0, un anion simple, correspondant à $Y^-$-$F_i$, notamment choisi parmi : $OH^-$, $F^-$, $CN^-$, RO- ou $RS^-$, R représentant un groupement alkyle comprenant de 1 à 20 atomes de carbone ou un groupement aryle comprenant de 6 à 30 atomes de carbone.

**5.** Utilisation selon la revendication 3, pour la préparation d'une composition stable contenant en solution :

- au moins une première partie dudit liquide ionique de formule $A_1^+X_1^-$, dont le cation et/ou l'anion correspond (ent) à une entité ionique liée à une ou des fonctions initiales $F_0$, jouant le rôle de matrice liquide, et
- au moins une deuxième partie dudit liquide ionique de formule $A_1^+X_1^-$, dans laquelle ladite ou lesdites fonctions initiales $F_0$ sont transformées en de premières nouvelles fonctions, conférant à ladite deuxième partie dudit liquide ionique le rôle de sel fonctionnalisé et de support de réaction,

le sel fonctionnalisé et la matrice liquide formant une phase homogène,
les susdites premières nouvelles fonctions de la deuxième partie dudit liquide ionique étant susceptibles d'être transformées ultérieurement en d'autres fonctions, sans qu'il y ait affectation de la ou des fonctions initiales $F_0$ de la première partie dudit liquide ionique.

**6.** Utilisation d'un liquide ionique selon la revendication 4, **caractérisé en ce que** le cation $A_2^+$ et/ou l'anion $X_2^-$ du ou des sels fonctionnalisés, correspondant à une entité ionique Y- liée à au moins une fonction $F_i$, sont immobilisés dans la matrice liquide et ne peuvent pas être extraits de la matrice liquide par des moyens conventionnels d'extraction, notamment par solvant, et dans laquelle la(les) fonction(s) $F_i$ du ou des sels fonctionnalisés peuvent être transformée(s) à l'issue d'au moins une réaction résultant de l'addition d'au moins un réactif dans la susdite composition.

**7.** Utilisation d'un liquide ionique selon la revendication 6, **caractérisé en ce que** plusieurs sels fonctionnalisés sont immobilisés.

**8.** Utilisation selon l'une des revendications 4, 6 ou 7, **caractérisée en ce que** le cation $A_2^+$ est fonctionnel.

**9.** Utilisation selon l'une des revendications 4, 6 ou 7, **caractérisée en ce que** l'anion $X_2^-$ est fonctionnel.

**10.** Utilisation selon l'une des revendications 4, 6 ou 7 à 9, **caractérisée en ce que** $A_2^+$ et $X_2^-$ sont fonctionnels.

**11.** Utilisation selon l'une des revendications 1, 2, 4, 6 à 10, **caractérisée en ce que**:

- soit le liquide ionique de formule $A_1^+X_1^-$ est solide à température ambiante et est liquéfiable dans une gamme de températures allant d'environ 25°C à environ 250°C, notamment d'environ 30°C à environ 150°C, et le sel fonctionnalisé $A_2^+X_2^-$ est solide à température ambiante et est soluble dans le liquide ionique $A_1^+X_1^-$ liquéfié, pour former une phase homogène,
- soit le liquide ionique de formule $A_1^+X_1^-$ est solide à température ambiante et est liquéfiable dans une gamme de températures allant d'environ 25°C à environ 250°C, notamment d'environ 30°C à environ 150°C, et le sel fonctionnalisé $A_2^+X_2^-$ est liquide à température ambiante, et est miscible avec le liquide ionique $A_1^+X_1^-$ liquéfié pour former une phase homogène,
- soit le liquide ionique $A_1^+X_1^-$ est liquide à température ambiante et le sel fonctionnalisé $A_2^+X_2^-$ est liquide à température ambiante et miscible avec le liquide ionique $A_1^+X_1^-$ pour former une phase homogène,
- soit le liquide ionique $A_1^+X_1^-$ est liquide à température ambiante et le sel fonctionnalisé $A_2^+X_2^-$ est solide à température ambiante et est soluble ou partiellement soluble dans le liquide ionique $A_1^+X_1^-$ dans une gamme de températures allant d'environ 25°C à environ 250°C, notamment d'environ 30°C à environ 150°C, pour former une phase homogène.

**12.** Utilisation selon la revendication 5, **caractérisée en ce que** :

- soit le liquide ionique de formule $A_1^+X_1^-$ est liquide à température ambiante,
- soit le liquide ionique de formule $A_1^+X_1^-$ est solide à température ambiante et est liquéfiable dans une gamme de températures allant d'environ 25°C à environ 250°C, notamment d'environ 30°C à environ 150°C.

**13.** Utilisation selon l'une des revendications 1 à 12, **caractérisée en ce que** le liquide ionique de formule $A_1^+X_1^-$, jouant le rôle de matrice liquide, présente une viscosité inférieure ou égale à environ 1500 cp (15 $N.s/m^2$), notamment inférieure à environ 500 cp (5 $N.s/m^2$) et de préférence inférieure à environ 200 cp (2 $N.s/m^2$).

**14.** Composition stable contenant en solution :

- au moins ledit liquide ionique de formule $A_1^+X_1^-$, jouant le rôle de matrice liquide et,
- au moins un sel fonctionnalisé (sel à tâche dédiée), notamment sel d'onium fonctionnalisé, de formule $A_2^+X_2^-$, comme support de réaction,

le sel fonctionnalisé, notamment le sel d'onium fonctionnalisé, étant dissous dans la matrice liquide, pour former une phase homogène,
$A_1^+$ représentant un cation non fonctionnel ou un mélange de cations dans lequel
aucun des cations n'est fonctionnel, et $X_1^-$ représentant un anion non fonctionnel ou un mélange d'anions dans lequel aucun des anions n'est fonctionnel,
$A_2^+$ représentant un cation, fonctionnel ou non, ou un mélange de cations dans lequel aucun des cations n'est fonctionnel ou dans lequel l'un au moins des cations est fonctionnel, et $X_2^-$ représentant un anion, fonctionnel ou non, ou un mélange d'anions dans lequel aucun des anions n'est fonctionnel ou dans lequel l'un au moins des anions est fonctionnel,
sous réserve que $A_2^+$ et/ou $X_2^-$ représente(nt) ou comporte(nt) respectivement un cation fonctionnel et un anion fonctionnel,
lesdits cations fonctionnels et anions fonctionnels correspondant à une entité ionique Y-, à savoir respectivement cationique $Y^+$- ou anionique $Y^-$-, liée, éventuellement par l'intermédiaire d'un bras L, notamment un groupe alkyle comprenant de 1 à 20 atomes de carbone, à au moins une fonction $F_i$, $F_i$ variant de $F_0$ à $F_n$, n étant un nombre entier variant de 1 à 10, le cation fonctionnel pouvant être représenté sous la forme $Y^+$-L-$F_i$, et l'anion fonctionnel sous la forme Y -$(L)_k$-$F_i$, k étant égal à 0 ou 1, et l'anion fonctionnel pouvant représenter, lorsque k est égal à 0, un anion simple, correspondant à $Y^-$-$F_i$, notamment choisi parmi : $OH^-$, $F^-$, $CN^-$, $RO^-$ ou $RS^-$, R représentant un groupement alkyle comprenant de 1 à 20 atomes de carbone ou un groupement aryle comprenant de 6 à 30 atomes de carbone.

**15.** Composition stable contenant en solution :

- au moins une première partie dudit liquide ionique de formule $A_1^+X_1^-$, dont le cation et/ou l'anion correspond (ent) à une entité ionique liée à une ou des fonctions initiales $F_0$, jouant le rôle de matrice liquide, et
- au moins une deuxième partie dudit liquide ionique de formule $A_1^+X_1^-$, dans laquelle ladite ou lesdites fonctions

initiales $F_0$ sont transformées en de premières nouvelles fonctions, conférant à ladite deuxième partie dudit liquide ionique le rôle de sel fonctionnalisé et de support de réaction,

le sel fonctionnalisé et la matrice liquide formant une phase homogène,
les susdites premières nouvelles fonctions de la deuxième partie dudit liquide ionique étant susceptibles d'être transformées ultérieurement en d'autres fonctions, sans qu'il y ait affectation de la ou des fonctions initiales $F_0$ de la première partie dudit liquide ionique.

**16.** Composition selon la revendication 14, **caractérisée en ce que** le cation $A_2^+$ et/ou l'anion $X_2^-$ du ou des sels fonctionnalisés, correspondant à une entité ionique Y-liée à au moins une fonction $F_i$, sont immobilisés dans la matrice liquide et ne peuvent pas être extraits de la matrice liquide par des moyens conventionnels d'extraction, notamment par solvant.

**17.** Composition selon l'une quelconque des revendications 14 à 16, **caractérisée en ce que** la matrice liquide est non réactive vis-à-vis du sel fonctionnalisé.

**18.** Composition selon l'une des revendications 14, 16 et 17, **caractérisée en ce que** $A_2^+$ est un cation fonctionnel.

**19.** Composition selon la revendication 18, **caractérisée en ce que** les anions $X_1$- et $X_2$- sont identiques.

**20.** Composition selon l'une des revendications 18 ou 19, **caractérisée en ce que** :

- les anions $X_1$- et $X_2^-$ sont choisis parmi les deux familles suivantes :

  * les anions non complexes, choisis notamment parmi les anions $BF_4^-$, $PF_6^-$, $CF_3SO_3^-$, $CH_3COO^-$, $CF_3CO_2^-$, $^-N(SO_2CF_3)_2$ (ou $NTf_2^-$), les halogénures, les anions $BR_4^-$, $RCO_2^-$ ou $RSO_3^-$, R étant un groupe alkyle comprenant de 1 à 20 atomes de carbone ou un groupe aryle comprenant de 6 à 30 atomes de carbone, ledit groupe R pouvant également représenter un groupe perfluoré ou partiellement fluoré, ou les anions $R'SO_4^-$, R' étant un atome d'hydrogène, un groupe méthyle ou un groupe éthyle ;
  * les anions complexes, résultant de la combinaison d'un acide de Lewis et d'un halogénure, de préférence $Cl^-$ ou $F^-$, de formule générale $MX_j$, j étant un nombre entier compris entre 1 et 7, et M représentant un métal, notamment choisi parmi l'aluminium, l'étain, le zinc, le bismuth, le manganèse, le fer, le cuivre, le molybdène, l'antimoine, le gallium ou l'indium ;

- les cations $A_1^+$ et $A_2^+$ sont choisis parmi les cations onium, tels que les cations pyridinium, imidazolium, ammonium, phosphonium ou sulfonium, substitués ou non, et de préférence ammonium ou phosphonium.

**21.** Composition selon l'une quelconque des revendications 14 et 16 à 20, **caractérisée en ce que** le cation fonctionnel $A_2^+$ correspond à une entité cationique $Y^+$-, liée, éventuellement par l'intermédiaire d'un bras L, notamment un groupe alkyle comprenant de 1 à 20 atomes de carbone, à une fonction $F_0$, ladite fonction $F_0$ étant choisie parmi les fonctions classiques de la chimie organique, telles que les fonctions hydroxyle, carboxylique, amide, sulfone, amine primaire, amine secondaire, aldéhyde, cétone, éthényle, éthynyle, diényle, éther, époxyde, phosphine (primaire, secondaire ou tertiaire), azoture, imine, cétène, cumulène, hétérocumulène, thiol, thioéther, sulfoxyde, groupements phosphorés, hétérocycles, acide sulfonique, silane, stannane ou aryle fonctionnel.

**22.** Composition selon l'une quelconque des revendications 14 à 21, **caractérisée en ce que** le liquide ionique est choisi parmi les suivants :

$(Ra)_3N^+\text{-}R_b, X_1^-$

$(Ra)_3P^+\text{-}R_b, X_1^-$

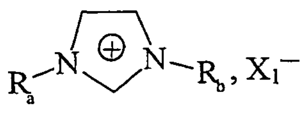 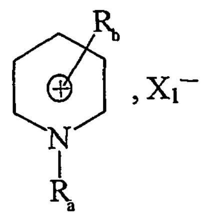

$R_a$ et $R_b$ représentant des groupes alkyle linéaires ou ramifiés, comprenant de 1 à 20 atomes de carbone, notamment un groupe éthyle, propyle, butyle, pentyle, hexyle, heptyle ou octyle, ou des groupes alkyle fonctionnels comprenant de 1 à 20 atomes de carbone, ou des groupes aryle fonctionnels ou non comprenant de 6 à 30 atomes de carbone,

$Bu_3P^+$-Me, $X_1^-$

$^{\oplus}P(C_6H_{13})_3C_{14}H_{29}$, $X_1^-$

$(C_8H_{17})_3N^+Me$ , $X_1^-$

$X_1^-$ étant notamment choisi parmi : $NTf_2^-$, $PF_6^-$ , $BF_4^-$ ou $CF_3SO_3^-$ .

**23.** Composition selon l'une quelconque des revendications 14 et 16 à 22, **caractérisée en ce que** le sel fonctionnalisé est choisi parmi les suivants :

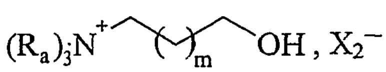 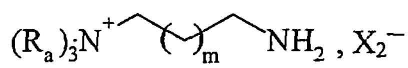

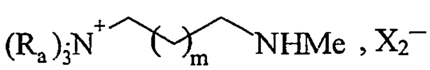 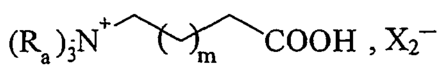

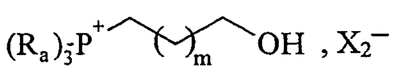 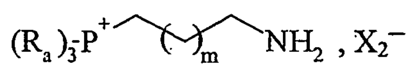

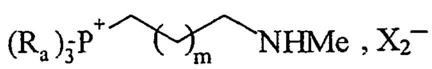 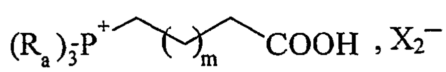

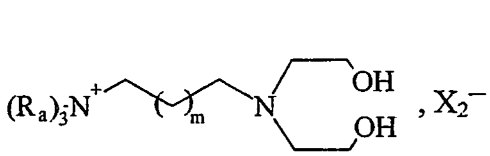 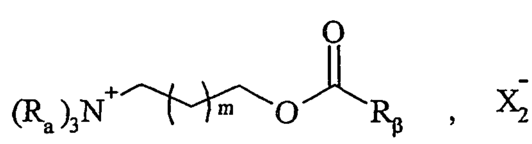

$X_2^-$ étant choisi parmi : $NTf_2^-$, $PF_6^-$, $BF_4^-$, $Cl^-$, $Br^-$ , $I^-$, $CF_3SO_3^-$, $MeSO_4^-$, $EtSO_4^-$, $MeSO_3^-$, $C_6H_5SO_3^-$, $pMeC_6H_4SO_3^-$, m étant un nombre entier compris de 0 à 20,
$R_\beta$ représentant un groupe vinyle, substitué ou non, aryle fonctionnel comprenant de 1 à 20 atomes de carbone, ou alkyle fonctionnel comprenant de 6 à 30 atomes de carbone,

et $R_a$ représentant un groupe alkyle ramifié ou non comprenant de 1 à 20 atomes de carbone, notamment un groupe éthyle, propyle, butyle, pentyle, hexyle, heptyle ou octyle.

**24.** Composition selon l'une des revendications 14 et 16 à 23, **caractérisée en ce que** $X_2^-$ est un anion fonctionnel, correspondant en particulier à un anion dont le $pK_A$ de l'acide conjugué est inférieur à 30, et est notamment choisi parmi les anions suivants :

$OH^-$, $F^-$, $R_cBZ_3^-$ , $N3^-$, $CN^-$, ou $W\bar{C}R_cV$

Z représentant un groupement -F, -OH, -OR, R représentant un groupe alkyle comprenant de 1 à 20 atomes de carbone,
V et W représentant, indépendamment l'un de l'autre, un groupement électroattracteur, notamment un groupement cyano, alcoxycarbonyle comprenant de 2 à 20 atomes de carbone, acyle comprenant de 2 à 20 atomes de carbone, benzoyle, alkyle sulfonyle comprenant de 1 à 20 atomes de carbone, aryle sulfonyle comprenant de 6 à 30 atomes de carbone, dialcoxyphosphonyle comprenant de 2 à 20 atomes de carbone,
$R_c$ représentant un groupement alkyle, ramifié ou non, cyclique ou non, comprenant de 1 à 20 atomes de carbone, ou un groupement aryle comprenant de 6 à 30 atomes de carbone,
et **en ce que** le cation $A_2^+$ est choisi parmi les cations ammonium et phosphonium, notamment parmi les cations suivants :

$Me_3\overset{+}{N}-R_d$ $Et_3\overset{+}{N}-R_d$ $Bu_4\overset{+}{P}-R_d$

$R_d$ étant un groupe alkyle comprenant de 1 à 20 atomes de carbone.

**25.** Utilisation d'une composition selon l'une quelconque des revendications 14 à 24, pour la synthèse organique, en continu, en discontinu, combinatoire, ou parallèle, et/ou pour la préparation de banques de produits.

**26.** Utilisation d'une composition selon l'une quelconque des revendications 14 et 16 à 25, pour la mise en oeuvre d'un procédé de préparation d'une molécule G à partir d'une fonction initiale $F_0$, liée, éventuellement par l'intermédiaire d'un bras L, notamment un groupe alkyle comprenant de 1 à 20 atomes de carbone, à une entité ionique $Y^+$-, faisant partie du cation $A_2^+$ du sel fonctionnalisé $A_2^+X_2^-$, et/ou $Y^-$-, faisant partie de l'anion $X_2$- du sel fonctionnalisé $A_2^+X_2^-$, le cation étant sous la forme $Y^+$-L-$F_0$ et/ou l'anion étant sous la forme $Y^-$-$(L)_k$-$F_0$, k étant égal à 0 ou 1, lequel procédé comprend les étapes :

- d'une première addition d'un réactif $B_1$ dans la composition susmentionnée et la réaction entre ladite fonction $F_0$, et le réactif $B_1$, conduisant à une fonction $F_1$, liée à l'entité ionique. $Y^+$-, faisant partie du cation $A_2^+$ du sel fonctionnalisé $A_2^+X_2^-$, et/ou à l'entité ionique $Y^-$-, faisant partie de l'anion $X_2^-$ du sel fonctionnalisé $A_2^+X_2^-$, selon l'un des schémas réactionnels suivants :

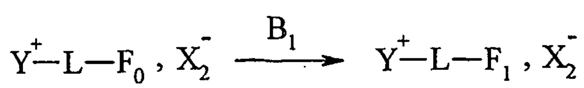


ou

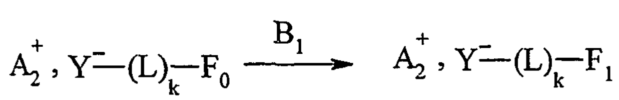


- de n-1 additions successives de réactifs $B_i$, $1 < i \leq n$, n variant de 2 à 10, à la composition susmentionnée, permettant, à chaque addition, la réaction entre le réactif $B_i$ et une fonction $F_{i-1}$, conduisant à l'obtention d'une fonction $F_i$, la $(n-1)^{ième}$ addition du réactif $B_n$ sur la fonction $F_{n-1}$ conduisant à l'obtention de la fonction $F_n$, les n-1 additions pouvant être représentées selon l'un des schémas réactionnels suivants :

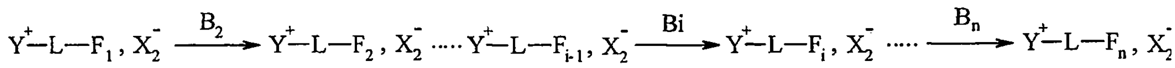


ou

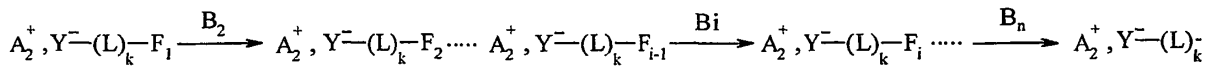


- de clivage de la fonction $F_n$, liée à l'entité ionique $Y^+$- ou $Y^-$ - respectivement du cation $A_2^+$ et/ou de l'anion $X_2^-$ , permettant de récupérer

d'une part le sel fonctionnalisé $A_2^+X_2$- sous la forme $Y^+$-L-$F_0$, $X_2^-$ ou $A_2^+$, $Y^-$-$(L)_k$-$F_0$, en solution dans la matrice liquide ionique $A_1^+X_1^-$, ou sous la forme $Y^+$-L-$F'_0$, $X_2^-$ ou $A_2^+$, $Y^-$-$(L)_k$-$F'_0$, dans lesquelles $F'_0$ représente une fonction différente de $F_0$,
et d'autre part la molécule G,
selon l'un des schémas réactionnels suivants :

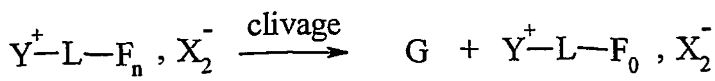


ou

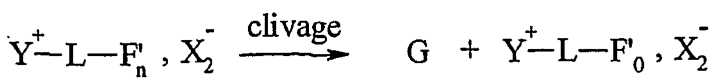


ou

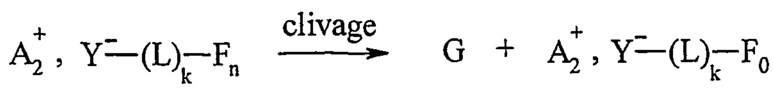


ou

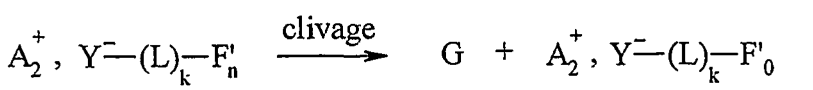


**27.** Utilisation selon la revendication 26, pour la mise en oeuvre de la réaction de Diels-Alder, selon l'un des schémas réactionnels suivants :

a)

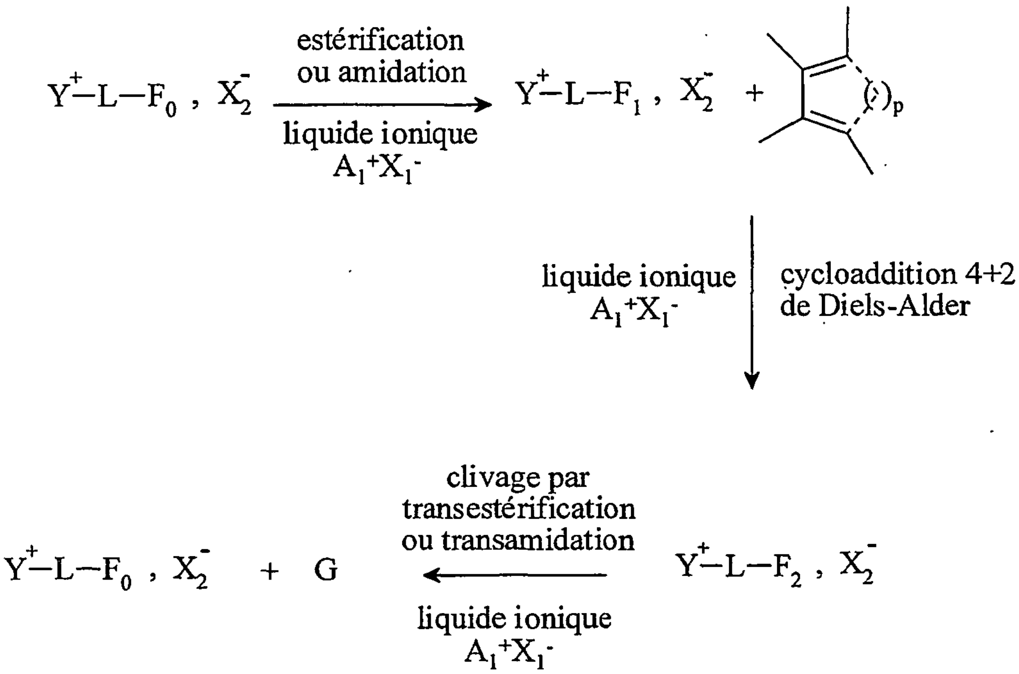


p étant un nombre entier variant de 0 à 2,
$Y^+$- représentant un cation onium tel que défini dans l'une des revendications 20 à 25, et étant de préférence un cation triméthylalkylammonium, triéthylalkylammonium ou tributylalkylphosphonium,
L représentant un bras, notamment un groupe alkyle linéaire ou ramifié comprenant de 1 à 20 atomes de carbone, ou un groupe aralkyle éventuellement fonctionnel, comprenant de 6 à 30 atomes de carbone, et étant de préférence un groupe alkyle linéaire de préférence un groupe alkyle linéaire de type $(CH_2)_r$, r variant de 1 à 20, et de préférence de 3 à 6,
$X_2^-$ étant tel que défini dans l'une des revendications 1 à 26, et étant notamment $NTf_2^-$ , $BF_4^-$, $PF_6^-$, $Cl^-$, $Br^-$, $CH_3COO^-$, $CF_3CO_2$-, $CF_3SO_3^-$, $BR_4^-$, R étant tel que défini dans la revendication 20,
le liquide ionique $A_1^+X_1^-$ étant notamment de la forme :

$(Ra)_3N^+-R_b, X_1^-$

$(Ra)_3P^+-R_b, X_1^-$

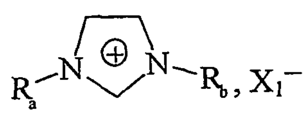

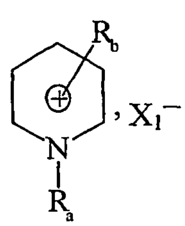

$R_a$ et $R_b$ étant tels que définis dans la revendication 22, et représentant de préférence des groupes alkyle comprenant de 1 à 20 atomes de carbone,
$X_1$- étant choisi parmi : $BF_4$-, $PF_6^-$, $NTf_2^-$, $Cl^-$, $Br^-$, $CH_3COO^-$, $CF_3CO_2^-$, $GF_3SO_3$ , $BR_4^-$ , R étant tel que défini dans la revendication 20,
les fonctions $F_0$, $F_1$ et $F_2$ étant telles que définies ci-dessous :

- $F_0$ correspond à un groupe $-\chi_1H$, dans lequel $\chi1$ représente un atome d'oxygène ou un groupe $-NR_f$, $R_f$ correspondant à un groupe alkyle, linéaire ou ramifié, comprenant de 1 à 20 atomes de carbone, ou un groupe aryle comprenant de 6 à 30 atomes de carbone,
- $F_1$ répond à la formule suivante :

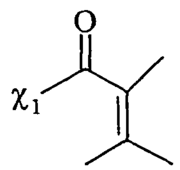

$\chi_1$ étant tel que défini ci-dessus,
- $F_2$ répond à la formule suivante :

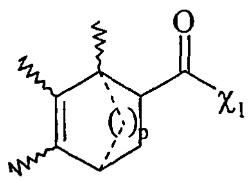

$\chi_1$ étant tel que défini ci-dessus,

G répondant à la formule suivante :

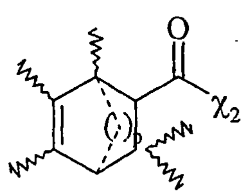

dans laquelle $\chi_2$ représente soit un groupe ORg, Rg représentant un atome d'hydrogène ou un groupe alkyle comprenant de 1 à 20 atomes de carbone, soit un groupe $-NR_hR_u$, $R_h$ et $R_u$ représentant indépendamment l'un de l'autre un atome d'hydrogène, un groupe alkyle comprenant de 1 à 20 atomes de carbone ou un groupe aryle comprenant de 6 à 30 atomes de carbone,

b) 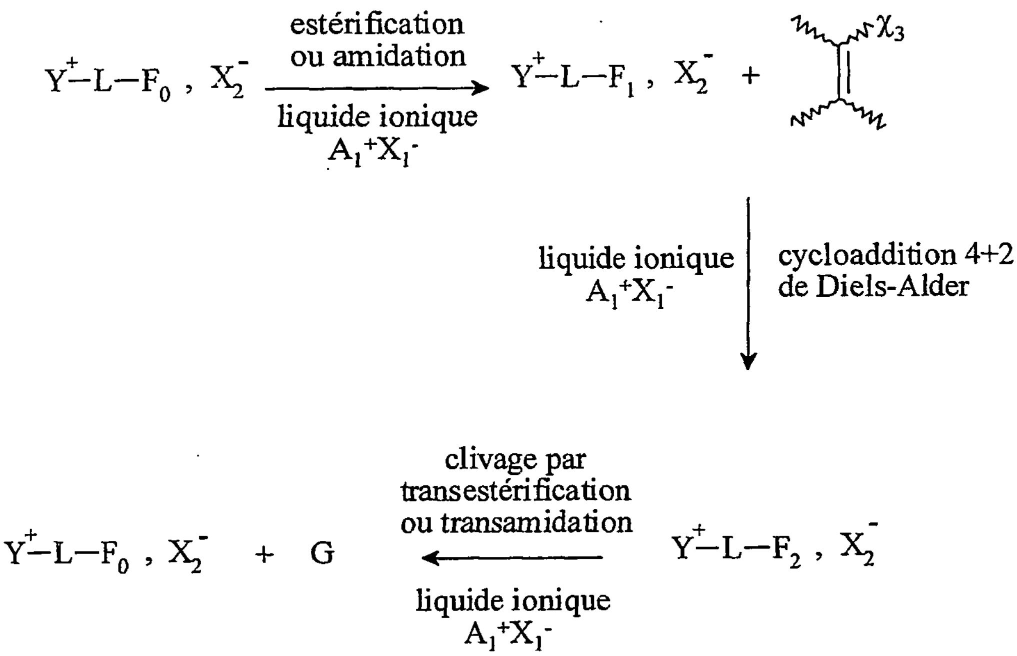


$Y^+$-, L, $X_2^-$ et le liquide ionique $A_1^+X_1^-$ étant tels que définis précédemment, les fonctions $F_0$, $F_1$ et $F_2$ étant telles que définies ci-dessous :

- $F_0$ représente toute fonction permettant d'agrafer un diène-1,3, et est notamment choisi parmi les fonctions carbonyles, amines, alcoxy, silanes, stannanes et boranes, comprenant de 1 à 20 atomes de carbone,
- $F_1$ répond à la formule suivante :

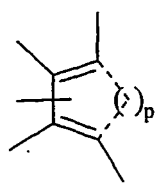

p étant un nombre entier variant de 0 à 2,
- $F_2$ répond à la formule suivante :

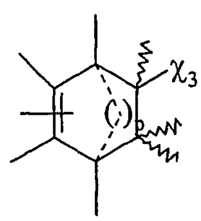

$\chi_3$ représentant un groupement électroattracteur, notamment choisi parmi les groupes cyano, alkoxycarbonyle, comprenant de 1 à 20 atomes de carbone, acyle comprenant de 2 à 20 atomes de carbone, benzoyle, sulfonyle, dialkoxyphosphonyle comprenant de 1 à 10 atomes de carbone,
G répondant à la formule suivante :

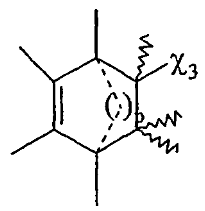

$\chi_3$ étant tel que défini ci-dessus.

**28.** Utilisation selon la revendication 26, pour la mise en oeuvre de la réaction de Heck, selon le schéma réactionnel suivant :

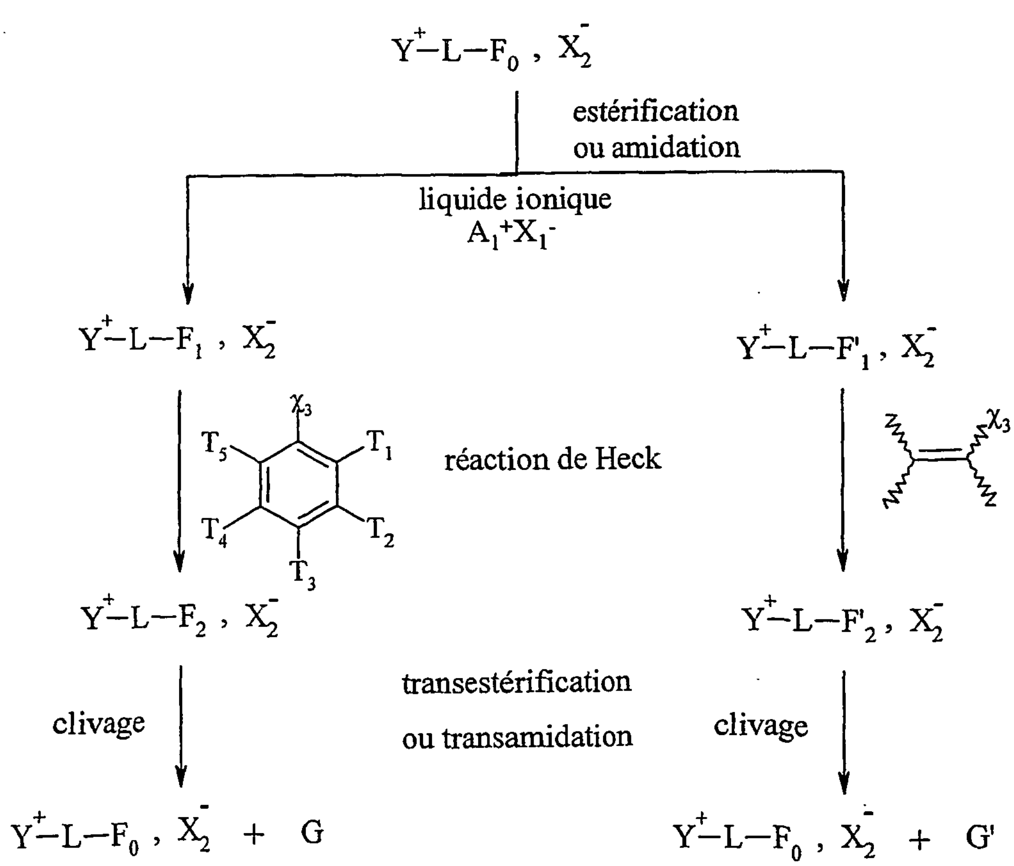


$Y^+$- représentant un cation onium tel que défini dans l'une des revendications 21 à 26, et étant de préférence un cation triméthylalkylammonium, triéthylalkylammonium ou tributylalkylphosphonium,
L représentant un bras, notamment un groupe alkyle linéaire ou ramifié comprenant de 1 à 20 atomes de carbone, ou un groupe aralkyle éventuellement fonctionnel comprenant de 1 à 20 atomes de carbone, et étant de préférence un groupe alkyle linéaire de préférence un groupe alkyle linéaire de type $(CH_2)_r$, r variant de 1 à 20, et de préférence de 3 à 6,
$X_2^-$ étant tel que défini dans l'une des revendications 1 à 26, et étant notamment $BF_4^-$, $PF_6^-$, $NTf_2^-$, $CF_3SO_3^-$, $Cl^-$, $Br^-$ , ou $I^-$,
le liquide ionique $A_1^+X_1^-$ étant notamment de la forme :

$(Ra)_3N^+\text{-}R_b, X_1^-$

$(Ra)_3P^+\text{-}R_b, X_1^-$

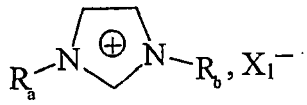

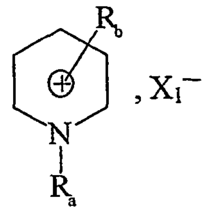

$R_a$ et $R_b$ étant tels que définis dans la revendication 22, et représentant de préférence des groupes alkyle comprenant de 1 à 20 atomes de carbone,
$X_1$- étant choisi parmi: $BF_4^-$, $PF_6^-$, $NTf_2^-$, $Cl^-$, $Br^-$, $CH_3COO^-$, $CF_3CO_2^-$, $CF_3SO_3^-$, $BR_4^-$, R étant tel que défini dans la revendication 20,
les fonctions $F_0$, $F_1$, $F'_1$, $F_2$ et $F'_2$ étant telles que définies ci-dessous :

- $F_0$ correspond à un groupe -$\chi_1$H, dans lequel $\chi_1$ représente un atome d'oxygène ou un groupe -$NR_f$, $R_f$ correspondant à un groupe alkyle, linéaire ou ramifié, comprenant de 1 à 20 atomes de carbone, ou un groupe aryle comprenant de 6 à 30 atomes de carbone,
- $F_1$ répond à la formule suivante :

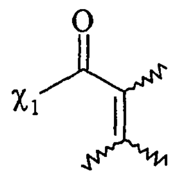

$\chi_1$ étant tel que défini ci-dessus,
- $F_2$ répond à la formule suivante :

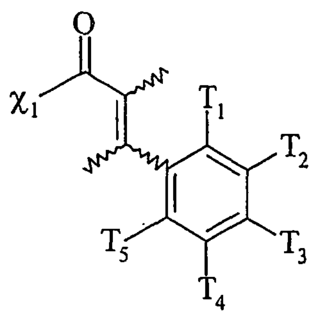

$\chi_1$ étant tel que défini ci-dessus,

G répondant à la formule suivante :

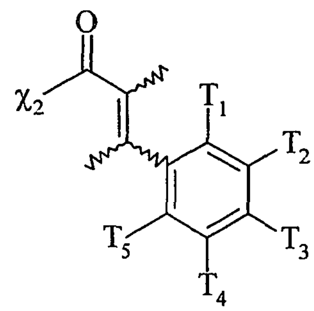

dans laquelle $\chi_2$ représente soit un groupe $-OR_g$, Rg représentant un atome d'hydrogène ou un groupe alkyle comprenant de 1 à 20 atomes de carbone, soit un groupe $-NR_hR_u$, $R_h$ et $R_u$ représentant indépendamment l'un de l'autre un atome d'hydrogène, un groupe alkyle comprenant de 1 à 20 atomes de carbone ou un groupe aryle comprenant de 6 à 30 atomes de carbone,

$\chi_3$ représentant un groupement partant, notamment choisi parmi les halogénures I, Cl et Br, les groupes mésylate, tosylate, triflate, sulfonate, sulfate ou phosphate,

$T_1$, $T_2$, $T_3$, $T_4$ et $T_5$ représentant indépendamment les uns des autres un atome d'hydrogène, un groupe alkyle, linéaire ou ramifié, comprenant de 1 à 20 atomes de carbone ou un groupe aryle comprenant de 6 à 30 atomes de carbone, ou un groupe fonctionnel notamment choisi parmi $NO_2$, CN, COOR, OR, COR, NHCOR, NRR'', $SO_2R$, I, Br, R et R'' représentant indépendamment l'un de l'autre un groupe alkyle comprenant de 1 à 20 atomes de carbone ou un groupe aryle comprenant de 6 à 30 atomes de carbone,

l'entité

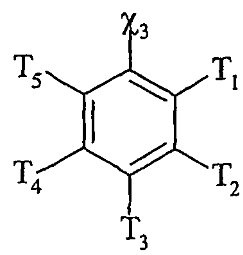

représentant notamment les groupes suivants :

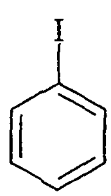
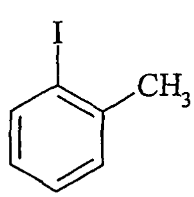
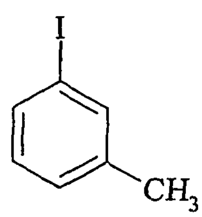
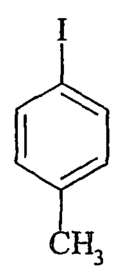
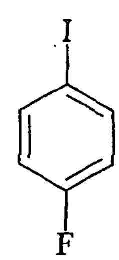

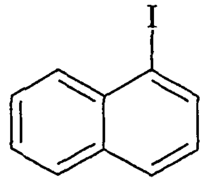
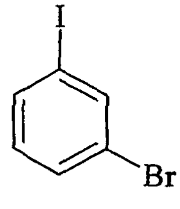
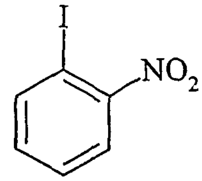
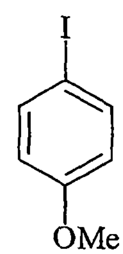

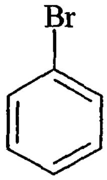

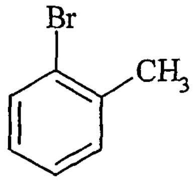

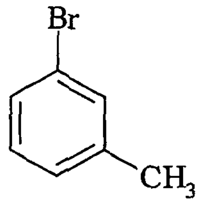

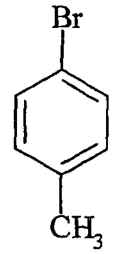

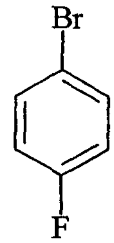

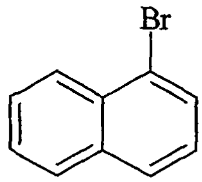

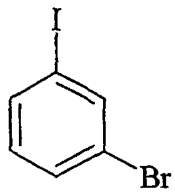

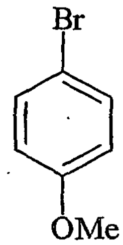

- $F'_1$ répond à la formule suivante :

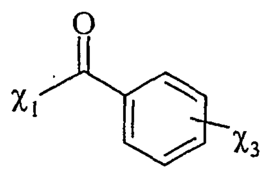

$\chi_1$O $\chi_3$ $\chi_1$ et $\chi_3$ étant tels que définis ci-dessus,
- $F'_2$ répond à la formule suivante :

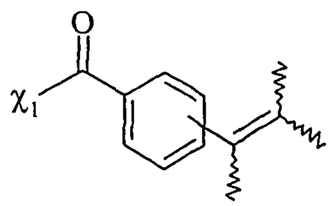

$\chi_1$ étant tel que défini ci-dessus,

G' répondant à la formule suivante :

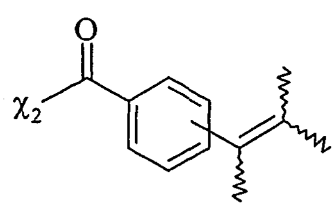

$\chi_2$ étant tel que défini ci-dessus.

**29.** Utilisation selon la revendication 26, pour la mise en oeuvre de la réaction de Baylis-Hihnan, selon le schéma

réactionnel suivant :

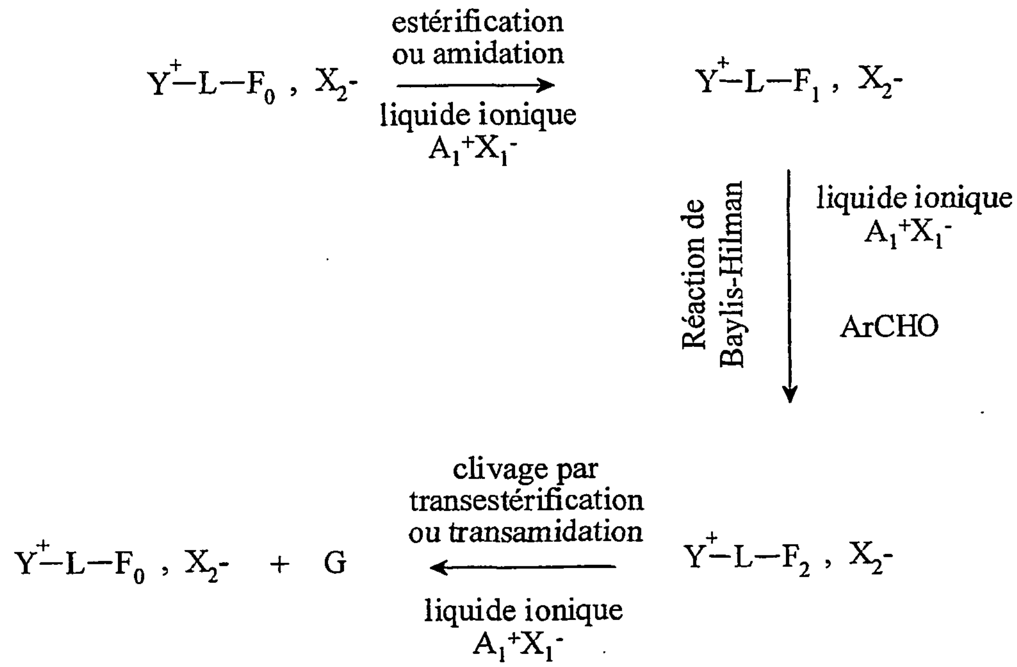


$Y^+$- représentant un cation onium tel que défini dans l'une des revendications 21 à 26, et étant de préférence un cation triméthylalkylammonium, triéthylalkylammonium ou tributylalkylphosphonium,

L représentant un bras, notamment un groupe alkyle linéaire ou ramifié comprenant de 1 à 20 atomes de carbone, ou un groupe aralkyle éventuellement fonctionnel, comprenant de 6 à 30 atomes de carbone, et étant de préférence un groupe alkyle linéaire de préférence un groupe alkyle linéaire de type $(CH_2)_r$, r variant de 1 à 20, et de préférence de 3 à 6,

$X_2^-$ étant tel que défini dans l'une des revendications 1 à 26, et étant notamment BF4-, $PF_6^-$, $NTf_2^-$, $CF_3SO_3^-$, $Cl^-$, $Br^-$, $I^-$, $CH_3CO_2^-$ ou $CF_3CO_2^-$,

le liquide ionique $A_1^+X_1^-$ étant notamment de la forme :

$(Ra)_3N^+$-$R_b$, $X_1^-$

$(Ra)_3P^+$-$R_b$, $X_1^-$

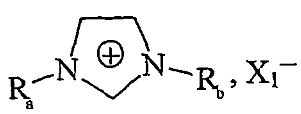

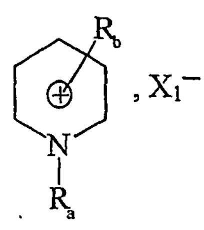

$R_a$ et $R_b$ étant tels que définis dans la revendication 22, et représentant de préférence des groupes alkyle comprenant de 1 à 20 atomes de carbone,

$X_1$- étant choisi parmi: $BF_4^-$, $PF_6^-$ , $NTf_2^-$, $Cl^-$, $Br^-$, $CH_3COO^-$, $CF_3CO_2^-$, $CF_3SO_3^-$, $BR_4^-$, R étant tel que défini dans la revendication 20,

les fonctions $F_0$, $F_1$ et $F_2$ étant telles que définies ci-dessous :

- $F_0$ représente un groupe -OH,
- $F_1$ répond à la formule suivante :

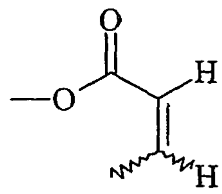

- $F_2$ répond à la formule suivante :

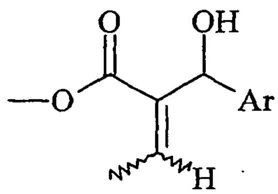

G répondant à la formule suivante :

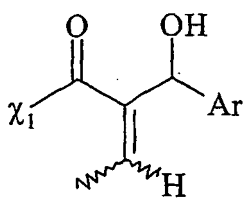

$\chi_1$ représentant un groupe -OH, ou un groupe -ORg, Rg représentant un groupe alkyle, linéaire ou ramifié, comprenant de 1 à 20 atomes de carbone,
Ar représentant un groupe aromatique ou hétéroaromatique, substitué ou non,
ArCHO étant notamment choisi parmi :

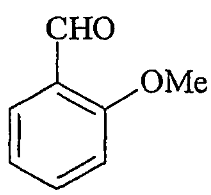 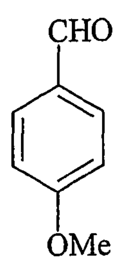 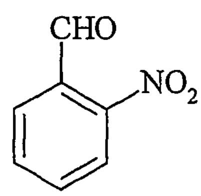 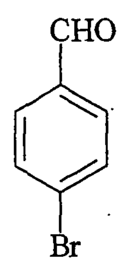

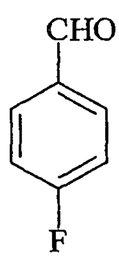 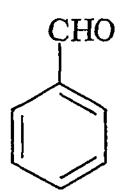 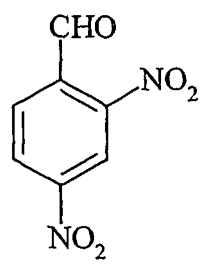 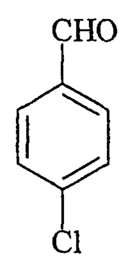

**30.** Utilisation selon la revendication 26, pour la mise en oeuvre du couplage de Suzuki, selon l'un des schémas

réactionnels suivants :

a)

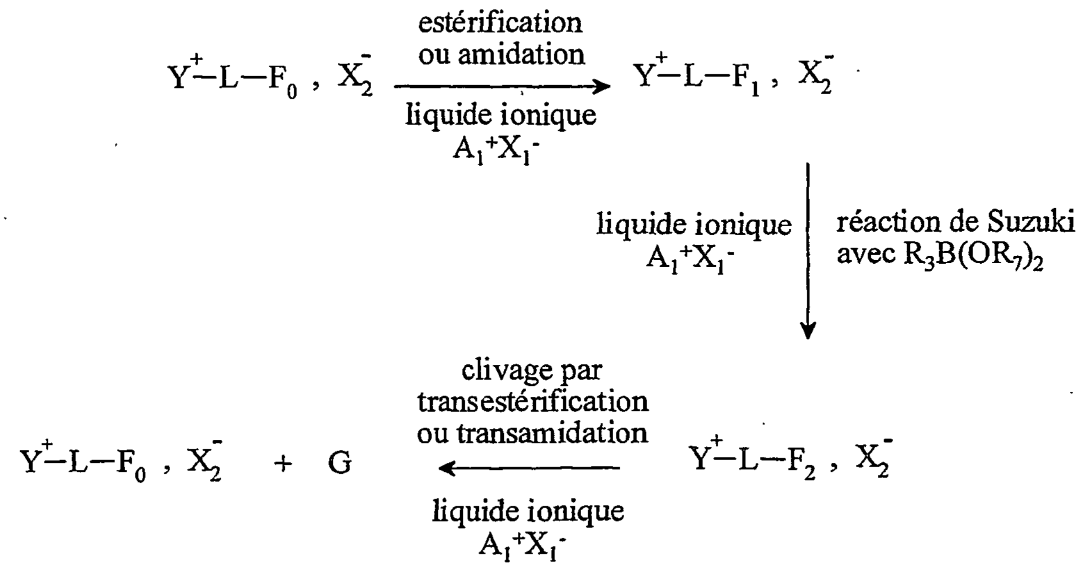


$R_3$ étant choisi parmi les groupes aryle, hétéroaryle, éthényle, diényle, allyle, éthynyle, substitués ou non, comprenant de 2 à 30 atomes de carbone,
$R_7$ représentent un groupe alkyle, ramifié ou linéaire ou un groupe cycloalkyle comprenant de 1 à 12 atomes de carbone,
$Y^+$- représentant un cation onium tel que défini dans l'une des revendications 21 à 26, et étant de préférence un cation triméthylalkylammonium, triéthylalkylammonium ou tributylalkylphosphonium,
L représentant un bras, notamment un groupe alkyle linéaire ou ramifié comprenant de 1 à 20 atomes de carbone, ou un groupe aralkyle éventuellement fonctionnel comprenant de 6 à 30 atomes de carbone, et étant de préférence un, groupe alkyle linéaire de préférence un groupe alkyle linéaire de type $(CH_2)_r$, r variant de 1 à 20, et de préférence de 3 à 6,
X2- étant tel que défini dans l'une des revendications 1 à 26, et étant notamment $NTf_2^-$, $BF_4^-$, $PF_6^-$ , $Cl^-$, $Br^-$, $CH_3COO^-$, $CF_3CO_2^-$, $CF_3SO_3^-$, $BR_4^-$, R étant tel que défini dans la revendication 20,
le liquide ionique $A_1^+X_1^-$ étant notamment de la forme :

$(Ra)_3N^+-R_b, X_1^-$

$(Ra)_3P^+-R_b, X_1^-$

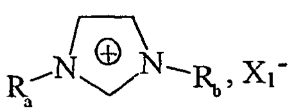

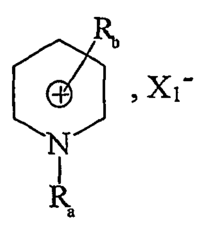

$R_a$ et $R_b$ étant tels que définis dans la revendication 22, et représentant de préférence des groupes alkyle comprenant de 1 à 20 atomes de carbone,
$X_1^-$ étant choisi parmi : $BF_4^-$ $PF_6^-$, $NTf_2^-$, $Cl^-$, $Br^-$, $CH_3COO^-$, $CF_3CO_2^-$, $CF_3SO_3^-$, $BR_4^-$, R étant tel que défini dans la revendication 20,
les fonctions $F_0$, $F_1$ et $F_2$ étant telles que définies ci-dessous :

- $F_0$ est de la forme $-\chi_1H$, $\chi_1$ représentant un atome d'oxygène ou un groupe
- $NR_f$, $R_f$ correspondant à un groupe alkyle, linéaire ou ramifié, comprenant de 1 à 20 atomes de carbone, ou un groupe aryle comprenant de 6 à 30 atomes de carbone,

- $F_1$ est de la forme $-R_e-\chi$, $R_e$ représentant un groupe aromatique ou hétéroaromatique comprenant de 6 à 30 atomes de carbone, $\chi$ représentant un groupe partant choisi de préférence parmi Cl, Br, I, OTf, $O-CO_2R^5$ ou $OSO_3-R^5$, $R^5$ représentant un groupe alkyle comprenant de 1 à 10 atomes de carbone ou un groupe aralkyle comprenant de 6 à 30 atomes de carbone, $F_1$ répondant de préférence à la formule suivante :

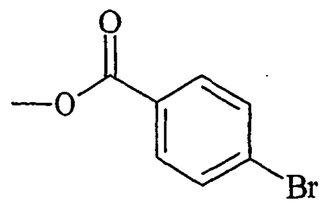

- $F_2$ est de la forme $-R_e-R_2$, $R_e$ étant tel que défini ci-dessus et $R_2$ étant choisi parmi les groupes aryle, hétéroaryle, éthényle, diényle, allyle, éthynyle, substitués ou non, comprenant de 2 à 30 atomes de carbone, $F_2$ répondant de préférence à la formule suivante:

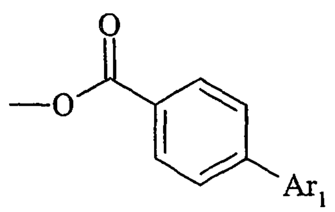

$Ar_1$ représentant un groupe aromatique choisi de préférence parmi :

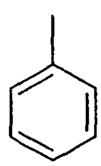
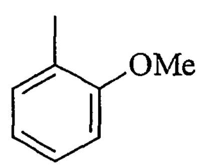
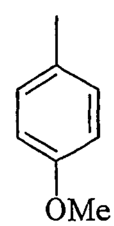
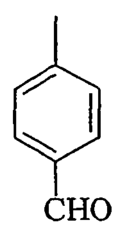
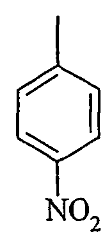

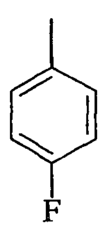
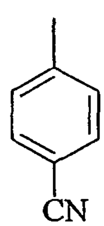
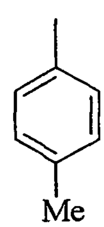
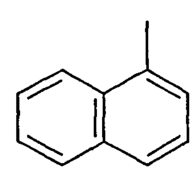

la molécule G étant de la forme $R_2-R_3$, $R_2$ et $R_3$ étant tels que définis ci-dessus, et répond notamment à la formule suivante :

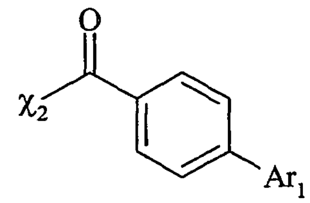

dans laquelle $\chi_2$ représente soit un groupe -ORg, Rg représentant un atome d'hydrogène ou un groupe alkyle comprenant de 1 à 20 atomes de carbone, soit un groupe $NR_hR_u$, $R_h$ et $R_u$ représentant indépendamment l'un de l'autre un atome d'hydrogène, un groupe alkyle comprenant de 1 à 20 atomes de carbone ou un groupe aryle comprenant de 6 à 30 atomes de carbone,
$Ar_1$ est tel que défini ci-dessus,

b)

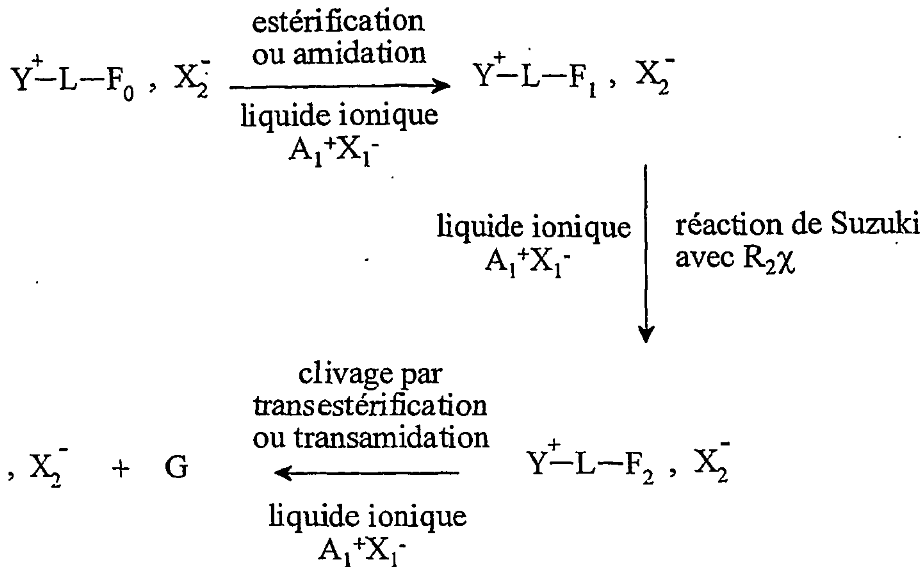


$Y^+$-, L, $X_2^-$, $A_1^+X_1^-$ et $R_2$ étant tels que définis ci-dessus,
les fonctions $F_0$, $F_1$ et $F_2$ étant telles que définies ci-dessous :

- $F_0$ est de la forme $-\chi_1H$, $\chi_1$ étant tel que défini ci-dessus,
- $F_1$ est de la forme $-Rq-B(OR_7)_2$, $R_7$ étant tel que défini ci-dessus, et $R_q$ correspondant à un groupe aryle comprenant de 6 à 30 atomes de carbone, hétéroaryle comprenant de 4 à 20 atomes de carbone, éthényle comprenant de 2 à 20 atomes de carbone, diényle comprenant de 3 à 20 atomes de carbone, allyle comprenant de 3 à 20 atomes de carbone, éthynyle comprenant de 2 à 20 atomes de carbone, substitués ou non, $F_1$ répondant de préférence à la formule suivante :

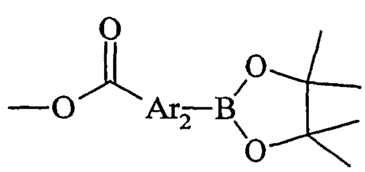

$Ar_2$ correspondant à un groupe aryle substitué ou non comprenant de 6 à 30 atomes de carbone,

- $F_2$ est de la forme $-R_q-R_e$, $R_q$ et $R_e$ étant tels que définis ci-dessus, $F_2$ répondant de préférence à la formule suivante :

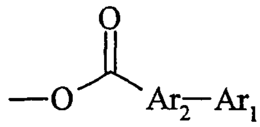

$Ar_1$ représentant un groupe aromatique choisi de préférence parmi :

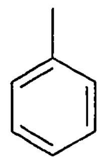
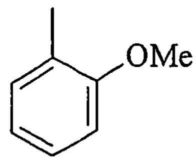
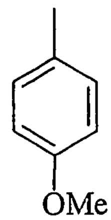
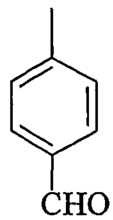
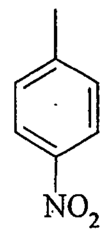

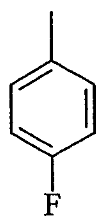
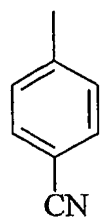
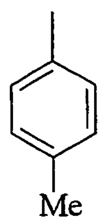
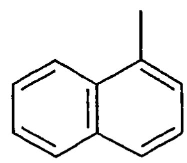

la molécule G étant de la forme $R_2$-$R_3$, $R_2$ et $R_3$ étant tels que définis ci-dessus, et répondant notamment à la formule suivante :

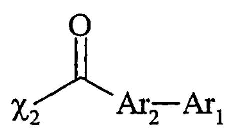

dans laquelle $\chi_2$, $Ar_1$ et $Ar_2$ sont tels que définis ci-dessus,

c)

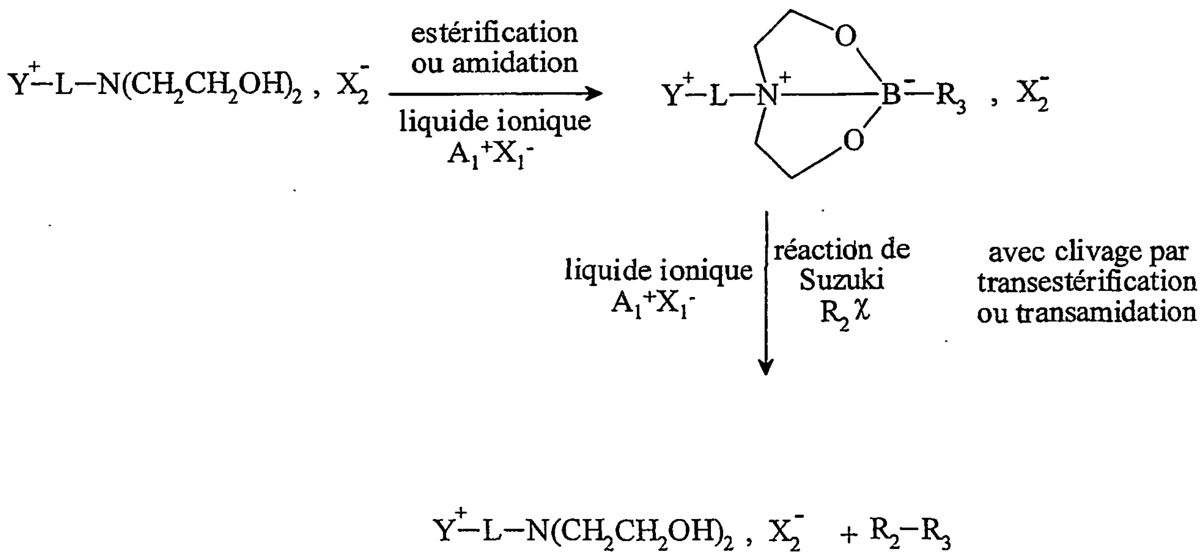


$Y^+$-, L, $X_2^-$; $A_1^+X_1^-$, $R_2$ et $R_3$ étant tels que définis ci-dessus,
$R_3$ étant de préférence un groupe phényle,

d)

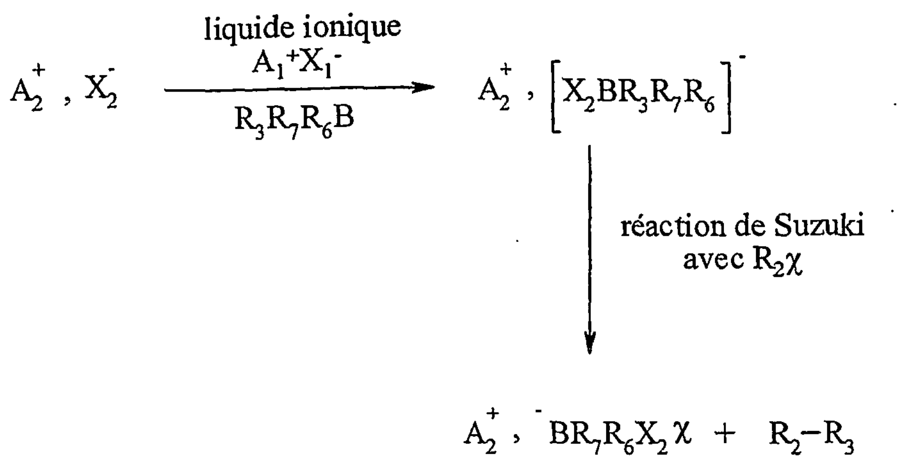


$A_2^+$ étant un cation ammonium $(R_a)_3N^+R_b$ ou phosphonium $(R_a)_3P^+R_b$, de préférence tétrabutylammonium et tétraméthylammonium, $R_a$ et $R_b$ étant tels que définis ci-dessus,
$X_2^-$ étant notamment choisi parmi OHT, $F^-$, $CN^-$, $R_sO^-$, $R_sS^-$, de préférence $OH^-$ ou $F^-$, $R_s$ représentant un groupe alkyle comprenant de 1 à 20 atomes de carbone ou un groupe aryle comprenant de 6 à 30 atomes de carbone,
$R_3$ et $R_4$ étant tels que définis ci-dessus,
$R_6$ et $R_7$ représentant indépendamment l'un de l'autre un groupe alkyle comprenant de 1 à 20 atomes de carbone ou un groupe aryle comprenant de 6 à 30 atomes de carbone,
la molécule borée de formule $R_3R_7R_6B$ étant un trialkyle ou aryle borane, le groupe alkyle comprenant de 1 à 20 atomes de carbone et le groupe aryle comprenant de 6 à 30 atomes de carbone, un acide ou ester borinique, de préférence un acide ou ester boronique choisi comme étant l'acide phényl boronique,
$R_2$ et $\chi$ sont tels que définis précédemment, $R_2\chi$ correspondant de préférence à un halogénure d'aryle choisi parmi :

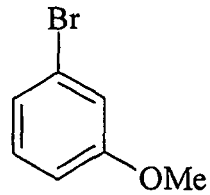

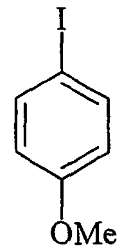

**31.** Utilisation selon les revendications 1 à 30 pour la synthèse de banques de molécules selon la technique de synthèse parallèle, selon le schéma suivant :

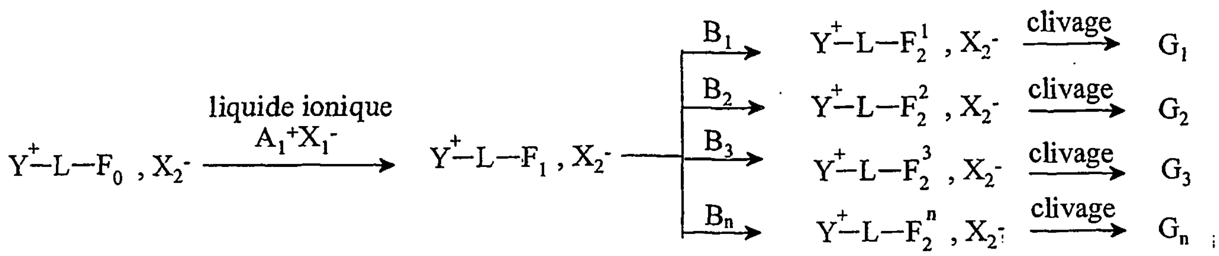


**caractérisée en ce que** le sel fonctionnalisé $Y^+$-L-$F_1$, $X_2^-$ dans le liquide ionique $A_1^+$, $X_1^-$ est séparé en n parties sensiblement égales, n variant de 2 à 1024, et **en ce que** chacune de ces parties est ensuite transformée selon une réaction de synthèse organique, de préférence une réaction de couplage de Heck ou de Suzuki, à l'aide chacune d'un réactif différent $B_i$ pour donner n solutions contenant chacune un composé défini $Y^+$-L-$F_2^i$, $X_2^-$, $F_2^i$ représentant une fonction choisie parmi les fonctions telles que définies dans la revendication 21, i variant de 1 à n, chaque solution étant traitée pour libérer les molécules $G_i$, i variant de 1 à n, qui sont chacune isolées et purifiées, constituant une banque de molécules.

**32.** Utilisation selon les revendications 1 à 30, pour la mise en oeuvre de la synthèse de banques de molécules par la technique de séparation-mélange selon le schéma suivant :

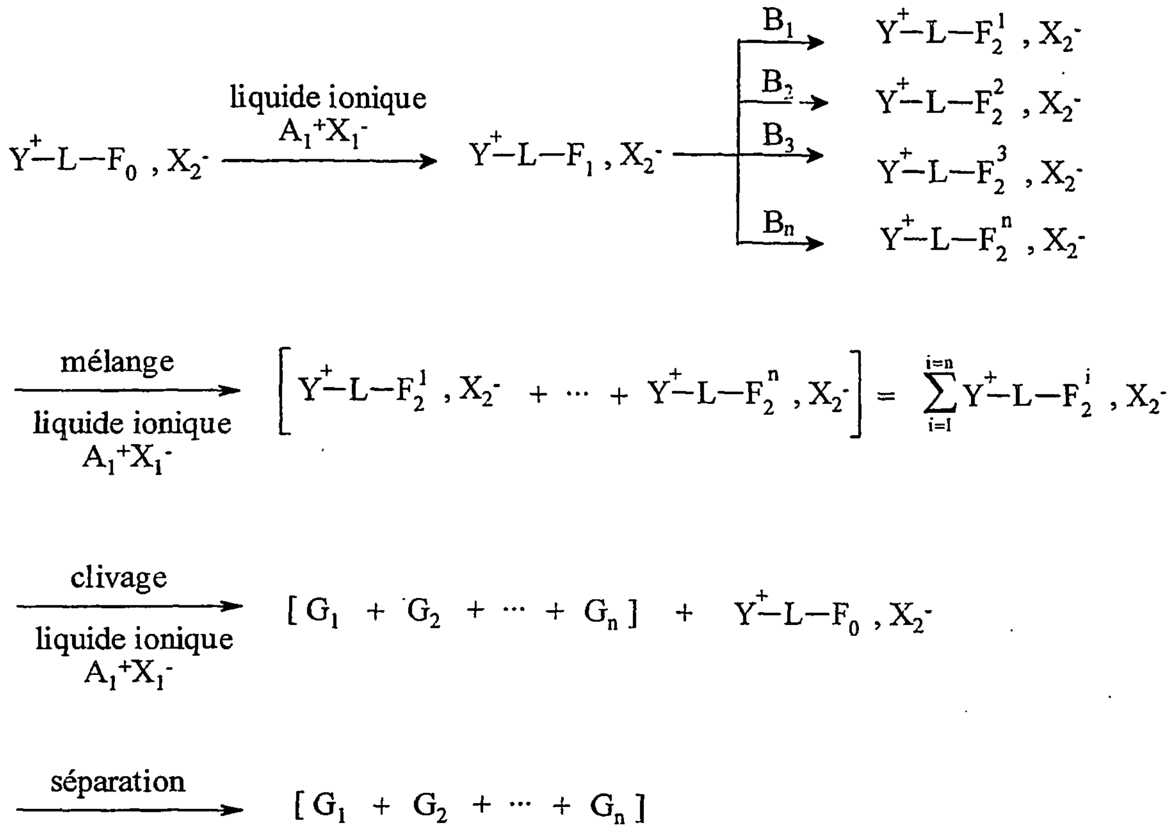


**caractérisée en ce que** :

- n fractions de la solution $Y^+$-L-$F_1$, $X_2^-$, obtenue à partir du sel fonctionnalisé de départ $Y^+$-L-$F_0$, $X_2^-$, dans le liquide ionique $A_1^+X_1^-$ sont transformées en parallèle selon une réaction de la chimie organique, de préférence une réaction de couplage de Heck ou de Suzuki, chacune à l'aide d'un réactif différent $B_i$ pour donner n solutions contenant chacune un composé défini $Y^+$-L-$F_2^i$, $X_2^-$, i variant de 1 à n, n variant de 2 à 1024, de préférence de 2 à 96, $F_2^1$ représentant une fonction choisie parmi les fonctions telles que définies dans la revendication 21,
- les n solutions obtenues à l'étape précédente sont mélangées pour donner une solution dans le liquide ionique $A_1^+X_1^-$ contenant les n produits $Y^+$-L-$F_2^i$, $X_2^-$, i variant de 1 à n, notée $\sum_{i=1}^{i=n} Y^+\!-L-F_2^i$ , , $X_2^-$, et cette solution est soumise à une étape de clivage, de préférence une transestérification ou une transamidation, afin d'obtenir en solution dans le liquide ionique $A_1^+X_1^-$, un mélange des n molécules $G_i$, i variant de 1 à n, et du sel fonctionnalisé de départ $Y^+$-L-$F_0$, $X_2^-$,
- le mélange tel qu'obtenu à l'étape précédente est séparé du liquide ionique $A_1^+X_1^-$ et du sel fonctionnalisé de départ $Y^+$-L- $F_0$, $X_2^-$, par les méthodes usuelles de séparation, de préférence par distillation sous vide, par extraction par un solvant classique tel que l'heptane ou le toluène suivie d'une évaporation de solvant, par chromatographie sur colonne, plaques ou sous pression, afin d'obtenir une banque contenant n molécules $G_i$,

cet enchaînement d'étapes mentionné ci-dessus pouvant être répété j fois, j étant compris de 2 à 10, afin d'obtenir j banques différentes de n produits.

## Claims

1. The use of an ionic liquid, as liquid matrix for organic synthesis in homogeneous phase on soluble support, without

volatile organic solvent, said ionic liquid being presented in liquid or solid form at ambient temperature, of formula $A_1^+X_1^-$, $A_1^+$ representing a cation, functional or non-functional, or a mixture of cations in which either none of the cations is functional or at least one of the cations is functional, and $X_1^-$ an anion, functional or non-functional, or a mixture of anions in which either none of the anions is functional or at least one of the anions is functional.

**2.** The use according to claim 1, **characterized in that** $A_1^+$ represents a non-functional cation or a mixture of non-functional cations and $X_1^-$ a non-functional anion or a mixture of non-functional anions.

**3.** The use according to claim 1, **characterized in that**
$A_1^+$ represents a functional cation or a mixture of cations at least one of which is functional,
and/or $X_1^-$ represents a functional anion or a mixture of anions at least one of which is functional,
said functional cations and functional anions corresponding to an ionic entity, namely cationic or anionic respectively, linked to at least one function $F_i$, $F_i$ varying from $F_0$ to $F_n$, n being an integer varying from 1 to 10.

**4.** The use of an ionic liquid according to one of claims 1 or 2, for the preparation of a stable composition containing in solution:

- at least said ionic liquid of formula $A_1^+X_1^-$, playing the role of liquid matrix and,
- at least one functionalized salt (salt with a dedicated task), in particular functionalized onium salt, of formula $A_2^+X_2^-$, as reaction support,

the functionalized salt, in particular the functionalized onium salt, being dissolved in the liquid matrix, in order to form a homogeneous phase,
$A_1^+$ representing a non-functional cation or a mixture of cations in which none of the cations is functional, and $X_1^-$ representing a non-functional anion or a mixture of anions in which none of the anions is functional,
$A_2^+$ representing a cation, functional or non-functional, or a mixture of cations in which none of the cations is functional or in which at least one of the cations is functional, and $X_2^-$ representing an anion, functional or non-functional, or a mixture of anions in which none of the anions is functional or in which at least one of the anions is functional,
provided that $A_2^+$ and/or $X_2^-$ represent(s) or comprise(s) a functional cation and a functional anion respectively,
said functional cations and functional anions corresponding to an ionic entity $Y^-$, namely cationic $Y^+$- or anionic $Y^-$- respectively, linked, optionally via an L linker, in particular an alkyl group comprising 1 to 20 carbon atoms, to at least one function F;, F; varying from $F_0$ to $F_n$, n being an integer varying from 1 to 10, the functional cation being representable in the form $Y^+$-L-$F_i$, and the functional anion in the form $Y^-$-$(L)_k$-$F_i$, k being equal to 0 or 1, and the functional anion possibly representing, when k is equal to 0, a single anion, corresponding to $Y^-$-$F_i$, in particular chosen from: $OH^-$, $F^-$, $CN^-$, RO- or $RS^-$, R representing an alkyl group comprising 1 to 20 carbon atoms or an aryl group comprising 6 to 30 carbon atoms.

**5.** The use according to claim 3, for the preparation of a stable composition containing in solution:

- at least one first part of said ionic liquid of formula $A_1^+X_1^-$,the cation and/or the anion of which correspond(s) to an ionic entity linked to one or more initial functions $F_0$, playing the role of liquid matrix, and
- at least one second part of said ionic liquid of formula $A_1^+X_1^-$, in which said initial function or functions $F_0$ are converted into first novel functions, conferring upon said second part of said ionic liquid the role of functionalized salt and of reaction support,

the functionalized salt and the liquid matrix forming a homogeneous phase,
the abovementioned first novel functions of the second part of said ionic liquid being capable of being converted subsequently into other functions, without affecting the initial function or functions $F_0$ of the first part of said ionic liquid.

**6.** The use of an ionic liquid according to claim 4, **characterized in that** the $A_2^+$ cation and/or the $X_2^-$ anion of the functionalized salt or salts, corresponding to a Y-ionic entity linked to at least one function $F_i$, are immobilized in the liquid matrix and cannot be extracted from the liquid matrix by standard extraction means, in particular by solvent, and in which the function(s) $F_i$ of the functionalized salt or salts can be converted at the end of at least one reaction resulting from the addition of at least one reagent to said composition.

**7.** The use of an ionic liquid according to claim 6, **characterized in that** several functionalized salts are immobilized.

**8.** The use according to one of claims 4, 6 or 7, **characterized in that** the $A_2^+$ cation is functional.

**9.** The use according to one of claims 4, 6 or 7, **characterized in that** the $X_2^-$ anion is functional.

**10.** The use according to one of claims 4, 6 or 7 to 9, **characterized in that** $A_2^+$ and $X_2$- are functional.

**11.** The use according to one of claims 1, 2, 4, 6 to 10, **characterized in that**:

- either the ionic liquid of formula $A_1^+X_1^-$ is solid at ambient temperature and is liquefiable within a temperature range from approximately 25°C to approximately 250°C, in particular from approximately 30°C to approximately 150°C, and the $A_2^+X_2^-$ functionalized salt is solid at ambient temperature and is soluble in the liquefied $A_1^+X_1^-$ ionic liquid, in order to form a homogeneous phase,
- or the ionic liquid of formula $A_1^+X_1^-$ is solid at ambient temperature and is liquefiable within a temperature range from approximately 25°C to approximately 250°C. in particular from approximately 30°C to approximately 150°C, and the $A_2^+X_2^-$ functionalized salt is liquid at ambient temperature, and is miscible with the liquefied $A_1^+X_1^-$ ionic liquid, in order to form a homogeneous phase,
- or the $A_1^+X_1^-$ ionic liquid is liquid at ambient temperature and the $A_2^+X_2^-$ functionalized salt is liquid at ambient temperature and miscible with the $A_1^+X_1^-$ ionic liquid, in order to form a homogeneous phase,
- or the $A_1^+X_1^-$ ionic liquid is liquid at ambient temperature and the $A_2^+X_2^-$ functionalized salt is solid at ambient temperature and is soluble or partially soluble in the $A_1^+X_1^-$ ionic liquid within a temperature range from approximately 25°C to approximately 250°C, in particular from approximately 30°C to approximately 150°C, in order to form a homogeneous phase.

**12.** The use according to claim 5, **characterized in that**:

- either the ionic liquid of formula $A_1$+$X_1$- is liquid at ambient temperature,
- or the ionic liquid of formula $A_1^+X_1$- is solid at ambient temperature and is liquefiable within a temperature range from approximately 25°C to approximately 250°C, in particular from approximately 30°C to approximately 150°C.

**13.** The use according to one of claims 1 to 12, **characterized in that** the ionic liquid of formula $A_1^+X_1^-$, playing the role of liquid matrix, has a viscosity less than or equal to approximately 1500 cp (15 $N.s/m^2$), in particular less than approximately 500 cp (5 $N.s/m^2$) and preferably less than approximately 200 cp (2 $N.s/m^2$).

**14.** A stable composition containing in solution:

- at least said ionic liquid of formula $A_1^+X_1^-$, playing the role of liquid matrix and,
- at least one functionalized salt (salt with a dedicated task), in particular functionalized onium salt, of formula $A_2^+X_2^-$, as reaction support,

the functionalized salt, in particular the functionalized onium salt, being dissolved in the liquid matrix, in order to form a homogeneous phase,
$A_1^+$ representing a non-functional cation or a mixture of cations in which none of the cations is functional, and $X_1^-$ representing a non-functional anion or a mixture of anions in which none of the anions is functional,
$A_2^+$ representing a cation, functional or non-functional, or a mixture of cations in which none of the cations is functional or in which at least one of the cations is functional, and $X_2^-$ representing an anion, functional or non-functional, or a mixture of anions in which none of the anions is functional or in which at least one of the anions is functional, provided that $A_2^+$ and/or $X_2^-$ represent(s) or comprise(s) a functional cation and a functional anion respectively, said functional cations and functional anions corresponding to an ionic entity Y-, namely cationic $Y^+$- or anionic $Y^-$- respectively, linked, optionally via an L linker, in particular an alkyl group comprising 1 to 20 carbon atoms, to at least one function $F_i$, $F_i$ varying from $F_0$ to $F_n$, n being an integer varying from 1 to 10, the functional cation being representable in the form $Y^+$-L-$F_i$, and the functional anion in the form $Y^-$-$(L)_k$-$F_i$, k being equal to 0 or 1, and the functional anion possibly representing, when k is equal to 0, a single anion, corresponding to $Y^-$-$F_i$, in particular chosen from: $OH^-$, $F^-$, $CN^-$, $RO^-$ or $RS^-$, R representing an alkyl group comprising 1 to 20 carbon atoms or an aryl group comprising 6 to 30 carbon atoms.

**15.** A stable composition containing in solution:

- at least one first part of said ionic liquid of formula $A_1^+X_1^-$, the cation and/or anion of which correspond(s) to an ionic entity linked to one or more initial functions $F_0$, playing the role of liquid matrix, and

- at least one second part of said ionic liquid of formula $A_1^+X_1^-$, in which said initial function or functions $F_0$ are converted into first novel functions, conferring on said second part of said ionic liquid the role of functionalized salt and reaction support,

the functionalized salt and the liquid matrix forming a homogeneous phase,
the abovementioned first novel functions of the second part of said ionic liquid being capable of being subsequently converted to other functions, without affecting the initial function or functions $F_0$ of the first part of said ionic liquid.

**16.** The composition of claim 14, **characterized in that** the $A_2^+$ cation and/or the $X_2^-$ anion of the functionalized salt or salts, corresponding to a Y- ionic entity linked to at least one function $F_i$, are immobilized in the liquid matrix and cannot be extracted from the liquid matrix by standard extraction means, in particular by solvent.

**17.** The composition of any one of claims 14 to 16, **characterized in that** the liquid matrix is non-reactive vis-à-vis the functionalized salt.

**18.** The composition of one of claims 14, 16 and 17, **characterized in that** $A_2^+$ is a functional cation.

**19.** The composition of claim 18, **characterized in that** the $X_1^-$ and $X_2^-$ anions are identical.

**20.** The composition of one of claims 18 or 19, **characterized in that**:

- the $X_1^-$ and $X_2^-$ anions are chosen from the following two families:

  * the non-complex anions, chosen in particular from the $BF_4^-$, $PF_6^-$, $CF_3SO_3^-$, $CH_3COO^-$, $CF_3CO_2^-$, $^-N(SO_2CF_3)_2$ (or $NTf_2^-$) anions, the halides, the $BR_4^-$, $RCO_2^-$ or $RSO_3^-$ anions, R being an alkyl group comprising 1 to 20 carbon atoms or an aryl group comprising 6 to 30 carbon atoms, said R group also possibly representing a perfluorinated or partially fluorinated group, or the $R'SO_4^-$ anions, R' being a hydrogen atom, a methyl group or an ethyl group;
  * the complex anions, resulting from the combination of a Lewis acid and a halide, preferably $Cl^-$ or $F^-$, of general formula $MX_j$, j being an integer comprised between 1 and 7, and M representing a metal, in particular chosen from aluminium, tin, zinc, bismuth, manganese, iron, copper, molybdenum, antimony, gallium or indium;

- the $A_1^+$ and $A_2^+$ cations are chosen from the onium cations, such as the substituted or non-substituted pyridinium, imidazolium, ammonium, phosphonium or sulphonium cations, , and preferably ammonium or phosphonium.

**21.** The composition of any one of claims 14 and 16 to 20, **characterized in that** the $A_2^+$ functional cation corresponds to a $Y^+$- cationic entity, linked, via an L linker, in particular an alkyl group comprising 1 to 20 carbon atoms, to a function $F_0$, said function $F_0$ being chosen from the standard functions of organic chemistry, such as the hydroxyl, carboxylic, amide, sulphone, primary amine, secondary amine, aldehyde, ketone, ethenyl, ethynyl, dienyl, ether, epoxide, phosphine (primary, secondary or tertiary), azide, imine, ketene, cumulene, heterocumulene, thiol, thioether, sulphoxide, phosphorus-containing moieties, heterocycles, sulphonic acid, silane, stannane or functional aryl functions.

**22.** The composition of any one of claims 14 to 21, **characterized in that** the ionic liquid is chosen from the following:

$(Ra)_3N^+-R_b$, $X_1^-$

$(Ra)_3P^+-R_b$, $X_1^-$

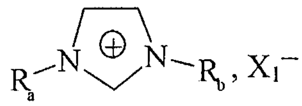

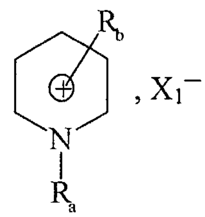

$R_a$ and $R_b$ representing linear or branched alkyl groups, comprising 1 to 20 carbon atoms, in particular an ethyl, propyl, butyl, pentyl, hexyl, heptyl or octyl group, or functional alkyl groups comprising 1 to 20 carbon atoms, or functional or non-functional aryl groups comprising 6 to 30 carbon atoms,

$Bu_3P^+$-Me , $X_1^-$

$^{\oplus}P(C_6H_{13})_3C_{14}H_{29}$ , $X_1^-$

$(CaHm)_3N^+Me$ , $X_1^-$

$X_1^-$ being in particular chosen from: $NTf_2$-, $PF_6^-$, $BF_4^-$ or $CF_3SO_3^-$.

**23.** The composition of any one of claims 14 and 16 to 22, **characterized in that** the functionalized salt is chosen from the following:

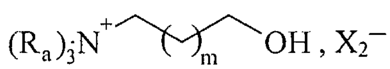

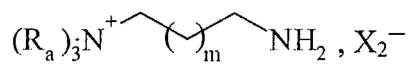

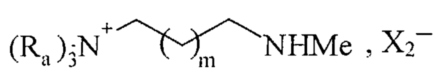

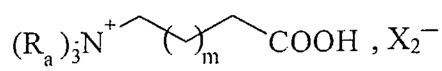

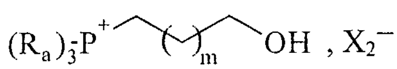

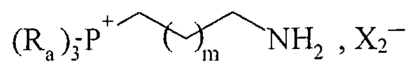

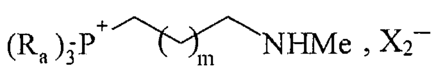

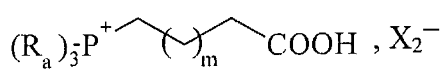

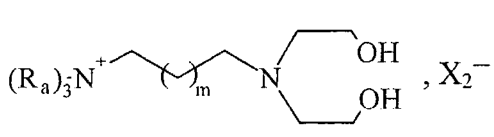

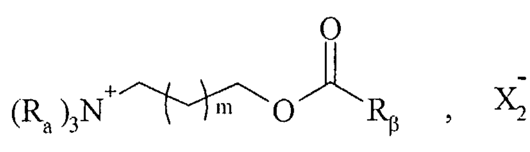

$X_2^-$ being chosen from: $NTf_2^-$, $PF_6^-$, $BF_4^-$, $Cl^-$, $Br^-$, $I^-$, $CF_3SO_3^-$, $MeSO_4^-$ , $EtSO_4^-$, $MeSO_3^-$, $C_6H_5SO_3^-$, $pMeC_6H_4SO_3^-$, m being an integer comprised between 0 and 20,
$R_\beta$ representing a substituted or non-substituted vinyl group, functional aryl group comprising 1 to 20 carbon atoms, or functional alkyl group comprising 6 to 30 carbon atoms,
and $R_a$ representing a branched or non-branched alkyl group comprising 1 to 20 carbon atoms, in particular an

ethyl, propyl, butyl, pentyl, hexyl, heptyl or octyl group.

**24.** The composition of one of claims 14 and 16 to 23, **characterized in that** $X_2$ is a functional anion, corresponding in particular to an anion the $pK_A$ of the conjugated acid of which is less than 30, and is chosen in particular from the following anions:

$OH^-$, $F^-$, $R_cBZ_3^-$, $N_3^-$, $CN^-$, or $W\bar{C}R_cV$

Z representing an -F, -OH, -OR group, R representing an alkyl group comprising 1 to 20 carbon atoms,
V and W representing, independently of each other, an electroattractive group, in particular a cyano, alkoxycarbonyl moiety comprising 2 to 20 carbon atoms, acyl moiety comprising 2 to 20 carbon atoms, benzoyl, alkyl sulphonyl moiety comprising 1 to 20 carbon atoms, aryl sulphonyl moiety comprising 6 to 30 carbon atoms, dialkoxyphosphonyl moiety comprising 2 to 20 carbon atoms,
$R_c$ representing a branched or non-branched, cyclic or non-cyclic alkyl moiety, comprising 1 to 20 carbon atoms, or an aryl moiety comprising 6 to 30 carbon atoms,
and **in that** the $A_2^+$ cation is chosen from the ammonium and phosphonium cations, in particular from the following cations:

$Me_3\overset{+}{N}-R_d$ $Et_3\overset{+}{N}-R_d$ $Bu_4\overset{+}{P}-R_d$

$R_d$ being an alkyl group comprising 1 to 20 carbon atoms.

**25.** The use of a composition according to any one of claims 14 to 24, for continuous, discontinuous, combinatorial, or parallel organic synthesis, and/or for the preparation of libraries of products.

**26.** The use of a composition according to any one of claims 14 and 16 to 25, for the implementation of a process for the preparation of a molecule G with an initial function $F_0$, linked, optionally via an L linker, in particular an alkyl group comprising 1 to 20 carbon atoms, to a $Y^+$-ionic entity, forming part of the $A_2^+$ cation of the $A_2^+$+$X_2^-$, and/or $Y^-$- functionalized salt, forming part of the $X_2$- anion of the $A_2^+X_2^-$ functionalized salt, the cation being in the form $Y^+$-L-$F_0$ and/or the anion being in the form $Y^-$-$(L)_k$-$F_0$, k being equal to 0 or 1, which process comprises the stages:

- of a first addition of a reagent $B_1$ into the abovementioned composition and the reaction between said function $F_0$, and the reagent $B_1$, leading to a function $F_1$, linked to the $Y^+$- ionic entity, forming part of the $A_2^+$ cation of the $A_2^+X_2^-$ functionalized salt, and/or to the $Y^-$- ionic entity, forming part of the $X_2^-$ anion of the $A_2^+X_2^-$ functionalized salt, according to one of the following reaction diagrams:

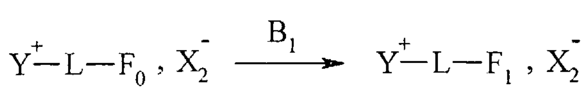

or

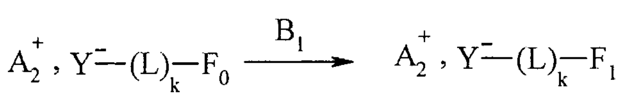

- of n-1 successive additions of B; reagents, $1 < i \leq n$, n varying from 2 to 10, to the abovementioned composition, allowing, at each addition, the reaction between the reagent $B_i$ and a function $F_{i-1}$, leading to the obtaining of a function $F_i$, the (n-1)$^{th}$ addition of the reagent $B_n$ to the function $F_{n-1}$ leading to the obtaining of the function $F_n$, the n-1 additions being representable according to one of the following reaction diagrams:

$$Y^{+}{-}L{-}F_1\,,\,X_2^- \xrightarrow{B_2} Y^{+}{-}L{-}F_2\,,\,X_2^- \cdots\cdots Y^{+}{-}L{-}F_{i-1}\,,\,X_2^- \xrightarrow{Bi} Y^{+}{-}L{-}F_i\,,\,X_2^- \cdots\cdots \xrightarrow{B_n} Y^{+}{-}L{-}F_n\,,\,X_2^-$$

or

$$A_2^+\,,Y^-{-}(L)_k{-}F_1 \xrightarrow{B_2} A_2^+\,,Y^-{-}(L)_k{-}F_2 \cdots\cdots A_2^+\,,Y^-{-}(L)_k{-}F_{i-1} \xrightarrow{Bi} A_2^+\,,Y^-{-}(L)_k{-}F_i \cdots\cdots \xrightarrow{B_n} A_2^+\,,Y^-{-}(L)_k{-}F_n$$

- of cleavage of the function $F_n$, linked to the $Y^+$- or $Y^-$- ionic entity respectively of the $A_2^+$ cation and/or of the $X_2$ - anion, making it possible to recover

on the one hand the $A_2^+X_2^-$ functionalized salt in the form $Y^+$-L-$F_0$, $X_2$- or $A_2^+$, $Y^-$-$(L)_k$-$F_0$, in solution in the $A_1^+X_1^-$ ionic liquid matrix, or in the form $Y^+$-L-$F'_0$, $X_2^-$ or $A_2^+$, $Y^-$-$(L)_k$-$F'_0$, in which $F'_0$ represents a function different from $F_0$, and on the other hand the molecule G,
according to one of the following reaction diagrams:

$$Y^{+}{-}L{-}F_n\,,X_2^- \xrightarrow{\text{cleavage}} G \;+\; Y^{+}{-}L{-}F_0\,,X_2^-$$

or

$$Y^{+}{-}L{-}F'_n\,,X_2^- \xrightarrow{\text{cleavage}} G \;+\; Y^{+}{-}L{-}F'_0\,,X_2^-$$

or

$$A_2^+\,,Y^-{-}(L)_k{-}F_n \xrightarrow{\text{cleavage}} G \;+\; A_2^+\,,Y^-{-}(L)_k{-}F_0$$

or

$$A_2^+\,,Y^-{-}(L)_k{-}F'_n \xrightarrow{\text{cleavage}} G \;+\; A_2^+\,,Y^-{-}(L)_k{-}F'_0$$

**27.** The use according to claim 26, for implementation of the Diels-Alder reaction, according to one of the following reaction diagrams:

a)

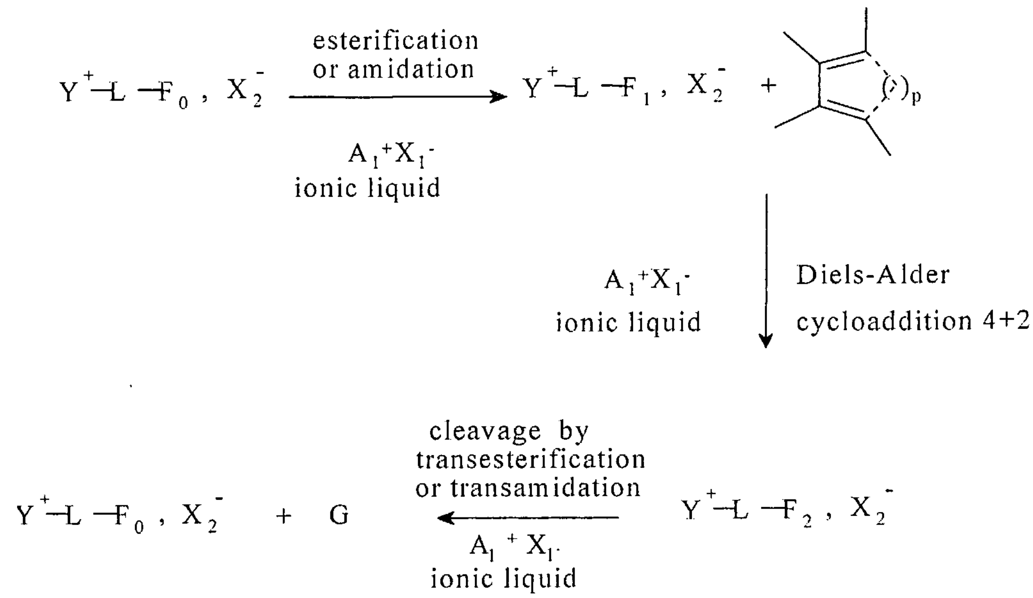


p being an integer varying from 0 to 2,
$Y^+$- representing an onium cation as defined in one of claims 20 to 25, and preferably being a trimethylalkylammonium, triethylalkylammonium or tributylalkylphosphonium cation,
L representing an linker, in particular a linear or branched alkyl group comprising 1 to 20 carbon atoms, or an optionally functional aralkyl group, comprising 6 to 30 carbon atoms, and preferably being a linear alkyl group preferably a linear alkyl group of(CH2)r type, r varying from 1 to 20, and preferably from 3 to 6,
$X_2^-$ being as defined in one of claims 1 to 26, and being in particular $NTf_2^-$, $BF_4^-$, $PF_6^-$, $Cl^-$, $Br^-$, $CH_3COO^-$, $CF_3CO_2)^-$, $CF_3SO_3^-$, $BR_4^-$, R being as defined in claim 20,
the $A_1{}^+X_1{}^-$ ionic liquid being in particular in the form:

$(Ra)_3N^+\text{-}R_b, X_1^-$

$(Ra)_3P^+\text{-}R_b, X_1^-$

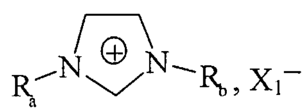

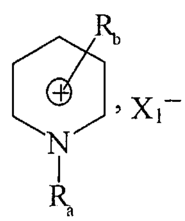

$R_a$ and $R_b$ being as defined in claim 22, and preferably representing alkyl groups comprising 1 to 20 carbon atoms, $X_1^-$ being chosen from: $BF_4^-$, $PF_6^-$, $NTf_2^-$, $Cl^-$, $Br^-$, $CH_3COO^-$, $CF_3CO_2^-$, $CF_3SO_3^-$, $BR_4^-$, R being as defined 111 claim 20,
the functions $F_0$, $F_1$ and $F_2$ being as defined below:

- $F_0$ corresponds to a $-\chi_1H$ group, in which $\chi_1$ represents an oxygen atom or an
- $NR_f$ group, $R_f$ corresponding to a linear or branched alkyl group, comprising 1 to 20 carbon atoms, or an aryl group comprising 6 to 30 carbon atoms,
- $F_1$ corresponds to the following formula:

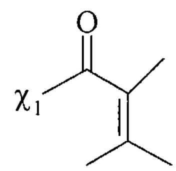

$\chi_1$ being as defined above,

- $F_2$ corresponds to the following formula:

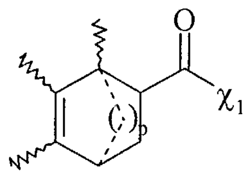

$\chi_1$ being as defined above,

G corresponding to the following form

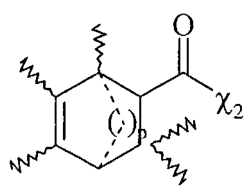

in which $\chi_2$ represents either an ORg group, Rg representing a hydrogen atom or an alkyl group comprising 1 to 20 carbon atoms, or an $-NR_hR_u$ group, $R_h$ and $R_u$ representing independently of each other a hydrogen atom, an alkyl group comprising 1 to 20 carbon atoms or an aryl group comprising 6 to 30 carbon atoms,

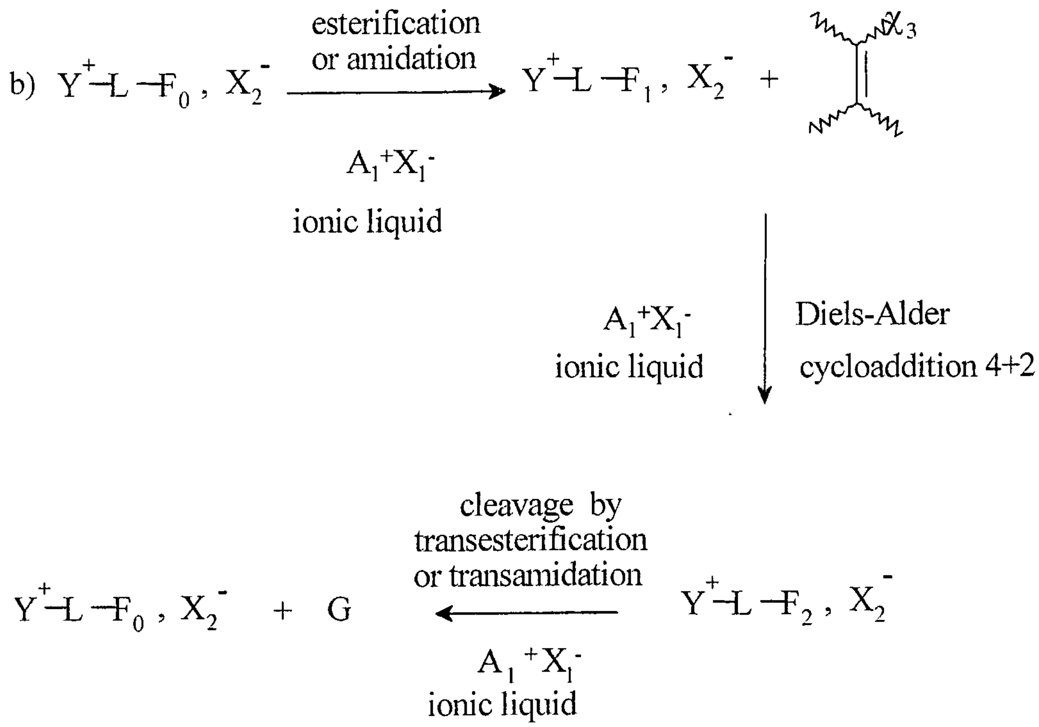


$Y^+$-, L, $X_2^-$ and the $A_1^+X_1^-$ ionic liquid being as defined previously,
the functions $F_0$, $F_1$ and $F_2$ being as defined below:

- $F_0$ represents any function making it possible to attach a 1,3-diene, and is in particular chosen from the carbonyl, amine, alkoxy, silane, stannane and borane functions, comprising 1 to 20 carbon atoms,
- $F_1$ corresponds to the following formula:

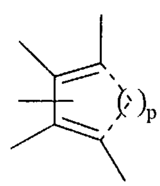

p being an integer varying from 0 to 2,
- $F_2$ corresponds to the following formula:

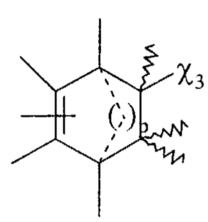

$\chi_3$ representing an electroattractive group, in particular chosen from the cyano, alkoxycarbonyl groups, comprising 1 to 20 carbon atoms, acyl groups comprising 2 to 20 carbon atoms, benzoyl, sulphonyl, dialkoxyphosphonyl groups comprising 1 to 10 carbon atoms,

G corresponding to the following formula:

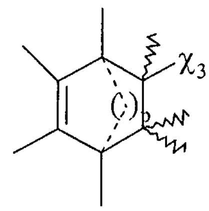

$\chi_3$ being as defined above.

**28.** The use according to claim 26, for implementation of Heck's reaction, according to the following reaction diagram:

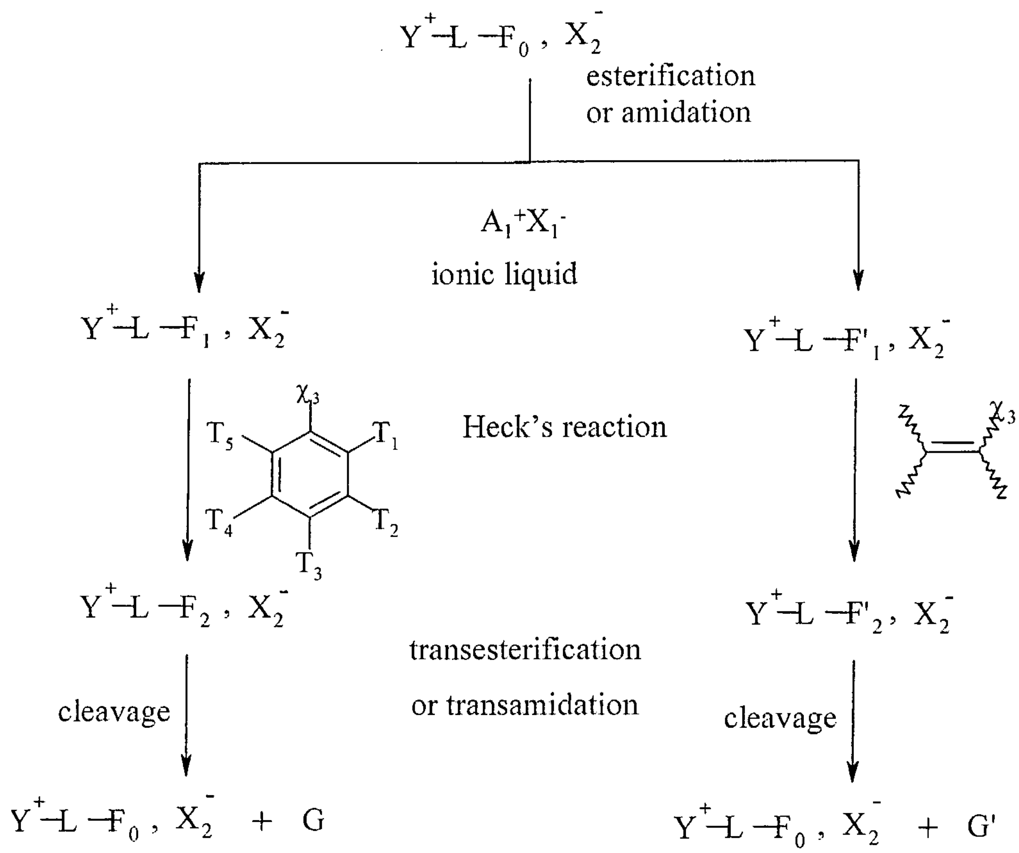


$Y^+$- representing an onium cation as defined in one of claims 21 to 26, and preferably being a trimethylalkylammonium, triethylalkylammonium or tributylalkylphosphonium cation,
L representing an linker, in particular a linear or branched alkyl group comprising 1 to 20 carbon atoms, or an optionally functional aralkyl group comprising 1 to 20 carbon atoms, and preferably being a linear alkyl group preferably a linear alkyl group of $(CH_2)_r$ type, r varying from 1 to 20, and preferably from 3 to 6,
$X_2$- being as defined in one of claims 1 to 26, and being in particular $BF_4^-$, $PF_6^-$, $NTf_2$-, $CF_3SO_3$-, $Cl^-$, $Br^-$, or $I^-$,
the $A_1^+X_1^-$ ionic liquid being in particular in the form:

$(Ra)_3N^+$-$R_b$, $X_1^-$

$(Ra)_3P^+$-$R_b$, $X_1^-$

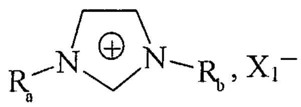

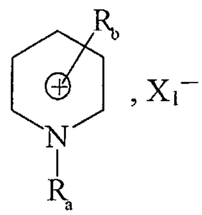

$R_a$ and $R_b$ being as defined in claim 22, and preferably representing alkyl groups comprising 1 to 20 carbon atoms, $X_1$- being chosen from: $BF_4^-$, $PF_6^-$, $NTif_2^-$, $Cl^-$, $Br^-$, $CH_3COO^-$, $CF_3CO_2^-$, $CF_3SO_3^-$, $BR_4^-$, R being as defined in claim 20, the functions $F_0$, $F_1$, $F'_1$, $F_2$ and $F'_2$ being as defined below:

- $F_0$ corresponds to a $-\chi_1H$ group, in which $\chi_1$ represents an oxygen atom or an $-NR_f$ group, $R_f$ corresponding to a linear or branched alkyl group, comprising 1 to 20 carbon atoms, or an aryl group comprising 6 to 30 carbon atoms,
- $F_1$ corresponds to the following formula:

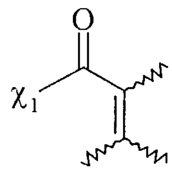

$\chi_1$ being as defined above,
- $F_2$ corresponds to the following formula:

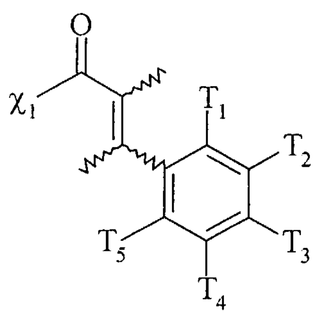

$\chi_1$ being as defined above,

G corresponding to the following formula:

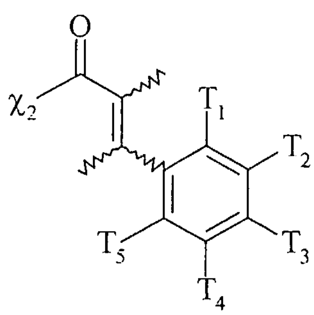

in which $\chi_2$ represents either an $-OR_g$ group, $R_g$ representing a hydrogen atom or an alkyl group comprising 1 to

20 carbon atoms, or an $-NR_hR_u$ group, $R_h$, and $R_u$ representing independently of each other a hydrogen atom, an alkyl group comprising 1 to 20 carbon atoms or an aryl group comprising 6 to 30 carbon atoms,
$\chi_3$ representing a leaving moiety, in particular chosen from the halides I, Cl and Br, the mesylate, tosylate, triflate, sulphonate, sulphate or phosphate groups,
$T_1$, $T_2$, $T_3$, $T_4$ and $T_5$ representing independently of one another a hydrogen atom, a linear or branched alkyl group, comprising 1 to 20 carbon atoms or an aryl group comprising 6 to 30 carbon atoms, or a functional group in particular chosen from $NO_2$, CN, COOR, OR, COR, NHCOR, NRR", $SO_2R$, I, Br, R and R" representing independently of each other an alkyl group comprising 1 to 20 carbon atoms or an aryl group comprising 6 to 30 carbon atoms,
the entity

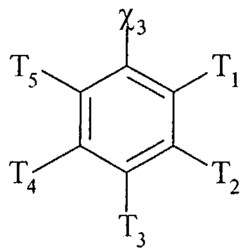

representing in particular the following groups :

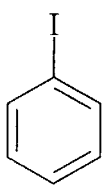
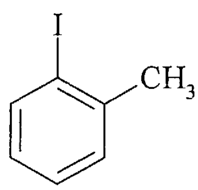
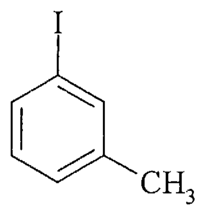
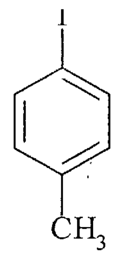
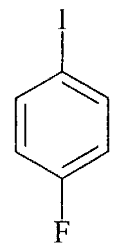

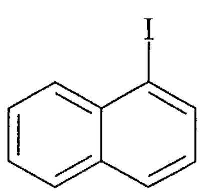
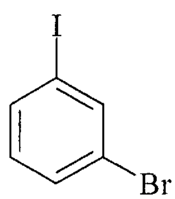
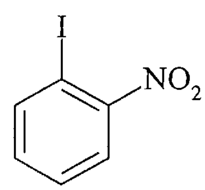
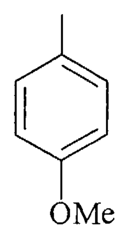

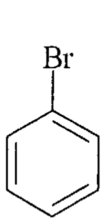
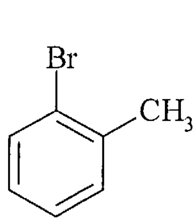
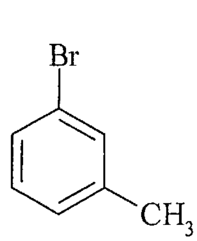
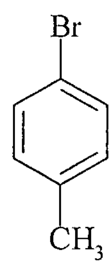
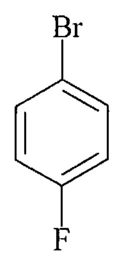

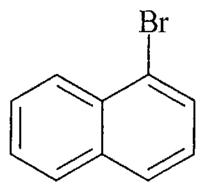 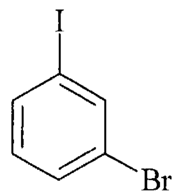 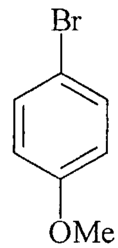

- F'$_1$ corresponds to the following formula:

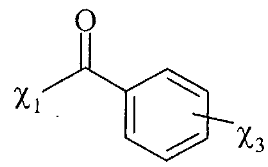

$\chi_1$ and $\chi_3$ being as defined above,
- F'$_2$ corresponds to the following formula:

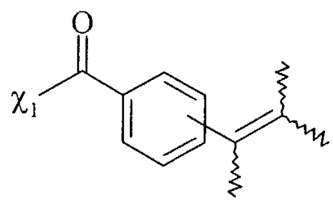

$\chi_1$ being as defined above,

G' corresponding to the following formula:

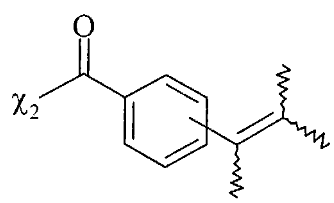

$\chi_2$ being as defined above.

**29.** The use according to claim 26, for implementation of the Baylis-Hillman reaction, according to the following reaction diagram:

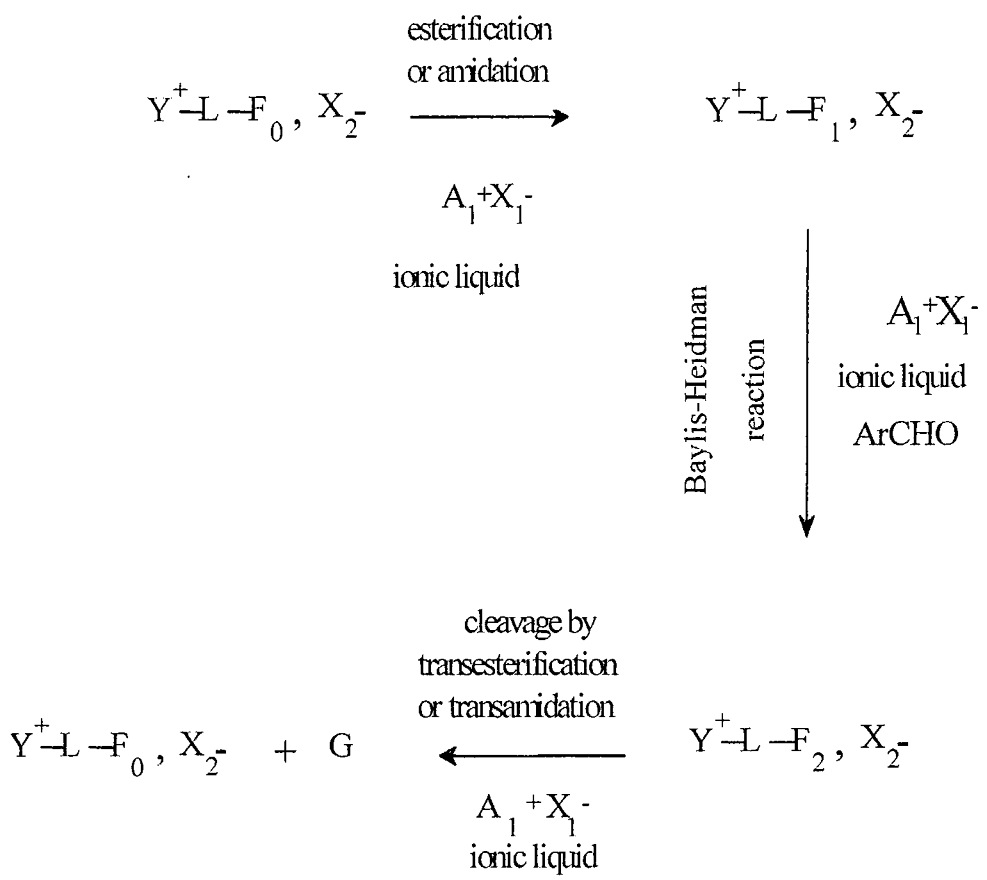


$Y^+$- representing an onium cation as defined in one of claims 21 to 26, and preferably being a trimethylalkylammonium, triethylalkylammonium or tributylalkylphosphonium cation,
L representing an linker, in particular a linear or branched alkyl group comprising 1 to 20 carbon atoms, or an optionally functional aralkyl group, comprising 6 to 30 carbon atoms, and preferably being a linear alkyl group preferably a linear alkyl group of $(CH_2)_r$ type, r varying from 1 to 20, and preferably from 3 to 6,
$X_2^-$ being as defined in one of claims 1 to 26, and being in particular $BF_4^-$, $PF_6^-$, $NTf_2^-$, $CF_3SO_3^-$, $Cl^-$, $Br^-$ , $I^-$, $CH_3CO_2^-$ or $CF_3CO_2^-$,
the $A_1^+X_1^-$ ionic liquid being in particular in the form:

$(Ra)_3N^+\text{-}R_b, X_1^-$

$(Ra)_3P^+\text{-}R_b, X_1^-$

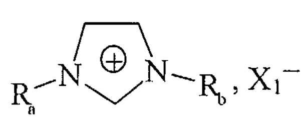

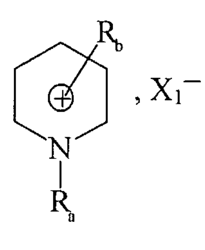

$R_a$ and $R_b$ being as defined in claim 22, and preferably representing alkyl groups comprising 1 to 20 carbon atoms, $X_1$- being chosen from: $BF_4^-$, $PF_6^-$, $NTf_2^-$, $Cl^-$, $Br^-$, $CH_3COO^-$, $CF_3CO_2^-$, $CF_3SO_3^-$, $BR_4^-$, R being as defined in claim 20, the functions $F_0$, $F_1$ and $F_2$ being as defined below:

- $F_0$ represents an -OH group,
- $F_1$ corresponds to the following formula:

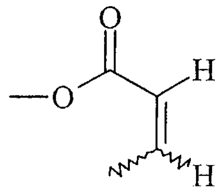

- $F_2$ corresponds to the following formula:

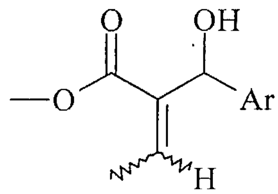

G corresponding to the following formula: i

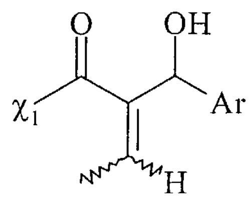

$\chi_1$ representing an -OH group, or an -$OR_g$ group, $R_g$ representing a linear or branched alkyl group, comprising 1 to 20 carbon atoms,
Ar representing a substituted or non-substituted, aromatic or heteroaromatic group,
ArCHO being in particular chosen from:

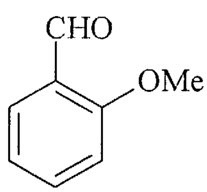
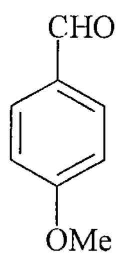
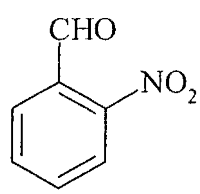
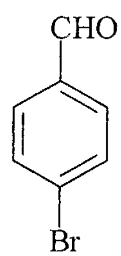

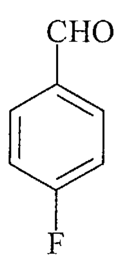
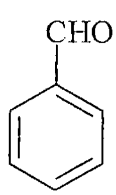
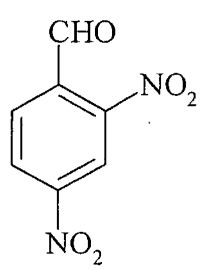
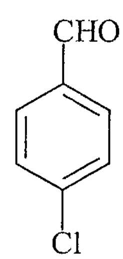

**30.** The use according to claim 26, for implementation of Suzuki coupling, according to one of the following reaction diagrams:

a)

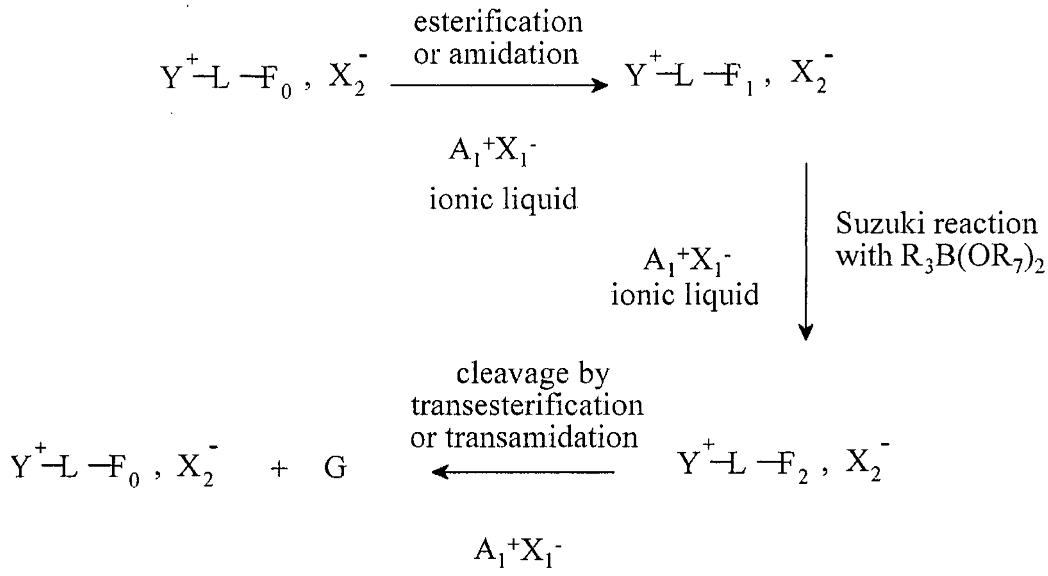


$R_3$ being chosen from the substituted or non-substituted aryl, heteroaryl, ethenyl, dienyl, allyl, ethynyl groups, comprising 2 to 30 carbon atoms,
$R_7$ represents a branched or linear alkyl group or a cycloalkyl group comprising 1 to 12 carbon atoms,
$Y^+$- representing an onium cation as defined in one of claims 21 to 26, and preferably being a trimethylalkylammonium, triethylalkylammonium or tributylalkylphosphonium cation,
L representing an linker, in particular a linear or branched alkyl group comprising 1 to 20 carbon atoms, or an optionally functional aralkyl group comprising 6 to 30 carbon atoms, and preferably being a linear alkyl group preferably a linear alkyl group of $(CH_2)_r$ type, r varying from 1 to 20, and preferably from 3 to 6,
$X_2^-$ being as defined in one of claims 1 to 26, and being in particular $NTf_2^-$, $BF_4^-$ , $PF_6^-$, $Cl^-$, $Br^-$, $CH_3COO^-$, $CF_3CO_2^-$, $CF_3SO_3^-$, $BR_4^-$, R being as defined in claim 20,
the $A_1^+X_1^-$ ionic liquid being in particular in the form:

$(Ra)_3N^+-R_b$, $X_1^-$

$(Ra)_3P^+-R_b$, $X_1^-$

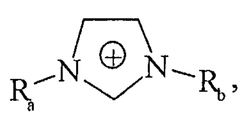 , $X_1^-$ 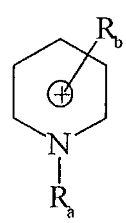 , $X_1^-$

$R_a$ and $R_b$ being as defined in claim 22, and preferably representing alkyl groups comprising 1 to 20 carbon atoms,
$X_1^-$ being chosen from: $BF_4^-$, $PF_6^-$, $NTf_2^-$, $Cl^-$, $Br^-$, $CH_3COO^-$. $CF_3CO_2^-$, $CF_3SO_3^-$, $BR_4^-$, R being as defined in claim 20,
the functions $F_0$, $F_1$ and $F_2$ being as defined below:

- $F_0$ is in the form $-\chi_1H$, $\chi_1$ representing an oxygen atom or an
- $NR_f$ group, $R_f$ corresponding to a linear or branched alkyl group, comprising 1 to 20 carbon atoms, or an aryl group comprising 6 to 30 carbon atoms,
- $F_1$ is in the form $-R_e-\chi$, $R_e$ representing an aromatic or heteroaromatic group comprising 6 to 30 carbon atoms, $\chi$ representing a leaving group preferably chosen from Cl, Br, I, OTf, $O-CO_2R^5$ or $OSO_3-R^5$, $R^5$ representing an alkyl group comprising 1 to 10 carbon atoms or an aralkyl group comprising 6 to 30 carbon atoms, $F_1$ preferably corresponding to the following formula:

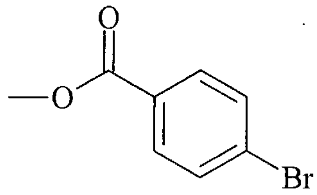

- $F_2$ is in the form $-R_e-R_2$, $R_e$ being as defined above and $R_2$ being chosen from the substituted or non-substituted aryl, heteroaryl, ethenyl, dienyl, allyl, ethynyl groups, comprising 2 to 30 carbon atoms, $F_2$ preferably corresponding to the following formula:

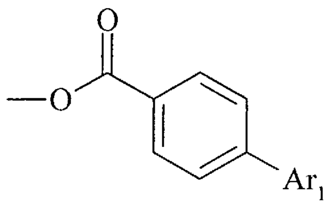

$Ar_1$ representing an aromatic group preferably chosen from:

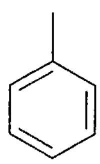 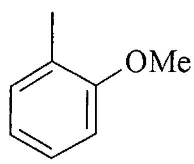 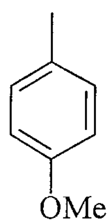 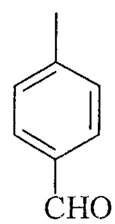 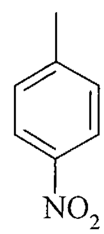

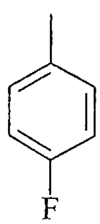 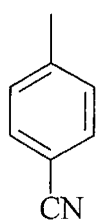 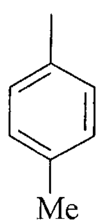 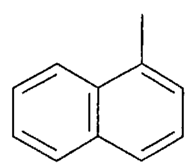

the G molecule being in the form $R_2-R_3$, $R_2$ and $R_3$ being as defined above, and corresponds in particular to the following formula:

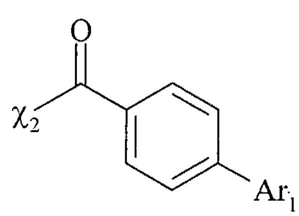

in which $\chi_2$ represents either an -ORg group, Rg representing a hydrogen atom or an alkyl group comprising 1 to 20 carbon atoms, i.e. an $-NR_hR_u$ group, $R_h$ and $R_u$ representing independently of each other a hydrogen atom, an alkyl group comprising 1 to 20 carbon atoms or an aryl group comprising 6 to 30 carbon atoms,

$Ar_1$ is as defined above,

**b)**

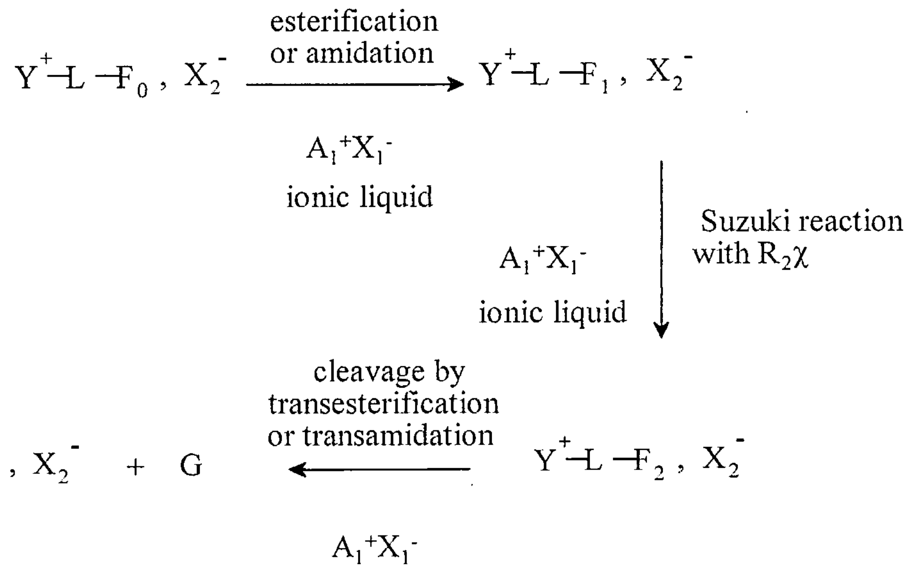


$Y^+$-, L, $X_2^-$, $A_1^+X_1^-$ and $R_2$ being as defined above, the functions $F_0$, $F_1$ and $F_2$ being as defined below:

- $F_0$ is in the form $-\chi_1H$, $\chi_1$ being as defined above,
- $F_1$ is in the form $-R_q-B(OR_7)_2$, $R_7$ being as defined above, and $R_q$ corresponding to an aryl group comprising 6 to 30 carbon atoms, heteroaryl group comprising 4 to 20 carbon atoms, ethenyl group comprising 2 to 20 carbon atoms, dienyl group comprising 3 to 20 carbon atoms, allyl group comprising 3 to 20 carbon atoms, ethynyl group comprising 2 to 20 carbon atoms, substituted or non-substituted, $F_1$ preferably corresponding to the following formula:

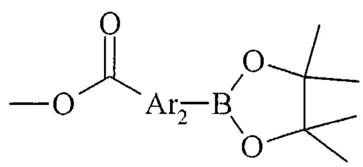

$Ar_2$ corresponding to a substituted or non-substituted aryl group comprising 6 to 30 carbon atoms,

- $F_2$ is in the form $-R_q-R_e$, $R_q$ and $R_e$ being as defined above, $F_2$ preferably corresponding to the following formula:

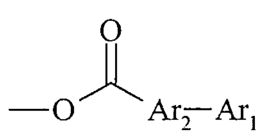

$Ar_1$ representing an aromatic group preferably chosen from:

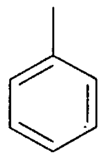
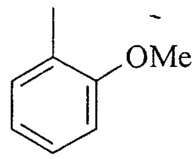
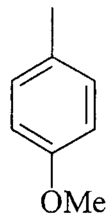
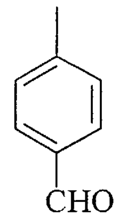
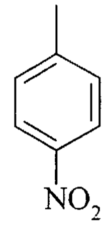

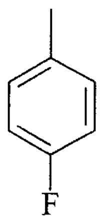
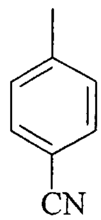
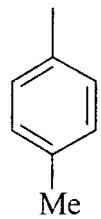
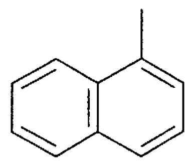

the G molecule being in the form $R_2$-$R_3$, $R_2$ and $R_3$ being as defined above, and corresponding in particular to the following formula:

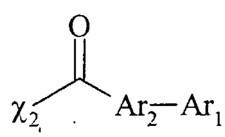

in which $\chi_2$, $Ar_1$ and $Ar_2$ are as defined above,

**c)**

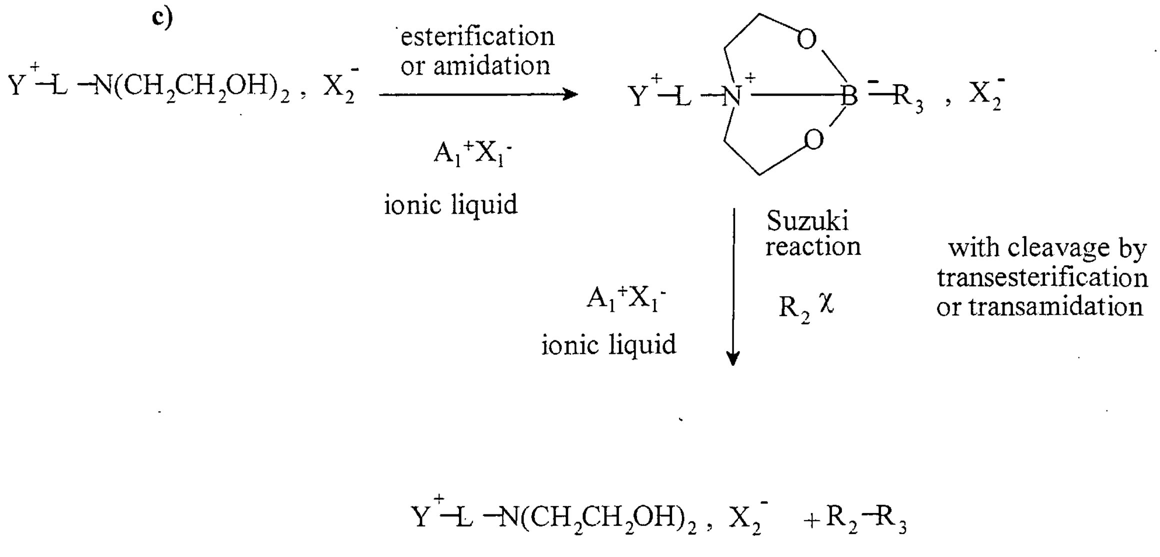


$Y^+$-, L, $X_2^-$, $A_1^+X_1^-$, $R_2$ and $R_3$ being as defined above,
$R_3$ preferably being a phenyl group,

d)

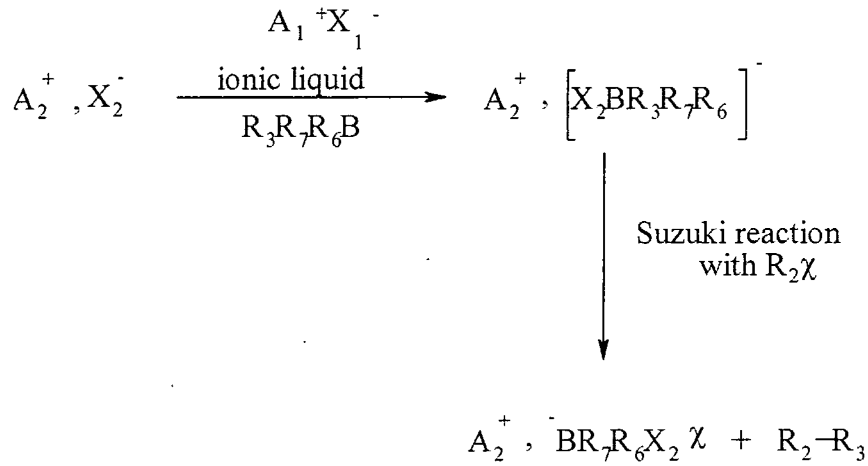


$A_2^+$ being an $(R_a)_3N^+R_b$ ammonium or $(R_a)_3P^+R_b$ phosphonium cation, preferably tetrabutylammonium and tetramethylammonium, $R_a$ and $R_b$ being as defined above,

$X_2^-$ being in particular chosen from $OH^-$, $F^-$, $CN^-$, $R_sO^-$, $R_sS^-$, preferably $OH^-$ or $F^-$, $R_s$ representing an alkyl group comprising 1 to 20 carbon atoms or an aryl group comprising 6 to 30 carbon atoms,

$R_3$ and $R_4$ being as defined above,

$R_6$ and $R_7$ representing independently of each other an alkyl group comprising 1 to 20 carbon atoms or an aryl group comprising 6 to 30 carbon atoms,

the boronic molecule of formula $R_3R_7R_6B$ being a trialkyl or aryl borane, the alkyl group comprising 1 to 20 carbon atoms and the aryl group comprising 6 to 30 carbon atoms, a boronic acid or ester, preferably a boronic acid or ester chosen as being phenyl boronic acid,

$R_2$ and $\chi$ are as defined previously, $R_2\chi$ preferably corresponding to an aryl halide chosen from:

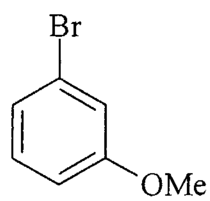

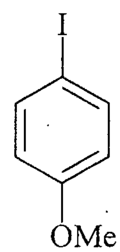

**31.** The use according to claims 1 to 30 for the synthesis of molecule libraries according to the parallel synthesis technique, according to the following diagram:

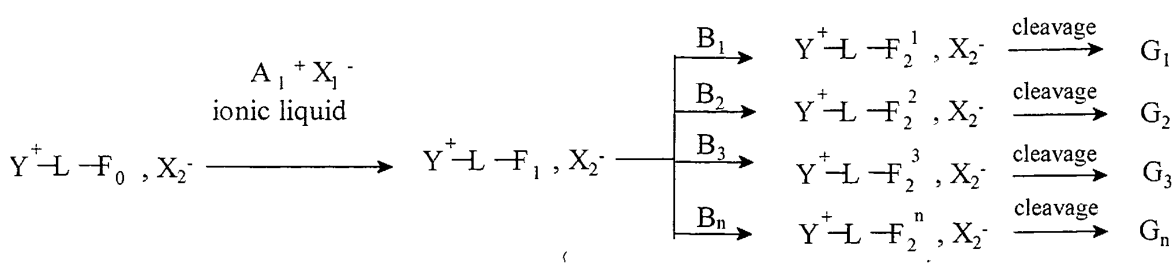


**characterized in that** the $Y^+$-L-$F_1$, $X_2^-$ functionalized salt in the $A_1^+$, $X_1^-$ ionic liquid is separated into n approximately

equal parts, n varying from 2 to 1024, and **in that** each of these parts is then converted according to an organic synthesis reaction, preferably a Heck or Suzuki coupling reaction, each using a different reagent $B_i$ in order to produce n solutions each containing a defined $Y^+$-L-$F_2^i$, $X_2^-$ compound, $F_2^i$ representing a function chosen from the functions as defined in claim 21, i varying from 1 to n, each solution being treated in order to release the $G_i$ molecules, i varying from 1 to n, which are each isolated and purified, constituting a molecule library.

**32.** The use according to claims 1 to 30, for implementation of the synthesis of molecule libraries by the split-and-mix technique according to the following diagram:

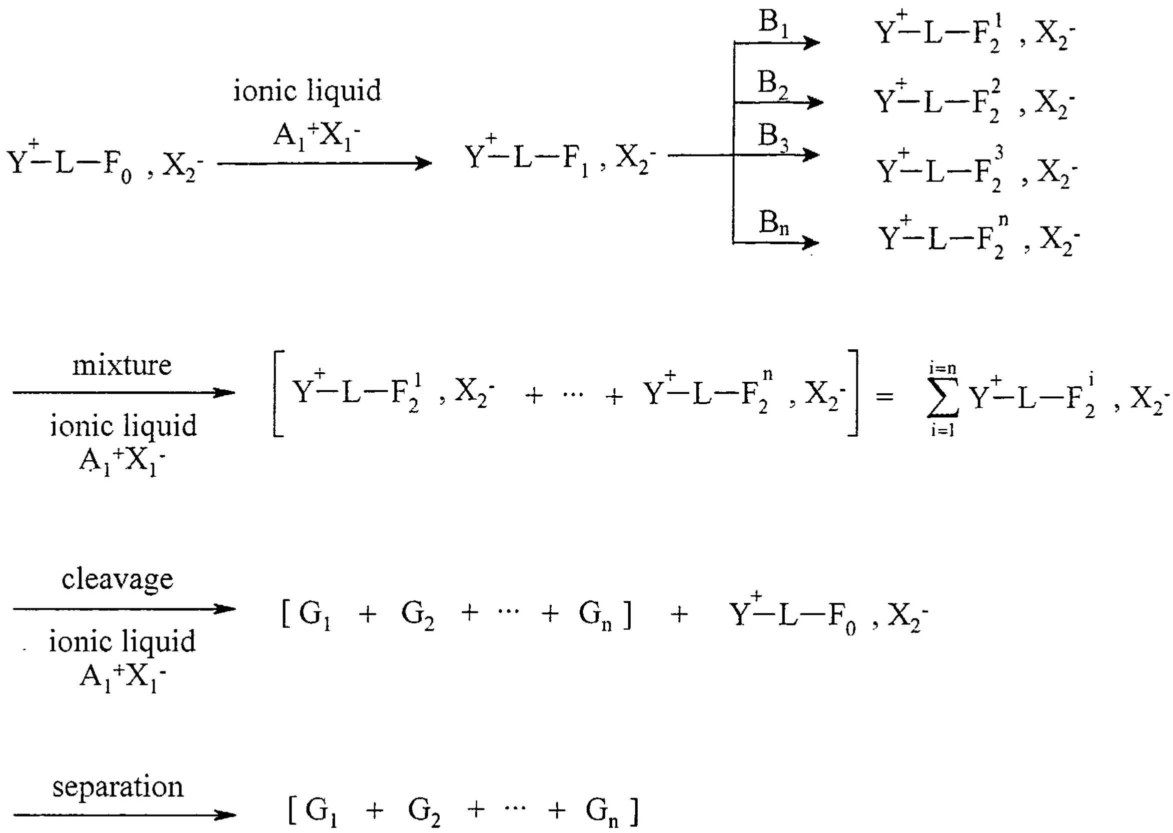


**characterized in that**:

- n fractions of the $Y^+$-L-$F_1$, $X_2$- solution, obtained from the starting $Y^+$-L-$F_0$, $X_2^-$ functionalized salt, in the $A_1^+X_1$- ionic liquid are converted in parallel according to an organic chemistry reaction, preferably a Heck or Suzuki coupling reaction, each using a different reagent B; in order to produce n solutions each containing a defined $Y^+$-L-$F_2^i$, $X_2^-$ compound, i varying from 1 to n, n varying from 2 to 1024, preferably from 2 to 96, $F_2^i$ representing a function chosen from the functions as defined in claim 21,
- the n solutions obtained in the preceding stage are mixed in order to produce a solution in the $A_1$+$X_1$- ionic liquid containing the n $Y^+$-L-$F_2^i$, $X_2^-$ products, i varying from 1 to n, annotated $\sum_{i=1}^{i=n} Y^+{-}L{-}F_2^i$, $X_2^-$, and this solution is subjected to a cleavage stage, preferably a transesterification or a transamidation, in order to obtain in solution in the $A_1^+X_1^-$ ionic liquid, a mixture of the n G; molecules, i varying from 1 to n, and the starting $Y^+$-L-$F_0$, $X_2^-$ functionalized salt,
- the mixture as obtained in the preceding stage is separated from the $A_1^+X_1^-$ ionic liquid and from the starting $Y^+$-L-$F_0$, $X_2^-$ functionalized salt by the usual separation methods, preferably by vacuum distillation, by extraction with a standard solvent such as heptane or toluene followed by evaporation of the solvent, by chromatography on a column, plates or under pressure, in order to obtain a library containing n G; molecules

this sequence of stages mentioned above being possibly repeated j times, j being comprised between 2 and 10, in order to obtain j different libraries of n products.

## Patentansprüche

1. Verwendung einer ionischen Flüssigkeit als flüssige Matrix für die organische Synthese in homogener Phase auf löslichem Träger ohne flüchtiges organisches Lösungsmittel, wobei sich die ionische Flüssigkeit bei Umgebungstemperatur in flüssiger oder fester Form zeigt, mit der Formel $A_1^+X_1^-$, wobei $A_1^+$ ein funktionelles Kation oder ein nicht funktionelles Kation oder eine Mischung von Kationen ist, in welcher entweder keines der Kationen funktionell ist, oder mindestens eines der Kationen funktionell ist, und wobei $X_1^-$ ein funktionelles Anion oder ein nicht funktionelles Anion, oder eine Mischung von Anionen ist, in welcher entweder keines der Anionen funktionell ist, oder mindestens eines der Anionen funktionell ist.

2. Verwendung gemäß Anspruch 1, **dadurch gekennzeichnet, dass** $A_1^+$ ein nicht funktionelles Kation oder eine Mischung nicht funktioneller Kationen und $X_1^-$ ein nicht funktionelles Anion oder eine Mischung nicht funktioneller Anionen darstellt.

3. Verwendung gemäß Anspruch 1, **dadurch gekennzeichnet, dass**
$A_1^+$ ein funktionelles Kation oder eine Mischung von Kationen, von denen mindestens eines funktionell ist, darstellt, und/oder $X_1^-$ ein funktionelles Anion oder eine Mischung von Anionen, von denen mindestens eines funktionell ist, darstellt,
wobei die funktionellen Kationen und die funktionellen Anionen einer ionischen Gesamtheit, nämlich jeweils einer kationischen oder anionischen, entsprechen, die an mindestens eine Gruppe F; gebunden ist, wobei $F_i$ von $F_0$ bis $F_n$ variiert, wobei n eine ganze Zahl ist, die zwischen 1 und 10 variiert.

4. Verwendung einer ionischen Flüssigkeit gemäß einem der Ansprüche 1 oder 2 für die Herstellung einer stabilen Zusammensetzung enthaltend in Lösung:

    - mindestens die ionische Flüssigkeit der Formel $A_1^+ X_1^-$, die die Rolle der flüssigen Matrix spielt und,
    - mindestens ein funktionalisiertes Salz (Salz mit zugeordneter Aufgabe), insbesondere ein funktionalisiertes Oniumsalz, mit der Formel $A_2^+X_2^-$ als Reaktionsträger,

    wobei das funktionalisierte Salz, insbesondere das funktionalisierte Oniumsalz in der flüssigen Matrix aufgelöst ist, um eine homogene Phase zu bilden,
    wobei $A_1^+$ ein nicht funktionelles Kation oder eine Mischung von Kationen, in welcher keines der Kationen funktionell ist, darstellt, und $X_1^-$ ein nicht funktionelles Anion oder eine Mischung von Anionen, in welcher keines der Anionen funktionell ist, darstellt,
    wobei $A_2^+$ ein funktionelles Kation oder ein nicht funktionelles Kation oder eine Mischung von Kationen, in welcher keines der Kationen funktionell ist oder, in welcher mindestens eines der Kationen funktionell ist, darstellt, und $X_2$- ein funktionelles Anion oder ein nicht funktionelles Anion oder eine Mischung von Anionen, in welcher keines der Anionen funktionell ist oder, in welcher mindestens eines der Anionen funktionell ist, darstellt,
    unter dem Vorbehalt dass $A_2^+$ und/oder $X_2^-$ jeweils ein funktionelles Kation und ein funktionelles Anion darstellen oder enthalten,
    wobei die funktionellen Kationen und funktionellen Anionen einer ionischen Gesamtheit Y-entsprechen, nämlich jeweils kationisch $Y^+$- oder anionisch $Y^-$-, die gegebenenfalls über einen L-Arm, insbesondere eine Alkylgruppe mit 1 bis 20 Kohlenstoffatomen, mit mindestens einer Gruppe $F_i$ verbunden ist, wobei $F_i$ zwischen $F_0$ und $F_n$ variiert, wobei n eine ganze Zahl ist, die zwischen 1 und 10 variiert, wobei das funktionelle Kation in der Form $Y^+$- L- $F_i$ und das funktionelle Anion in der Form $Y^-$-$(L)_k$ -$F_i$ dargestellt werden kann, wobei k gleich 0 oder 1 ist, und das funktionelle Anion, wenn k gleich 0 ist, ein einfaches Anion darstellen kann, entsprechend $Y^-$- $F_i$, insbesondere ausgewählt aus: $OH^-$, $F^-$, $CN^-$, RO- oder $RS^-$, wobei R eine Alkylgruppierung mit 1 bis 20 Kohlenstoffatomen oder eine Arylgruppierung mit 6 bis 30 Kohlenstoffatomen darstellt.

5. Verwendung gemäß Anspruch 3 für die Herstellung einer stabilen Zusammensetzung, enthaltend in Lösung:

    - mindestens einen ersten Teil der ionischen Flüssigkeit der Formel $A_1^+X_1^-$, deren Kation und/oder Anion einer ionischen Gesamtheit entspricht (entsprechen), die an eine oder mehrere Anfangsgruppen $F_0$ gebunden ist, welcher die Rolle einer flüssigen Matrix spielt, und

- mindestens einen zweiten Teil der ionischen Flüssigkeit der Formel $A_l^+X_1^-$, in welchem die genannte(n) Anfangsgruppe(n) $F_0$ in erste neue Gruppen transformiert wird (werden), was dem zweiten Teil der ionischen Flüssigkeit die Rolle eines funktionalisierten Salzes und eines Reaktionsträgers verleiht,

wobei das funktionalisierte Salz und die flüssige Matrix eine homogene Phase bilden,
wobei die oben genannten ersten neuen Gruppen des zweiten Teils der ionischen Flüssigkeit fähig sind, weiter in andere Gruppen transformiert zu werden, ohne dass es eine Auswirkung auf die eine oder mehreren Anfangsfunktionen $F_0$ des ersten Teils der ionischen Flüssigkeit hat.

**6.** Verwendung einer ionischen Flüssigkeit gemäß Anspruch 4, **dadurch gekennzeichnet, dass** das Kation $A_2^+$ und/oder das Anion $X_2^-$ des oder der funktionalisierten Salze, entsprechend einer ionischer Gesamtheit Y-, gebunden an mindestens eine Gruppe $F_i$, in der flüssigen Matrix immobilisiert sind, und nicht durch herkömmliche Extraktionsmittel, insbesondere durch Lösungsmittel aus der flüssigen Matrix extrahiert werden können, und in welcher die Gruppe(n) $F_i$ des oder der funktionalisierten Salze am Ende mindestens einer Reaktion, die aus der Zugabe von mindestens einem Reagenz zu der genannten Zusammensetzung resultiert, transformiert werden kann (können).

**7.** Verwendung einer ionischen Flüssigkeit gemäß Anspruch 6, **dadurch gekennzeichnet, dass** mehrere funktionalisierte Salze immobilisiert sind.

**8.** Verwendung gemäß einem der Ansprüche 4, 6 oder 7, **dadurch gekennzeichnet, dass** das Kation $A_2^+$ funktionell ist.

**9.** Verwendung gemäß einem der Ansprüche 4, 6 oder 7, **dadurch gekennzeichnet, dass** das Anion $X_2^-$ funktionell ist.

**10.** Verwendung gemäß einem der Ansprüche 4, 6 oder 7 bis 9, **dadurch gekennzeichnet, dass** $A_2^+$ und $X_2^-$ funktionell sind.

**11.** Verwendung gemäß einem der Ansprüche 1, 2, 4, 6 bis 10, **dadurch gekennzeichnet, dass**

- entweder die ionische Flüssigkeit der Formel $A_1^+X_1^-$ bei Umgebungstemperatur fest ist, und in einem Temperaturbereich zwischen ungefähr 25 °C und ungefähr 250 °C, insbesondere zwischen ungefähr 30°C und ungefähr 150 °C verflüssigbar ist, und das funktionalisierte Salz $A_2^+$X2 bei Umgebungstemperatur fest ist, und in der verflüssigten ionischen Flüssigkeit $A_1^+X_1^-$ löslich ist, um eine homogene Phase zu bilden,
- oder die ionische Flüssigkeit der Formel $A_1^+X_1^-$ bei Umgebungstemperatur fest ist und in einem Temperaturbereich zwischen ungefähr 25 °C und ungefähr 250 °C, insbesondere zwischen ungefähr 30 °C und ungefähr 150 °C verflüssigbar ist und das funktionalisierte Salz $A_2^+X_2^-$ ist bei Umgebungstemperatur flüssig ist und mit der verflüssigten ionischen Flüssigkeit $A_1^+X_1^-$ mischbar ist, um eine homogene Phase zu bilden,
- oder die ionische Flüssigkeit $A_1^+X_1^-$ bei Umgebungstemperatur flüssig ist und das funktionalisierte Salz $A_2^+X_2^-$ bei Umgebungstemperatur flüssig ist und mit der ionischen Flüssigkeit $A_1^+X_1^-$ mischbar ist, um eine homogene Phase zu bilden,
- oder die ionische Flüssigkeit $A_1^+X_1^-$ bei Umgebungstemperatur flüssig ist und das funktionalisierte Salz $A_2^+X_2^-$ bei Umgebungstemperatur fest ist und in der ionischen Flüssigkeit $A_1^+X_1^-$ in einem Temperaturbereich, der zwischen ungefähr 25 °C und ungefähr 250 °C, insbesondere zwischen ungefähr 30 °C und ungefähr 150 °C liegt, löslich oder teilweise löslich ist, um eine homogene Phase zu bilden.

**12.** Verwendung gemäß Anspruch 5, **dadurch gekennzeichnet, dass**:

- entweder die ionische Flüssigkeit der Formel $A_1^+X_1^-$ bei Umgebungstemperatur flüssig ist,
- oder die ionische Flüssigkeit der Formel $A_1^+X_1^-$ bei Umgebungstemperatur fest ist, und in einem Temperaturbereich zwischen ungefähr 25 °C und ungefähr 250 °C, insbesondere zwischen ungefähr 30 °C und ungefähr 150 °C verflüssigbar ist.

**13.** Verwendung gemäß einem der Ansprüche 1 bis 12, **dadurch gekennzeichnet, dass** die ionische Flüssigkeit der Formel $A_1^+X_1^-$, die die Rolle einer flüssigen Matrix spielt, eine Viskosität unter oder gleich ungefähr 1500 cp (15 N.s/$m^2$), insbesondere unter ungefähr 500 cp (5 N.s/$m^2$) und bevorzugt unter ungefähr 200 cp (2 N.s/$m^2$) aufweist.

**14.** Stabile Zusammensetzung enthaltend in Lösung:

- mindestens die genannte ionische Flüssigkeit der Formel $A_1^+X_1^-$, die die Rolle einer flüssigen Matrix spielt, und

- mindestens ein funktionalisiertes Salz (Salz mit zugeordneter Aufgabe), insbesondere funktionalisiertes Oniumsalz, der Formel $A_2^+X_2^-$ als Reaktionsträger,

wobei das funktionalisierte Salz, insbesondere das funktionalisierte Oniumsalz in der flüssigen Matrix aufgelöst ist, um eine homogene Phase zu bilden,
wobei $A_1^+$ ein nicht funktionelles Kation oder eine Mischung von Kationen, in welcher keines der Kationen funktionell ist, darstellt, und $X_1^-$ ein nicht funktionelles Anion oder eine Mischung von Anionen, in welcher keines der Anionen funktionell ist, darstellt,
wobei $A_2^+$ ein funktionelles oder nicht funktionelles Kation oder eine Mischung von Kationen, in welcher keines der Kationen funktionell ist, oder in welcher mindestens eines der Kationen funktionell ist, darstellt, und $X_2^-$ ein funktionelles oder nicht funktionelles Anion oder eine Mischung von Anionen, in welcher keines der Anionen funktionell ist, oder in welcher mindestens eines der Anionen funktionell ist, darstellt,
unter dem Vorbehalt, dass $A_2^+$ und/oder $X_2^-$ jeweils ein funktionelles Kation und ein funktionelles Anion darstellen oder enthalten,
wobei die funktionellen Kationen und funktionellen Anionen einer ionischen Gesamtheit Y- entsprechen, nämlich jeweils kationisch $Y^+$- oder anionisch $Y^-$-, die gegebenenfalls über einen L-Arm, insbesondere eine Alkylgruppe mit 1 bis 20 Kohlenstoffatomen, mit mindestens einer Gruppe $F_i$ verbunden ist, wobei F; zwischen $F_0$ und $F_n$ variiert, wobei n eine ganze Zahl ist, die zwischen 1 und 10 variiert, wobei das funktionelle Kation in der Form $Y^+$- L-$F_i$ dargestellt werden kann und das funktionelle Anion in der Form $Y^-$-$(L)_k$-$F_i$ dargestellt werden kann, wobei k 0 oder 1 ist und das funktionelle Anion, wenn k gleich 0 ist, ein einfaches Anion entsprechend $Y^-$-$F_i$ darstellen kann, insbesondere ausgewählt aus: $OH^-$, $F^-$, $CN^-$, $RO^-$ oder $RS^-$, wobei R eine Alkylgruppierung mit 1 bis 20 Kohlenstoffatomen oder eine Arylgruppierung mit 6 bis 30 Kohlenstoffatomen darstellt.

**15.** Stabile Zusammensetzung enthaltend in Lösung:

- mindestens einen ersten Teil der ionischen Flüssigkeit der Formel $A_1^+X_1^-$, deren Kation und/oder Anion einer ionischen Gesamtheit entspricht (entsprechen), die an eine oder mehrere Anfangsgruppen $F_0$ gebunden ist, der die Rolle einer flüssigen Matrix spielt, und
- mindestens einen zweiten Teil der ionischen Flüssigkeit der Formel $A_1^+X_1^-$, in welchem die eine oder die mehreren Anfangsgruppen $F_0$ in erste neue Gruppen transformiert werden, was dem zweiten Teil der ionischen Flüssigkeit, die Rolle eines funktionalisierten Salzes und eines Reaktionsträgers verleiht,

wobei das funktionalisierte Salz und die flüssige Matrix eine homogene Phase bilden,
wobei die oben genannten ersten neuen Gruppen des zweiten Teils der ionischen Flüssigkeit fähig sind weiter in andere Gruppen transformiert zu werden, ohne dass es eine Auswirkung auf die eine oder mehreren Anfangsgruppen $F_0$ des ersten Teils der genannten ionischen Flüssigkeit hat.

**16.** Zusammensetzung gemäß Anspruch 14, **dadurch gekennzeichnet, dass** das Kation $A_2^+$ und/oder das Anion $X_2^-$ des oder der funktionalisierten Salze, entsprechend einer ionischer Gesamtheit Y-, gebunden an mindestens eine Gruppe $F_i$ in der flüssigen Matrix immobilisiert sind, und nicht durch herkömmliche Extraktionsmittel, insbesondere durch Lösungsmittel, aus der flüssigen Matrix extrahiert werden können.

**17.** Zusammensetzung gemäß irgendeinem der Ansprüche 14 bis 16, **dadurch gekennzeichnet, dass** die flüssige Matrix gegenüber dem funktionalisierten Salz nicht reaktiv ist.

**18.** Zusammensetzung gemäß einem der Ansprüche 14, 16 und 17, **dadurch gekennzeichnet, dass** $A_2^+$ ein funktionelles Kation ist.

**19.** Zusammensetzung gemäß Anspruch 18, **dadurch gekennzeichnet, dass** die Anionen $X_1^-$ und $X_2^-$ identisch sind.

**20.** Zusammensetzung gemäß einem der Ansprüche 18 oder 19, **dadurch gekennzeichnet, dass**

- die Anionen $X_1^-$ und $X_2^-$ aus den folgenden Familien ausgewählt sind

* den nicht komplexen Anionen, ausgewählt insbesondere unter den Anionen $BF_4^-$, $PF_6^-$, $CF_3SO_3^-$, $CH_3COO^-$, $CF_3CO_2^-$,$^-N(SO_2CF_3)_2$ (oder $NTf_2^-$), den Halogeniden, den Anionen $BR_4^-$ , $RCO_2^-$ oder $RSO_3^-$, wobei R eine Alkylgruppe ist, die 1 bis 20 Kohlenstoffatome enthält oder eine Arylgruppe ist, die 6 bis 30 Kohlenstoffatome enthält, wobei die Gruppe R auch eine perfluorierte oder partiell fluorierte Gruppe dargestellen kann, oder den Anionen $R'SO_4^-$, wobei R' ein Wasserstoffatom, eine Methylgruppe oder eine Ethylgruppe ist;

* den komplexen Anionen, die aus der Kombination einer Lewissäure und einem Halogenid, vorzugsweise $Cl^-$ oder F- resultieren, mit der allgemeinen Formel $MX_j$, wobei j eine ganze Zahl zwischen 1 und 7 ist, und M ein Metall darstellt, insbesondere ausgewählt unter Aluminium, Zinn, Zink, Wismut, Mangan, Eisen, Kupfer, Molybdän, Antimon, Gallium oder Indium;

- die Kationen $A_1^+$ und $A_2^+$ ausgewählt sind aus den Oniumkationen, wie den substituierten oder nicht substituierten Pyridinium-, Imidazolium-, Ammonium-, Phosphonium-, und Sulfonium-Kationen und vorzugsweise den Ammonium-, oder Phosphonium-Kationen.

**21.** Zusammensetzung gemäß irgendeinem der Ansprüche 14 und 16 bis 20, **dadurch gekennzeichnet, dass** das funktionelle Kation $A_2^+$ einer kationischen Gesamtheit $Y^+$- entspricht, die gegebenenfalls über einen L-Arm, insbesondere eine Alkylgruppe, die 1 bis 20 Kohlenstoffatome enthält, an eine Gruppe $F_0$ gebunden ist, wobei die genannte Gruppe $F_0$ ausgewählt ist aus den klasischen Gruppen der organischen Chemie, wie den Hydroxyl-, Carboxyl-, Amid-, Sulfon-, Primäramin-, Sekundäramin-, Aldehyd-, Keton-, Ethenyl-, Ethynyl-, Dienyl-, Ether-, Epoxyd-, Phosphin- (primär, sekundär oder tertiär), Nitrid-, Imin-, Keten-, Cumulen-, Heterocumulen-, Thiol-, Thiolether-, Sulfoxydgruppen, phosphorisierten hetrozyklischen Gruppierungen, Sulfonsäure, Silan, Stannan, oder funktionelles Aryl.

**22.** Zusammensetzung gemäß irgendeinem der Ansprüche 14 bis 21, **dadurch gekennzeichnet, dass** die ionische Flüssigkeit ausgewählt ist aus den Folgenden:

$(Ra)_3N^+-R_b, X_1^-$

$(Ra)_3P^+-R_b, X_1^-$

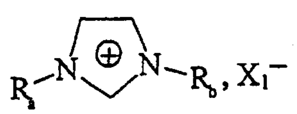

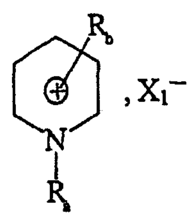

wobei $R_a$ und $R_b$ lineare oder verzweigte Alkylgruppen darstellen mit 1 bis 20 Kohlenstoffatomen, insbesondere eine Ethyl-, Propyl-, Butyl-, Pentyl-, Hexyl-, Heptyl- oder Octylgruppe oder funktionelle Alkylgruppen mit 1 bis 20 Kohlenstoffatomen oder funktionelle oder nicht funktionelle Arylgruppen mit 6 bis 30 Kohlenstoffatomen,

$Bu_3P^+-Me, X_1^-\ {}^{\oplus}P(C_6H_{13})_3C_{14}H_{29}, X_1^-\ (C_8H_{17})_3N^+Me, X_1^-$

wobei $X_1^-$ insbesondere ausgewählt ist aus: $NTf_2^-$, $PF_6^-$, $BF_4^-$, oder $CF_3SO_3^-$.

**23.** Zusammensetzung nach irgendeinem der Ansprüche 14 und 16 bis 22, **dadurch gekennzeichnet, dass** das funktionalisierte Salz aus den folgenden ausgewählt ist:

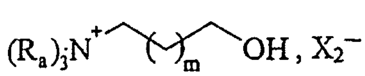

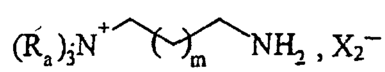

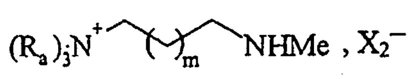

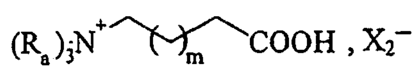

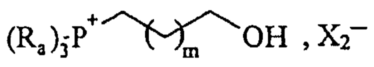

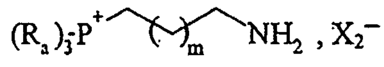

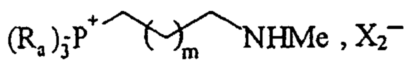

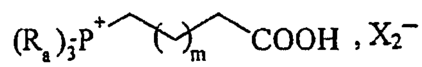

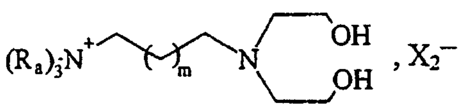

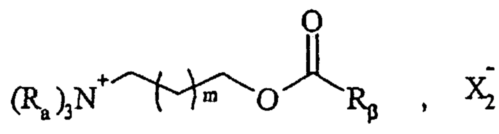

$X_2^-$ausgewählt aus: $NTf_2^-$, $PF_6^-$, $BF_4^-$, $Cl^-$, $Br^-$, $I^-$, $CF_3SO_3^-$, $MeSO_4^-$, $EtSO_4^-$, Me-$SO_3^-$, $C_6H_5SO_3^-$, p$MeC_6H_4SO_3^-$, wobei m eine ganze Zahl zwischen 0 und 20 ist,
$R_\beta$ eine substituierte oder nicht substituierte Vinylgruppe, funktionelle Arylgruppe mit 1 bis 20 Kohlenstoffatomen, oder eine funktionelle Alkylgruppe mit 6 bis 30 Kohlenstoffatomen darstellt,
und $R_a$ eine verzweigte oder nicht verzweigte Alkylgruppe mit 1 bis 20 Kohlenstoffatomen, insbesondere eine Ethyl-, Propyl-, Butyl-, Pentyl-, Hexyl-, Heptyl- oder Octylgruppe darstellt.

**24.** Verbindung gemäß einem der Ansprüche 14 und 16 bis 23, **dadurch gekennzeichnet, dass** $X_2^-$ ein funktionelles Anion ist, das einem Anion, dessen $pK_A$ der zugeordneten Säure geringer als 30 ist, entspricht und insbesondere aus den folgenden Anionen ausgewählt ist:

$OH^-$, F-, $R_cBZ_3^-$, $N_3^-$, $CN^-$, oder $W\,\overline{C}\,R_cV$

wobei Z eine -F, -OH, -OR-Gruppe darstellt, R eine Alkylgruppe mit 1 bis 20 Kohlenstoffatomen darstellt,
V und W unabhängig voneinander eine elektroattraktive Gruppe darstellen, insbesondere eine Cyanogruppe, eine Alkoxycarbonylgruppe mit 2 bis 20 Kohlenstoffatomen, eine Acylgruppe mit 2 bis 20 Kohlenstoffatomen, eine Benzoylgruppe, eine Alkylsulfonylgruppe mit 1 bis 20 Kohlenstoffatomen, eine Arylsulfonylgruppe mit 6 bis 30 Kohlenstoffatomen, eine Dialkoxyphosphonylgruppe mit 2 bis 20 Kohlenstoffatomen,
$R_c$ eine verzweigte oder nicht verzweigte, zyklische oder nicht zyklische Alkylgruppe mit 1 bis 20 Kohlenstoffatomen oder eine Arylgruppe mit 6 bis 30 Kohlenstoffatomen darstellt,
und, dass das $A_2^+$-Kation ausgewählt ist aus Ammonium- und Phosphoniumkationen, insbesondere aus den folgenden Kationen:

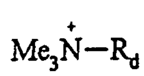

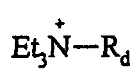

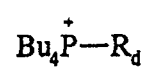

wobei $R_d$ eine Alkylgruppe mit 1 bis 20 Kohlenstoffatomen ist.

**25.** Verwendung einer Zusammensetzung gemäß irgendeinem der Ansprüche 14 bis 24 für die kontinuierliche, diskontinuierliche, kombinatorische oder parallele organische Synthese und/oder die Herstellung von Produktbanken.

**26.** Verwendung einer Zusammensetzung gemäß irgendeinem der Ansprüche 14 und 16 bis 25 für die Ausführung eines Herstellungsverfahrens eines Moleküls G ausgehend von eine Anfangsgruppe $F_0$, die gegebenenfalls über einen L-Arm, insbesondere eine Alkylgruppe mit 1 bis 20 Kohlenstoffatomen, an eine ionische Gesamtheit $Y^{+-}$, die Teil des Kations $A_2^+$ des funktionalisierten Salzes $A_2^+X_2^-$ ist, oder $Y^{--}$, die Teil des Anions $X_2^-$ des funktionalisierten Salzes $A_2^+X_2^-$ ist, gebunden ist, wobei das Kation die Form $Y^+$-L-$F_0$ und/oder das Anion die Form $Y^-$-$(L)_k$-$F_0$ hat, wobei k gleich 0 oder 1 ist, welches Verfahren die Schritte umfasst:

- eine erste Zugabe eines Reagenz $B_1$ in die obengenannte Zusammensetzung und die Reaktion zwischen der

genannten Gruppe $F_0$ und dem Reagenz $B_1$, die zu einer Gruppe $F_1$ führt, die an die ionische Gesamtheit $Y^+$-, die Teil des Kations $A_2^+$ des funktionalisierten Salzes $A_2^+X_2^-$ ist, und/oder $Y^-$-, die Teil des Anions $X_2^-$ des funktionalisierten Salzes $A_2^+X_2^-$ ist, gebunden ist, gemäß einem der folgenden Reaktionsschemata:

$$Y^+\!-L-F_0,\ X_2^- \xrightarrow{B_1} Y^+\!-L-F_1,\ X_2^-$$

oder

$$A_2^+,\ Y^-\!-(L)_k-F_0 \xrightarrow{B_1} A_2^+,\ Y^-\!-(L)_k-F_1$$

- n-1 folgende Zugaben von Reagenzien $B_i$, $1 < i \leq n$, wobei n von 2 bis 10 variiert, zu der obengenannten Zusammensetzung, welche bei jeder Zugabe die Reaktion zwischen dem Reagenz $B_i$ und einer Gruppe $F_{i-1}$, erlauben, die zum Erhalt einer Gruppe $F_i$ führt, wobei die (n-1)te Zugabe des Reagenz $B_n$ zur Gruppe $F_{n-1}$ zum Erhalt der Gruppe $F_n$ führt, wobei die n-1 Zugaben durch eines der folgenden Reaktionsschemata dargestellt werden können:

$$Y^+\!-L-F_1,\ X_2^- \xrightarrow{B_2} Y^+\!-L-F_2,\ X_2^- \cdots\cdots Y^+\!-L-F_{i-1},\ X_2^- \xrightarrow{Bi} Y^+\!-L-F_i,\ X_2^- \cdots\cdots \xrightarrow{B_n} Y^+\!-L-F_n,\ X_2^-$$

oder

$$A_2^+,\ Y^-\!-(L)_k-F_1 \xrightarrow{B_2} A_2^+,\ Y^-\!-(L)_k-F_2 \cdots\cdots A_2^+,\ Y^-\!-(L)_k-F_{i-1} \xrightarrow{Bi} A_2^+,\ Y^-\!-(L)_k-F_i \cdots\cdots \xrightarrow{B_n} A_2^+,\ Y^-\!-(L)_k^-$$

- Spaltung der Funktion $F_n$, die jeweils an die ionische Gesamtheit $Y^+$- oder $Y^-$- des Kations $A_2^+$ und/oder des Anions $X_2^-$ gebunden ist, was die Gewinnung von einerseits dem funktionalisierten Salz $A_2^+X_2^-$ in der Form $Y^+$-L-$F_0$, $X_2^-$ oder $A_2^+$, $Y^-$-$(L)_k$-$F_0$ in Lösung in der ionischen flüssigen Matrix $A_1^+X_1$ oder in der Form $Y^+$-L-$F'_0$, $X_2^-$ oder $A_2^+$, $Y^-$- $(L)_k$-$F'_0$, in welcher $F'_0$ eine von $F_0$ verschiedene Gruppe darstellt, und andererseits dem Molekül G,

gemäß einem der folgenden Reaktionsschemata:

$$Y^+\!-L-F_n,\ X_2^- \xrightarrow{\text{Spaltung}} G + Y^+\!-L-F_0,\ X_2^-$$

oder

$$Y^+\!-L-F'_n,\ X_2^- \xrightarrow{\text{Spaltung}} G + Y^+\!-L-F'_0,\ X_2^-$$

oder

$$A_2^+ , Y^- -(L)_k-F_n \xrightarrow{\text{Spaltung}} G + A_2^+ , Y^- -(L)_k-F_0$$

oder

$$A_2^+ , Y^- -(L)_k-F'_n \xrightarrow{\text{Spaltung}} G + A_2^+ , Y^- -(L)_k-F'_0$$

erlaubt.

**27.** Verwendung gemäß Anspruch 26 für das Ausführen der Diels-Alder-Reaktion gemäß einem der folgenden Reaktionsschemata:

a)

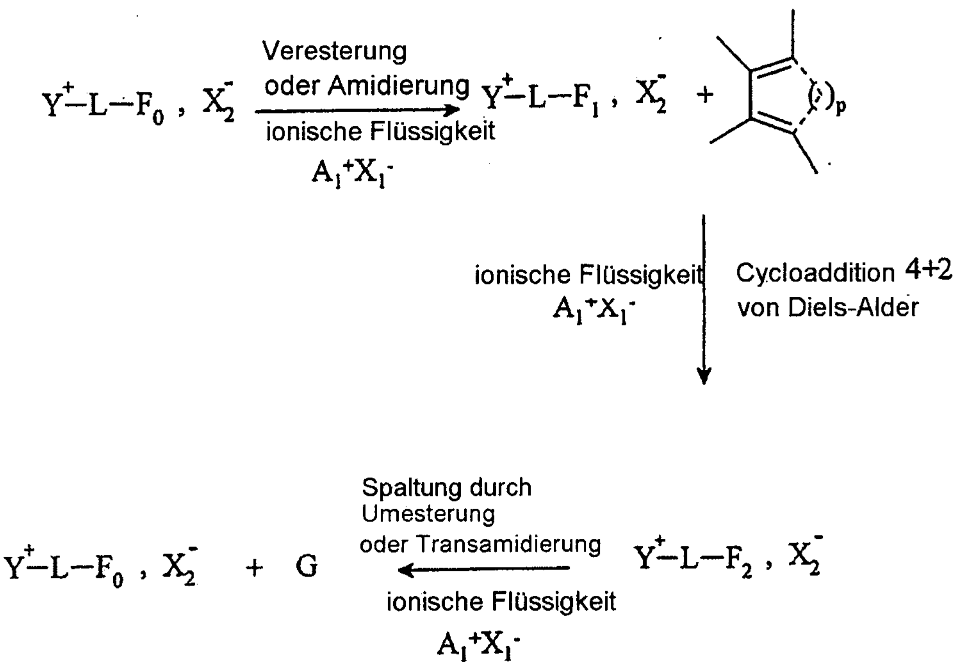


wobei p eine ganze Zahl von 0 bis 2 ist,
$Y^+$- ein Oniumkation, wie es in einem der Ansprüche 20 bis 25 definiert ist, und vorzugsweise ein Trimethylalkylammonium-, ein Triethylalkylammonium- oder ein Tributylalkylphosphoniumkation ist,
L einen Arm darstellt, insbesondere eine lineare oder verzweigte Alkylgruppe mit 1 bis 20 Kohlenstoffatomen, oder eine gegebenenfalls funktionelle Aralkylgruppe mit 6 bis 30 Kohlenstoffatomen, und vorzugsweise eine lineare Alkylgruppe vorzugsweise eine lineare Alkylgruppe des Typs $(CH_2)_r$, wobei r von 1 bis 20, und vorzugsweise von 3 bis 6, variiert,
$X_2^-$ so ist, wie es in einem der Ansprüche 1 bis 26 definiert ist, und insbesondere $NTf_2^-$, $BF_4^-$, $PF_6^-$, $Cl^-$, $Br^-$, $CH_3COO^-$, $CF_3CO_2^-$, $CF_3SO_3^-$, $BR_4^-$ ist, wobei R wie in Anspruch 20 definiert ist,
die ionische Flüssigkeit $A_1^+X_1^-$ insbesondere die Form hat:

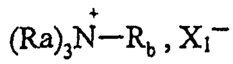
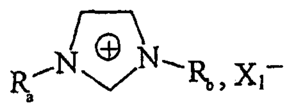
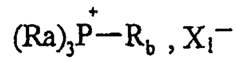
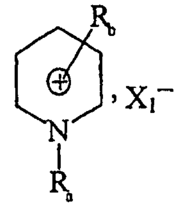

wobei $R_a$ und $R_b$ wie in Anspruch 22 definiert sind, und insbesondere Alkylgruppen mit 1 bis 20 Kohlenstoffatomen darstellen,
X1- ausgewählt ist aus: $BF_4^-$, $PF_6^-$, $NTf_2^-$, $Cl^-$, $Br^-$, $CH_3COO^-$, $CF_3CO_2^-$, $CF_3SO_3^-$, BR4-, wobei R wie in Anspruch 20 definiert ist,
die Funktionen $F_0$, $F_1$ und $F_2$ wie unten definiert sind:

- $F_0$ entspricht einer - $\chi_1$H-Gruppe, in welcher $\chi_1$ ein Sauerstoffatom oder eine -$NR_f$-Gruppe darstellt, wobei $R_f$ einer linearen oder verzweigten Alkylgruppe mit 1 bis 20 Kohlenstoffatomen, oder einer Arylgruppe mit 6 bis 30 Kohlenstoffatomen entspricht,
- $F_1$ der folgenden Formel entspricht:

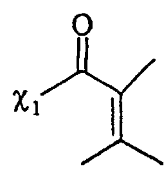

wobei $\chi_1$ wie oben definiert ist,
- $F_2$ der folgenden Formel entspricht:

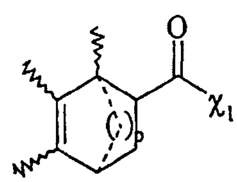

wobei $\chi_1$ wie oben definiert ist,

G der folgenden Formel entspricht:

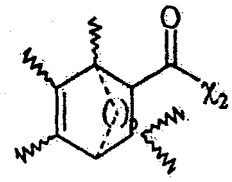

in der $\chi_2$ entweder eine ORg-Gruppe, wobei $R_g$ ein Wasserstoffatom oder eine Alkylgruppe mit 1 bis 20 Kohlenstoffatomen ist, oder eine -$NR_hR_u$-Gruppe darstellt, wobei $R_h$ und $R_u$ unabhängig voneinander ein Wasserstoffatom, eine Alkylgruppe mit 1 bis 20 Kohlenstoffatomen, oder eine Arylgruppe mit 6 bis 30 Kohlenstoffatomen darstellen,

b)

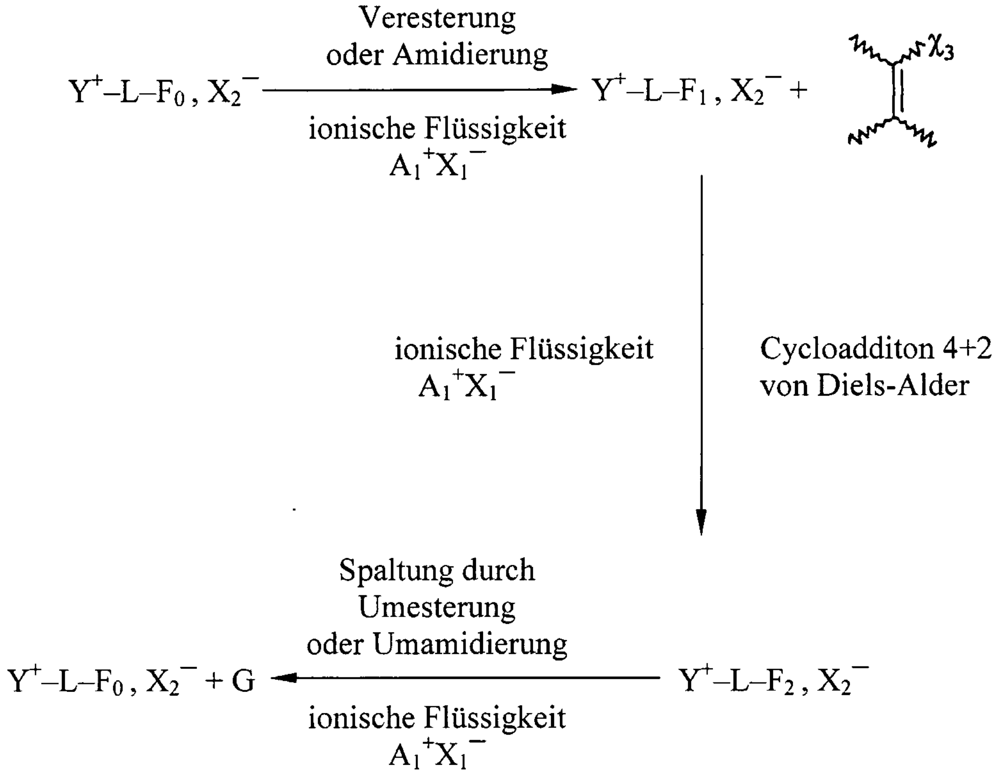


wobei $Y^+$-, L, $X_2^-$ und die ionische Flüssigkeit $A_1^+X_1^-$ wie zuvor definiert sind,
die Gruppen $F_0$, $F_1$ und $F_2$ wie unten definiert sind:

- $F_0$ stellt jede Gruppe dar, die das Anbinden eines 1,3-Diens möglich macht, und insbesondere aus den Carbonyl-, Amin-, Alkoxy-, Silan-, Stannan- und Borangruppen, die 1 bis 20 Kohlenstoffatome enthalten, ausgewählt ist,
- $F_1$ entspricht der folgenden Formel:

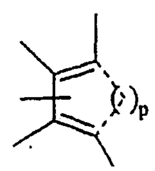

wobei p eine ganze Zahl ist, die zwischen 0 und 2 variiert,
- $F_2$ entspricht der folgenden Formel:

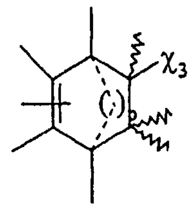

wobei $\chi_3$ eine elektroattraktive Gruppe darstellt, insbesondere ausgewählt aus den Cyano-, Alkoxycarbonylgruppen mit 1 bis 20 Kohlenstoffatomen, den Acylgruppen mit 1 bis 20 Kohlenstoffatomen, den Benzoyl-, Sulfonyl-, Dialkoxyphosphonylgruppen mit 1 bis 10 Kohlenstoffatomen,

G der folgenden Fonnel entspricht:

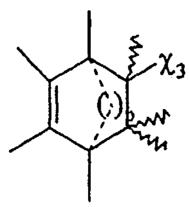

wobei $\chi_3$ wie oben definiert ist.

**28.** Verwendung gemäß Anspruch 26 für das Ausführen der Heckreaktion gemäß dem folgenden Reaktionsschema:

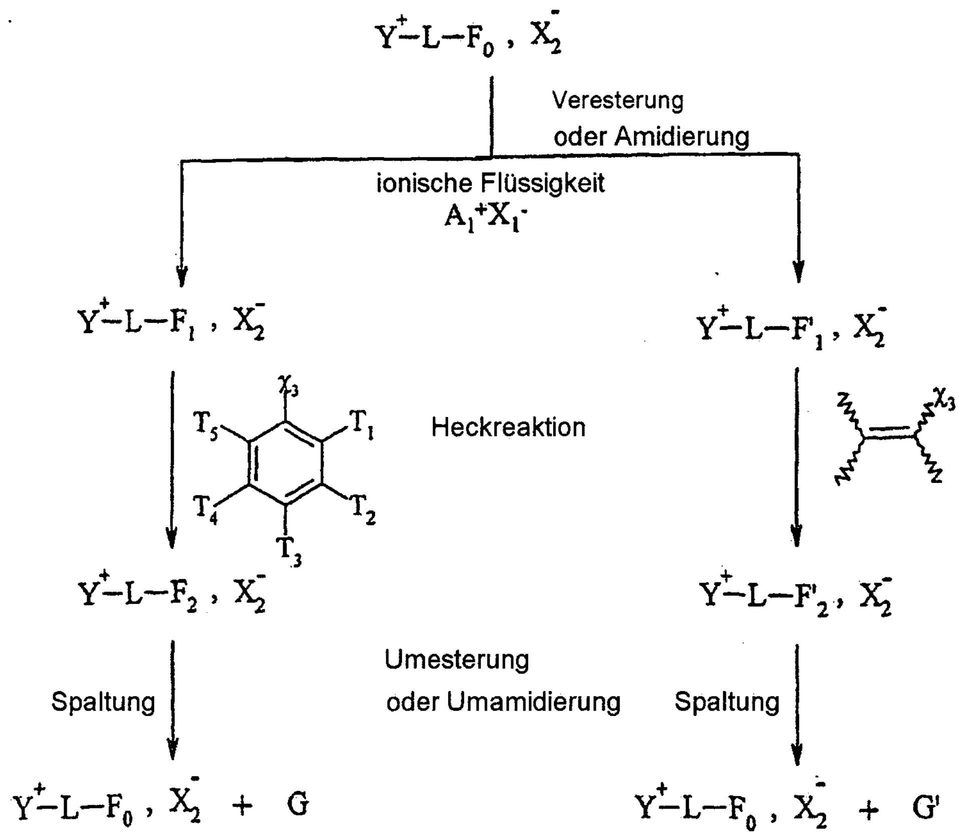


wobei $Y^+$- ein Oniumkation, wie es in einem der Ansprüche 21 bis 26 definiert ist, und vorzugsweise ein Trimethylalkylammonium-, ein Triethylalkylammonium- oder ein Tributylalkylphosphoniumkation ist,
L einen Arm darstellt, insbesondere eine lineare oder verzweigte Alkylgruppe mit 1 bis 20 Kohlenstoffatomen, oder eine gegebenenfalls funktionelle Aralkylgruppe mit 1 bis 20 Kohlenstoffatomen, und vorzugsweise eine lineare Alkylgruppe vorzugsweise eine lineare Alkylgruppe des Typs $(CH2)_r$, wobei r von 1 bis 20, und vorzugsweise von 3 bis 6, variiert, ist,
$X_2^-$ so ist, wie es in einem der Ansprüche 1 bis 26 definiert ist und insbesondere $BF_4^-$, $PF_6^-$, $NTf_2^-$, $CF_3SO_3^-$, $Cl^-$, $Br^-$ oder $I^-$,
die ionische Flüssigkeit $A_1^+X_1^-$ insbesondere die Form hat:

$(Ra)_3N^+$-$R_b$, $X_1^-$

$(Ra)_3P^+$-$R_b$, $X_1^-$

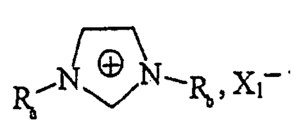

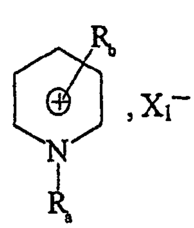

wobei $R_a$ und $R_b$ wie in Anspruch 22 definiert sind, und insbesondere Alkylgruppen mit 1 bis 20 Kohlenstoffatomen darstellen,
$X_1$ ausgewählt ist aus: $BF_4^-$, $PF_6^-$, $NTf_2^-$, $Cl^-$, $Br^-$, $CH_3COO^-$, $CF_3CO_2^-$, $CF_3SO_3^-$, $BR_4^-$, wobei R wie in Anspruch 20 definiert ist,
die Funktionen $F_0$, $F_1$, $F'_1$, $F_2$ und $F'_2$ wie unter definiert sind:

- $F_0$ entspricht einer $-\chi_1H$-Gruppe, in welcher $\chi_1$ ein Sauerstoffatom oder eine $-NR_f$-Gruppe darstellt, wobei $R_f$ einer linearen oder verzweigten Alkylgruppe mit 1 bis 20 Kohlenstoffatomen, oder einer Arylgruppe mit 6 bis 30 Kohlenstoffatomen entspricht,
- $F_1$ entspricht der folgenden Formel:

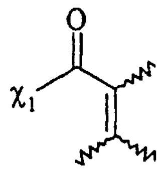

wobei $\chi_1$ wie oben definiert ist,
- $F_2$ entspricht der folgenden Formel:

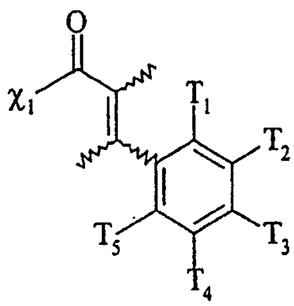

wobei $\chi$ 1 wie oben definiert ist,

G der folgenden Formel entspricht:

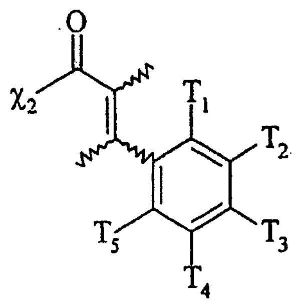

in der $\chi$ entweder eine $-OR_g$-Gruppe, wobei $R_g$ ein Wasserstoffatom oder eine Alkylgruppe mit 1 bis 20 Kohlenstoffatomen ist, oder eine $-NR_hR_u$-Gruppe darstellt, wobei $R_h$ und $R_u$ unabhängig voneinander ein Wasserstoffatom, eine Alkylgruppe mit 1 bis 20 Kohlenstoffatomen oder eine Arylgruppe mit 6 bis 30 Kohlenstoffatomen darstellen,
$\chi_3$ eine flüchtige Gruppierung darstellt, insbesondere ausgewählt aus den I-, Cl- und Br- Halogeniden, den Mesylat-, Tosylat-, Triflat-, Sulfonat-, Sulfat- oder Phosphatgruppen,
$T_1$, $T_2$, $T_3$, $T_4$ und $T_5$ unabhängig voneinander ein Wasserstoffatom, eine lineare oder verzweigte Alkylgruppe mit 1 bis 20 Kohlenstoffatomen, oder eine Arylgruppe mit 6 bis 30 Kohlenstoffatomen, oder eine funktionelle Gruppe darstellen, die insbesondere ausgewählt ist aus $NO_2$, CN, COOR, OR, COR, NHCOR, NRR", $SO_2R$, I, Br, wobei R und R" unabhängig voneinander eine Alkylgruppe mit 1 bis 20 Kohlenstoffatomen, oder eine Arylgruppe mit 6 bis 30 Kohlenstoffatomen darstellen,
die Gesamtheit

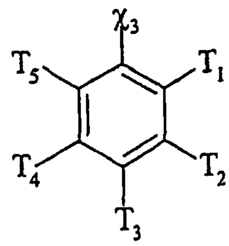

insbesondere die folgenden Gruppen darstellt:

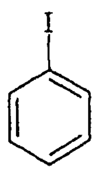
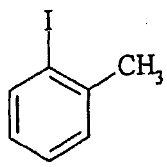
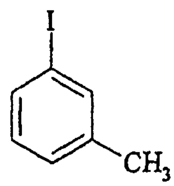
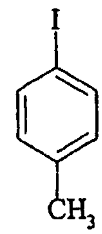
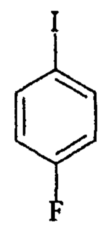

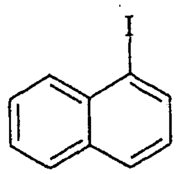
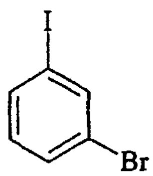
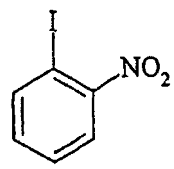
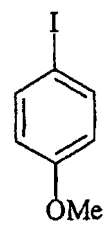

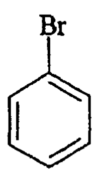
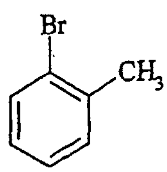
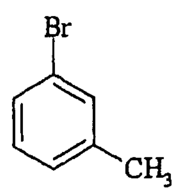
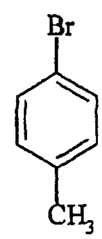
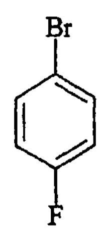

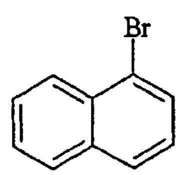
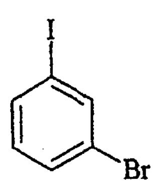
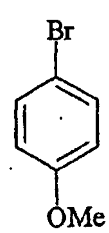

- wobei $F'_1$ der folgenden Formel entspricht:

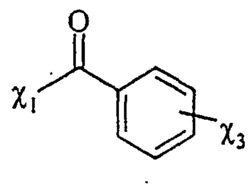

und $\chi_1$ und $\chi_3$ wie oben definiert sind,
- $F'_2$ der folgenden Formel entspricht:

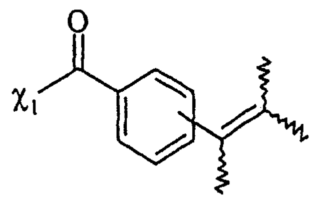

und $\chi_1$ wie oben definiert ist,

G' der folgenden Formel entspricht:

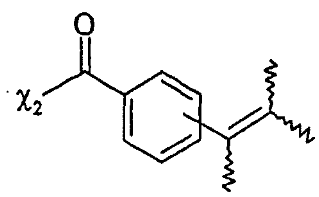

und $\chi_2$ wie oben definiert ist.

**29.** Verwendung gemäß Anspruch 26 für die Ausführung der Baylis-Hilman-Reaktion gemäß dem folgenden Reaktionsschema:

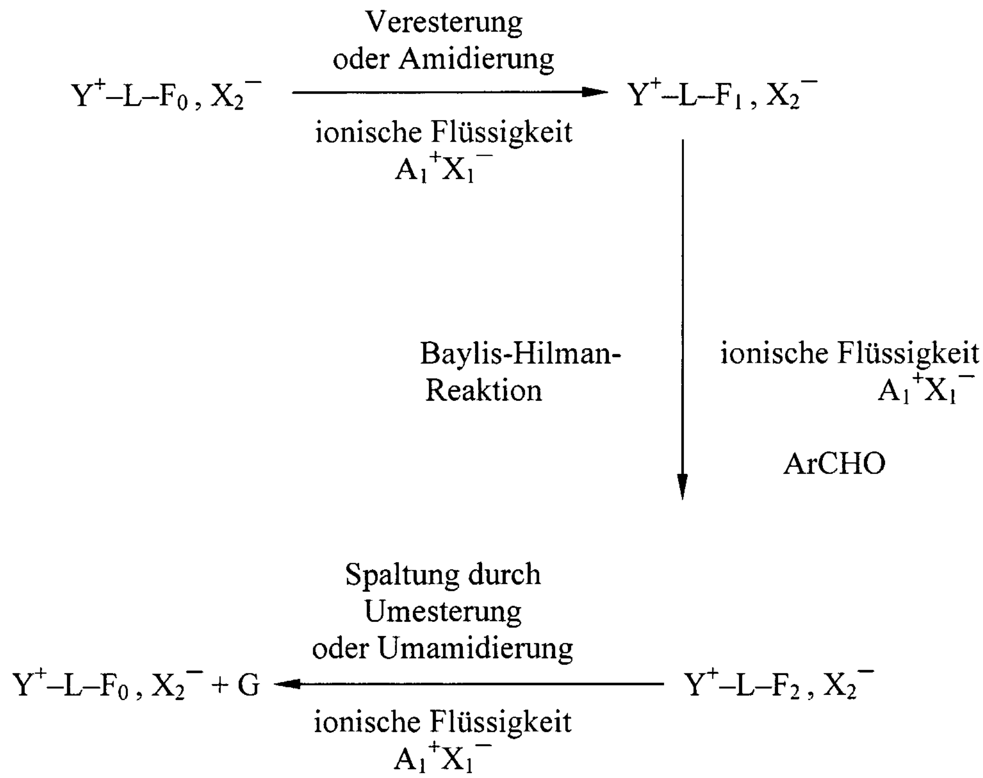


wobei $Y^+$- ein Oniumkation, wie es in einem der Ansprüche 21 bis 26 definiert ist, und vorzugsweise ein Trimethylalkylammonium-, ein Triethylalkylammonium- oder ein Tributylalkylphosphoniumkation ist,
L einen Arm darstellt, insbesondere eine lineare oder verzweigte Alkylgruppe mit 1 bis 20 Kohlenstoffatomen, oder eine gegebenenfalls funktionelle Aralkylgruppe mit 6 bis 30 Kohlenstoffatomen, und vorzugsweise eine lineare Alkylgruppe vorzugsweise eine lineare Alkylgruppe des Typs $(CH_2)_r$, wobei r von 1 bis 20, und vorzugsweise von 3 bis 6, variiert, ist,
$X_2^-$ so ist, wie es in einem der Ansprüche 1 bis 26 definiert ist, und insbesondere $BF_4^-$, $PF_6^-$, $NTf_2^-$, $CF_3SO_3^-$ , $Cl^-$, $Br^-$, $I^-$, $CH_3CO_2^-$ oder $CF_3CO_2^-$ ist,
die ionische Flüssigkeit $A_1^+X_1^-$ insbesondere die Form hat:

$(Ra)_3N^+$-$R_b$, $X_1^-$

$(Ra)_3P^+$-$R_b$, $X_1^-$

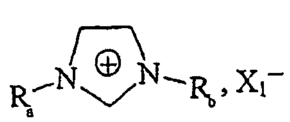

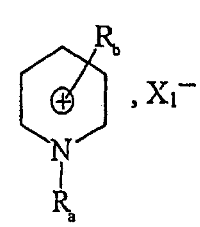

wobei $R_a$ und $R_b$ wie in Anspruch 22 definiert sind, und insbesondere Alkylgruppen mit 1 bis 20 Kohlenstoffatomen darstellen,
$X_1^-$ ausgewählt ist aus: $BF_4^-$, $PF_6^-$, $NTf_2^-$, $Cl^-$, $Br^-$, $CH_3COO^-$, $CF_3CO_2^-$, $CF_3SO_3^-$, $BR_4^-$, wobei R wie in Anspruch 20 definiert ist,
die Funktionen $F_0$, $F_1$ und $F_2$ wie unter definiert sind:

- $F_0$ entspricht einer -OH-Gruppe
- $F_1$ entspricht der folgenden Formel:

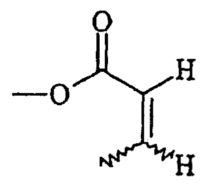

- $F_2$ entspricht der folgenden Formel:

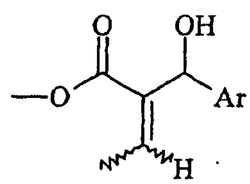

G der folgenden Formel entspricht:

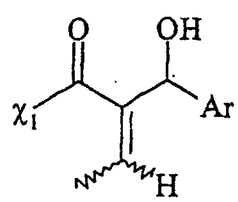

in der $\chi_1$ eine -OH-Gruppe oder eine -ORg-Gruppe darstellt, wobei Rg eine lineare oder verzweigte Alkylgruppe mit 1 bis 20 Kohlenstoffatomen ist,
Ar eine substituierte oder nicht substituierte, aromatische oder hetereoaromatische Gruppe darstellt,
ArCHO insbesondere ausgewählt ist aus:

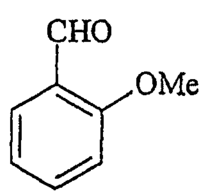
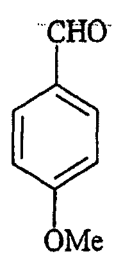
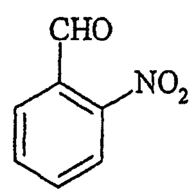
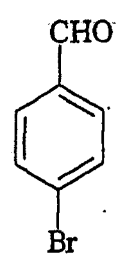

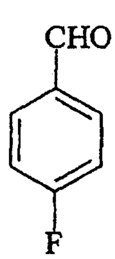
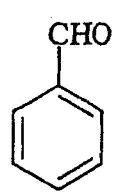
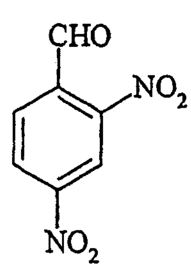
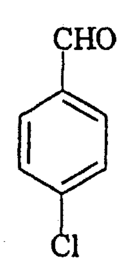

**30.** Verwendung gemäß Anspruch 26 für die Ausführung der Suzuki-Kopplung gemäß einem der folgenden Reaktions-

schemata:

a)

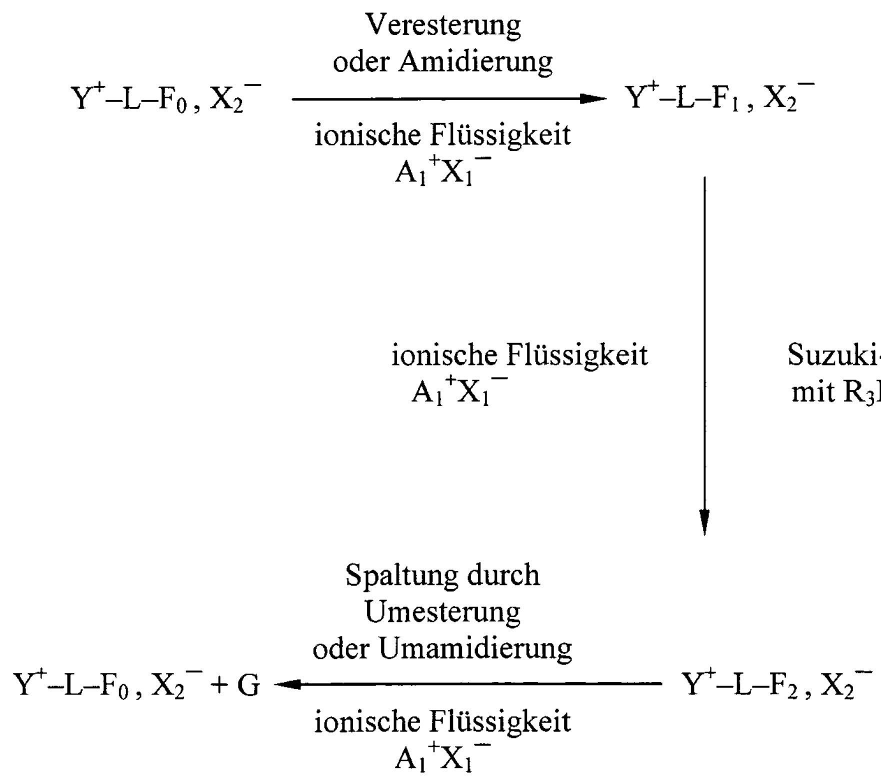


wobei $R_3$ ausgewählt ist aus den subsituierten oder nichtsubstituierten Aryl-, Heteroaryl-, Ethenyl-, Dienyl-, Allyl, Ethynylgruppen mit 2 bis 30 Kohlenstoffatomen,
$R_7$ eine verzweigte oder lineare Alkylgruppe oder eine Cycloalkylgruppe mit 1 bis 12 Kohlenstoffatomen darstellt,
$Y^+$- ein Oniumkation, wie es in einem der Ansprüche 21 bis 26 definiert ist, und vorzugsweise ein Trimethylalkylammonium-, ein Triethylalkylammonium- oder ein Tributylalkylphosphoniumkation ist,
L einen Arm darstellt, insbesondere eine lineare oder verzweigte Alkylgruppe mit 1 bis 20 Kohlenstoffatomen, oder eine gegebenenfalls funktionelle Aralkylgruppe mit 6 bis 30 Kohlenstoffatomen, und vorzugsweise eine lineare Alkylgruppe vorzugsweise eine lineare Alkylgruppe des Typs $(CH_2)_r$, wobei r von 1 bis 20, und vorzugsweise von 3 bis 6, variiert, ist,
$X_2^-$ so ist, wie es in einem der Ansprüche 1 bis 26 definiert ist, und insbesondere $NTf_2^-$, $BF_4^-$, $PF_6^-$, $Cl^-$, $Br^-$, $CH_3COO^-$, $CF_3CO_2^-$, $CF_3SO_3^-$, $BR_4^-$, wobei R wie in Anspruch 20 definiert ist, ist,
die ionische Flüssigkeit $A_1^+X_1^-$ insbesondere die Form hat:

$(Ra)_3N^+\text{-}R_b, X_1^-$

$(Ra)_3P^+\text{-}R_b, X_1^-$

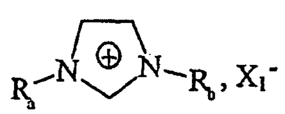

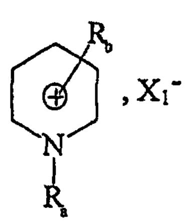

wobei $R_a$ und $R_b$ wie in Anspruch 22 definiert sind, und insbesondere Alkylgruppen mit 1 bis 20 Kohlenstoffatomen

darstellen,
$X_1^-$ ausgewählt ist aus: $BF_4^-$, $PF_6^-$, $NTf_2^-$, $Cl^-$, $Br^-$, $CH_3COO^-$, $CF_3CO_2^-$, $CF_3SO_3^-$ , $BR_4^-$, wobei R wie in Anspruch 20 definiert ist,
die Funktionen $F_0$, $F_1$ und $F_2$ wie unter definiert sind:

- $F_0$ hat die Form $-\chi_1H$, wobei $\chi_1$ ein Sauerstoffatom oder eine $-NR_f$-Gruppe darstellt, wobei $R_f$ einer linearen oder verzweigten Alkylgruppe mit 1 bis 20 Kohlenstoffatomen, oder einer Arylgruppe mit 6 bis 30 Kohlenstoffatomen entspricht,
- $F_1$ hat die Form $-R_e-\chi$, wobei $R_e$ eine aromatische oder heteroaromatische Gruppe mit 6 bis 30 Kohlenstoffatomen darstellt, $\chi$ eine flüchtige Gruppe vorzugsweise ausgewählt aus Cl, Br, I, OTf, $O-CO_2R^5$ oder $OSO_3-R^5$ darstellt, wobei $R^5$ eine Alkylgruppe mit 1 bis 10 Kohlenstoffatomen, oder eine Aralkylgruppe mit 6 bis 30 Kohlenstoffatomen darstellt, wobei $F_1$ vorzugsweise der folgenden Formel entspricht:

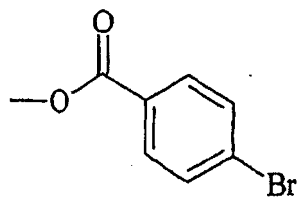

- $F_2$ hat die Form $-R_e-R_2$, wobei $R_e$ wie oben definiert ist und $R_2$ ausgewählt ist aus den substituierten oder nichtsubstituierten Aryl-, Heteroaryl-, Ethenyl-, Dienyl-, Allyl-, Ethynylgruppen mit 2 bis 30 Kohlenstoffatomen, wobei $F_2$ vorzugsweise der folgenden Formel entspricht:

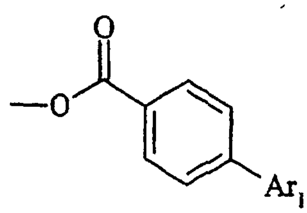

wobei $Ar_1$ eine aromatische Gruppe darstellt, die vorzugsweise ausgewählt ist aus:

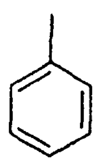 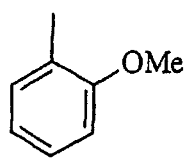 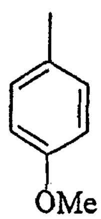 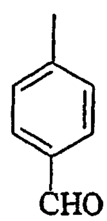 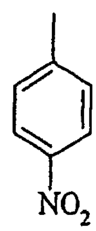

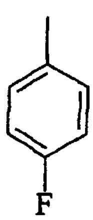 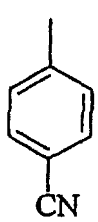 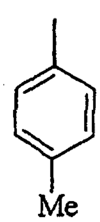 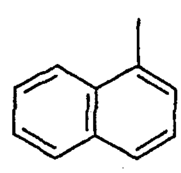

das Molekül G die Form $R_2-R_3$ hat, wobei $R_2$ und $R_3$ wie oben definiert sind, und vorzugsweise der folgenden Formel entspricht:

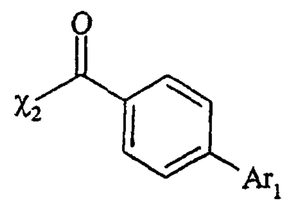

in der $\chi_2$ entweder eine -ORg-Gruppe, wobei $R_g$ ein Wasserstoffatom oder eine Alkylgruppe mit 1 bis 20 Kohlenstoffatomen ist, oder eine $-NR_hR_u$-Gruppe darstellt, wobei $R_h$ und $R_u$ unabhängig voneinander ein Wasserstoffatom, eine Alkylgruppe mit 1 bis 20 Kohlenstoffatomen oder eine Arylgruppe mit 6 bis 30 Kohlenstoffatomen darstellen, $Ar_1$ wie oben definiert ist,

b)

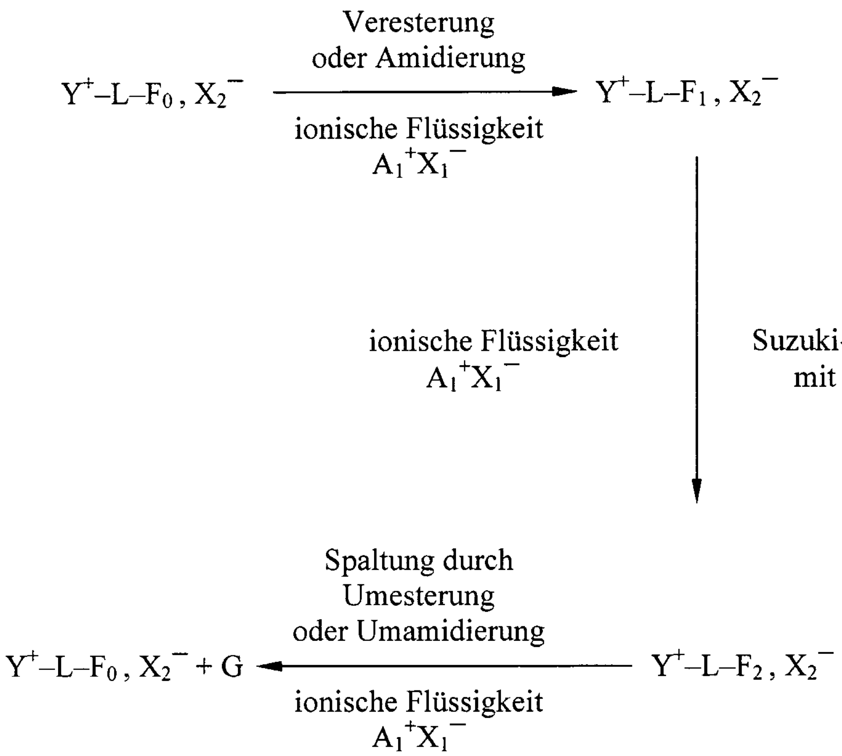


wobei $Y^+$-, L, $X_2^-$, $A_1^+X_1^-$ und $R_2$ wie oben definiert sind,
die Funktionen $F_0$, $F_1$ und $F_2$ wie unten definiert sind:

- $F_0$ hat die Form - $\chi_1H$, wobei $\chi_1$ wie oben definiert ist,
- $F_1$ hat die Form $-R_q-B(OR_7)_2$, wobei $R_7$ wie oben definiert ist und $R_q$ einer substituierten oder nicht substituierten Arylgruppe mit 6 bis 30 Kohlenstoffatomen, Heteroarylgruppe mit 4 bis 20 Kohlenstoffatomen, Ethenylgruppe mit 2 bis 20 Kohlenstoffatomen, Dienylgruppe mit 3 bis 20 Kohlenstoffatomen, Allylgruppe mit 3 bis 20 Kohlenstoffatomen, Ethynylgruppe mit 2 bis 20 Kohlenstoffatomen entspricht, wobei $F_1$ vorzugsweise der folgenden Formel entspricht:

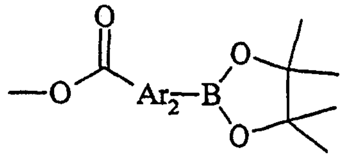

wobei $Ar_2$ einer substituierten oder nicht substituierten Arylgruppe mit 6 bis 30 Kohlenstoffatomen entspricht,
- $F_2$ hat die Form $-R_q-R_e$, wobei $R_q$ und $R_e$ wie oben definiert sind, wobei $F_2$ vorzugsweise der folgenden Formel entspricht:

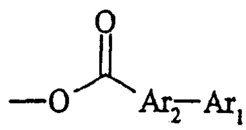

wobei $Ar_1$ eine aromatische Gruppe darstellt, die vorzugsweise ausgewählt ist aus:

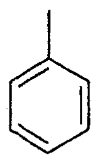 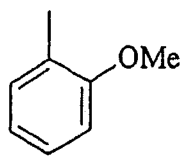 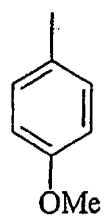 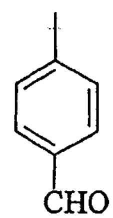 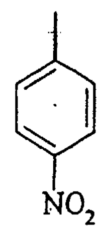

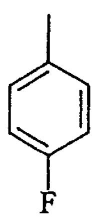 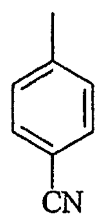 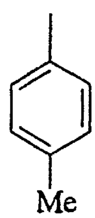 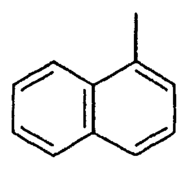

das Molekül G die Fonn $R_2-R_3$ hat, wobei $R_2$ und $R_3$ wie oben definiert sind, und vorzugsweise der folgenden Formel entspricht:

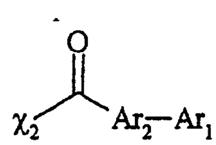

in welcher $\chi_2$, $Ar_1$ und $Ar_2$ wie oben definiert sind,

c)

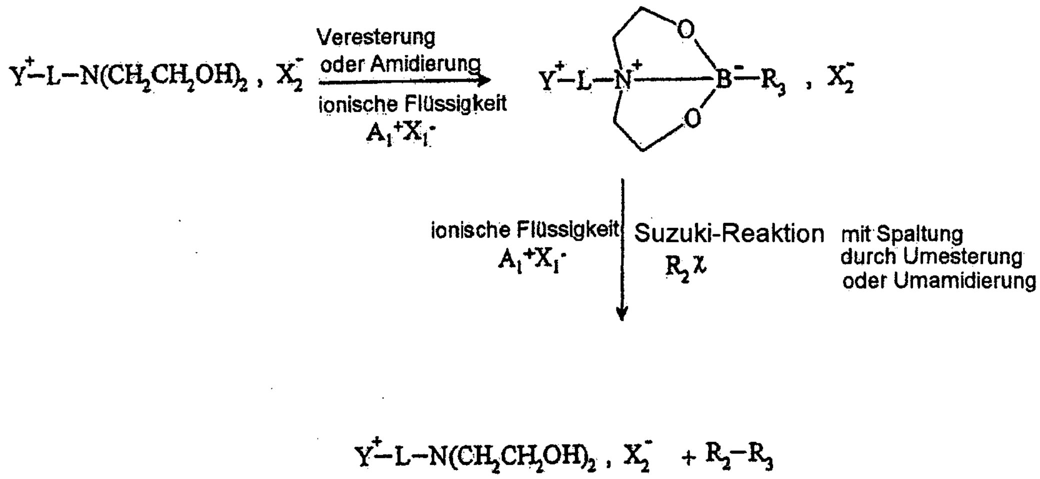


wobei $Y^+$-, L, $X_2^-$, $A_1^+X_1^-$, $R_2$ und $R_3$ wie oben definiert sind,
$R_3$ vorzugsweise eine Phenylgruppe ist,

d)

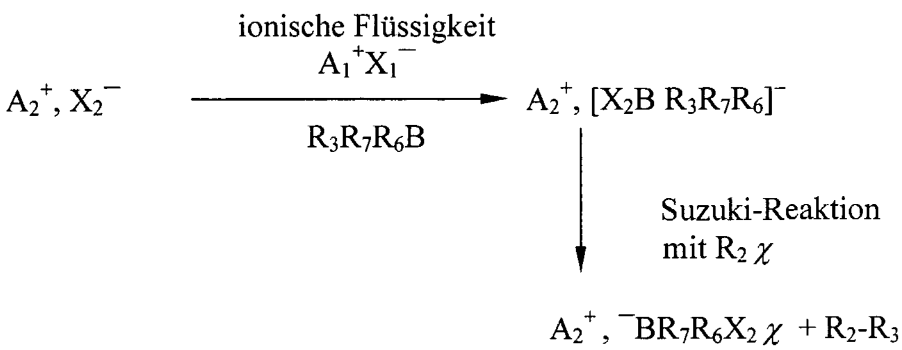


wobei $A_2^+$ ein Ammoniumkation $(R_a)_3N^+R_b$ oder ein Phosphoniumkation $(R_a)_3P^+R_b$, vorzugsweise Tetrabutylammonium und Tetramethylammonium ist, wobei $R_a$ und $R_b$ wie oben definiert sind,
$X_2^-$ ausgewählt ist aus $OH^-$, $F^-$, $CN^-$, $R_sO^-$, $R_sS^-$, vorzugsweise $OH^-$ oder $F^-$, wobei $R_s$ eine Alkylgruppe mit 1 bis 20 Kohlenstoffatomen, oder eine Aralkylgruppe mit 6 bis 30 Kohlenstoffatomen darstellt,
$R_3$ und $R_4$ wie oben definiert sind,
$R_6$ und $R_7$ unabhängig voneinander eine Alkylgruppe mit 1 bis 20 Kohlenstoffatomen, oder einer Arylgruppe mit 6 bis 30 Kohlenstoffatomen darstellen,
das borierte Molekül der Formel $R_3R_7R_6B$ ein Trialkyl- oder Arylboran, wobei die Alkylgruppe 1 bis 20 Kohlenstoffatomen und die Arylgruppe 6 bis 30 Kohlenstoffatomen enthält, eine Boronsäure oder ein Boronester, vorzugsweise eine Boronsäure oder ein Boronester , die als Phenylboronsäure ausgewählt ist, ist
$R_2$ und $\chi$ wie oben definiert sind, $R_2\chi$ vorzugsweise einem Arylhalogenid entspricht, das ausgewählt ist aus:

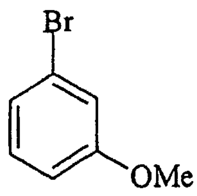

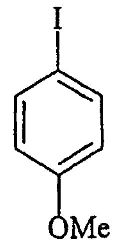

**31.** Verwendung gemäß den Ansprüchen 1 bis 30 für die Synthese von Molekülbanken nach der Technik der parallelen Synthese gemäß dem folgenden Schema:

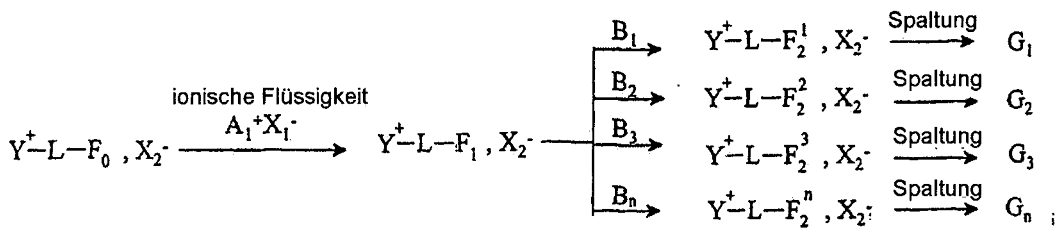


**dadurch gekennzeichnet, dass** das funktionalisierte Salz $Y^+$-L-$F_1$, $X_2^-$ in der ionischen Flüssigkeit $A_1^+$,$X_1^-$ in n im Wesentliche gleiche Teile aufgeteilt wird, wobei n von 2 bis 1024 variiert, und dass jeder dieser Teile dann mit Hilfe eines anderen Reagenz $B_i$ gemäß einer organischen Synthesereaktion, insbesondere einer Kopplungsreaktion nach Heck oder Suzuki, transformiert wird um n Lösungen zu ergeben, die jeweils eine bestimmte Verbindung $Y^+$-L-$F_2^i$ , $X_2^-$ enthalten, wobei $F_2^i$ eine Gruppe darstellt, die aus den im Anspruch 21 definierten Gruppen ausgewählt ist, wobei i von 1 bis n variiert, wobei jede Lösung behandelt wird um die Moleküle G; freizusetzen, wobei i von 1 bis n variiert, die jeweils isoliert und gereinigt werden, und eine Molekülbank bilden.

**32.** Verwendung gemäß einem der Ansprüche 1 bis 30 zur Ausführung der Synthese der Molekülbank durch die Technik Trennung-Mischung gemäß dem folgenden Schema:

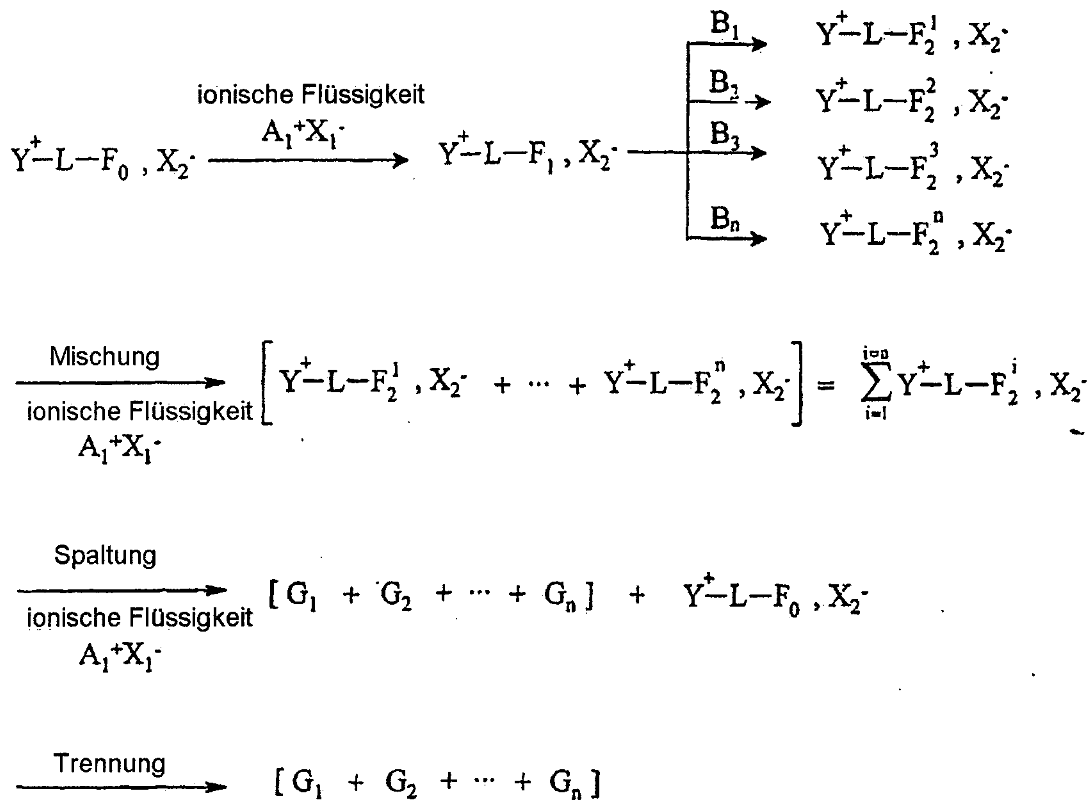


**dadurch gekennzeichnet, dass**:

- n Fraktionen der Lösung $Y^+$-L-$F_1$, $X_2^-$, die ausgehend von einem funktionalisierten Ausgangssalz $Y^+$-L-$F_0$, $X_2^-$ erhalten wurden, in der ionischen Flüssigkeit $A_1^+X_1^-$ jeweils mit Hilfe eines verschiedenen Reagenz $B_i$ parallel gemäß einer Reaktion der organischen Chemie, vorzugsweise einer Kopplungsreaktion nach Heck oder Suzuki, transformiert werden, um n Lösungen zu ergeben, die jeweils eine bestimmte Verbindung $Y^+$-L-$F_2^i$, $X_2^-$ enthalten, wobei i von 1 bis n variiert, n von 2 bis 1024, vorzugsweise 2 bis 96 , variiert, wobei $F_2^i$ eine Gruppe darstellt, die aus den in Anspruch 21 definierten Gruppen ausgewählt ist,
- die im voran gegangenen Schritt erhaltenen n Lösungen gemischt werden, um eine Lösung in der ionischen Flüssigkeit $A_1^+X_1^-$ zu ergeben, die n Produkte $Y^+$-L-$F_2^i$, $X_2^-$ enthält, wobei i von 1 bis n variiert, bezeichnet $\sum_{i=1}^{i=n} Y^+\text{–L–}F_2{}^i, X_2^-$, und diese Lösung einem Spaltungsschritt, vorzugsweise einer Umesterung oder einer Umamidierung unterworfen wird, um in der ionischen Flüssigkeit $A_1^+X_1^-$ gelöst eine Mischung von n Molekülen $G_i$, wobei i von 1 bis n variiert, und funktionalisiertes Ausgangssalz $Y^+$-L-$F_0$ , $X_2^-$ zu erhalten,
- die im vorherigen Schritt erhaltene Mischung von der ionischen Flüssigkeit $A_1^+X_1$ und dem funktionalisierten Ausgangssalz $Y^+$-L-$F_0$, $X_2^-$ durch übliche Trennverfahren, vorzugsweise durch Vakuumdestillation, durch Extraktion mit einem klassischen Lösungsmittel wie Heptan oder Toluol gefolgt von einer Verdampfung des Lösungsmittels, durch Chromatografie auf einer Kolonne, einer Platte oder unter Druck, getrennt wird, um eine Bank zu erhalten, die n Moleküle G; enthält,

wobei diese oben genannte Reihe von Schritten j mal wiederholt werden kann, wobei j zwischen 2 und 10 liegt, um j verschiedene Banken von n Produkten zu erhalten.

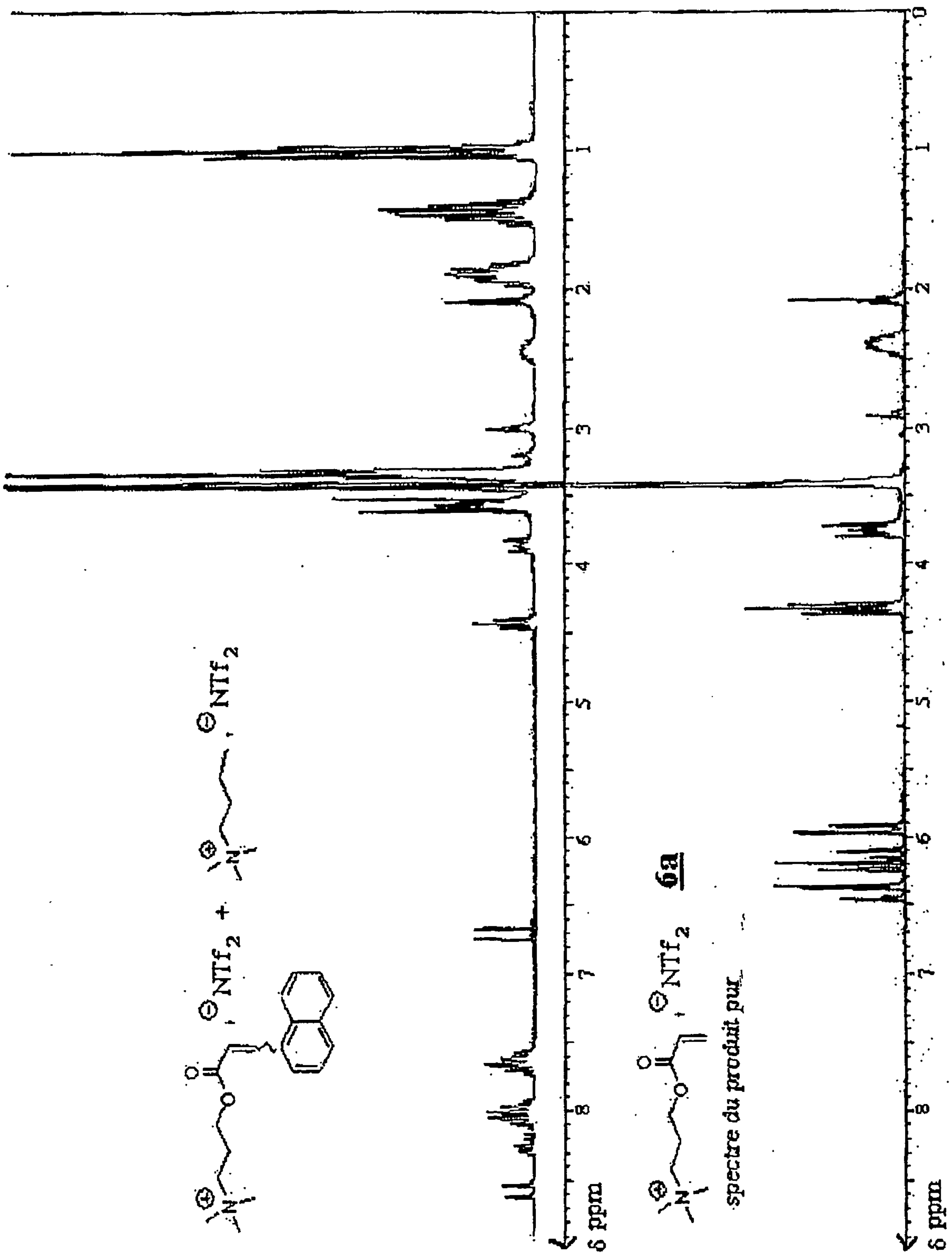


FIGURE 1

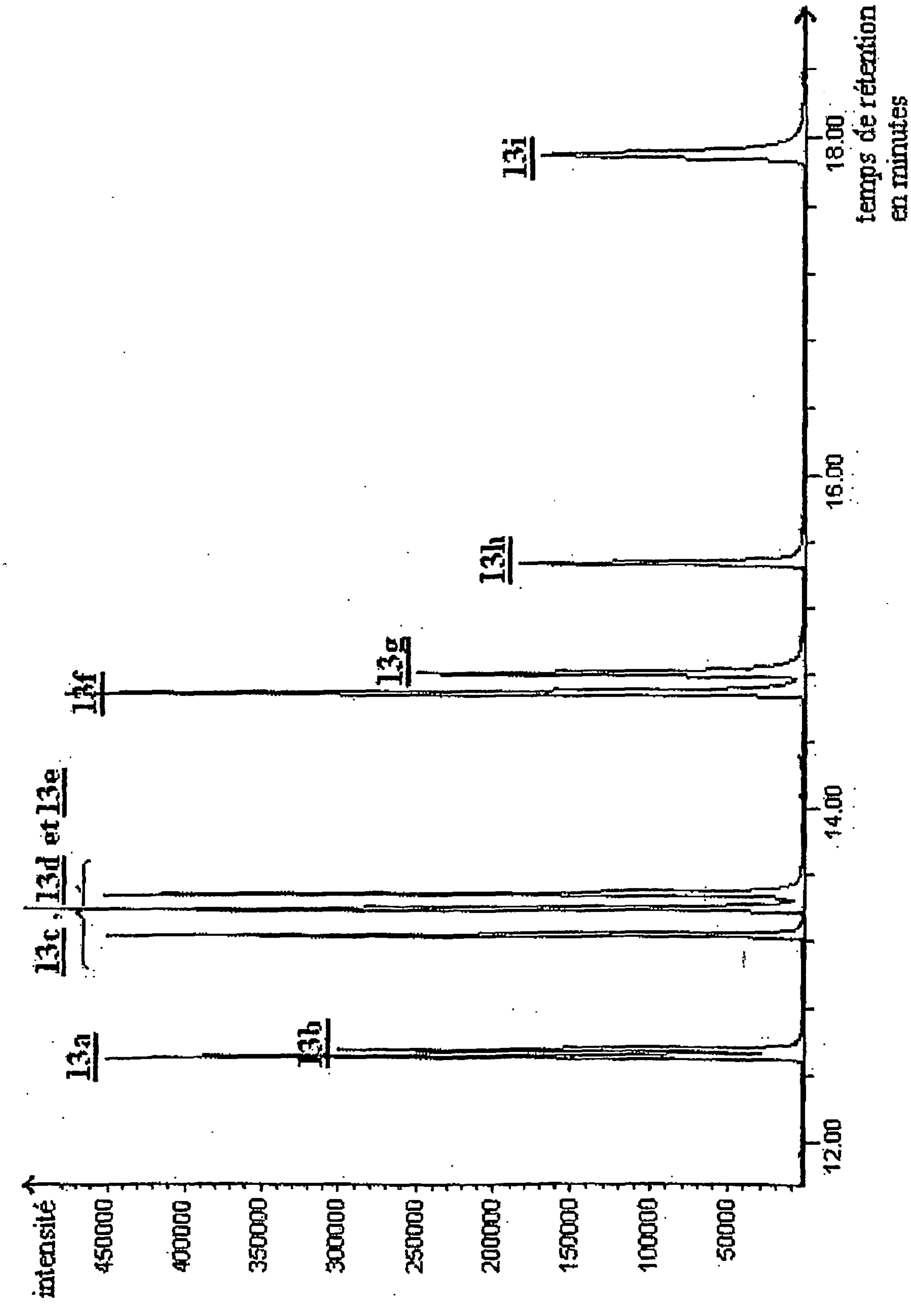
intensité
450000
400000
350000
300000
250000
200000
150000
100000
50000
12.00
14.00
16.00
18.00
temps de rétention en minutes
13a
13b
13c, 13d et 13e
13f
13g
13h
13i

FIGURE 2

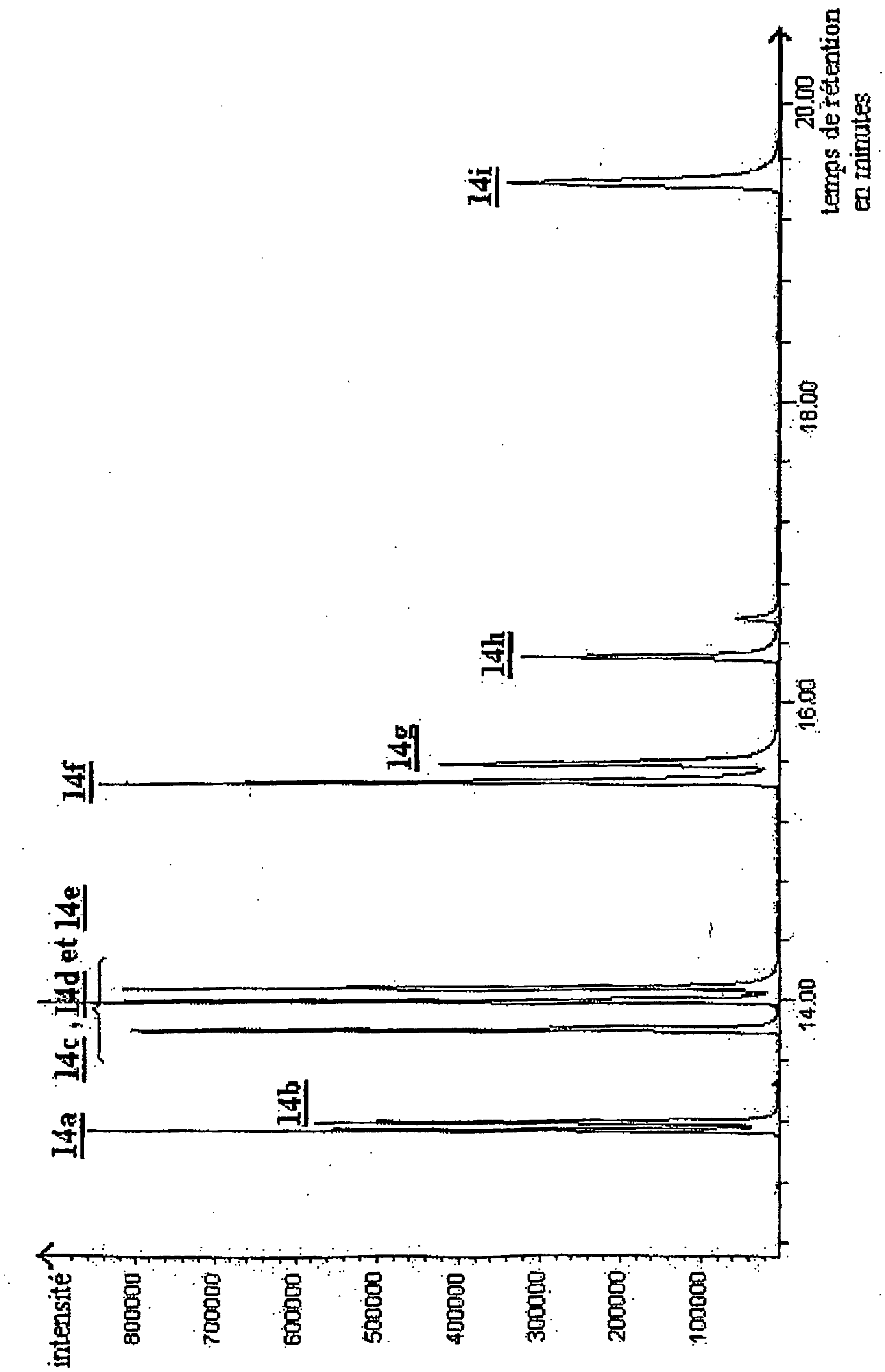
intensité
800000
700000
600000
500000
400000
300000
200000
100000
14a
14b
14c , 14d et 14e
14f
14g
14h
14i
14.00
16.00
18.00
20.00
temps de rétention
en minutes

FIGURE 3

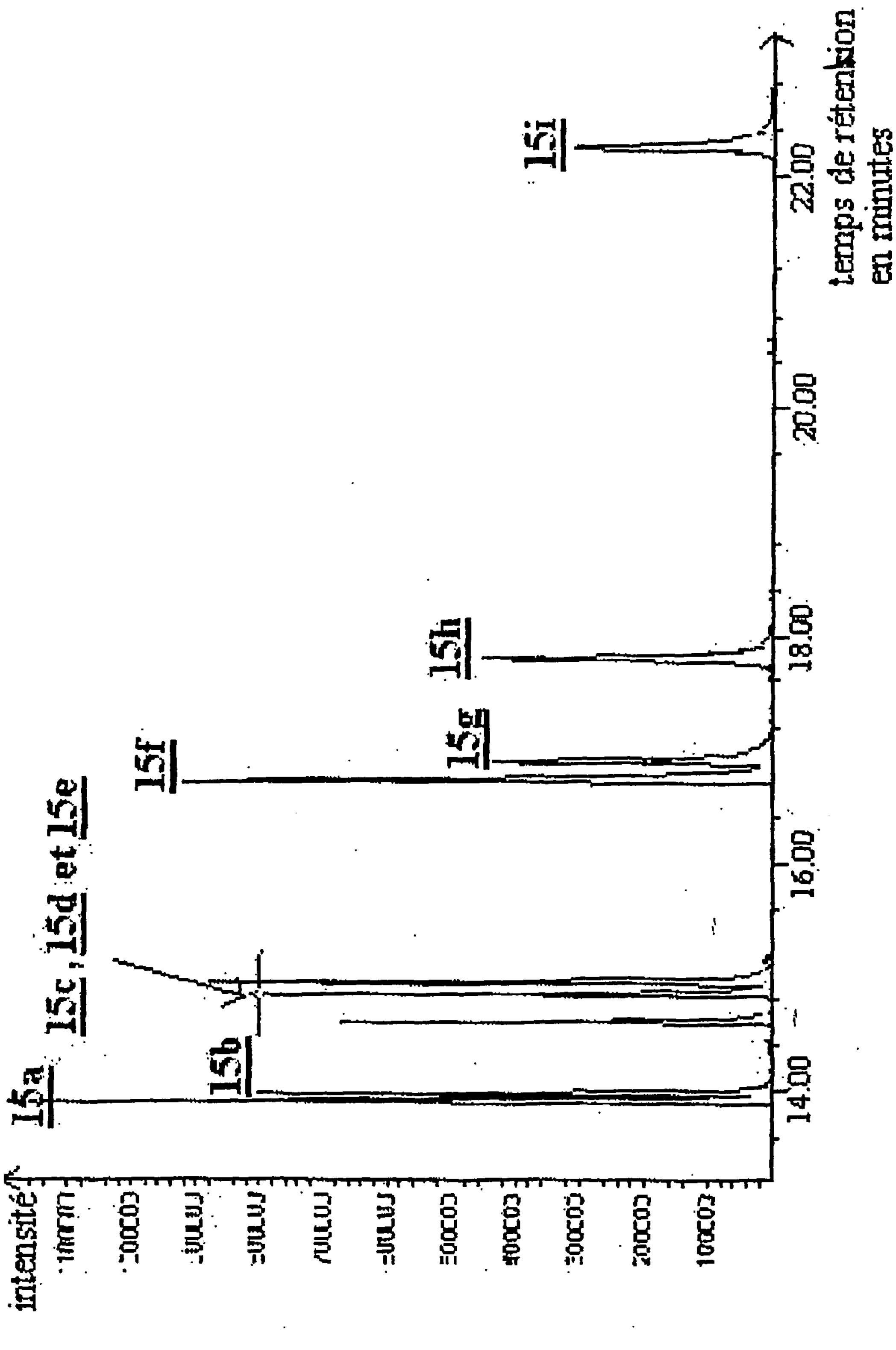
intensité
15a
15b
15c, 15d et 15e
15f
15g
15h
15i
14.00
16.00
18.00
20.00
22.00
temps de rétention
en minutes

FIGURE 4

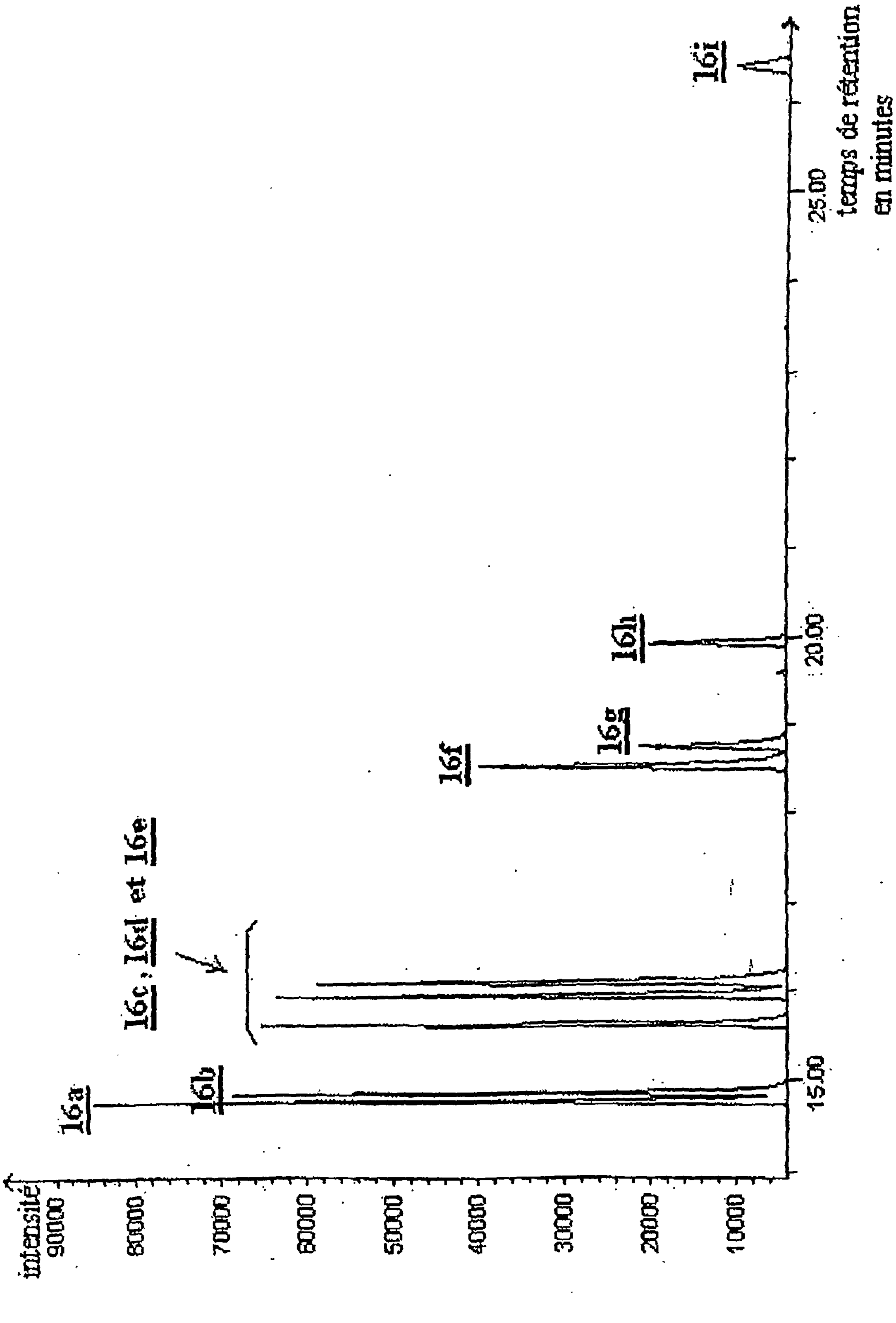
intensité
90000
80000
70000
60000
50000
40000
30000
20000
10000
15.00
20.00
25.00
temps de rétention
en minutes
16a
16b
16c, 16d et 16e
16f
16g
16h
16i

FIGURE 5

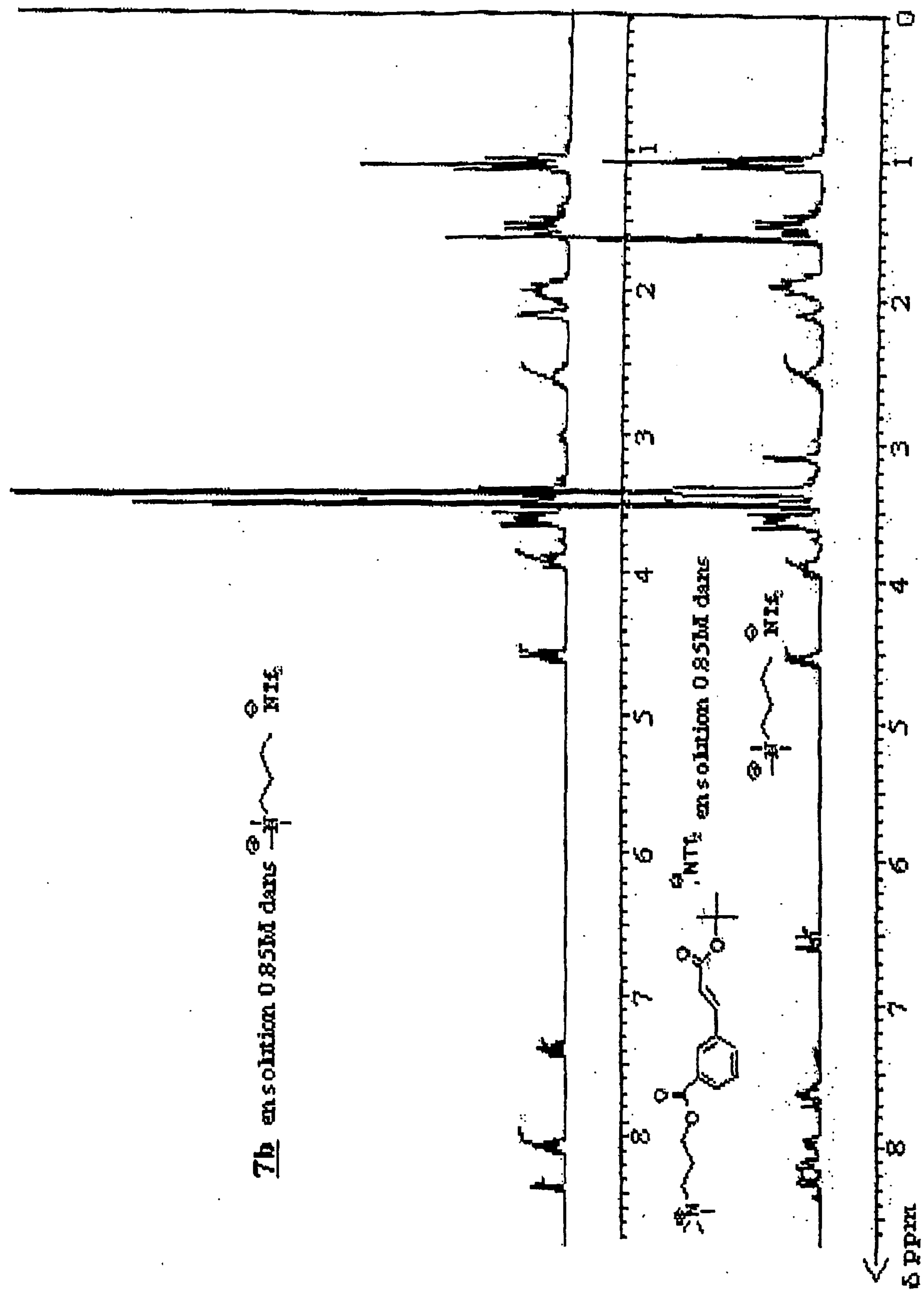
7b en solution 0.85M dans
en solution 0.85M dans
δ ppm
0
1
2
3
4
5
6
7
8

FIGURE 6

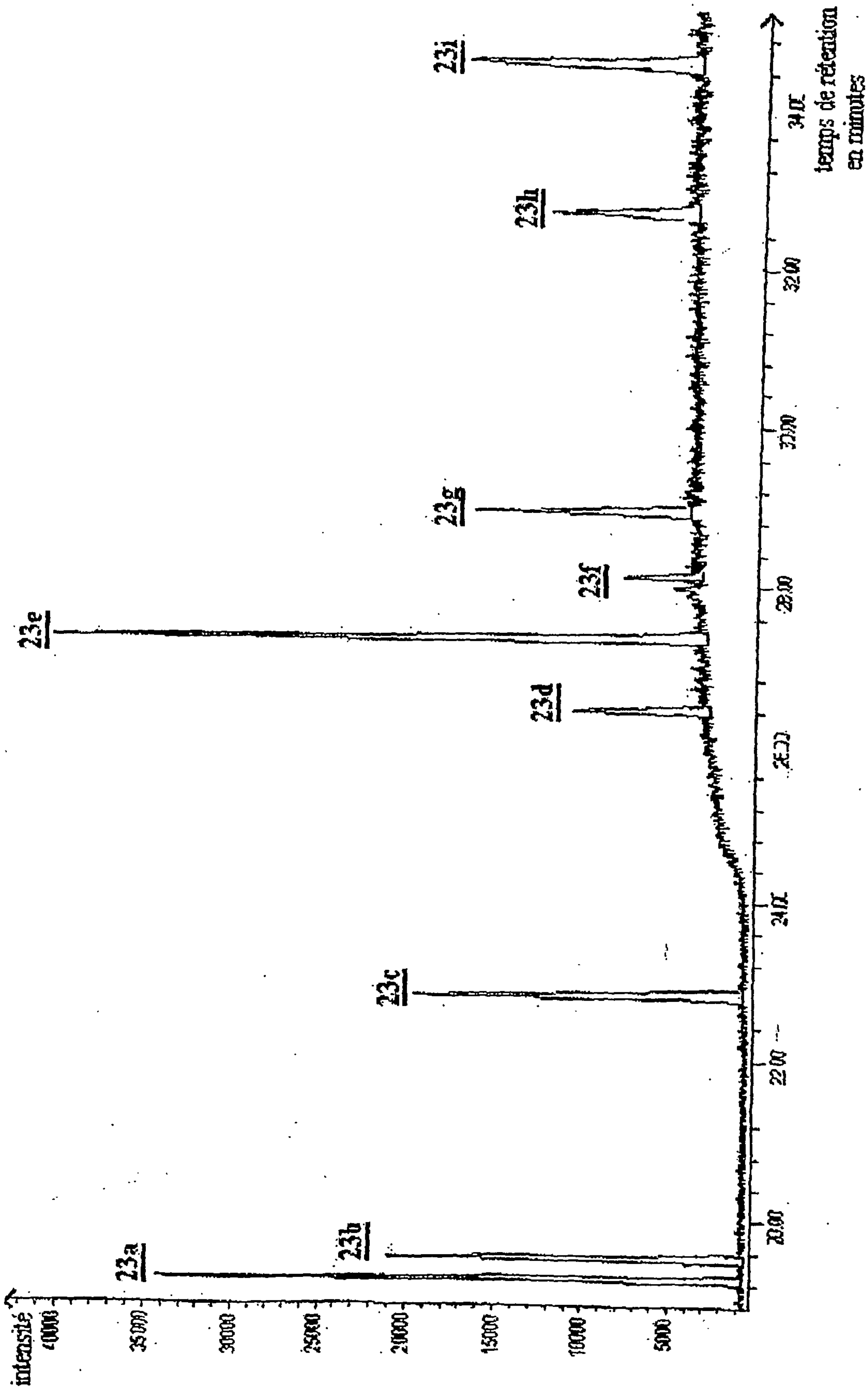
intensité
40000
35000
30000
25000
20000
15000
10000
5000
23a
23b
23c
23d
23e
23f
23g
23h
23i
temps de rétention
en minutes

FIGURE 7

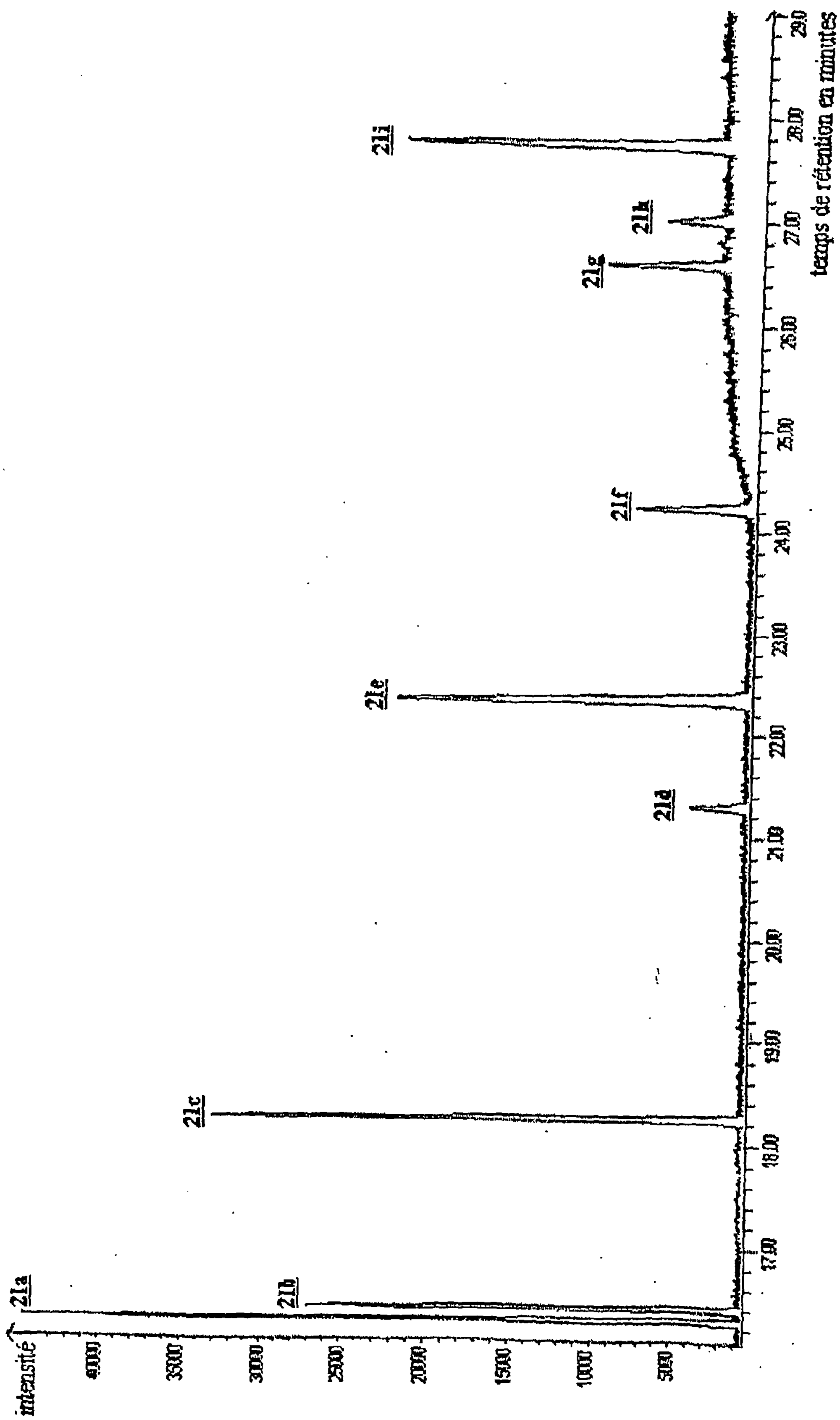
intensité
40000
35000
30000
25000
20000
15000
10000
5000
21a
21b
21c
21d
21e
21f
21g
21h
21i
17.00
18.00
19.00
20.00
21.00
22.00
23.00
24.00
25.00
26.00
27.00
28.00
29.0
temps de rétention en minutes

FIGURE 8

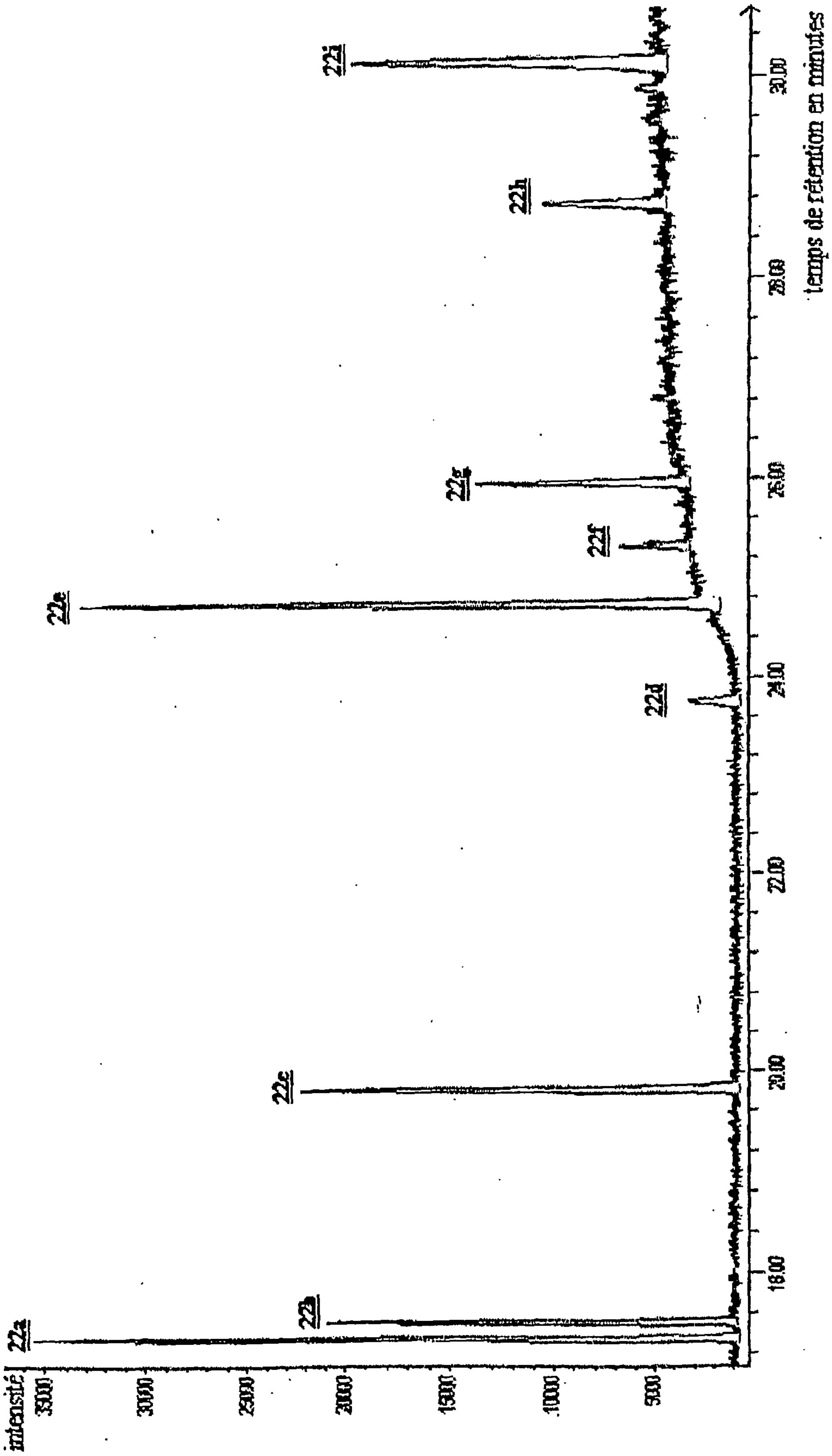
intensité
22a
22b
22c
22d
22e
22f
22g
22h
22i
16.00
20.00
22.00
24.00
26.00
28.00
30.00
temps de rétention en minutes

FIGURE 9

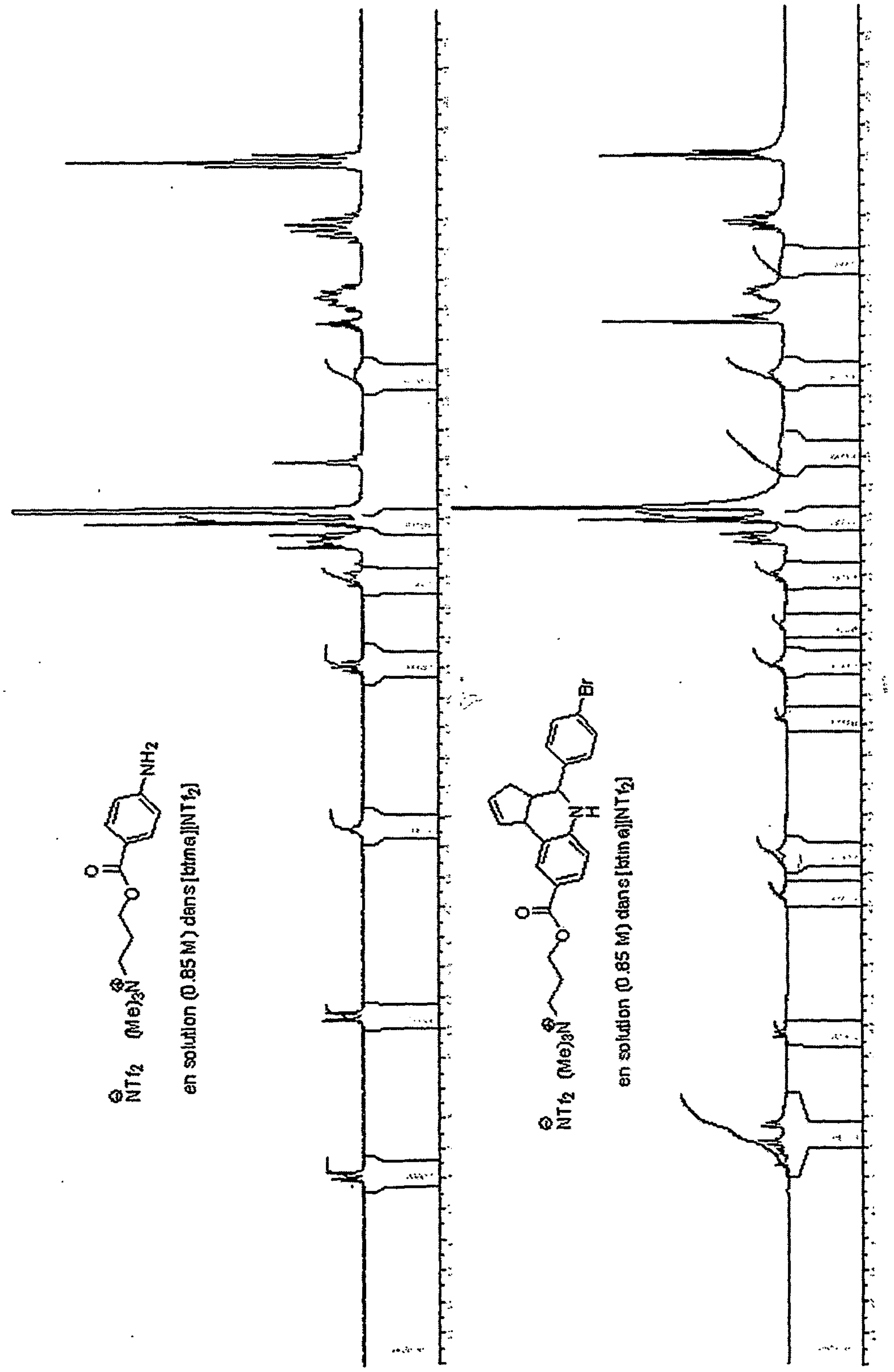

en solution (0.85 M) dans [btma][NTf2]
en solution (0.85 M) dans [btma][NTf2]


FIGURE 10

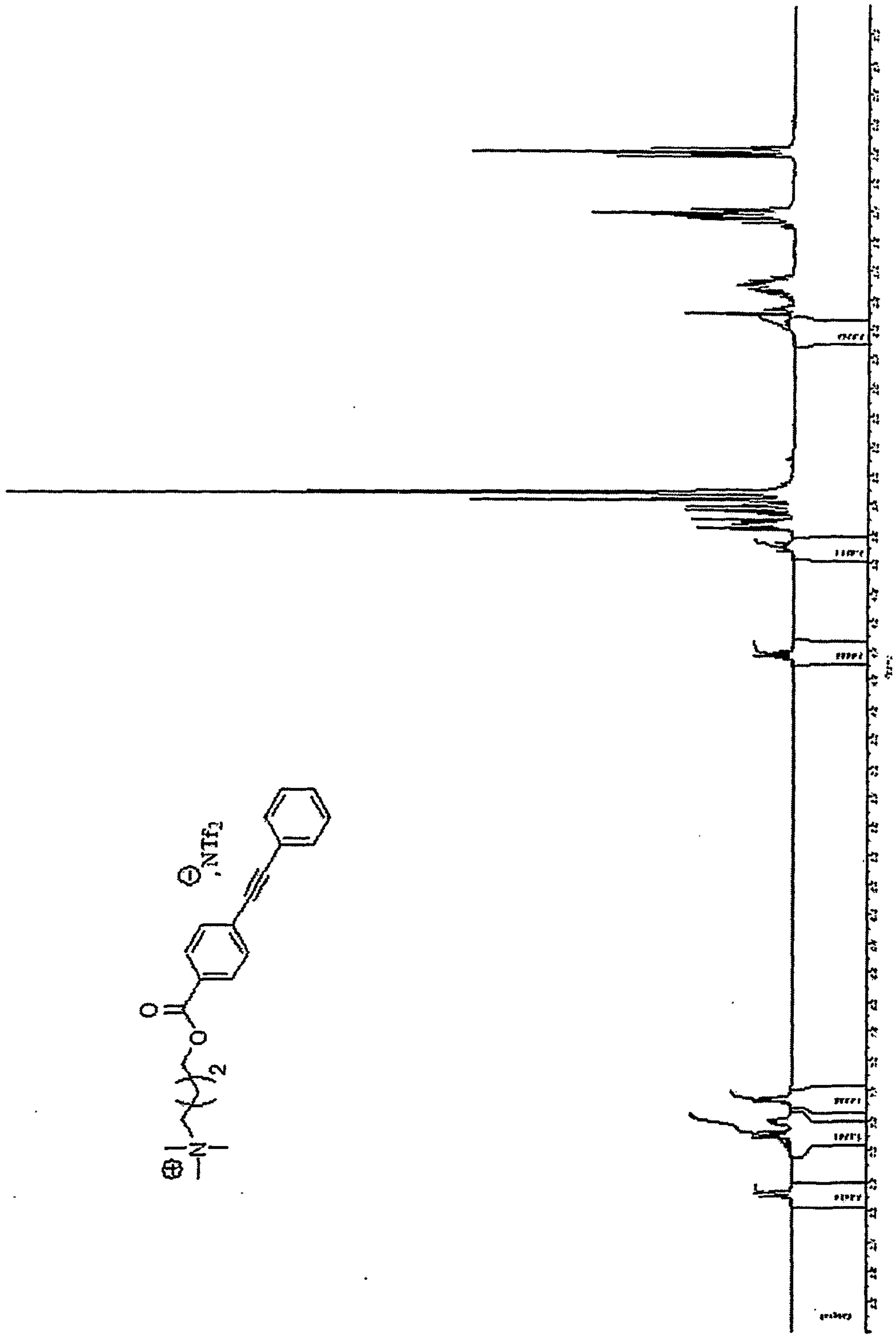

FIGURE 11

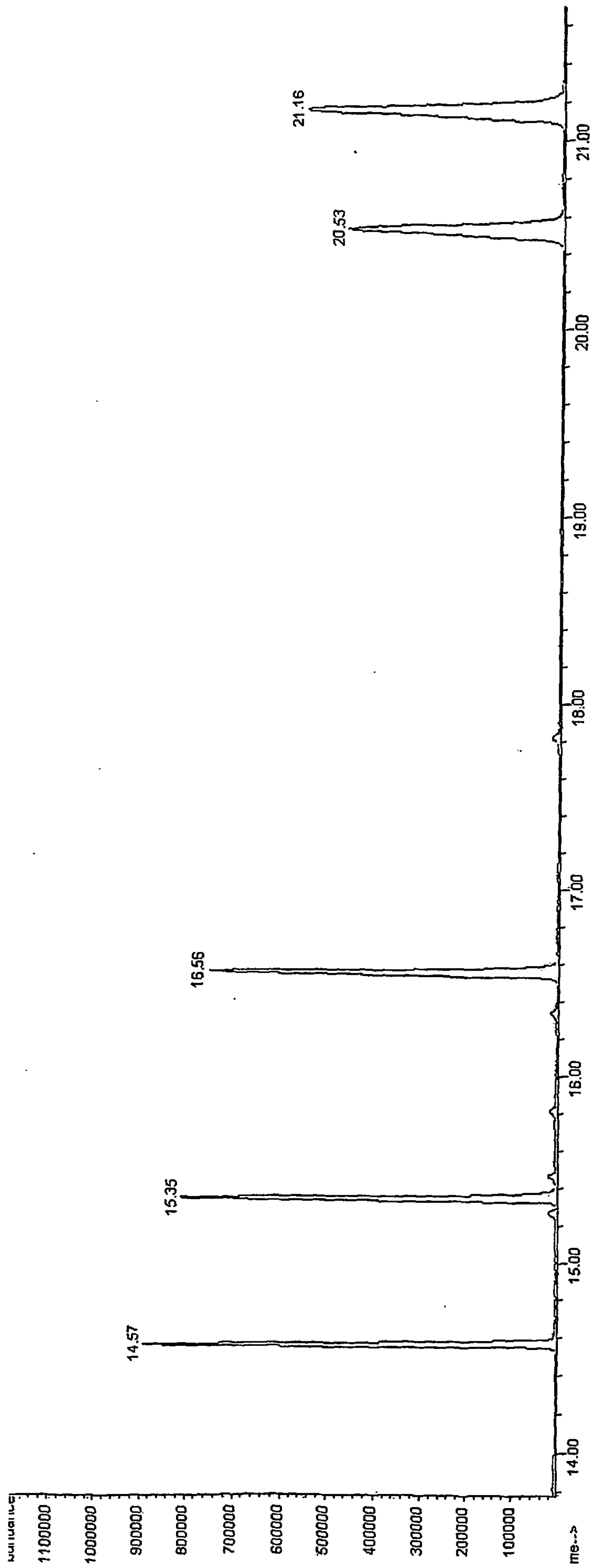
21.16
20.53
16.56
15.35
14.57
14.00
15.00
16.00
17.00
18.00
19.00
20.00
21.00
1100000
1000000
900000
800000
700000
600000
500000
400000
300000
200000
100000
me-->


FIGURE 12

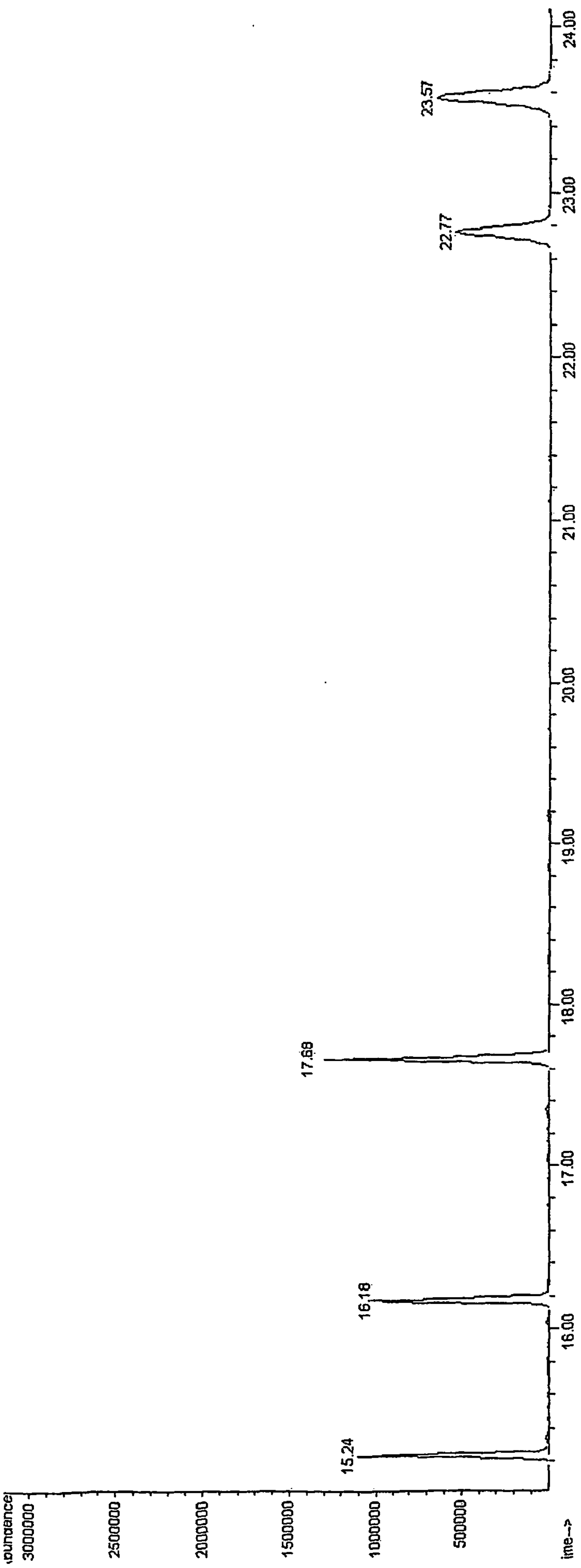

Abundance
3000000
2500000
2000000
1500000
1000000
500000
Time-->
15.24
16.18
17.68
22.77
23.57
16.00
17.00
18.00
19.00
20.00
21.00
22.00
23.00
24.00


FIGURE 13